(19) Europäisches Patentamt
European Patent Office
Office européen des brevets

(11) EP 1 296 992 B1

(12) **EUROPÄISCHE PATENTSCHRIFT**

(45) Veröffentlichungstag und Bekanntmachung des Hinweises auf die Patenterteilung:
**23.04.2008 Patentblatt 2008/17**

(21) Anmeldenummer: **01960378.6**

(22) Anmeldetag: **25.06.2001**

(51) Int Cl.:
***C07F 9/6568*** *(2006.01)* ***C07C 45/50*** *(2006.01)*
***B01J 31/24*** *(2006.01)*

(86) Internationale Anmeldenummer:
**PCT/EP2001/007219**

(87) Internationale Veröffentlichungsnummer:
**WO 2002/000669 (03.01.2002 Gazette 2002/01)**

(54) **PHOSPHACYCLOHEXANE UND IHRE VERWENDUNG IN DER HYDROFORMYLIERUNG VON OLEFINEN**

PHOSPHACYCLOHEXANES AND THE USE THEREOF IN THE HYDROFORMYLATION OF OLEFINS

PHOSPHACYCLOHEXANES ET LEUR UTILISATION DANS L'HYDROFORMYLATION D'OLEFINES

(84) Benannte Vertragsstaaten:
**BE DE ES NL**

(30) Priorität: **26.06.2000 DE 10031108**

(43) Veröffentlichungstag der Anmeldung:
**02.04.2003 Patentblatt 2003/14**

(73) Patentinhaber: **BASF SE**
**67056 Ludwigshafen (DE)**

(72) Erfinder:
- **MACKEWITZ, Thomas**
  **68219 Mannheim (DE)**
- **AHLERS, Wolfgang**
  **67549 Worms (DE)**
- **ZELLER, Edgar**
  **68165 Mannheim (DE)**
- **RÖPER, Michael**
  **67157 Wachenheim (DE)**
- **PACIELLO, Rocco**
  **67098 Bad Dürkheim (DE)**
- **KNOLL, Konrad**
  **67069 Ludwigshafen (DE)**
- **PAPP, Rainer**
  **67346 Speyer (DE)**
- **VOSS, Hartwig**
  **67227 Frankenthal (DE)**

(74) Vertreter: **Pohl, Michael Friedrich**
**Reitstötter, Kinzebach & Partner (GbR)**
**Patentanwälte**
**Ludwigsplatz 4**
**67059 Ludwigshafen (DE)**

(56) Entgegenhaltungen:
**EP-A- 0 646 588**
**EP-A- 0 982 314**
**EP-A- 1 036 796**
**WO-A-00/26220**
**WO-A-00/55164**
**WO-A-97/46507**
**DE-A- 19 743 197**
**DE-C- 313 876**
**DE-C- 889 591**
**US-A- 3 496 204**
**US-A- 5 177 044**

- **PATENT ABSTRACTS OF JAPAN vol. 011, no. 163 (C-424), 26. Mai 1987 (1987-05-26) -& JP 61 291532 A (AGENCY OF IND SCIENCE & TECHNOL), 22. Dezember 1986 (1986-12-22)**
- **VAN DER VEEN, LARS A. ET AL: "New Phosphacyclic Diphosphines for Rhodium-Catalyzed Hydroformylation" ORGANOMETALLICS, Bd. 18, Nr. 23, 8. November 1999 (1999-11-08), Seiten 4765-4777, XP000964505 ISSN: 0276-7333**
- **DAHLENBURG, LUTZ ET AL: "Chiral bisphosphines. IX. Cationic rhodium(I) complexes with (1S,2S)-cyclopentane-1,2-diylbis (dialkylph osphine) ligands, [(1S,2S)-C5H8(PR2) 2Rh(1,5-COD)]O3SCF3. Synthesis, structure and catalytic applications" JOURNAL OF ORGANOMETALLIC CHEMISTRY, Bd. 585, Nr. 2, 1999, Seiten 315-325, XP004177475 ISSN: 0022-328X**
- **OHGOMORI, YUJI ET AL: "Electronic and steric effects of phosphine ligands in the formation of ethylene glycol from synthesis gas catalyzed by rhodium" JOURNAL OF MOLECULAR CATALYSIS, Bd. 43, Nr. 2, 1987, Seiten 249-258, XP001021288 ISSN: 0304-5102**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

- LE FLOCH, PASCAL ET AL: "Transition metals in phosphinine chemistry" COORDINATION CHEMISTRY REVIEWS, Bd. 178-180, Nr. 1, 1998, Seiten 771-791, XP001029264 ISSN: 0010-8545
- BOEHM, D. ET AL: ".eta.6-Phosphinine- and.eta. 6-1,3-diphosphinine iron complexes" PHOSPHORUS, SULFUR AND SILICON AND THE RELATED ELEMENTS (PROCEEDINGS OF THE THIRTEENTH INTERNATIONAL CONFERENCE ON PHOSPHORUS CHEMISTRY, 1995), Bd. 109-110, Nr. 1-4, 1996, Seiten 173-176, XP001029494 ISSN: 1042-6507
- BREIT, BERNHARD: "Probing new classes of.pi.-acceptor ligands for rhodium catalyzed hydroformylation of styrene" JOURNAL OF MOLECULAR CATALYSIS, SECTION A: CHEMISTRY, Bd. 143, Nr. 1-3, 1999, Seiten 143-154, XP001021289 ISSN: 1381-1169
- SHOOK H E ET AL: "Geometrical Isomerism in 4-Phosphorinanols" JOURNAL OF THE AMERICAN CHEMICAL SOCIETY, Bd. 89, 1967, Seiten 1841-6, XP001027692 ISSN: 0002-7863
- MATHEY F ET AL: "REACTIONS DES AMINOLITHIENS AVEC LES PHOSPHINES INSATUREES" TETRAHEDRON, Bd. 27, 1972, Seiten 5645-8, XP001027653 ISSN: 0040-4020
- PELLERIN B ET AL: "METHYLIDENEPHOSPHINE" TETRAHEDRON LETTERS, Bd. 28, Nr. 47, 1987, Seiten 5811-4, XP001027627 ISSN: 0040-4039
- QUIN L D ET AL: "THERMOLYSIS OF 2-PHOSPHABICYCLO[2.2.2]OCTA-5,7-DIENES: GENERATION AND TRAPPING OF P-METHYL- AND P-PHENYLPHOSPHAETHENE" TETRAHEDRON LETTERS, Bd. 28, Nr. 47, 1987, Seiten 5783-6, XP001027625 ISSN: 0040-4039
- BACCOLINI G ET AL: "A One-Pot Synthesis of 1-Substituted Cyclic Phosphine Sulfides by Simultaneous Additionof Bis- and Mono-Grignard Reagents to a New Efficient Phosphorus Donating Agent" SYNLETT, Nr. 11, 2000, Seiten 1685-7, XP001026329 ISSN: 0936-5214
- KEGLEVICH G ET AL: "One-pot transformation of cycli phosphine oxides to phosphine boranes by dimethyl sulfide-borane" JOURNAL OF THE CHEMICAL SOCIETY, PERKIN TRANSACTIONS 1, Nr. 24, 19. Dezember 2000 (2000-12-19), Seiten 4451-5, XP001027484 ISSN: 0300-922X
- HACKLIN H ET AL: "1-HALOGENPHOSPHORINANE: DARSTELLUNG UND DERIVATE" PHOSPHORUS AND SULFUR, Bd. 36, 1988, Seiten 165-9, XP001026957 ISSN: 1042-6507
- MACDONELL G D ET AL: "Nuclear Magnetic Resonance Studies of Phosphorus Inversion in and Conformational Analysis of cis- and trans-4-tert-Butyl-1-phenylphosphorinane" JOURNAL OF THE AMERICAN CHEMICAL SOCIETY, Bd. 100, Nr. 14, 5. Juli 1978 (1978-07-05), Seiten 4535-40, XP001027619 ISSN: 0002-7863
- QUIN L D ET AL: "Stereochemical Consequences of C-Methylation of 1-Methylphosphorinane and Its Sulfide and Oxide: A Carbon-13 and Phosphorus-31 Nuclear Magnetic Resonance Study" JOURNAL OF ORGANIC CHEMISTRY, Bd. 43, Nr. 7, 14. April 1978 (1978-04-14), Seiten 1424-9, XP001027610 ISSN: 0022-3263
- FEATHERMAN S I ET AL: "Carbon-13 Magnetic Resonance Spectral Study of Some Phosphorinanes and Their 1-Sulfides" JOURNAL OF ORGANIC CHEMISTRY, Bd. 39, Nr. 19, 1974, Seiten 2899-904, XP001027615 ISSN: 0022-3263
- ISSLEIB K ET AL: "ALKALI-PHOSPHORVERBINDUNGEN UND IHR REAKTIVES VERHALTEN, VIII UEBER DIE BILDUNG CYCLISCHER PHOSPHINE" CHEMISCHE BERICHTE, Bd. 94, 1961, Seiten 113-117, XP001021338 ISSN: 0009-2940 in der Anmeldung erwähnt
- WELCHER R P ET AL: "4-PHOSPHORINANONES II" JOURNAL OF ORGANIC CHEMISTRY, Bd. 27, Mai 1962 (1962-05), Seiten 1824-1827, XP001021630 ISSN: 0022-3263 in der Anmeldung erwähnt
- AITKEN R A ET AL: "Flash Vacuum Pyrolysis of Dichlorophosphines over Magnesium: Generation and Reactivity of Simple Phosphinidenes" TETRAHEDRON LETTERS, Bd. 38, Nr. 48, 1. Dezember 1997 (1997-12-01), Seiten 8417-8420, XP004095961 ISSN: 0040-4039
- PLASSERAUD L ET AL: "Catalytic hydrogenation of benzene derivatives under biphasic conditions" JOURNAL OF ORGANOMETALLIC CHEMISTRY, ELSEVIER-SEQUOIA S.A. LAUSANNE, CH, Bd. 539, Nr. 1-2, 1. Juli 1997 (1997-07-01), Seiten 163-170, XP004082205 ISSN: 0022-328X
- HAYAO IMAMURA ET AL: "PROPERTIES AND REACTIVITY OF LANTHANIDE (EU AND YB) CATALYSTS DISPERSED ON SIO2" JOURNAL OF THE CHEMICAL SOCIETY. FARADAY TRANSACTIONS, ROYAL SOCIETY OF CHEMISTRY, CAMBRIDGE, GB, Bd. 88, Nr. 15, 7. August 1992 (1992-08-07), Seiten 2251-2256, XP000289793 ISSN: 0956-5000
- SCHULZ J ET AL: "Unprecedented efficient hydrogenation of arenes in biphasic liquid-liquid catalysis by re-usable aqueous colloidal suspensions of rhodium" CHEMICAL COMMUNICATIONS, Nr. 6, 1999, Seiten 535-6, XP002188271 ISSN: 1359-7345


Bemerkungen:

Die Akte enthält technische Angaben, die nach dem Eingang der Anmeldung eingereicht wurden und die nicht in dieser Patentschrift enthalten sind.

## Beschreibung

Die vorliegende Erfindung betrifft Phosphacyclohexane, Verfahren zu ihrer Herstellung und ihre Verwendung in Komplexen von Übergangsmetallen der VIII. Nebengruppe des Periodensystems bei der Hydroformylierung von Olefinen.

Jährlich werden weltweit etwa 7 Mio. Tonnen chemischer Produkte mit Hilfe der Hydroformylierung von Olefinen hergestellt. Es besteht deshalb ein großes wirtschaftliches Interesse daran, die Reaktion mit maximaler Aktivität und Selektivität zu betreiben.

Für industrielle Verfahren fanden zunächst Cobalt-Katalysatoren Anwendung. In der Zwischenzeit haben sich auch Rhodium-Katalysatoren in der Technik etabliert. Derartige Systeme zeigen allgemein höhere Selektivitäten als Cobalt-haltige Systeme.

Um eine Stabilisierung des Rhodium-haltigen Katalysators bei der Reaktion zu bewirken und bei der Aufarbeitung die Zersetzung unter Abscheidung von metallischem Rhodium zu vermeiden, werden im Allgemeinen Phosphorhaltige Liganden zur Stabilisierung verwendet. Letzteres ermöglicht es außerdem, die Reaktion auch bei abgesenktem Synthesegasdruck mit hinreichender Aktivität durchzuführen.

Für niedere α-Olefine hat sich insbesondere der Einsatz von Triphenylphosphan und anderen Triarylphosphanen als Cokatalysatoren in homogener Phase oder im Zweiphasen-System bewährt. Die Reaktionen werden auch mit monodentaten Liganden meist so geführt, dass ein maximaler Anteil an n-Aldehyden resultiert. Obwohl niedere α-Olefine sehr gut mit Triarylphosphan-modifizierten Rhodium-Katalysatoren hydroformylierbar sind (J. Falbe, New Syntheses With Carbon Monoxide, Springer, Berlin, 1980, S. 55 ff), ist dieses Katalysatorsystem für interne und interne, verzweigte Olefine sowie für höhere α-Olefine wenig geeignet (J. Falbe, New Syntheses With Carbon Monoxide, Springer, Berlin, 1980, S. 95 ff). So werden interne und interne verzweigte Doppelbindungen nur sehrlangsam in Gegenwart eines derartigen Katalysators hydroformyliert. Des Weiteren kann bei der destillativen Aufarbeitung des Reaktionsgemischs der Hydroformylierung höherer Olefine auch Ligand verloren gehen, welcher ständig ergänzt werden müsste. Allgemein bekannt ist ferner, dass Triarylphosphane in Gegenwart von Rhodium und Olefin abgebaut werden können, was zu einer zunehmenden Desaktivierung des Katalysators führt. Die Desaktivierung des Katalysators ist besonderes ausgeprägt bei Triarylphosphanen mit elektronenschiebenden Substituenten. Der Ligandabbau von Triarylphosphanen wird allgemein durch eine Insertion des Rhodiummetalls in die Aryl/P-Bindung eingeleitet. Folgereaktionen unter Hydroformylierungsbedingungen führen dann zur Bildung von inaktiven Phosphido-verbrückten Dimerkomplexen oder zu Alkyldiarylphosphanen und Derivaten der phosphinigen Säure, welche als Katalysatorgifte wirken und gleichfalls zur Desaktivierung führen.

Phosphit-modifizierte Rhodium-Katalysatoren werden neuerdings für die Hydroformylierung von niedersiedenden Olefinen vorgeschlagen, siehe M. Beller, B. Cornils, C. D. Frohning, C. W. Kohlpaintner, J. Mol. Catal. 1995, 104, 17. Sowohl α-Olefine als auch kurzkettige interne Olefine wie 2-Buten oder 3-Pentensäuremethylester können sehr gut mit Rhodium-haltigen, Chelatphosphit-modifizierten Katalysatoren hydroformyliert werden. Besonders hohe Linearitäten werden mit Chelatliganden mit einem Bisswinkel von etwa 120° erzielt. Allerdings ist dieses Katalysatorsystem für langkettiginterne Olefine mit mehr als 7 Kohlenstoffatomen und interne verzweigte Olefine wenig geeignet. Für diese Olefine haben sich einzähnige, sterisch gehinderte Monophosphite experimentell bewährt. Allerdings weisen Phosphite generell den Nachteil der Hydrolyseempfindlichkeit und der Tendenz zu Abbaureaktionen (insbesondere bei höheren Temperaturen) auf, wodurch deren technischer Einsatz behindert wird.

Im Gegensatz zu Triarylphosphanen und Phosphiten besitzen die weit basischeren Trialkylphosphane den Vorteil, die oben aufgeführten Abbaureaktionen nicht einzugehen. Insertionsreaktionen in die Alkyl/P-Bindung können hier nicht auftreten. In Kombination mit CO als Liganden resultieren mit Trialkylphosphanen sehr stabile Komplexe, was auf die Kombination von Donor- und Akzeptorliganden zurückzuführen ist, womit eine Liganddissoziation unterbunden wird (J. A. Moulijn, P. W. N. M. Leeuwen, R. A. van Santen, Catalysis, An integrated Approach to Homogeneous, Heterogenous and Industrial Catalysis, Elsevier, Amsterdam, 1993, Chapter 6). Im Falle von sterisch anspruchsvollen Phosphanen wird angenommen, dass der aktive Komplex nicht wie im Triphenylphosphan-Fall zwei, sondern nur einen Phosphanliganden trägt. Die Katalysatoren weisen jedoch im Vergleich zu Triarylphosphanen eine geringere Aktivität auf, welche durch Druck und insbesondere Temperatur gesteigert werden muss. Rhodium-Katalysatoren mit sterisch anspruchsvollen Trialkylphosphanen wie beispielsweise Tricyclohexylphosphan sind bei höheren Temperaturen im Mitteldruckbereich auch in der Lage, interne Olefine zu hydroformylieren, sind aber für die Hydroformylierung interner verzweigter Olefine aufgrund ihrer geringen Aktivität nur wenig geeignet.

Die Vorteile von Trialkylphosphanen als Cokatalysatoren in der Hydroformylierung liegen in der enormen thermischen Stabilität. Nachteilig ist jedoch die geringe Aktivität der resultierenden Katalysatorsysteme, welche nur unzureichend durch Erhöhung des Oxogasdrucks und durch Temperaturerhöhung kompensiert werden kann.

Die WO-A-97/46507 beschreibt ein Verfahren zur Hydroformylierung in Gegenwart wenigstens eines Übergangsmetallkatalysators, wobei als Liganden Phosphorverbindungen eingesetzt werden, in denen das Phosphoratom ein freies Elektronenpaar sowie drei Bindungen zu zwei Nachbaratomen besitzt. Dabei handelt es sich speziell um

substituierte Phosphabenzole.

Die DE-A-19743197 beschreibt ein Verfahren zur Herstellung von Phosphabenzolverbindungen durch Umsetzung entsprechender Pyryliumsalze mit $PH_3$.

K. Issleib und Siefried Häusler beschreiben in Chem. Ber. 1961, 94, 113 - 117 cyclische Phosphine, wie 1-substituierte Phosphacyclohexane.

R. P. Welcher und N. E. Day beschreiben in J. Org. Chem. 1962, 27, 1824 - 1827 2,6-substituierte 1-Phosphacyclohexan-4-one.

G. Märkl et al. beschreiben in Tetrahedron Letters 1970, Nr. 9, S. 645 - 648 unter anderem 1-substituierte 2,6-Diphenyl-1-phosphacyclohexan-4-one.

Die US 3,105,096 beschreibt Phosphacyclohexan-4-ole und ein Verfahren zur ihrer Herstellung aus den entsprechenden Phosphacyclohexan-4-onen.

K. Sommer beschreibt in Z. anorg. allg. Chem. 376, 37, S. 56 - 62 (1970) die Spaltung tertiärer Phosphine und speziell die Herstellung von 1-Phenylphosphacyclohexan durch Reduktion von 1-Phenylphosphacyclohexan-4-on sowie die Herstellung von unsubstituiertem Phosphacyclohexan.

S. D. Pastor et al. beschreiben in J. Org. Chem. 1984, 49, 2906 - 2909 die Synthese von 2,2,6,6-Tetramethylphosphacyclohexan-4-ol.

M. M. Rauhut et al. beschreiben in J. Org. Chem. 1961, 26, 5138 die radikalische Addition von primären oder sekundären Phosphanen an Olefine.

G. Märkl und A. Merz beschreiben in Tetrahedron Letters 1971, Nr. 17, S. 1215 - 1218 die Umsetzung von 2,4,6-Triaryl-substituierten Phosphabenzolen mit Organolithium- und Grignard-Verbindungen. Eine Hydrierung der dabei als Zwischenprodukte resultierenden Phosphacyclohexadiene wird nicht beschrieben.

G. Märkl et al. beschreiben in Angew. Chem., 79, 1967, S. 59 die Umsetzung von 2,4,6-Triphenylphosphabenzol mit Lithium-Alkylen und Lithium-Arylen.

A. J. Ashe und T. W. Smith beschreiben in Tetrahedron Letters 1977, Nr. 5, S. 407 - 410 die Reaktion von Phosphabenzol mit Methyllithium.

Es ist bekannt, katalytisch aktive Verbindungen an polymere Träger zu binden, um Katalysatorverluste bei der Aufarbeitung zu vermeiden. Die WO-A-00/53305 beschreibt sogenannte polymervergrößerte Katalysatoren, die ein Homo- oder Copolymerisat des Butadiens oder Isoprens aufweisen.

D. E. Bergbreiter et al. beschreiben in J. Polym. Sci., Teil A, Polym. Chem. 1989, 27, S. 4205 - 4226 die anionische Polymerisation von Ethen in Gegenwart von Alkyllithium-Reagenzien und deren Folgeumsetzung mit Phosphor-Elektrophilen.

Es ist bekannt, Pyryliumsalze mit primären Phosphinen umzusetzen. So beschreiben C. C. Price et al. in J. Am. Chem. Soc. 1966, 88, S. 1034 - 1037 die Umsetzung von 2,4,6-Triphenylpyryliumsalzen mit Phenylphosphin.

Keines der zuvor genannten Dokumente beschreibt den Einsatz von Phosphacyclohexanen als Liganden in Katalysatoren zur Hydroformylierung.

Die WO 00/52017 beschreibt ein Verfahren zur Herstellung von 9-Hydrocarbyl-9-phosphabicyclo[3.3.1]nonanen, die sich als Liganden für die Cobalt-katalysierte Hydroformylierung von Olefinen eignen.

Die US 3,496,204 beschreibt den Einsatz von Kobaltkomplexen mit tertiären 6-gliedrigen heterocyclischen Phosphinen als Liganden zur Hydroformylierung.

Die EP-A-0 646 588 beschreibt Phosphorverbindungen, die zwei zur Komplexbildung befähigte Phosphor (III)-atome enthalten. Diese sind Teil eines 5- oder 6-gliedrigen cyclischen Rests, wobei zwei dieser cyclischen Reste über eine Diarylbrücke miteinander verbunden sind. Diese Phosphorverbindungen eignen sich unter Anderem für Hydroformylierungskatalysatoren.

Die EP-A-0 982 314 beschreibt zweizähnige Phosphinliganden mit zwei verbrückten Phosphacyclohexadienen und deren Einsatz zur Herstellung linearer Aldehyde durch Hydroformylierung.

Die WO 00/26220 beschreibt asymmetrische Diphosphine, bei denen die Phosphoratome Teil eines Heterocyclus sein können und deren Einsatz in Katalysatoren für asymmetrische Umwandlungen, wie asymmetrische Hydrierungen.

Die US 5,177, 044 beschreibt Katalysatoren von Metallen der VIII. Nebengruppe mit Diphosphorverbindungen, bei denen zwei Phosphacyclohexylgruppen über eine verbrückende Gruppe miteinander verbunden sind.

Die JP 61 291 532 A beschreibt ein Verfahren zur Herstellung von Ethylenglykol durch Umsetzung von Kohlenmonoxid mit Wasserstoff in Gegenwart eines Rhodiumkatalysators.

Es besteht ein Bedarf an Katalysatorsystemen, die eine effektive Hydroformylierung auch bei mittleren und niedrigen Drücken ermöglichen, um den hohen apparativen Aufwand und die damit verbundenen Investitions- und Betriebskosten von Hochdruckverfahren zu vermeiden.

Der vorliegenden Erfindung lag daher die Aufgabe zugrunde, alkylsubstituierte Phosphane zu entwickeln, die als Cokatalysatoren für die Hydroformylierung von Olefinen geeignet sind und sich durch eine hohe Stabilität und vorzugsweise durch eine deutlich höhere Aktivität im Vergleich zu bisher bekannten Systemen auszeichnen. Daraus resultieren verschiedene Vorteile: Im Vergleich zu bisherigen Systemen ist aufgrund der höheren Raum-Zeit-Ausbeute ein kleinerer Reaktionsraum für die gleiche Produktmenge ausreichend, was sich in geringeren Investitionskosten niederschlägt. Darüber hinaus können aufgrund der höheren Aktivität kürzere Verweilzeiten eingestellt werden, was eine Reduzierung der Hochsiederbildung zur Folge hat. Die Hydrierrate soll möglichst niedrig sein.

Überraschenderweise wurde nun gefunden, dass diese Aufgabe durch die Verwendung von Verbindungen mit wenigstens einem unverbrückten Phosphacyclohexan- und/oder Phosphacyclohexen-Strukturelement als Liganden in Komplexen von Übergangsmetallen der VIII. Nebengruppe des Periodensystems der Elemente gelöst wird. Diese Komplexe eignen sich vorteilhaft als Katalysatoren zur Hydroformylierung von Olefinen durch Umsetzung mit CO und $H_2$.

Nicht zu den "unverbrückten" Strukturelementen zählen Ringsysteme, bei denen zwei nicht benachbarte Kohlenstoffatome des Phosphacyclohexan- oder Phosphacyclohexenrings zumindest zwei verschiedenen Ringen angehören.

Gegenstand der ERfindung ist daher ein Verfahren zur Hydroformylierung von Olefinen durch Umsetzung von Olefinen mit CO und $H_2$ in Gegenwart eines Rhodiumkomplexes als Katalysator bei Temperaturen von 50 bis 180°C und Drücken von 5 bis 100 bar, wobei der Katalysator wenigstens einen Liganden mit wenigstens einem unverbrückten Phosphacyclohexan-Strukturelement umfasst, ausgewählt unter

Phosphacyclohexanen der allgemeinen Formeln I und II

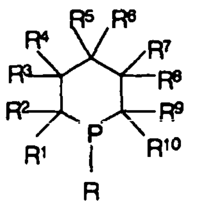

(I)

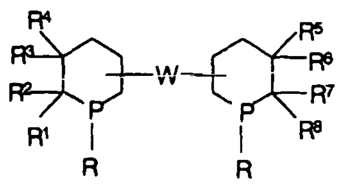

(II)

mit der Bedeutung

R

Wasserstoff, $C_{1-100}$-Alkyl, Cycloalkyl, Heterocycloalkyl, $C_{7-20}$-Aralkyl, $C_{7-20}$-Alkaryl, $C_{6-12}$-Aryl, Hetaryl, W'COO-$M^+$, W'$SO_3^-M^+$, W'$PO_3^{2-}M^+_2$, W'$NR'_3{}^+X^-$, W'OR', W'$NR'_2$, W'COOR', W'SR', W'$(CHR'CH_2O)_xR'$, W'$(CH_2NR')_xR'$, W'$(CHyCH_2NR')_xR'$, W'$((CH_2)_4O)_xR'$ oder W'COR',

wobei in der Formel II die Reste R auch anstelle von oder zusätzlich zu der Gruppe W zusammen eine Brücke mit 1 bis 20 Kohlenstoffatomen bilden können, die Bestandteil einer cyclischen oder aromatischen Gruppe sein kann und durch Heteroatome unterbrochen sein kann,

wobei in der Formel II die Reste R auch anstelle von oder zusätzlich zu der Gruppe W zusammen für eine Polyoxyalkylen- oder Polyalkylenimin-Brücke mit mindestens 21 Kohlenstoffatomen stehen können,

$R^1$ bis $R^{10}$

unabhängig voneinander Wasserstoff, $C_{1-20}$-Alkyl, $C_{7-20}$-Aralkyl, $C_{7-20}$-Alkaryl, $C_{6-12}$-Aryl, wobei eines oder mehrere Kohlenstoffatome durch Heteroatome ersetzt sein können, W'$COO^-M^+$, W'$SO_3^-M^+$, W'$PO_3^{2-}M^+_2$, W'$NR'_3{}^+X^-$, W'OR', W'$NR'_2$, W'COOR', W'SR', W'$(CHR'CH_2O)_xR'$, W'$(CH_2NR')_xR'$, W'$(CH_2CH_2NR')_xR'$, W'$((CH_2)_4O)_xR'$, W'Halogen, W'$NO_2$, W'COR' oder W'CN, wobei mindestens zwei der Reste $R^1$ bis $R^{10}$ von Wasserstoff verschieden sind, wobei in den Resten R und $R^1$ bis $R^{10}$ ein oder mehrere Wasserstoffatome durch Fluor ersetzt sein können,

W und W' unabhängig voneinander Einfachbindungen oder Brücken mit 1 bis 20 Kohlenstoffatomen, die Bestandteil einer cyclischen oder aromatischen Gruppe sein können und durch Heteroatome unterbrochen sein können, wobei W auch für eine Polyoxyalkylen- oder Polyalkylenimin-Brücke mit mindestens 21 Kohlenstoffatomen stehen kann,

R' Wasserstoff, $C_{1-20}$-Alkyl, Carbonylalkyl, Cycloalkyl oder Aryl,

M+ Kationäquivalent,

$X^-$ Anionäquivalent,

x Zahl von 1 bis 240,

wobei zwei geminale Reste $R^1$ bis $R^{10}$ eine Oxo-Gruppe bilden können und einer oder mehrere der Reste R und $R^1$ bis $R^{10}$ eine zusätzliche, zur Koordination befähigte dreibindige Phosphor- oder Stickstoffgruppierung aufweisen können,
wobei jeweils zwei vicinale Reste zu anellierten aliphatischen Ringen verbunden sein können,
wobei in den Verbindungen der allgemeinen Formel II auch zwei oder mehr Brücken W vorliegen können und wobei die nicht an die Brücke(n) W gebundenen Atome der Phosphacyclohexanringe auch wie für $R^1$ bis $R^{10}$ definiert substituiert sein können,
wobei in den Verbindungen der Formel I auch einer der Reste R oder $R^1$ bis $R^{10}$ und in den Verbindungen der Formel II auch einer der Reste R oder $R^1$ bis $R^8$ oder die beiden Reste R gemeinsam oder eine Gruppe W auch für einen Polymerrest mit einem zahlenmittleren Molekulargewicht im Bereich von 500 bis 50000 stehen kann, aufgebaut aus Wiederholungseinheiten, die sich von Monomeren ableiten, die ausgewählt sind unter Mono- und Diolefinen, Vinylaromaten, Estern a,β-ethylenisch ungesättigter Mono- und Dicarbonsäuren mit $C_1$-$C_{30}$-Alkanolen, N-vinylamiden, N-Vinyllactamen, unter Ringöffnung polymerisierbaren heterocyclischen Verbindungen und Mischungen davon.

Im Rahmen der vorliegenden Erfindung umfasst der Ausdruck 'Alkyl' geradkettige und verzweigte Alkylgruppen. Vorzugsweise handelt es sich dabei um geradkettige oder verzweigte $C_1$-$C_{100}$-Alkyl-, bevorzugterweise $C_1$-$C_{20}$-Alkyl- und besonders bevorzugt $C_1$-$C_{10}$-Alkyl- und ganz besonders bevorzugt $C_1$-$C_4$-Alkylgruppen. Beispiele für Alkylgruppen sind insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 2-Heptyl, 3-Heptyl, 2-Ethylpentyl, 1-Propylbutyl, Octyl, Nonyl, Decyl.
Substituierte Alkylreste weisen vorzugsweise 1, 2, 3, 4 oder 5, insbesondere 1, 2 oder 3 Substituenten auf. Diese sind beispielsweise ausgewählt unter Cycloalkyl, Aryl, Hetaryl, Halogen, $NE^1E^2$, $(NE^1E^2E^3)^+$, Carboxyl, Carboxylat, $-SO_3H$ und Sulfonat.
Bei der Cycloalkylgruppe handelt es sich vorzugsweise um eine $C_5$-$C_7$-Cycloalkylgruppe, wie Cyclopentyl, Cyclohexyl oder Cycloheptyl.
Bei der Heterocycloalkylgruppe handelt es sich vorzugsweise um eine $C_{5-7}$-Heterocycloalkylgruppe. Bevorzugt weist die Heterocycloalkylengruppe 1, 2, 3 oder 4 gegebenenfalls substituierte Heteroatome auf. Dazu zählen beispielsweise Pyrrolidin, Tetrahydrofuran, Pyrazolidin, Imidazolidin, Piperidin, Piperazin und Morpholin.
Wenn die Cycloalkylgruppe oder Heterocycloalkylgruppe substituiert ist, weist sie vorzugsweise 1, 2, 3, 4 oder 5, insbesondere 1, 2 oder 3 Substituenten, ausgewählt unter Alkyl, Alkoxy oder Halogen auf.
Aryl steht vorzugsweise für Phenyl, Tolyl, Xylyl, Mesityl, Naphthyl, Anthracenyl, Phenanthrenyl, Naphthacenyl und insbesondere für Phenyl, Naphthyl oder Xylyl.
Substituierte Arylreste weisen vorzugsweise 1, 2, 3, 4 oder 5, insbesondere 1, 2 oder 3 Substituenten, ausgewählt unter Alkyl, Alkoxy, Carboxyl, Carboxylat, Trifluormethyl, $-SO_3H$, Sulfonat, $NE^1E^2$, Alkylen-$NE^1E^2$, Nitro, Cyano oder Halogen auf.
Hetaryl steht vorzugsweise für Pyrrolyl, Pyrazolyl, Imidazolyl, Indolyl, Carbazolyl, Pyridyl, Chinolinyl, Acridinyl, Pyridazinyl, Pyrimidinyl oder Pyrazinyl.
Substituierte Hetarylreste weisen vorzugsweise 1, 2 oder 3 Substituenten, ausgewählt unter Alkyl, Alkoxy, Carboxyl, Carboxylat, $-SO_3H$, Sulfonat, $NE^1E^2$, Alkylen-$NE^1E^2$, Trifluormethyl oder Halogen auf.
Die obigen Ausführungen zu Alkyl-, Cycloalkyl- und Arylresten gelten entsprechend für Alkoxy-, Cycloalkyloxy- und Aryloxyreste.
$E^1$, $E^2$ und $E^3$ sind vorzugsweise unabhängig ausgewählt unter Wasserstoff, Alkyl und Cycloalkyl. Die Reste $NE^1E^2$ stehen vorzugsweise für N,N-Dimethyl, N,N-Diethyl, N,N-Dipropyl, N,N-Diisopropyl, N,N-Di-n-butyl, N,N-Di-tert.-butyl, N,N-Dicyclohexyl oder N,N-Diphenyl.
Heteroatom steht vorzugsweise für ein Sauerstoff-, Schwefel-, zweifach substituiertes Silicium- oder einfach substituiertes Stickstoffatom, wobei die Substituenten unabhängig voneinander für Wasserstoff, Alkyl, Cycloalkyl, Heterocycloalkyl, Aryl, Hetaryl, Alkoxy, Cycloalkoxy oder Aryloxy stehen.
Halogen steht für Fluor, Chlor, Brom und Iod, bevorzugt für Fluor, Chlor und Brom.
Carboxylat und Sulfonat stehen im Rahmen dieser Erfindung vorzugsweise für ein Derivat einer Carbonsäurefunktion bzw. einer Sulfonsäurefunktion, insbesondere für ein Metallcarboxylat oder -sulfonat, eine Carbonsäure- oder Sulfonsäureesterfunktion oder eine Carbonsäure- oder Sulfonsäureamidfunktion. Dazu zählen z. B. die Ester mit $C_1$-$C_4$-Alkanolen, wie Methanol, Ethanol, n-Propanol, Isopropanol, n-Butanol, sec.-Butanol und tert.-Butanol.
Unter Polybutadienen werden im Rahmen dieser Erfindung immer auch deren teilweise oder vollständigen

Hydrierungsprodukte verstanden.

Polyoxyalkylen steht vorzugsweise für Verbindungen mit Wiederholungseinheiten, die ausgewählt sind unter $\{CH_2CH_2O\}_{x1}$, $\{CH(CH_3)CH_2O\}_{x2}$ und $\{(CH_2)_4O\}_{x3}$, wobei $x_2$, $x_2$ und $x_3$ unabhängig voneinander für eine ganze Zahl von 0 bis 240, vorzugsweise 0 bis 100, stehen. Die Summe aus $x_1$, $x_2$ und $x_3$ steht für eine ganze Zahl von 1 bis 240, vorzugsweise 2 bis 100. In Polyoxyalkylenen, die zwei oder drei verschiedenartige Wiederholungseinheiten aufweisen, ist die Reihenfolge beliebig, d. h. es kann sich um statistisch verteilte, alternierende oder blockförmige Wiederholungseinheiten handeln. Das zuvor für die Polyoxyalkylene Gesagte gilt analog für Polyalkylenimine, wobei das Sauerstoffatom jeweils durch eine Gruppe $NR^i$ ersetzt ist, worin $R^i$ für Wasserstoff oder $C_{1-4}$-Alkyl steht.

$M^+$ steht für ein Kationäquivalent, d. h. für ein einwertiges Kation oder den einer positiven Einfachladung entsprechenden Anteil eines mehrwertigen Kations. Vorzugsweise steht $M^+$ für ein Alkalimetallkation, wie z. B. $Li^+$, $Na^+$ oder $K^+$ oder für ein Erdalkalimetallkation, für $NH_4{}^+$ oder eine quartäre Ammonium-Verbindung, wie sie durch Protonierung oder Quarternierung von Aminen erhältlich ist. Bevorzugt handelt es sich um Alkalimetallkationen, insbesondere um Natrium- oder Kaliumionen.

$X^-$ steht für ein Anionäquivalent, d. h. für ein einwertiges Anion oder den einer negativen Einfachladung entsprechenden Anteil eines mehrwertigen Anions. Vorzugsweise steht $X^-$ für ein Carbonat, Carboxylat oder Halogenid, besonders bevorzugt für $Cl^-$ und $Br^-$.

Die Werte für x stehen für eine ganze Zahl von 1 bis 240, vorzugsweise für eine ganze Zahl von 1 bis 100, insbesondere 1 bis 50, speziell 3 bis 40.

Wenn der Rest R für einen substituierten $C_{1-100}$-Alkylrest steht, so kann dieser z. B. ein- oder mehrfach durch wie für $R^1$ bis $R^{10}$ angegebene Reste substituiert sein.

Bei den Resten R kann eines oder können mehrere der im Rest enthaltenen Kohlenstoffatome durch Heteroatome ersetzt sein.

Der Rest R ist vorzugsweise ausgewählt aus Phenyl- oder $C_{1-12}$-Alkylresten, wobei es sich um lineare, verzweigte oder cyclische Alkylreste handeln kann, die auch Sauerstoffatome als Heteroatome in der Kette enthalten können, z. B. in Form von Alkylenoxid-, insbesondere Ethylenoxideinheiten, die endständig alkyliert sein können.

Bevorzugt steht der Rest R für einen $C_{2-14}$-Alkylrest, insbesondere für Propyl, n-Butyl, sek.-Butyl, tert.-Butyl, Pentyl, Hexyl, Heptyl, Octyl, 2-Ethylhexyl oder Dodecyl.

Bevorzugt steht der Rest R weiterhin für einen $C_{5-8}$-Cycloalkylrest, insbesondere Cyclohexyl.

Bevorzugt steht der Rest R weiterhin für einen Polyoxyalkylen- oder Polyalkyleniminrest. Geeignete Polyoxyalkylene leiten sich z. B. von Formaldehyd (Polyoxymethylene), cyclischen Ethern wie Tetrahydrofuran, Alkylenoxiden mit 2 bis 4 Kohlenstoffatomen im Alkylrest und Kombinationen davon ab. Geeignete Alkylenoxide sind beispielsweise Ethylenoxid, 1,2-Propylenoxid, Epichlorhydrin, 1,2- und 2,3-Butylenoxid. Geeignete Polyalkylenimine leiten sich z. B. von Aziridinen (Alkyleniminen) der Formel

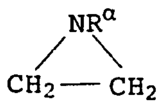

ab, worin $R^\alpha$ für Wasserstoff oder Alkyl steht. Das zahlenmittlere Molekulargewicht der Polyoxyalkylen- oder Polyalkyleniminreste liegt vorzugsweise in einem Bereich von etwa 400 bis 50000, besonders bevorzugt 800 bis 20000, speziell 2000 bis 10000.

Weist ein Substituent mehrere Reste R auf, so können diese gleiche oder verschiedene Bedeutungen aufweisen.

Bei den Strukturen der Formeln I und II kann es sich um Phosphacyclohexanone handeln, sofern zwei geminale Reste, ausgewählt $R^1$ bis $R^{10}$, für =O stehen. Vorzugsweise stehen in diesem Fall $R^5$ und $R^6$ in Formel I für ein doppelt gebundenes Sauerstoffatom.

Vorzugsweise sind in den Phosphacyclohexanen der Formeln I und II mindestens zwei oder drei oder vier der Reste $R^1$ bis $R^{10}$ von Wasserstoff verschieden. Vorzugsweise enthalten mindestens einer, zwei oder drei der Reste R und $R^1$ bis $R^{10}$ cyclische Strukturen, die aliphatisch, aromatisch oder heterocyclisch sein können. In den Verbindungen der Formel I liegen die cyclischen Strukturen beispielsweise in den Positionen 2, 4 und 6 vor. Die Strukturen können auch beispielsweise in den Positionen 1, 2 und 6 vorliegen.

Bevorzugt stehen die Reste $R^1$ bis $R^{10}$ für Wasserstoff und Reste, wie sie für R definiert sind, insbesondere $C_{1-12}$-Alkylreste, $C_{7-13}$-Aralkylreste, $C_{7-13}$-Alkarylreste und/oder $C_{6-12}$-Arylreste. Die Alkylreste können cyclische Strukturen enthalten. Die Arylgruppen der Aralkylreste, Alkarylreste und Arylreste leiten sich vorzugsweise von Benzol oder Naphthalin ab. Beispielsweise kann es sich um Phenylreste ($R^1$ bis $R^{10}$) oder Naphthylreste handeln. Alkarylreste weisen dabei vorzugsweise einen, zwei oder drei Alkylsubstituenten auf, bei denen es sich insbesondere um Methyl- oder

Ethylreste handelt.

Wenn R und/oder $R^1$ bis $R^{10}$ für Alkyl- und Arylreste stehen, so können diese fluoriert oder perfluoriert sein. Ein bevorzugter fluorierter Alkylrest ist Trifluormethyl.

In einer geeigneten Ausführungsform der Erfindung steht in den Verbindungen der allgemeinen Formel I oder II wenigstens einer der Reste R oder $R^1$ bis $R^{10}$ für eine polare (hydrophile) Gruppe, wobei dann in der Regel wasserlösliche Katalysatoren resultieren. Bevorzugt sind die polaren Gruppen ausgewählt unter $W'COO^-M^+$, $W'SO_3^-M^+$, $W'PO_3^{2-}M^+_2$, $W'NR'_3{}^+X^-$, W'OR', $W'NR'_2$, W'COOR', W'SR', $W'(CHR'CH_2O)_xR'$, $W'(CH_2NR')_xR'$, $W'(CH_2CH_2NR')_xR'$ oder $W'((CH_2)_4O)_xR'$, worin W', $M^+$, $X^-$, x und R' die zuvor angegebenen Bedeutungen besitzen.

Mindestens einer der Substituenten R und $R^1$ bis $R^{10}$ kann eine zusätzliche, zur Koordination befähigte dreibindige Phosphor- oder Stickstoffgruppierung aufweisen, wodurch ein zwei- oder mehrzähniger Ligand entsteht. Besonders bevorzugt sind Phosphan-, Phosphinit-, Phosphonit- und Phosphitgruppen sowie $\eta^5$-Phospholylkomplexe oder Phosphabenzolgruppierungen.

Vorzugsweise handelt es sich bei den Resten $R^1$ bis $R^{10}$ um Kohlenwasserstoffreste, die keine weiteren Heteroatome aufweisen.

Bei den Brücken W und W' handelt es sich nach einer bevorzugten Ausführungsform um Einfachbindungen oder Brücken mit 1 bis 6 Kohlenstoffatomen, die Bestandteil einer cyclischen oder aromatischen Gruppe sein können. Es kann sich dabei um Einfachbindungen handeln, wie auch um niedere Alkylengruppen, wie z. B. $C_{1-10}$-Alkylen.

In den Verbindungen der Formel II können die beiden Reste R gemeinsam und/oder eine Gruppe W für eine Brücke mit 1 bis 20 Kohlenstoffatomen stehen, die Bestandteil einer cyclischen oder aromatischen Gruppe sein kann und/oder die durch Heteroatome unterbrochen sein kann. Nach einer ersten bevorzugten Ausführungsform stehen die verbrückenden Gruppen für eine $C_{1-20}$-Alkylenkette. Verbrückende Alkylenketten sind gegebenenfalls mit Cycloalkyl, Heterocycloalkyl, Aryl und/oder Hetaryl substituiert, welche gegebenenfalls 1, 2 oder 3 der zuvor genannten Substituenten tragen können. Verbrückende Alkylenketten können in Abhängigkeit von der Kohlenstoffatomanzahl der Alkylenkette 1, 2, 3 oder 4 Doppelbindungen aufweisen und/oder durch 1 bis 10, z. B. 1, 2 oder 3, nicht benachbarte, gegebenenfalls substituierte Heteroatome unterbrochen sein und/oder ein-, zwei- oder dreifach mit Cycloalkyl, Aryl oder Hetaryl anelliert sein. Bevorzugt handelt es sich um $C_{1-15}$-, besonders bevorzugt um $C_{1-10}$-Alkylenketten, wie beispielsweise $C_6$- oder $C_3$-Alkylenketten.

Wenn in den Verbindungen der Formel II die beiden Reste R gemeinsam und/oder eine Gruppe W für eine Aryl-anellierte Alkylenbrücke stehen, so handelt es sich bei den anellierten Arylen bevorzugt um Benzol oder Naphthalin.

Anellierte Benzolringe sind vorzugsweise unsubstituiert oder weisen einen, zwei oder drei, insbesondere einen oder zwei Substituenten auf, die ausgewählt sind unter Alkyl, Alkoxy, Halogen, $SO_3H$, Sulfonat, $NE^1E^2$, Alkylen-$NE^1E^2$, Trifluormethyl, Nitro, Carboxyl, Alkoxycarbonyl, Acyl und Cyano. Anellierte Naphthaline sind vorzugsweise unsubstituiert oder weisen im nicht anellierten Ring und/oder im anellierten Ring jeweils einen, zwei oder drei, insbesondere einen oder zwei der zuvor bei den anellierten Benzolringen genannten Substituenten auf.

Vorzugsweise handelt es sich bei den verbrückenden Gruppen um eine unsubstituierte $C_{2-6}$-Alkylenbrücke.

Bevorzugt stehen die beiden Reste R gemeinsam und/oder eine Gruppe W weiterhin für eine $C_{2-20}$-Alkylenbrücke, die durch bis zu 20, insbesondere durch bis zu 10, nicht benachbarte, gegebenenfalls substituierte Heteroatome unterbrochen ist. Diese sind vorzugsweise ausgewählt unter O, S, $NR^\alpha$ oder $SiR^\beta R^\gamma$, wobei die Reste $R^\alpha$, $R^\beta$ oder $R^\gamma$ unabhängig voneinander für Alkyl, Cycloalkyl oder Aryl stehen. Vorzugsweise handelt es sich um oligomere Polyoxyalkylen- oder Polyalkyleniminbrücken. Diese weisen beispielsweise die zuvor beschriebenen Wiederholungseinheiten auf.

Nach einer weiteren Ausführungsform können in den Verbindungen der Formel II die beiden Reste R gemeinsam und/oder eine Gruppe W auch eine höhermolekulare Polyoxyalkylen- oder Polyalkylenimin-Brücke mit mindestens 21 Kohlenstoffatomen bilden. Das zahlenmittlere Molekulargewicht der Polyoxyalkylen- oder Polyalkyleniminreste liegt vorzugsweise in einem Bereich von etwa 400 bis 50000, besonders bevorzugt 800 bis 20000 und speziell 1000 bis 10000. Besonders bevorzugt handelt es sich um Polyethylenoxide, Copolymere aus Ethylenoxid und 1,2-Propylenoxid, in denen die Alkylenoxide in beliebiger Reihenfolge, alternierend oder in Form von Blöcken eingebaut sein können sowie Polyethylenimine.

Nach einer geeigneten Ausführungsform kann in den Verbindungen der Formel I auch einer der Reste R oder $R^1$ bis $R^{10}$ und in den Verbindungen der Formel II auch einer der Reste R oder $R^1$ bis $R^8$ oder die beiden Reste R gemeinsam oder eine Gruppe W auch für einen von den zuvor genannten Definitionen für diese Reste und Gruppen verschiedenen Polymerrest mit einem zahlenmittleren Molekulargewicht im Bereich von etwa 500 bis 50000 stehen. Die Wiederholungseinheiten dieser Polymerreste leiten sich formal ab von Monomeren, die ausgewählt sind unter Mono- und Diolefinen, Vinylaromaten, Estern $\alpha,\beta$-ethylenisch ungesättigter Mono- und Dicarbonsäuren mit $C_1$-$C_{30}$-Alkanolen, N-Vinylamiden, N-Vinyllactamen, unter Ringöffnung polymerisierbaren heterocyclischen Verbindungen und Mischungen davon.

Bevorzugt weisen die Polymerreste ein zahlenmittleres Molekulargewicht im Bereich von 800 bis 20000, besonders bevorzugt 2000 bis 10000, auf.

Als Monomere bevorzugte Monoolefine sind $C_{2-8}$-Monoolefine, wie Ethen, Propen, n-Buten, Isobuten sowie

Aromaten-substituierte Monoolefine, wie 1,1-Diphenylethylen, 1,2-Diphenylethylen und Mischungen der zuvor genannten Monoolefine. Als Monomere bevorzugte Diolefine sind konjugierte Diene, wie Butadien, Isopren, 2,3-Dimethylbutadien, Piperylen(1,3-Pentadien) und Mischungen davon. Die Ester α,β-ethylenisch ungesättigter Mono- und Dicarbonsäuren sind vorzugsweise ausgewählt unter den Estern der Acrylsäure, Methacrylsäure, Maleinsäure, Fumarsäure, Itaconsäure und Crotonsäure. Bevorzugt sind die Ester mit $C_{1-20}$-Alkanolen. Dazu zählen beispielsweise Methyl(meth)acrylat, Ethyl(meth)acrylat, n-Butyl(meth)acrylat, tert.-Butyl(meth)acrylat, n-Hexyl(meth)acrylat, n-Octyl(meth)acrylat, Ethylhexyl(meth)acrylat, etc. Als Monomere geeignete Vinylaromaten sind beispielsweise Styrol, α-Methylstyrol, o-Chlorstyrol, Vinyltoluole und Mischungen davon. Geeignete N-Vinylamide sind beispielsweise N-Vinylformamid, N-Vinylacetamid, N-Vinylpropionamid und Mischungen davon. Geeignete N-Vinyllactame sind beispielsweise N-Vinylpyrrolidin, N-Vinylpiperidon, N-Vinylcaprolactam und Mischungen davon. Geeignete Monomere für die Ringöffnungspolymerisation sind z. B. cyclische Ether, wie Ethylenoxid und Propylenoxid, cyclische Amine, cyclische Sulfide (Ethylensulfid, Thietane), Lactone und Lactame. Bevorzugt sind ε-Caprolacton und ε-Caprolactam.

Die zuvor genannten Monomere können einzeln, in Form von Gemischen aus der jeweiligen Monomerklasse sowie allgemein als Mischungen eingesetzt werden.

Die Herstellung der als Reste geeigneten Polymere erfolgt nach üblichen, dem Fachmann bekannten Polymerisationsverfahren. In Abhängigkeit von den zu polymerisierenden Monomeren zählt dazu die radikalische, kationische und anionische Polymerisation, einschließlich der kationischen und anionischen Ringöffnungspolymerisation.

Erfolgt die Herstellung der Polymerreste durch anionische Polymerisation, beispielsweise durch die entsprechende im Folgenden beschriebene Reaktionsvariante zur Herstellung der erfindungsgemäßen Phosphacyclohexane, so werden als Monomere vorzugsweise Akzeptor-aktivierte ethylenisch ungesättigte Verbindungen und Ethen eingesetzt.

Wenn in den Verbindungen der Formel I einer der Reste R oder $R^1$ bis $R^{10}$ und in den Verbindungen der Formel II einer der Reste R oder $R^1$ bis $R^8$ oder die beiden Reste R gemeinsam oder eine Gruppe W für einen Polymerrest stehen, so handelt es sich vorzugsweise um einen Polyolefinrest (Polyalkenrest). Diese Polyolefine weisen Wiederholungseinheiten auf, die sich von einpolymerisierten Monomeren ableiten, die vorzugsweise ausgewählt sind unter $C_{2-6}$-Alkenen, wie Ethen, Propen, n-Buten, Isobuten, Olefinen mit zwei Doppelbindungen, wie Butadien, Isopren, 1,3-Pentadien und Mischungen davon. Polyolefine, die konjugierte Diene eingebaut enthalten, können im Wesentlichen ausschließlich die 1,2- und 1,4-Polymerisationsprodukte als auch Mischformen mit beliebigen 1,2- und 1,4-Anteilen aufweisen. Verfahren zur Einstellung der 1,2- und 1,4-Anteile bei der Polymerisation konjugierte Diene sind dem Fachmann bekannt. Dazu zählt beispielsweise bei der anionischen Polymerisation der Zusatz von Donorlösungsmitteln, z. B. Ether, wie THF, oder Aminen. Polyolefine mit Wiederholungseinheiten von 1,2-Additionsprodukten konjugierter Diene weisen seitenständige ethylenisch ungesättigte Gruppen auf. Polyolefine mit Wiederholungseinheiten von 1,4-Additionsprodukten weisen in der Hauptkette ethylenisch ungesättigte Gruppen auf. Diese können gewünschtenfalls teilweise oder vollständig hydriert werden. Möglich ist jedoch auch die Verwendung von Phosphacyclohexanen mit Polyolefinresten mit ethylenisch ungesättigten Seitenketten als Liganden in Übergangsmetallkomplexen für die Hydroformylierung. Dabei erfolgt unter Hydroformylierungsbedingungen in der Regel zumindest eine teilweise Umsetzung der ethylenisch ungesättigten Seitenketten in Alkoholgruppen, d. h. es resultieren Liganden mit polaren Seitenketten.

Wenn in den Verbindungen der Formel I einer der Reste R oder $R^1$ bis $R^{10}$ und in den Verbindungen der Formel II einer der Reste R oder $R^1$ bis $R^8$ oder die beiden Reste R gemeinsam oder eine Gruppe W für einen Polyolefinrest stehen, so handelt es sich vorzugsweise um einen Polyethylen- oder Polybutadienrest.

Die in Struktur II nicht an die Brücke W gebundenen Positionen der Phosphorcyclohexanringe können zudem einen der Reste R bzw. $R^1$ bis $R^{10}$ tragen.

Der Rest R' ist vorzugsweise Wasserstoff oder ein $C_{1-6}$-Alkyl, wie ein Methyl- oder Ethylrest. Weist ein Substituent mehrere Reste R' auf, so können diese gleiche oder verschiedene Bedeutungen aufweisen.

Vorzugsweise liegt, abgesehen von C=O in 4-Position und $C(CH_3)_2$ in 2- und/oder 6-Position an jedem Ringkohlenstoffatom des Phosphacyclohexans maximal ein von Wasserstoff verschiedener Substituent vor. Beispielsweise können in 2- und 6-Position, 2-, 4- und 6-Position, 2-, 3-, 5- und 6-Position Substituenten vorliegen. Besonders bevorzugt sind Substituenten, speziell Aryl, in 2- und 6-Position.

Bevorzugt werden als Liganden Phosphacyclohexane verwendet, die ausgewählt sind unter Verbindungen der allgemeinen Formel III

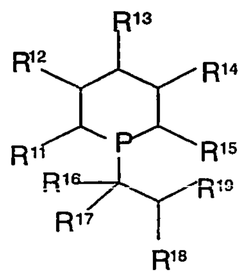

(III)

mit der Bedeutung:

$R^{11}$ bis $R^{19}$ unabhängig voneinander Wasserstoff, $C_{1-20}$-Alkyl, $C_{7-20}$-Aralkyl, $C_{7-20}$-Alkaryl, $C_{6-12}$-Aryl, wobei eines oder mehrere Kohlenstoffatome durch Heteroatome ersetzt sein können, $W'COO^-M^+$, $W'SO_3^-M^+$, $W'PO_3^{2-}M^+_2$, $W'NR'_3{}^+X^-$, W'OR', $WNR'_2$, W'COOR', W'SR', $W'(CHR'CH_2O)_x$ R', $W'(CH_2NR')_xR'$, $W'(CH_2CH_2NR')_xR'$,

wobei jeweils zwei vicinale Reste $R^{11}$ bis $R^{15}$ und/oder $R^{17}$ und $R^{18}$ und/oder $R^{16}$ und $R^{17}$ und/oder $R^{16}$ und $R^{19}$ und/oder $R^{18}$ und $R^{19}$ zu Ringen verbunden sein können,

W' Einfachbindung oder Brücke mit 1 bis 20 Kohlenstoffatomen, die Bestandteil einer cyclischen oder aromatischen Gruppe sein kann,

R' Wasserstoff oder $C_{1-6}$-Alkyl,

M+ Kation,

$X^-$ Anion,

x Zahl von 1 bis 240,

wobei einer oder mehrere der Reste $R^{11}$ bis $R^{19}$ eine zusätzliche, zur Koordination befähigte dreibindige Phosphor- oder Stickstoffgruppierung aufweisen können,
wobei $R^{18}$ auch $-W'-CR^{20}=CR^{21}R^{22}$, wobei $R^{20}$, $R^{21}$, $R^{22}$ wie vorstehend für $R^{11}$ bis $R^{19}$ definiert sind, bedeuten kann
Besonders bevorzugt werden als Liganden Phosphacyclohexane verwendet, die ausgewählt sind unter Verbindungen der allgemeinen Formeln I.a bis I.g und II.a

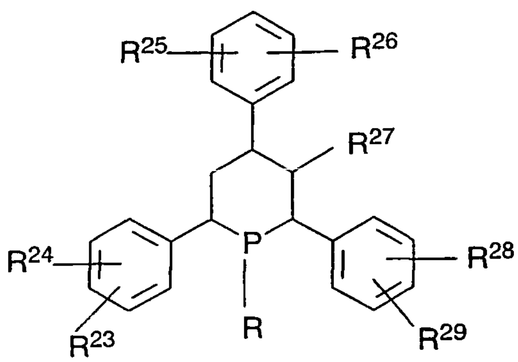

(I.a)

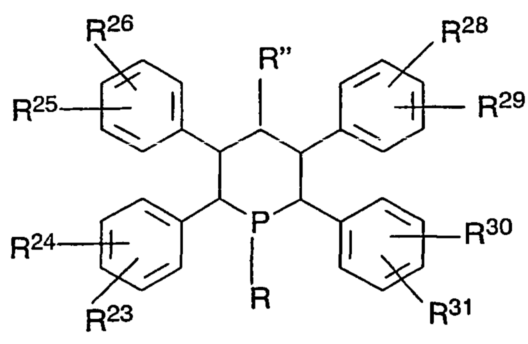

(I.b)

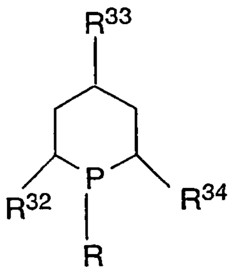

(I.c)

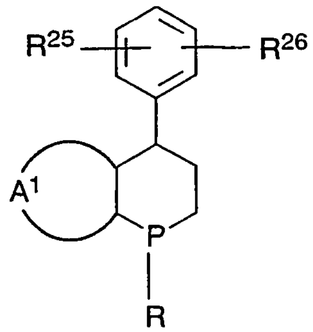

(I.d)

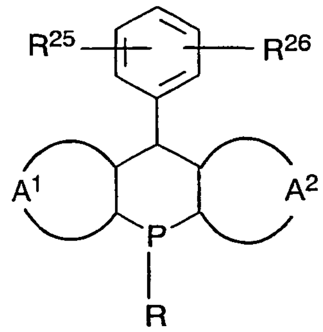

(I.e)

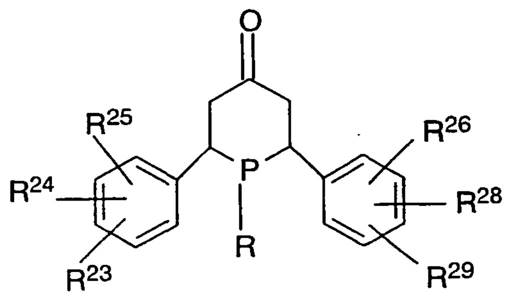

(I.f)

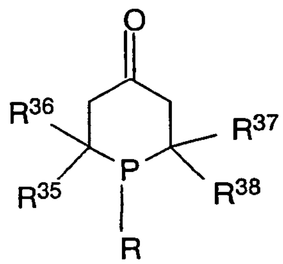

(I.g)

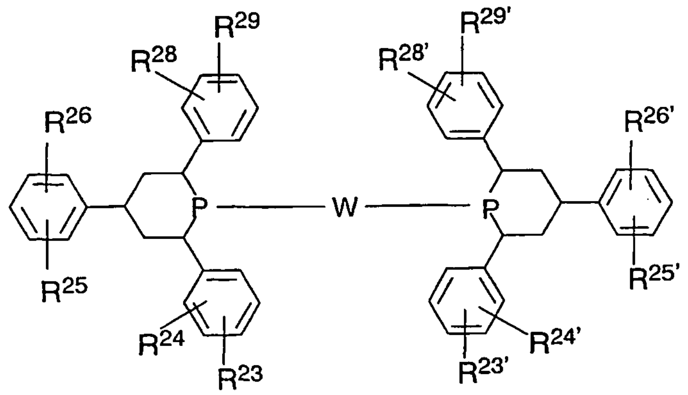

(II.a)

worin

R für $C_{1-20}$-Alkyl, Cycloalkyl, $C_{6-12}$-Aryl, W'$(CHR'CH_2O)_xR'$, $W'((CH_2)_4O)_xR'$ oder einen Polymerrest mit einem zahlenmittleren Molekulargewicht im Bereich von 500 bis 50000, der aufgebaut ist aus Ethylen und/ oder Butadien, steht, wobei
W' für eine Einfachbindung oder $C_{1-4}$-Alkylen steht, und
R' für Wasserstoff oder $C_{1-20}$-Alkyl steht,

x für eine ganze Zahl von 1 bis 240 steht,

$R^{23}$, $^{23'}$, $R^{24}$, $R^{24'}$, $R^{25}$, $R^{25'}$, $R^{26}$, $R^{26'}$, $R^{28}$, $R^{28'}$, $R^{29}$, $R^{29'}$, $R^{30}$ und $R^{31}$ R unabhängig voneinander für Wasserstoff, Alkyl, Alkoxy, Carboxyl, Carboxylat, Trifluormethyl, $-SO_3H$, Sulfonat, $NE^1E^2$ oder Alkylen-$NE^1E^2$ stehen, wobei $E^1$ und $E^2$ unabhängig voneinander für Wasserstoff, Alkyl oder Cycloalkyl stehen,

$R^{27}$ für Wasserstoff, Alkyl, Cycloalkyl, Aryl oder Aralkyl steht,

$R^{32}$, $R^{33}$, $R^{34}$, $R^{35}$, $R^{36}$, $R^{37}$ und $R^{38}$ unabhängig voneinander für Alkyl oder Cycloalkyl stehen,

R" für Wasserstoff oder Phenyl steht,

$A^1$ und $A^2$ zusammen mit den benachbarten Kohlenstoffatomen des Phosphacyclohexans, an die sie gebunden wird, für ein anelliertes Ringsystem mit je 1 oder 2 weiteren Ringen stehen,

W für eine Brücke mit 1 bis 20 Kohlenstoffatomen steht, die durch Heteroatome unterbrochen sein kann.

In den Verbindungen der Formel I.a bis I.f und II.a stehen die Reste $R^{23}$, $R^{23'}$, $R^{24}$, $R^{24'}$, $R^{25}$, $R^{25'}$, $R^{26}$, $R^{26'}$, $R^{28}$, $R^{28'}$, $R^{29}$, $R^{29'}$, $R^{30}$ und $R^{31}$ vorzugsweise unabhängig voneinander für Wasserstoff, $C_{1-4}$-Alkyl, bevorzugt Methyl, Ethyl, Isopropyl, tert.-Butyl, $C_{1-4}$-Alkoxy, bevorzugt Methoxy.

$R^{27}$ steht vorzugsweise für Aralkyl, insbesondere Benzyl.

$R^{32}$, $R^{33}$ und $R^{34}$ stehen vorzugsweise unabhängig voneinander für $C_{1-4}$-Alkyl, besonders bevorzugt tert.-Butyl.

$R^{35}$, $R^{36}$, $R^{37}$ und $R^{38}$ stehen vorzugsweise unabhängig voneinander für $C_{1-4}$-Alkyl, besonders bevorzugt Methyl.

Bei den Ringen der anellierten Ringsystemen $A^1$ und $A^2$ handelt es sich vorzugsweise um 5- bis 7-gliedrige Ringe. Bevorzugt sind Ringsysteme, die sich von Benzol, Naphthalin und deren teilweisen Hydrierungsprodukten oder Perhydrierungsprodukten ableiten. Ankondensierte Ringe sind vorzugsweise unsubstituiert oder weisen je Ring 1,2 oder 3, insbesondere 1 oder 2 Substituenten auf, die ausgewählt sind unter Alkyl, Alkoxy, Halogen, $SO_3H$, Sulfonat, $NE^1E^2$, Alkylen-$NE^1E^2$, Trifluormethyl, Nitro, Carboxyl, Alkoxycarbonyl, Acyl und Cyano.

In der Formel II.a steht der Rest W vorzugsweise für eine $C_{1-10}$-Alkylengruppe, wie beispielsweise eine $C_2$-$C_8$-Alkylengruppe. Die Gruppe W steht vorzugsweise weiterhin für eine Brücke mit 1 bis 20 Kohlenstoffatomen, die durch bis zu 10 nicht benachbarte Sauerstoffatome unterbrochen sein kann. Dabei handelt es sich dann um niedermolekulare Polyoxyalkylengruppen, die Wiederholungseinheiten aufweisen, die sich von Ethylenoxid, Propylenoxid, Tetrahydrofuran und beliebigen Kombinationen davon ableiten. Die Gruppe W kann weiterhin für eine Polyoxyalkylengruppe mit mehr als 21 Kohlenstoffatomen stehen.

Bevorzugt sind Verbindungen der Formel I.a, worin $R^{23}$, $R^{24}$, $R^{25}$, $R^{26}$, $R^{27}$, $R^{28}$, und $R^{29}$ für Wasserstoff stehen. Bevorzugt sind weiterhin Verbindungen der Formel I.a, worin $R^{25}$, $R^{26}$ und $R^{27}$ für Wasserstoff und $R^{23}$, $R^{24}$, $R^{28}$ und $R^{29}$ für $C_{1-4}$-Alkyl, besonders bevorzugt Methyl, stehen. Bevorzugt sind weiterhin Verbindungen der Formel I.a, worin $R^{23}$ und $R^{28}$ für $C_{1-4}$-Alkyl, besonders bevorzugt Methyl, und $R^{24}$, $R^{25}$, $R^{26}$, $R^{27}$ und $R^{29}$ für Wasserstoff stehen. Vorzugsweise weisen die Phenylreste in 2- und 6-Position des Phosphacyclohexanrings der Verbindungen der Formel I.a je einen Alkylrest in der 2-Position oder zwei Alkylreste in 2- und 4-Position auf. Vorzugsweise steht der Rest R in den Verbindungen der Formel I.a (wie auch I.b bis I.f und II.a) für $C_{1-14}$-Alkyl, wie Propyl, n-Butyl, sek.-Butyl, Isobutyl, n-Octyl, 2-Ethylhexyl, Dodecyl, sowie Cyclohexyl, Methoxyethoxyethyl oder für einen Polymerrest, beispielsweise einen Polyethylen- oder Polybutadienrest.

Bevorzugt sind in den Verbindungen der Formel I.b die Reste $R^{23}$, $R^{24}$, $R^{25}$, $R^{26}$, $R^{28}$, $R^{29}$, $R^{30}$ und $R^{31}$ ausgewählt unter Wasserstoff und $C_{1-4}$-Alkyl, besonders bevorzugt Wasserstoff.

Besonders bevorzugt stehen in den Verbindungen der Formel I.c die Reste $R^{32}$, $R^{33}$ und $R^{34}$ für tert.-Butyl.

Besonders bevorzugt steht die Verbindung der Formel I.e für ein 1,2,7,8-Dibenzo-3,4,5,6-tetrahydro-9-phosphaanthracengerüst.

Besonders bevorzugt sind in den Verbindungen der Formel I.f die Reste $R^{23}$, $R^{24}$, $R^{25}$, $R^{26}$, $R^{28}$ und $R^{29}$ ausgewählt unter Wasserstoff und $C_{1-4}$-Alkyl, besonders bevorzugt Methyl. Vorzugsweise weisen die Phenylreste der Phosphacyclohexanone der Formel I.f einen von Wasserstoff verschiedenen Substituenten in der 2-Position, zwei von Wasserstoff verschiedene Substituenten in der 2- und 4-Position oder drei von Wasserstoff verschiedene Substituenten in der 2-, 4- und 6-Position auf.

Besonders bevorzugt sind Verbindungen der Formel I.g, worin $R^{35}$, $R^{36}$, $R^{37}$ und $R^{38}$ für $C_{1-4}$-Alkyl, insbesondere Methyl, stehen.

Bezüglich bevorzugter Substituenten der Verbindungen der Formel II.a gilt das zuvor für Verbindungen der Formel I.a Gesagte. Die verbrückende Gruppe W steht vorzugsweise für eine $C_{1-10}$-Alkylengruppe, wie beispielsweise eine $C_2$-$C_8$-Alkylengruppe.

Beispielhaft für bevorzugte Verbindungen sind folgende Phosphacyclohexane:

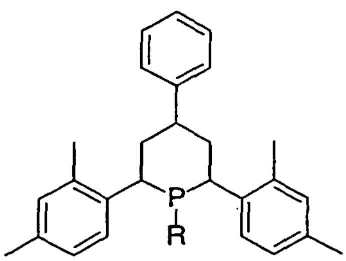

1 [a – i]

a: R = $C_2H_5$
b: R = $C_3H_7$
c: R = $C_4H_9$
d: R = $C_6H_{11}$
e: R = cyclo-$C_6H_{11}$
f: R = $C_8H_{17}$

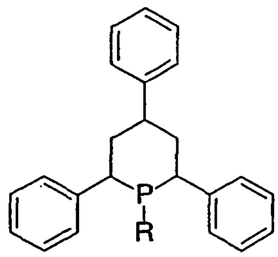

2 [a – i]

g: R = $CH_2CH_2O[CH_2CH_2O]_nCH_3$ mit n = 0 bis 100
h: R = Polyethylen, $M_n$ = 500-50000
i: R = Polybutadien, $M_n$ = 500-50000 (teilhydriert oder vollständig hydriert)
k: R = Polyisobuten, $M_n$ = 500-50000

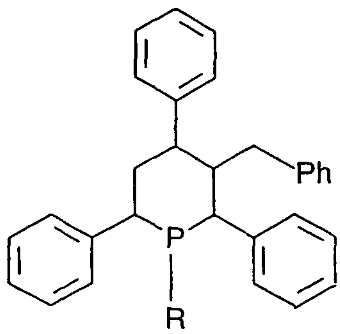

3 [a – i]

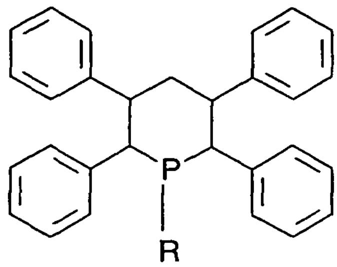

4 [a – i]

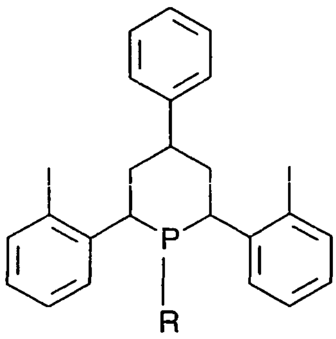

5 [a – i]

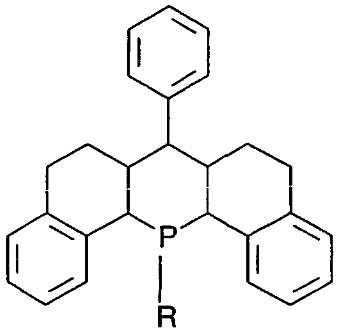

6 [a – i]

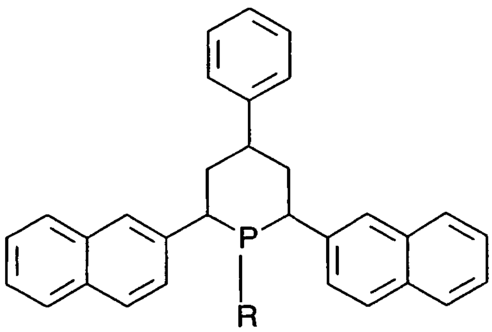

7 [a – i]

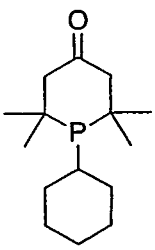

8

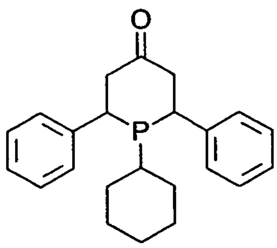

9

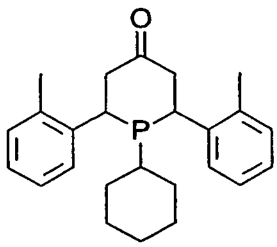

10

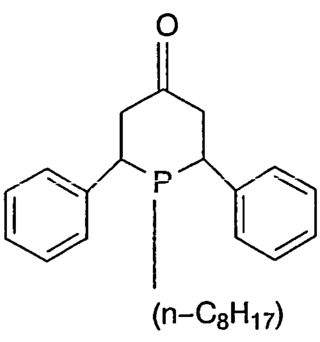

11

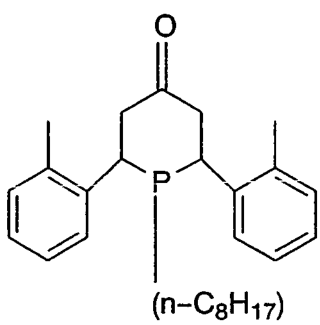

12

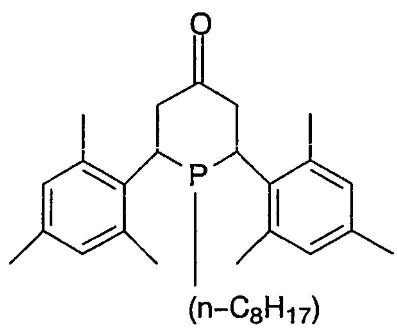

13

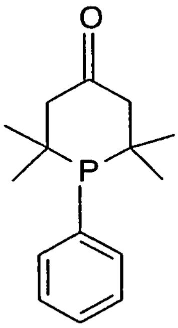

14

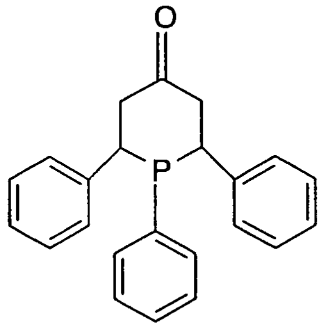

15

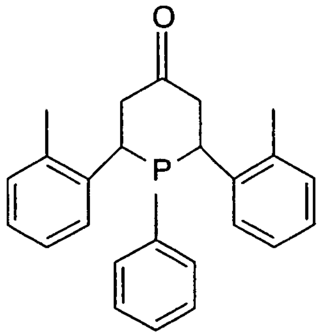

16

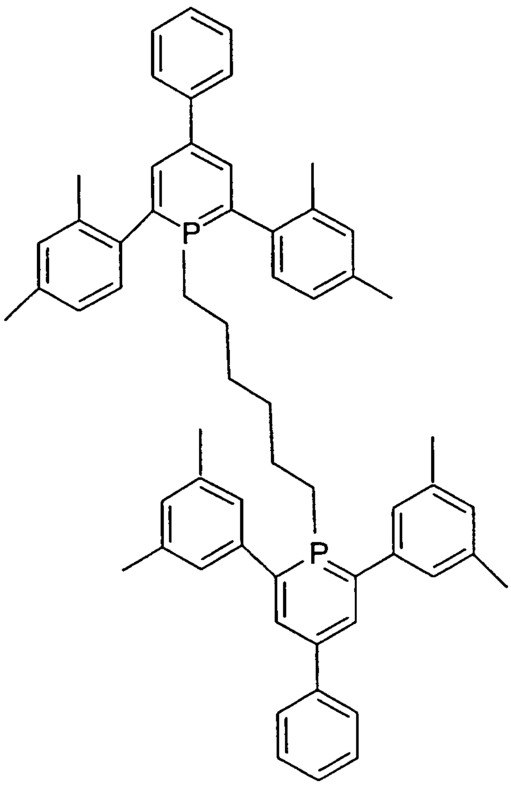

17

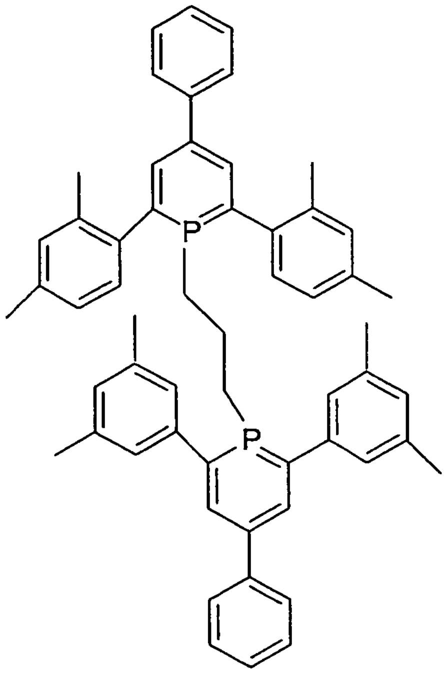

18

Besonders bevorzugt sind die Strukturen 1.

Ein weiterer Gegenstand der Erfindung sind Phosphacyclohexane der allgemeinen Formeln I, II und III, wie zuvor beschrieben, wobei mindestens drei der Reste $R^1$ bis $R^{10}$ von Wasserstoff verschieden sind und wobei mindestens zwei der Reste R und $R^1$ bis $R^{10}$ cyclische Strutkturen, die aliphatisch, aromatisch oder heterocyclisch sein können, enthalten, und Gemische davon, ausgenommen:

- Verbindungen der Formel I, worin $R^5$ und $R^6$ gemeinsam für eine Oxo-Gruppe stehen oder einer der Reste $R^5$ oder $R^6$ für Hydroxyl und der andere für Wasserstoff steht.

Auf geeignete und bevorzugte Ausführungsformen der zuvor für den Einsatz als Liganden beschriebenen Phosphacyclohexane wird Bezug genommen.

Für die Herstellung der erfindungsgemäß als Cokatalysatoren eingesetzten Phosphacyclohexane stehen verschiedene, bereits veröffentlichte Verfahren zur Verfügung. Exemplarisch seien folgende Verfahren genannt, auf die in vollem Umfang Bezug genommen wird:

1-Substituierte Phosphacyclohexane lassen sich nach Chem. Ber. 1961, 94, 113-117 durch Kondensation von Alkaliphosphiden des Typs $Li_2RP$ mit 1,5-Dihalogenalkanen herstellen.

Nach J. Org. Chem. 1962, 27, 1824-1827 lassen sich 2,6-substituierte 1-Phosphacyclohexan-4-one durch Kondensation von Phoronen oder Phoronderivaten mit sekundären Alkyl- und Arylphosphanen herstellen. Die erhaltenen Phosphacyclohexanone können durch eine anschließende Wolff-Kishner-Reduktion in 2,6-substituierte Phosphacyclohexane überführt werden.

Nach Tetrahedron Letters 1970, 645-648 lassen sich 1-substituierte 2,6-Diphenyl-1-phosphacyclohexan-4-one durch Kondensation von Bis(hydroxymethyl)phosphan mit Dibenzylidenaceton in siedendem Pyridin erhalten.

Phosphacyclohexanole und ein Verfahren zur Herstellung derartiger Verbindungen aus Phosphacyclohexanonen sind in US 3,105,096 beschrieben.

Die Anzahl der über die obigen Verfahren herstellbaren Phosphacyclohexane ist allerdings auf bestimmte Substitutionmuster beschränkt. Insbesondere sterisch anspruchsvolle Phosphacyclohexane sind nur schwer und nicht über die obigen Verfahren zugänglich.

Die Herstellung der erfindungsgemäßen Phosphacyclohexane gelingt durch ein Verfahren zur Herstellung wenigstens eines Phosphacyclohexans der allgemeinen Formel V.c

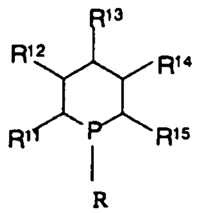

(V.c)

worin

R
für Wasserstoff, $C_{1-100}$-Alkyl, Cycloalkyl, Heterocycloalkyl, $C_{7-20}$-Aralkyl, $C_{7-20}$-Alkaryl, $C_{6-12}$-Aryl, Hetaryl, $W'COO^-M^+$, $W'SO_3^-M^+$, $W'PO_3^{2-}M^+_2$, $W'NR'_3{}^+X^-$, W'OR', $W'NR'_2$, W'COOR', W'SR', $W'(CHR'CH_2O)_xR'$, $W'(CH_2NR')_xR'$, $W'(CH_2CH_2NR')_xR'$, $W'((CH_2)_4O)_xR'$ oder W'COR', steht,
wobei in zwei gleichen oder verschiedenen Einheiten V.a, V.b oder V.c die Reste R gemeinsam oder je einer der Reste $R^{11}$ bis $R^{15}$ gemeinsam auch für eine verbrückende Gruppe W stehen können, die diese Einheiten kovalent miteinander verbindet,

$R^{11}$ bis $R^{15}$
unabhängig voneinander für Wasserstoff, $C_{1-20}$-Alkyl, $C_{7-20}$-Aralkyl, $C_{7-20}$-Alkaryl, $C_{6-12}$-Aryl, wobei eines oder mehrere Kohlenstoffatome durch Heteroatome ersetzt sein können, $W'COO^-M^+$, $W'SO_3^-M^+$, $W'PO_3^{2-}M^+_2$, $W'NR'_3{}^+X^-$, W'OR', $W'NR'_2$, W'COOR', W'SR', $W'(CHR'CH_2O)_xR'$, $W'(CH_2NR')_xR'$, $W'(CH_2CH_2NR')_xR'$, $W'((CH_2)_4O)_xR'$, W'Halogen, $W'NO_2$, W'COR' oder W'CN, stehen,
wobei in den Resten R und $R^{11}$ bis $R^{15}$ ein oder mehrere Wasserstoffatome durch Fluor ersetzt sein können,

W und W'
unabhängig voneinander für Einfachbindungen oder Brücken mit 1 bis 20 Kohlenstoffatomen stehen, die Bestandteil einer cyclischen oder aromatischen Gruppe sein können und durch Heteroatome unterbrochen sein können, wobei W auch für eine Polyoxyalkylen- oder Polyalkylenimin-Brücke mit mindestens 21 Kohlenstoffatomen stehen kann,

R' für Wasserstoff, $C_{1-20}$-Alkyl, Carbonylalkyl, Cycloalkyl oder Aryl steht,

M+ für ein Kationäquivalent steht,

$X^-$ für ein Anionäquivalent steht und,

x für eine ganze Zahl von 1 bis 240 steht,

wobei einer oder mehrere der Reste R und $R^{11}$ bis $R^{15}$ eine zusätzliche, zur Koordination befähigte dreibindige Phosphor- oder Stickstoffgruppe aufweisen können,
wobei in den Verbindungen der Formel V.c auch einer der Reste R oder $R^{11}$ bis $R^{15}$ oder, falls vorhanden, eine Gruppe W auch für einen Polymerrest mit einem zahlenmittleren Molekulargewicht im Bereich von 500 bis 50000 stehen kann, aufgebaut aus Wiederholungseinheiten, die sich von Monomeren ableiten, die ausgewählt sind unter Mono- und Diolefinen, Vinylaromaten, Estern α,β-ethylenisch ungesättigter Mono- und Dicarbonsäuren mit $C_{1-30}$-Alkanolen, N-Vinylamiden, N-Vinyllactamen, unter Ringöffnung polymerisierbaren heterocyclischen Verbindungen und Mischungen davon,
wobei man

a) wenigstens ein Phosphabenzol der allgemeinen Formel IV

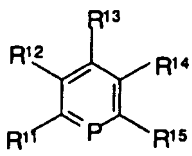

(IV)

worin $R^{11}$, $R^{12}$, $R^{13}$, $R^{14}$ und $R^{15}$ die zuvor angegebenen Bedeutungen besitzen, mit Wasserstoff in Gegenwart eines Hydrierkatalysators unter Erhalt wenigstens eines Phosphacyclohexans der Formel V.c, worin der Rest R für Wasserstoff steht, hydriert, und gegebenenfalls

b1) das/die in Schritt a) erhaltene(n) Hydrierungsprodukt(e) mit wenigstens einer ethylenisch ungesättigten Verbindung umsetzt, wobei der Reaktionsschritt b1)

- in Gegenwart wenigstens eines Radikalbildners erfolgt, oder
- in Gegenwart wenigstens einer Säure oder wenigstens einer Base oder wenigstens einer Übergangsmetallverbindung erfolgt, oder
- bei einer erhöhten Temperatur im Bereich von 100 bis 250 °C erfolgt, oder

b2) das/die in Schritt a) erhaltene(n) Hydrierungsprodukt(e) mit wenigstens einer Verbindung der Formel R-X oder X-W-Y umsetzt, wobei X und Y für nucleophil verdrängbare Gruppen stehen und R und W die zuvor angegebenen Bedeutungen, ausgenommen R für Wasserstoff, besitzen.

Schritt a) Hydrierung des Phosphabenzols

Prinzipiell können zur Hydrierung übliche Phosphabenzole und Bisphosphabenzole eingesetzt werden, deren Benzolringe Substituenten aufweisen, die denen der zuvor beschriebenen Verbindungen der Formeln I oder II entsprechen.
Bevorzugt als Ausgangsmaterialien sind Phosphabenzolverbindungen der allgemeinen Formel IV.a

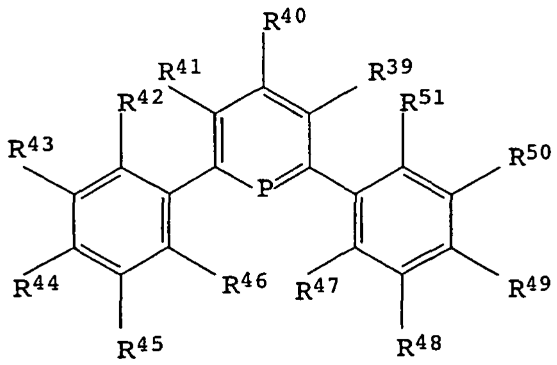

(IV.a)

in der die Reste $R^{39}$ bis $R^{51}$ unabhängig voneinander Wasserstoff, W'COOM, W'$SO_3$M, W'$NR_3$X, W'$NR_2$, W'OR, W'COOR oder W'SR (mit M = wasserstoff, $NH_4$ oder Alkalimetall, X = Anion, R = Wasserstoff oder $C_{1-6}$-Alkyl), oder $C_{1-12}$-Alkyl, $C_{6-12}$-Aryl, $C_{7-12}$-Aralkyl, $C_{7-12}$-Alkaryl oder $C_{3-6}$-Heteroaromaten, wobei die Alkyl-, Aryl-, Alkaryl- und Aralkylreste mit den davor genannten Resten substituiert sein können und zwei oder mehrere der Reste zu anellierten aliphatischen oder aromatischen Ringen verbunden sein können.

Vorzugsweise bedeuten mindestens einer der Reste $R^{42}$ und $R^{46}$ und mindestens einer der Reste $R^{47}$ und $R^{51}$ unabhängig voneinander $C_{1-12}$-Alkyl, $C_{6-12}$-Aryl, $C_{7-12}$-Aralkyl oder $C_{7-12}$-Alkaryl, oder $R^{42}$ und $R^{41}$ und/oder $R^{51}$ und $R^{39}$ bilden einen $C_{2-4}$-Alkylenrest.

Besonders bevorzugt bedeuten mindestens einer der Reste $R^{42}$ und $R^{46}$ und mindestens einer der Reste $R^{47}$ und $R^{51}$ $C_{1-6}$-Alkyl, oder ($R^{42}$ und $R^{41}$) und ($R^{51}$ und $R^{39}$) bilden jeweils einen $C_{2-3}$-Alkylenrest.

Vorzugsweise bedeutet $R^{40}$ einen Phenylrest.

Bevorzugt bedeuten die Reste $R^{39}$ und $R^{41}$ Wasserstoff und jeweils maximal drei der Reste $R^{42}$ bis $R^{46}$ und $R^{47}$ bis $R^{51}$ sind von Wasserstoff verschieden. Dabei liegen in den Resten $R^{42}$ bis $R^{46}$ und $R^{47}$ bis $R^{51}$ jeweils besonders bevorzugt maximal 6, insbesondere maximal 3 C-Atome vor.

Bevorzugt liegen in den Verbindungen der allgemeinen Formel (IV.a) neben dem Phosphabenzolkern 3 bis 5, insbesondere 3 weitere aromatische Kerne vor. Die Anzahl der Alkylreste in den Verbindungen der allgemeinen Formel (IV.a) beträgt vorzugsweise 0 bei rein cyclischen Strukturen, ansonsten vorzugsweise 2 bis 7, insbesondere 2 bis 6. Bei den Alkylresten kann es sich um lineare oder verzweigte Alkylreste handeln. Vorzugsweise liegen nur lineare Alkylreste vor. Gleiches gilt für verbrückende Alkylengruppen entsprechend.

Beispielhaft seien folgende Phosphabenzole genannt:

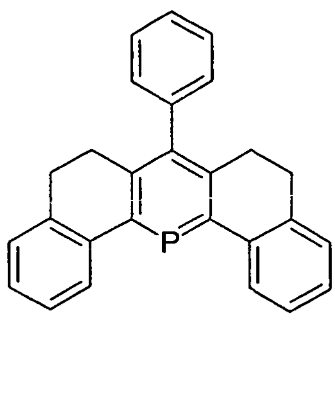

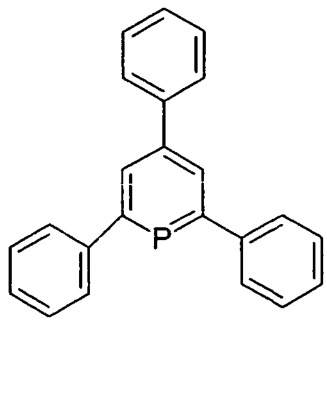

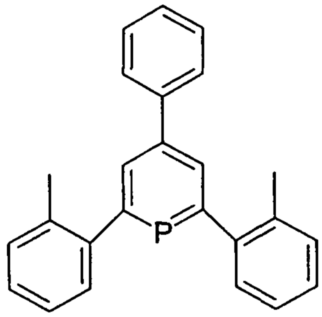

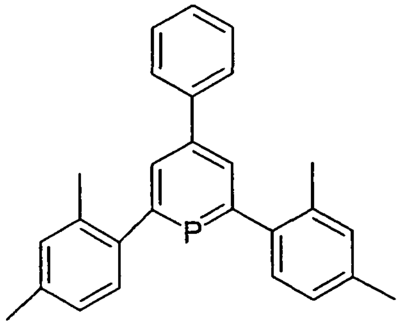

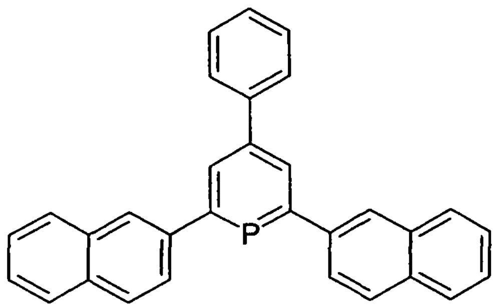

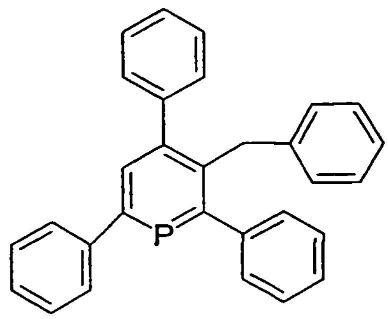

Als Phosphabenzole eignen sich neben den vorstehend beschriebenen Verbindungen insbesondere 2,4,6-Triarylphosphabenzole, 2,3,5,6-Tetraarylphosphabenzole und 2,3,4,5,6-Pentaarylphosphabenzole. Besonders bevorzugt sind 2,4,6-Triarylphosphabenzole. In 1-Position unsubstituierte Phosphacyclohexane und -phosphacyclohexene sind in der Regel von geringerer katalytischer Aktivität als entsprechende in 1-Position substituierte Verbindungen.

Die Phosphabenzole können nach allgemein beschriebenen Verfahren hergestellt werden, welche z. B. zu finden sind in G. Märkl in Multiple Bonds and Low Coordination in Phosphorus Chemistry (Hrsg.: M. Regitz, O. J. Scherer), Thieme, Stuttgart, 1990, S. 220 ff (und dort zitierte Literatur). Ein besonders einfaches Verfahren zur Herstellung von Phosphabenzolverbindungen aus Pyryliumsalzen durch Umsetzung mit Phosphan ist beschrieben in WO 97/46507, DE-A-196 21 967 sowie in der DE-A-197 43 197. Auf die in diesen Dokumenten beschriebenen Phosphabenzole und Bisphosphabenzole wird hier Bezug genommen.

Beispielhaft für geeignete Bisphosphabenzole seine folgende Verbindungen genannt:

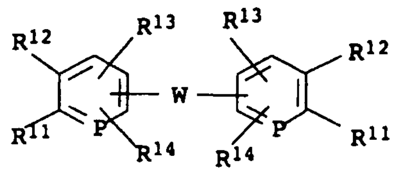

worin

$R^{11}$ bis $R^{14}$ und W die zuvor angegebenen Bedeutungen besitzen.

Als Katalysatoren für die Hydrierung von Phosphabenzolen zu in 1-Position unsubstituierten Phosphacyclohexanen und/oder Phosphacyclohexenen eignen sich im Allgemeinen alle homogenen oder heterogenen Katalysatoren, welche üblicherweise in der Kernhydrierung von Aromaten Anwendung finden. Dazu zählen Katalysatoren auf Basis von Edelmetallen, wie Pt, Pd, Ru und Rh oder Übergangsmetallen, wie Mo, W, Cr, Fe, Co und Ni, die einzeln oder im Gemisch eingesetzt werden können und/oder die zur Erhöhung der Aktivität und/oder Stabilität auf Trägern, wie Aktivkohle, Aluminiumoxid, Kieselgur etc., aufgebracht sein können. Geeignet sind beispielsweise Raney-Nickel, an Aktivkohle gebundenes Pd, metallisches Pt, Platin- und Zinkoxid etc.

Nach einer bevorzugten Ausführungsform werden in Schritt a) Hydrierkatalysatoren auf Basis von Ruthenium eingesetzt, die eine vorteilhafte Hydrierung der eingesetzten Phosphabenzole zu in 1-Position (d. h. am P-Atom) unsubstituierten Phosphacyclohexanen und gegebenenfalls Phosphacyclohexenen ermöglichen.

Nach einer weiteren geeigneten Ausführungsform werden im Reaktionsschritt a) Hydrierkatalysatoren eingesetzt, die auch eine sich gegebenenfalls anschließende Folgeumsetzung in den Reaktionsschritten b1) oder b2) katalysieren oder zumindest nicht negativ beeinflussen. In diesem Fall kann gegebenenfalls auf eine Abtrennung des Hydrierkatalysators nach der Hydrierung verzichtet werden. Besonders bevorzugt wird ein Hydrierkatalysator auf Basis eines Übergangsmetalls eingesetzt, das befähigt ist, mit den resultierenden Phosphacyclohexanen, gegebenenfalls nach einer Folgeumsetzung in den Schritten b1) oder b2), Übergangsmetallkomplexe oder -verbindungen zu bilden, die als Hydroformylierungskatalysatoren aktiv sind. Dazu zählen vorzugsweise Hydrierkatalysatoren auf Basis von Rhodium. Bevorzugt sind Rhodiumcarboxylate, wie Rhodiumacetat, Rhodiumethylhexanoat sowie Rhodiumdicarbonylacetylacetonat. Vorteilhafterweise kann dann die Herstellung des Hydroformylierungskatalysators in situ erfolgen, d. h. das nach Hydrierung und gegebenenfalls einer Folgeumsetzung erhaltene Reaktionsgemisch kann direkt zur Hydroformylierung eingesetzt werden.

Die Temperatur bei der Hydrierung liegt vorzugsweise in einem Bereich von 20 bis 250 °C, besonders bevorzugt 50 bis 180 °C und insbesondere 80 bis 160 °C. Sie ist in der Regel abhängig vom eingesetzten Übergangsmetall. Der Reaktionsdruck liegt vorzugsweise zwischen Umgebungsdruck und 600 bar, besonders bevorzugt 5 bis 100 bar und insbesondere 10 bis 80 bar. Durch geeignete Wahl der Reaktionstemperaturen und/oder Drücke kann der Fachmann den Anteil an Phosphacyclohexenen und Phosphacyclohexanen im Produktgemisch steuern.

Die Hydrierung in Reaktionsschritt a) kann in Gegenwart von reinem Wasserstoff oder in Gegenwart von Wasserstoff-haltigen Gasgemischen, wie beispielsweise Synthesegas, erfolgen.

Bei der Hydrierung von Phosphabenzolen im Reaktionsschritt a) resultieren Phosphacyclohexane und/oder Phosphacyclohexene, die ausgewählt sind unter den Verbindungen der allgemeinen Formeln V.a bis V.c

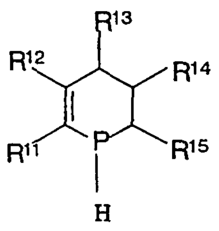

(V.a)

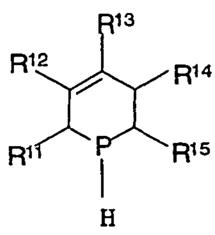

(V.b)

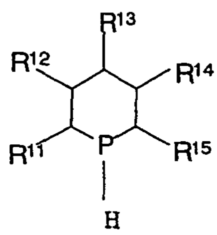

(V.c)

worin $R^{11}$, $R^{12}$, $R^{13}$, $R^{14}$ und $R^{15}$ die zuvor angegebenen Bedeutungen besitzen, und den Gemischen davon. In der Regel resultieren überwiegend Phosphacyclohexane oder Gemische mit Phosphacyclohexenen, die überwiegend, d. h. zu mindestens 80 Gew.-%, bevorzugt zu mindestens 90 Gew.-% Phosphacyclohexane enthalten. Derartige Gemische können in der Regel ohne vorherige Aufarbeitung für Folgeumsetzungen eingesetzt werden.

Gewünschtenfalls kann man das im Hydrierschritt a) erhaltene Reaktionsgemisch vor einer Folgeumsetzung einer Aufarbeitung unterziehen. Dazu zählt beispielsweise die Abtrennung von nicht umgesetztem Phosphabenzol, z. B. durch fraktionierte Destillation oder durch Kristallisation der Phosphacyclohexene und/oder Phosphacyclohexane.

Die Verbindungen V.c eignen sich auch als Liganden für Übergangsmetallkatalysatoren zum Einsatz in der Hydroformylierung. Dabei ist die katalytische Aktivität der resultierenden Hydroformylierungskatalysatoren in der Regel jedoch geringer, als die der in 1-Position substituierten Folgeprodukte. Die Verbindungen V.c sind des Weiteren wertvolle Zwischenprodukte bei der Herstellung von erfindungsgemäßen und erfindungsgemäß eingesetzten Liganden. Dabei können im Allgemeinen sowohl einzelne in 1-Position unsubstituierte Verbindungen als auch Produktgemische eingesetzt werden.

Schritt b1)

Eine erste Ausführungsform des Verfahrens zur Herstellung von in 1-Position substituierten Phosphacyclohexenen und/oder Phosphacyclohexanen umfasst die Reaktionsschritte a) Hydrierung wenigstens eines Phosphabenzols und b1) Umsetzung des/der Hydrierungsprodukt(e) mit wenigstens einer ethylenisch ungesättigten Verbindung. Dabei erfolgt der Reaktionsschritt b1) nach einer der drei folgenden Reaktionsvarianten:

I) in Gegenwart wenigstens eines Radikalbildners,

II) in Gegenwart wenigstens einer Säure oder wenigstens einer Base oder wenigstens einer Übergangsmetallverbindung,

III)bei einer erhöhten Temperatur im Bereich von 100 bis 250 °C.

Für die Umsetzung im Reaktionsschritt b1) eignen sich prinzipiell alle Verbindungen, welche eine oder mehrere ethylenisch ungesättigte Doppelbindungen enthalten. Dazu zählen z. B. Olefine, wie α-Olefine, interne geradkettige und interne verzweigte Olefine, funktionalisierte Olefine, oligomere und polymere Verbindungen, die wenigstens eine ethylenisch ungesättigte Doppelbindung aufweisen etc.

Der Reaktionsschritt b1) kann gewünschtenfalls in Gegenwart eines Lösungsmittels erfolgen. Dazu zählen Aromaten, wie Benzol, Toluol und Xylole, Cycloalkane, wie Cyclohexan, aliphatische Kohlenwasserstoffe etc. Nach einer geeigneten Ausführungsform erfolgt die Reaktion in dem zur Umsetzung eingesetzten Olefin als Lösungsmittel, wobei dieses im Allgemeinen in einem großen molaren Überschuss gegenüber dem Phosphacyclohexen bzw. Phosphacyclohexan eingesetzt wird.

Bevorzugt ist ein Verfahren zur Herstellung von Phosphacyclohexanen der allgemeinen Formel III durch

a) Hydrierung von Phosphabenzolen der allgemeinen Formel IV,

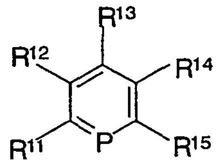

(IV)

und

b1) Umsetzung mit Olefinen der allgemeinen Formel $R^{16}R^{17}C = CR^{18}R^{19}$.

Nach dieser Ausführungsform lassen sich 1-Alkylphosphacyclohexane auf einfachem Wege gemäß dem folgenden Reaktionsschema durch a) Hydrierung von Phosphabenzolen und anschließender Anlagerung b1) eines Olefins an die gebildete PH-Funktion erhalten. Auch Phosphacyclohexene werden so gegebenenfalls teilweise gebildet. Auch sie sind katalytisch aktiv.

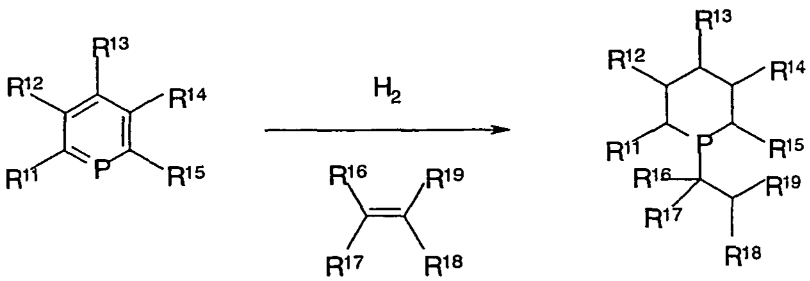


(IV) (III)

mit der Bedeutung:

$R^{11}$ bis $R^{19}$

unabhängig voneinander Wasserstoff, $C_{1-20}$-Alkyl, $C_{7-20}$-Aralkyl, $C_{7-20}$-Alkaryl, $C_{6-12}$-Aryl, wobei eines oder mehrere Kohlenstoffatome durch Heteroatome ersetzt sein können, $W'COO^-M^+$, $W'SO_3^-M^+$, $W'PO_3^{2-}M^+_2$, $W'NR'_3{}^+X^-$, W'OR', $WNR'_2$, W'COOR', W'SR', $W'(CHR'CH_2O)_xR'$, $W'(CH_2NR')_xR'$, $W'(CH_2CH_2NR')_xR'$,

wobei jeweils zwei vicinale Reste $R^{11}$ bis $R^{15}$ und/oder $R^{17}$ und $R^{18}$ und/oder $R^{16}$ und $R^{17}$ und/oder $R^{16}$ und $R^{19}$ und/oder $R^{18}$ und $R^{18}$ zu anellierten aliphatischen oder aromatischen Ringen verbunden sein können,

W'
Einfachbindungen oder eine Brücke mit 1 bis 20 Kohlenstoffatomen, die Bestandteil einer cyclischen oder aromatischen Gruppe sein kann,

R' Wasserstoff oder $C_{1-6}$-Alkyl,

M+ Kation,

$X^-$ Anion,

x Zahl von 1 bis 240,

wobei einer oder mehrere der Reste $R^{11}$ bis $R^{19}$ eine zusätzliche, zur Koordination befähigte dreibindige Phosphor- oder Stickstoffgruppierung aufweisen können, wobei $R^{18}$ auch -W'-$CR^{20}$=$CR^{21}R^{22}$, wobei $R^{20}$, $R^{21}$, $R^{22}$ wie vorstehend für $R^{11}$ bis $R^{19}$ definiert sind, bedeuten kann. In diesem Fall können eine oder beide Doppelbindungen angelagert werden.

Besonders bevorzugte Phosphabenzolverbindungen zur Herstellung der Verbindungen der Formel III sind die der Formel IV.a, wie zuvor beschrieben.

Als Olefine $R^{16}R^{17}C=CR^{18}R^{19}$ kommen beliebige Olefine, wie vorstehend angegeben, beispielsweise $\alpha$-Olefine, interne und interne verzweigte Olefine mit beispielsweise 2 bis 24 C-Atomen in Betracht. Bevorzugt sind $\alpha$-Olefine, wobei $R^{16}$ und $R^{17}$ bzw. $R^{18}$ und $R^{19}$ für Wasserstoff stehen. Besonders geeignet sind Ethen, Propen, Isobuten, 1-Hexen, 1-Octen, 1-Decen, 1-Dodecen und Cyclohexen. Geeignet sind weiterhin Polyisobutene, die noch wenigstens eine ethylenisch ungesättigte Doppelbindung aufweisen. Auch funktionalisierte Olefine können eingesetzt werden. Dazu zählen Polyalkylenglykole mit mindestens einer endständigen Vinylgruppe, wie vinylpolyethylenglykolmonomethylether und Vinylpolytetrahydrofuran. Geeignet sind weiterhin Divinylverbindungen, wobei dann über die P-Atome verbrückte Phosphacyclohexane resultieren.

Im Falle der in situ-Herstellung der Verbindungen der Formel III ist die Verwendung des Olefins zweckmäßig, welches auch im erfindungsgemäß angewendeten Hydroformylierungsverfahren Verwendung findet. Besonders bevorzugt sind $\alpha$-Olefine oder interne, lineare Olefine oder Gemische, die diese Olefine enthalten.

Als Phosphabenzole eignen sich neben den vorstehend beschriebenen Verbindungen insbesondere 2,4,6-Triarylphosphabenzole, 2,3,5,6-Tetraarylphosphabenzole und 2,3,4,5,6-Pentaarylphosphabenzole. Besonders bevorzugt sind 2,4,6-Triarylphosphabenzole. Gegebenenfalls in Spuren enthaltene in 1-Position unsubstituierte Phosphacyclohexane und -phosphacyclohexene sind, wie erwähnt, im Allgemeinen von geringerer katalytischer Aktivität. Durch Behandlung des Rhodium/Phosphacyclohexan-Rohkatalysatorgemischs mit einem Olefin oder Olefingemisch in Gegenwart von Synthesegas lassen sich derartige Verbindungen in ein noch aktiveres Katalysatorsystem überführen. Dadurch resultieren aktivere Katalysatorgemische, wie ein Vergleich verschiedener Hydroformylierungsversuche zeigt.

Die Hydrierung von Phosphabenzolen zu Phosphacyclohexanen erfolgt bei den zuvor angegebenen Temperaturen und Drücken. Sie kann in Gegenwart von reinem Wasserstoff oder Wasserstoff-haltigen Gasgemischen, wie Synthesegas, erfolgen.

Das Phosphabenzol/Katalysatormetall-Verhältnis liegt im Batchverfahren im Bereich von 1 bis 1000, vorzugsweise im Bereich von 2 bis 100 und insbesondere im Bereich von 2 bis 20. Das Olefin/Phosphabenzol-Verhältnis liegt im Bereich von 1 bis 500, vorzugsweise im Bereich von 5 bis 200 und insbesondere im Bereich von 10 bis 80. Im kontinuierlichen Verfahren liegt das Verhältnis im Bereich von 1 bis 5000, vorzugsweise 10 bis 2000, insbesondere 20 bis 1500.

Bei der Reaktionsdurchführung ist es möglich, das Olefin portionsweise zu zudosieren. Bevorzugt ist eine kontinuierliche Fahrweise.

Insbesondere bei Verwendung von Rhodium als Katalysatormetall in homogener Phase kann der Reaktionsaustrag direkt und ohne Isolierung des Cokatalysators in der Hydroformylierung eingesetzt werden. Besonders vorteilhaft ist es, den Rhodium/Phosphacyclohexanhaltigen Hydroformylierungskatalysator in situ unter Hydrier-Bedingungen aus dem entsprechenden Phosphabenzol oder aus einer anderen Phosphacyclohexan-Vorstufe und einem Rhodiumsalz oder einem Rhodiumkomplex in Gegenwart des entsprechenden Olefins herzustellen, wobei man diese Umwandlung vorzugsweise im gleichen Reaktor, der später auch für die Durchführung der Hydroformylierung verwendet wird, durchführt. Besonders bevorzugt wird zuerst die Hydrierung und nachfolgend die Hydroformylierung durchgeführt.

Die Umwandlung des Phosphabenzols IV zum Phosphacyclohexan III nach der zuvor beschriebenen bevorzugten Ausführungsform kann stufenweise oder einstufig erfolgen (siehe Reaktionsvariante III). Beispielsweise kann zunächst die Hydrierung des Phosphabenzols IV zum entsprechenden 1-unsubstituierten Phosphacyclohexan erfolgen, der sich die Addition, gegebenenfalls durch Radikalstarter initiiert, eines Olefins an die PH-Funktion der Zwischenstufe

unter Bildung des Cokatalysators III anschließt.

Variante I)

Nach einer bevorzugten Ausführungsform umfasst das Verfahren zwei separate Reaktionsschritte a) und b1), wobei der Reaktionsschritt b1) in Gegenwart wenigstens eines Radikalbildners erfolgt.

Geeignete Radikalbildner sind die üblichen, dem Fachmann bekannten Polymerisationsinitiatoren, wie sie für die radikalische Polymerisation eingesetzt werden. Beispiele für derartige Verbindungen sind organische Peroxide, z. B. Perester von Carbonsäuren, wie tert.-Amylperpivalat, tert.-Butylperpivalat, tert.-Butylperneohexanoat, tert.-Butylperisobutyrat, tert.-Butylper-2-ethylhexanoat, tert.-Butylperisononanoat, tert.-Butylpermaleat, tert.-Butylperbenzoat, Percarbonate, wie Di-(2-ethylhexyl)peroxodicarbonat, Dicyclohexylperoxodicarbonat, Di-(4-tert.-butylcyclohexyl)peroxodicarbonat, Keton-Peroxide, wie Acetylacetonperoxid, Methylethylketonperoxid, Hydroperoxide, wie tert.-Butylhydroperoxid und Cumolhydroperoxid und Azo-Initiatoren, wie 2,2'-Azobis(amidinopropan)dihydrochlorid, 2,2'-Azobis(N,N'-dimethylen)isobutyramidindihydrochlorid, 2-(Carbamoylazo)isobutyronitril, 2,2-Azobisisobutyronitril, 2,2-Azobisisovaleronitril, oder 4,4'-Azobis(4-cyanovaleriansäure). Dazu zählen z. B. die Vazo®-Marken der Fa. Du Pont, wie Vazo 52, 67 und 88, wobei die Zahl die Temperatur bezeichnet, bei der der Initiator eine Halbwertszeit von 10 Stunden aufweist.

Vorzugsweise wird der Radikalbildner in einer Menge von 0,001 bis 10 Gew.-%, besonders bevorzugt 0,01 bis 5 Gew.-%, bezogen auf die Gesamtmenge der ethylenisch ungesättigten Verbindungen, eingesetzt.

Wenn der Reaktionsschritt b1) in Gegenwart wenigstens eines Radikalbildners erfolgt, so beträgt die Reaktionstemperatur vorzugsweise 20 bis 200 °C, besonders bevorzugt 50 bis 150 °C. Der Fachmann wählt die geeignete Reaktionstemperatur anhand der Zerfallstemperatur, d, h. der entsprechenden Halbwertszeit des Initiators bei dieser Temperatur.

Variante II)

Nach einer weiteren geeigneten Ausführungsform umfasst das zuvor beschriebene Verfahren zwei separate Reaktionsschritte a) und b1), wobei der Schritt b1) in Gegenwart wenigstens einer Säure oder wenigstens einer Base oder wenigstens einer Übergangsmetallverbindung erfolgt. Geeignete Säuren sind beispielsweise Mineralsäuren, wie Salzsäure und Schwefelsäure. Bevorzugt werden Carbonsäuren, wie z. B. Essigsäure, eingesetzt. Geeignete Basen sind Alkalimetallbasen, wie Natronlauge, Kalilauge, Soda, Natriumhydrogencarbonat, Kaliumcarbonat oder Kaliumhydrogencarbonat und Erdalkalimetallbasen, wie Calciumhydroxid, Calciumoxid, Magnesiumhydroxid oder Magnesiumcarbonat sowie Ammoniak und Amine. Vorzugsweise werden Amine, wie Trimethylamin, Triethylamin, Triisopropylamin, etc. eingesetzt. Geeignete Übergangsmetallverbindungen sind Verbindungen und Komplexe von Edelmetallen oder Übergangsmetallen, wie sie üblicherweise auch in den zuvor genannten Hydrierkatalysatoren eingesetzt werden. Bevorzugt sind Rhodiumverbindungen und -komplexe. Letztere ermöglichen im Allgemeinen vorteilhafterweise eine in situ-Herstellung der erfindungsgemäßen und erfindungsgemäß eingesetzten Liganden und der auf ihnen basierenden Hydroformylierungskatalysatoren.

Variante III

Nach einer weiteren bevorzugten Ausführungsform umfasst das Verfahren zwei separate Reaktionsschritte a) und b1), wobei der Reaktionsschritt b1) bei einer Temperatur in einem Bereich von etwa 100 bis 250 °C erfolgt. Somit ist im Allgemeinen auch eine rein thermische Umsetzung der Phosphacyclohexene und/oder Phosphacyclohexane mit ethylenisch ungesättigten Verbindungen möglich.

Die Reaktion nach der Variante III kann mehrstufig ausgestaltet sein, wobei zunächst die Hydrierung (in Abwesenheit von ethylenisch ungesättigten Verbindungen) und anschließend die Umsetzung des/der Reaktionsprodukt (e) mit wenigstens einer ethylenisch ungesättigten Verbindung erfolgt. Die Reaktion kann auch einstufig ausgestaltet sein, wobei die Hydrierung in Anwesenheit der wenigstens einen ethylenisch ungesättigten Verbindung erfolgt. Vorzugsweise wird bei der einstufigen Reaktion ein Katalysator auf Basis von Rhodium eingesetzt.

Schritt b2)

Eine zweite Ausführungsform des Verfahrens zur Herstellung von in 1-Position substituierten Phosphacyclohexenen und/oder Phosphacyclohexanen umfasst die Reaktionsschritte a) Hydrierung wenigstens eines Phosphabenzols zu in 1-Position unsubstituierten Phosphacyclohexanen und/oder Phosphacyclohexenen und b2) Umsetzung des/der Hydrierungsprodukt(s/e) mit wenigstens einer Verbindung der Formel R-X oder X-W-Y, wobei X und Y für eine nucleophil verdrängbare Gruppe (Abgangsgruppe) stehen und R und W die zuvor angegebenen Bedeutungen besitzen.

Die nucleophil verdrängbaren Gruppen (Abgangsgruppen) X und Y der Verbindungen der Formeln R-X oder

X-W-Y sind die üblichen, dem Fachmann von der nucleophilen Substitution bekannten abgehenden Gruppen. Bevorzugt sind die Anionen starker Säuren, wie die Halogenide, z. B. -Cl, -Br und -I oder Gruppen wie OH, p-$CH_3C_6H_4$-$SO_3$-(Tosylate), p-$BrC_6H_4SO_3$-(Brosylate) und $F_3C$-$SO_3$-(Triflate).

Die Gruppen R und W können im Allgemeinen alle zuvor genannten Bedeutungen aufweisen. Auf geeignete und bevorzugte Ausführungen wird Bezug genommen.

Vorteilhafterweise eignet sich diese Verfahrensvariante zur Herstellung von in 1-Positon substituierten Phosphacyclohexenen und Phosphacyclohexanen, die durch die zuvor beschriebene Addition von ethylenisch ungesättigten Verbindungen nicht oder nur unzureichend zugängig sind. Beim Einsatz von Verbindungen der Formel X-W-Y resultieren zweikernige, jeweils in 1-Position verbrückte Phosphacyclohexene bzw. Phosphacyclohexane, wobei Brücken mit beliebiger Kohlenstoffatomanzahl zugängig sind. Somit gelingt nach diesem Verfahren auch insbesondere die Herstellung von zweikernigen Verbindungen mit Brücken mit ungeradzahligen Brückenatomen.

Nach einer geeigneten Ausführungsform umfasst das Verfahren die Schritte a) und b2), wobei man die in Schritt a) erhaltenen Hydrierungsprodukte vor der Umsetzung mit wenigstens einer Verbindung R-X oder X-W-Y zunächst am Phosphoratom metalliert. Dabei wird unter "Metallierung" der formale Austausch des Wasserstoffatoms gegen ein Metallatom verstanden. Geeignete Reagenzien für die Metallierung sind ganz allgemein starke Basen, z. B. Alkalimetall- und Erdalkalimetallhydride sowie Li-, Mg-, A1-, Sn-und Zn-organische Verbindungen. Bevorzugt sind Aluminium- und Lithium-organische Verbindungen. Dazu zählen beispielsweise n-Butyllithium, sek.-Butyllithium, tert.-Butyllithium, Phenyllithium etc. Geeignete Reagenzien für die Metallierung sind weiterhin Lithium-2,2,6,6-tetramethylpiperidin, Lithium-hexamethyldisilazan, Lithium-dicyclohexylamid und Lithium-diisopropylamid.

Das zuvor beschriebene Verfahren umfassend die Reaktionsschritte a) und b2) eignet sich vorteilhaft zur Herstellung der folgenden Verbindungen:

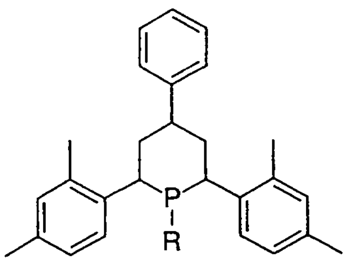

**19**

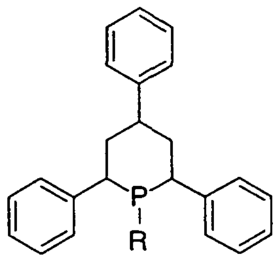

**20**

a: R = $C_8H_{17}$
b: R = $C_{12}H_{25}$

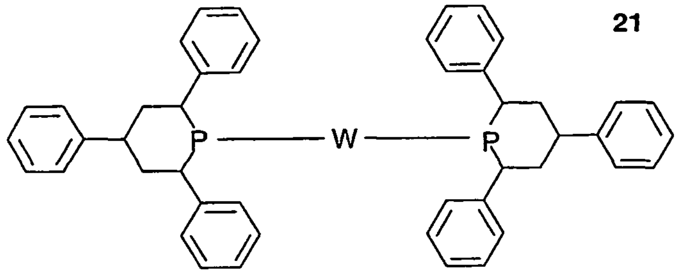

21

c: W = $CH_2CH_2$
d: W = $CH_2CH_2CH_2$
e: W = $CH_2CH_2CH_2CH_2$
f: W = $-(CH_2)_6-$
g: W = Polyalken, $M_n$ = 500-50000
h: W = $W'(CH_2CH_2O)_{x1}(CH(CH_3)CH_2O)_{x2}((CH_2)_4O)_{x3}R'$
$x_1, x_2, x_3$ = 0 bis 200, $\sum x_1+x_2+x_3$ = 1 bis 200, Reihenfolge der Alkylenoxideinheiten beliebig

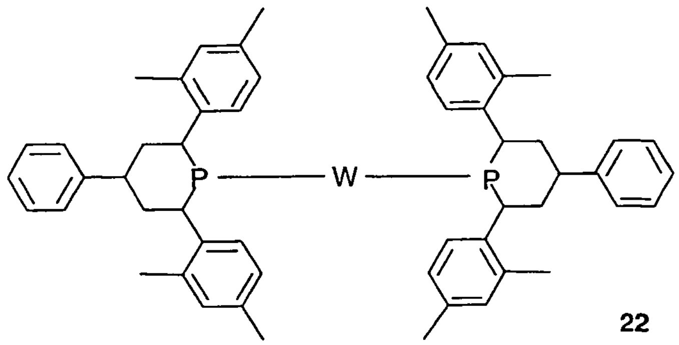

22

Zur Herstellung der erfindungsgemäßen und in dem erfindungsgemäßen Hydroformylierung verfahren eingesetzten Verbindungen eignet sich ein Verfahren zur Herstellung wenigstens eines Phosphacyclohexans der allgemeinen Formeln VI.a und/oder VI.b

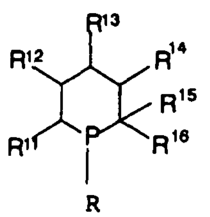

(VI.a)

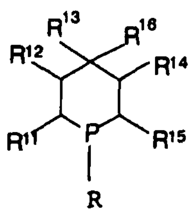

(VI.b)

worin

R
für $C_{1-100}$-Alkyl, Cycloalkyl, Heterocycloalkyl, $C_{7-20}$-Aralkyl, $C_{7-20}$-Alkaryl, $C_{6-12}$-Aryl, Hetaryl, $W'COO^-M^+$, $W'SO_3^-M^+$, $W'PO_3^{2-}M^+_2$. $W'NR'_3{}^+X^-$, W'OR', $W'NR'_2$, W'COOR', W'SR', $W'(CHR'CH_2O)_xR'$, $W'(CH_2NR')_xR'$, $W'(CH_2CH_2NR')_xR'$, $W'((CH_2)_4O)_xR'$ oder W'COR' steht,
wobei zwei Reste R auch gemeinsam für eine verbrückende Gruppe W stehen können, die zwei gleiche oder verschiedene Einheiten VI.a oder VI.b kovalent miteinander verbindet,

$R^{11}$ bis $R^{16}$
unabhängig voneinander für Wasserstoff, $C_{1-20}$-Alkyl, $C_{7-20}$-Aralkyl, $C_{7-20}$-Alkaryl, $C_{6-12}$-Aryl, wobei eines oder mehrere Kohlenstoffatome durch Heteroatome ersetzt sein können, $W'COO^-M^+$, $W'SO_3^-M^+$, $W'PO_3^{2-}M^+_2$, $W'NR'_3{}^+X^-$, W'OR', $W'NR'_2$, W'COOR', W'SR', $W'(CHR'CH_2O)_xR'$, $W'(CH_2NR')_xR'$, $W'(CH_2CH_2NR')_xR'$, $W'((CH_2)_4O)_xR'$, W'Halogen, $W'NO_2$, W'COR' oder W'CN stehen,
wobei in den Resten $R^{11}$ bis $R^{16}$ ein oder mehrere Wasserstoffatome durch Fluor ersetzt sein können,
wobei zwei Reste $R^{16}$ gemeinsam auch für eine verbrückende Gruppe W stehen können, die zwei gleiche oder verschiedene Einheiten VI.a oder VI.b kovalent miteinander verbindet,

W und W'

unabhängig voneinander für Einfachbindungen oder Brücken mit 1 bis 20 Kohlenstoffatomen stehen, die Bestandteil einer cyclischen oder aromatischen Gruppe sein können und durch Heteroatome unterbrochen sein können, wobei W auch für eine Polyoxyalkylen- oder Polyalkylenimin-Brücke mit mindestens 21 Kohlenstoffatomen stehen kann,

R' für Wasserstoff, $C_{1-20}$-Alkyl, Carbonylalkyl, Cycloalkyl oder Aryl steht,

M+ für ein Kationäquivalent steht,

$X^-$ für ein Anionäquivalent steht und,

x für eine ganze Zahl von 1 bis 240 steht,

wobei einer oder mehrere der Reste $R^{11}$ bis $R^{16}$ eine zusätzliche, zur Koordination befähigte dreibindige Phosphor- oder Stickstoffgruppe aufweisen können,
wobei in den Verbindungen der Formel VI.a oder VI.b auch einer der Reste R oder $R^{11}$ bis $R^{16}$ oder, falls vorhanden, eine Gruppe W auch für einen Polymerrest mit einem zahlenmittleren Molekulargewicht im Bereich von 500 bis 50000 stehen kann, aufgebaut aus Wiederholungseinheiten, die sich von Monomeren ableiten, die ausgewählt sind unter Mono- und Diolefinen, Vinylaromaten, Estern α,β-ethylenisch ungesättigter Mono- und Dicarbonsäuren mit $C_1$-$C_{30}$-Alkanolen, N-Vinylamiden, N-Vinyllactamen, unter Ringöffnung polymerisierbaren heterocyclischen Verbindungen und Mischungen davon,
wobei man

A) wenigstens ein Phosphabenzol der allgemeinen Formel IV

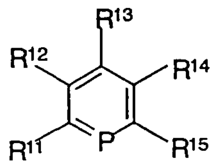

(IV)

worin $R^{11}$, $R^{12}$, $R^{13}$, $R^{14}$ und $R^{15}$ die zuvor angegebenen Bedeutungen besitzen, mit einer metallorganischen Verbindung R-Me oder Me-W-Me', worin R und W die zuvor angegebenen Bedeutungen besitzt und Me und Me' für ein Metallatom oder eine Metallatom-haltige Gruppe stehen, und anschließend mit wenigstens einer Verbindung der Formel $R^{16}$-X oder X-W-Y umsetzt,
wobei X und Y für nucleophil verdrängbare Gruppen stehen und $R^{16}$ und W die zuvor angegebenen Bedeutungen besitzen, und

B) das/die in Schritt A) erhaltene(n) Umsetzungsprodukt(e) mit Wasserstoff in Gegenwart eines Hydrierkatalysators hydriert.

Bezüglich geeigneter und bevorzugter Reste R, $R^{11}$ bis $R^{16}$, W und W' wird auf die obigen Ausführungen Bezug genommen.

Schritt A)

Als Ausgangsmaterialien geeignete und bevorzugte Phosphabenzolverbindungen der allgemeinen Formel IV sind die zuvor genannten.

In den Verbindungen R-Me und Me-W-Me' stehen Me und Me' für ein Metallatom oder eine Metallatom-haltige Gruppe. Wenn Me für ein Metallatom steht, so handelt es sich vorzugsweise um ein einwertiges Metall, insbesondere um Li. Geeignete Verbindungen R-Me, worin Me für ein einwertiges Metall steht, sind die zuvor als Reagenzien für die Metallierung genannten Lithium-organischen Verbindungen, wie n-Butyllithium, sek.-Butyllithium, tert.-Butyllithium und Phenyllithium.

Geeignete Verbindungen R-Me können auch durch Ummetallierung (Transmetallierung), d. h. Austausch der Metallatome erhalten werden. Werden zur Ummetallierung beispielsweise die metallhaltigen Produkte einer anionischen Polymerisation (wie im Folgenden beschrieben) eingesetzt, so resultieren Verbindungen R-Me, worin R für einen Polymerrest steht. Somit gelingt es, Phosphacyclohexane bereit zu stellen, die in 1-Position einen Polymerrest tragen.

Wenn in den Verbindungen R-Me und Me-W-Me' Me und Me' für eine Metallatom-haltige Gruppe stehen, so handelt es sich um ein zwei- oder mehrwertiges Metallatom, welches zusätzlich noch wenigstens einen weiteren Rest R oder einen von R verschiedenen Rest aufweist. Dabei handelt es sich z. B. um ein Halogenid. Bevorzugte Verbindungen R-Me und Me-W-Me', in denen Me und/oder Me' für eine Metallatom-haltige Gruppe stehen, sind Grignard-Verbindungen (Organometall-Verbindungen des Magnesiums). Grignard-Verbindungen und Verfahren zu ihrer Herstellung sind dem Fachmann bekannt. Bevorzugt sind n-$C_4H_9$-Mg-Cl und $C_8H_{17}$-Mg-Cl. Beim Einsatz von Verbindungen der Formel Me-W-Me' resultieren zweikernige, jeweils in 1-Position verbrückte Phosphacyclohexane.

Geeignete Verbindungen der Formel $R^{16}$-X und X-W-Y sind die zuvor genannten Verbindungen der Formel R-X oder X-W-Y, wobei X und Y für eine nucleophil verdrängbare Gruppe (Abgangsgruppe) steht. Geeignete Abgangsgruppen sind die zuvor genannten. Geeignete Verbindungen der Formel $R^{16}$-X sind Wasser und Alkohole. Beim Einsatz von Verbindungen der Formel X-W-Y resultieren zweikernige 2,2'-, 2,4'- und 4,4'-verknüpfte Phosphacyclohexadiene. Somit gelingt nach diesem Verfahren insbesondere die Herstellung von zweikernigen Verbindungen, die über andere Positionen als die 1-Position verbrückt sind.

Nach einer bevorzugten Ausführungsform setzt man in Schritt a) als metallorganische Verbindung eine Verbindung der allgemeinen Formel R-Me ein, worin R für einen Rest steht, der Wiederholungseinheiten aufweist, die sich von anionisch polymerisierbaren Monomeren ableiten.

Verfahren zur anionischen Oligomerisierung und Polymerisation von Monomeren in Gegenwart einer metallorganischen Verbindung sind prinzipiell bekannt.

H. L. Hsieh und R. P. Quirk beschreiben in Anionic Polyerisation, Principles and Practical Applications, Marcel Dekker-Verlag (1996), Kapitel II, 5, S. 93-116 geeignete Monomere und allgemeine Aspekte der anionischen Polymerisation. In Kapitel III, S. 131-258 wird die Polymerisation von Styrol-Butadien, in Kapitel IV, S. 261-368 die Herstellung Blockcopolymeren, in Kapitel V, 22, S. 621-638 werden Telechele und in Kapitel VI, 23, S. 641-684 werden (Meth) acrylate beschrieben. Kapitel VI, 24, S. 685-710 beschreibt die ringöffnende anionische Polymerisation. Auf die genannten Literaturstellen wird Bezug genommen.

Für die anionische Polymerisation geeignete ethylenisch ungesättigte Verbindungen sind Ethen und vorzugsweise Akzeptor-substituierte ethylenisch ungesättigte Verbindungen. Dazu zählen beispielsweise Vinylaromaten, wie Styrol, Aromaten-substituierte Monoolefine, wie 1,1-Diphenylethylen, 1,2-Diphenylethylen und Mischungen davon. Geeignet sind weiterhin konjugierte Diene, wie Butadien, Isopren, 2,3-Dimethylbutadien, 1,3-Pentadien und Mischungen davon. Geeignete anionisch polymerisierbare Monomere sind weiterhin die zuvor genannten Ester $\alpha,\beta$-ethylenisch ungesättigter Mono- und Dicarbonsäuren mit $C_{1-30}$-Alkanolen. Geeignete anionisch polymerisierbare Monomere sind weiterhin unter Ringöffnung polymerisierbare heterocyclische Verbindungen. Dazu zählen vorzugsweise die zuvor genannten Alkylenoxide, wie Ethylenoxid und 1,2-Propylenoxid, Aziridine, Lactone, wie ε-Caprolacton und Lactame, wie ε-Caprolactam. Beim Einsatz weniger reaktiver Monomere kann die Polymerisation in Gegenwart wenigstens eines Ethers oder Amins erfolgen, insbesondere eines Amins, das keine Aminwasserstoffe aufweist. Bevorzugt sind Amine, die zwei oder mehrere Aminogruppen aufweisen, die keine Aminwasserstoffe tragen. Bevorzugt ist beispielsweise Tetramethylethylendiamin (TMEDA).

Beim Einsatz konjugierter Diene, wie beispielsweise Butadien, kann der Anteil an 1,2- und 1,4-Wiederholungseinheiten im resultierenden Polymer nach üblichen, dem Fachmann bekannten Verfahren, z. B. durch Zusatz von Donorlösungsmitteln, wie Ethern, z. B. THF oder Dimethyoxyethan, oder Aminen eingestellt werden. Somit lassen sich Polymere mit variablem Kristallinitätsgrad von hochkristallin bis nicht kristallin (in der Regel > 40 Gew.-% 1,2-Vinylanteil) herstellen. Die anionische Polymerisation eignet sich vorteilhaft zur Herstellung von Ethen/Propen-Copolymeren durch Hydrierung der entsprechenden 1,4-Polyisoprene. Sie eignet sich weiterhin in vorteilhafter Weise zur Herstellung von ansonsten schwer zugängigen Blockcopolymeren, z. B. Styrol-Butadien-, Styrol-Isopren-, Butadien-(Meth)acrylat- oder Styrol-(Meth)acrylat-Blockcopolymeren. Sie eignet sich auch vorteilhaft zur Herstellung von statistischen Copolymeren, wie Styrol-Butadien-Copolymeren, wobei die Polymerisation dann gegebenenfalls in Gegenwart von sogenannten Randomizern, wie den zuvor genannten Donorlösungsmitteln oder Alkali- oder Erdalkalimetallsalzen, wie KOR oder Ba $(OR)_2$ erfolgt.

Beim Einsatz von Dienen, wie Butadien, zur anionischen Polymerisation resultieren Phosphacyclohexadiene, die in 1-Position einen Polyalkylenrest tragen, welcher noch ungesättigte Seitenketten (1,2-Produkt) oder Doppelbindungen in der Hauptkette (1,4-Produkt) aufweist. Diese werden im nachfolgenden Hydrierschritt b) vollständig oder nahezu vollständig in die entsprechenden Alkylreste überführt.

Das in Schritt A) erhaltene Umsetzungsprodukt enthält wenigstens ein Phosphacyclohexadien der allgemeinen Formel VII.a oder VII.b

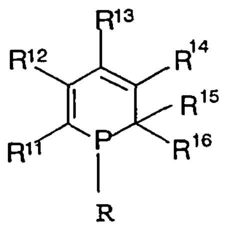

(VII.a)

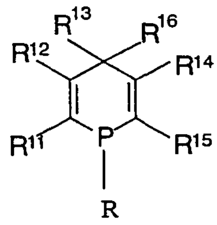

(VII.b)

oder ein Isomerengemisch davon, worin R und $R^{11}$ bis $R^{16}$ die zuvor angegebenen Bedeutungen besitzen.

Schritt B)

Geeignete Katalysatoren für die Hydrierung von Phosphacyclohexadienen zu Phosphacyclohexanen sind die zuvor für die Hydrierung von Phosphabenzolen genannten homogenen oder heterogenen Katalysatoren. Vorzugsweise werden Katalysatoren auf Basis von Ruthenium oder Rhodium und insbesondere Rhodium eingesetzt.

Die Temperatur bei der Hydrierung liegt vorzugsweise in einem Bereich von 20 bis 250 °C, besonders bevorzugt 50 bis 180 °C und insbesondere 80 bis 160 °C. Nach einer bevorzugten Ausführungsform führt man die Hydrierung zunächst bei einer Temperatur im Bereich von etwa 60 bis 120 °C durch, bis im Wesentlichen keine Cyclohexadiene mehr im Reaktionsgemisch enthalten sind, da diese zur Aromatisierung neigen. Dabei resultieren Hydrierprodukte, die überwiegend oder ausschließlich Phosphacyclohexene enthalten. Bevorzugt wird zur Herstellung von solchen Hydrierprodukten ein Katalysator auf Basis von Rhodium eingesetzt. Phosphacyclohexene sind wie die Phosphacyclohexane, gegebenenfalls nach einer Folgeumsetzung, als Liganden für Übergangsmetallkatalysatoren zur Hydroformylierung geeignet. Wird eine im Wesentlichen vollständige Hydrierung gewünscht, so erhöht man im Folgenden die Temperatur auf bis zu 250 °C, um die Hydrierung zu vervollständigen. Der Reaktionsdruck liegt vorzugsweise zwischen Umgebungsdruck und 600 bar, besonders bevorzugt 5 bis 100 bar und insbesondere 10 bis 80 bar.

Gewünschtenfalls kann zur Hydrierung wenigstens eine Olefin zugesetzt werden, um gegebenenfalls im Reaktionsgemisch noch enthaltenes, nicht umgesetztes Phosphabenzol, wie zuvor beschrieben, in katalytisch aktive Spezies zu überführen.

Zur Herstellung der erfindungsgemäßen und zur Hydroformylierung eingesetzten Verbindungen eignet sich weiter ein Verfahren zur Herstellung wenigstens eines Phosphacyclohexans der allgemeinen Formel V

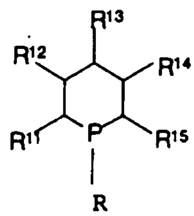

(V)

worin

R
für Wasserstoff, $C_{1-100}$-Alkyl, Cycloalkyl, Heterocycloalkyl, $C_{7-20}$-Aralkyl, $C_{7-20}$-Alkaryl, $C_{6-12}$-Aryl, Hetaryl, $W'COO^-M^+$, $W'SO_3^-M^+$, $W'PO_3^{2-}M^+_2$, $W'NR'_3{}^+X^-$, W'OR', $W'NR'_2$, W'COOR', W'SR', $W'(CHR'CH_2O)_xR'$, $W'(CH_2NR')_xR'$, $W'(CH_2CH_2NR)_xR'$, $W'((CH_2)_4O)_xR'$, oder W'COR' steht,
wobei in zwei gleichen oder verschiedenen Einheiten V die Reste R oder je einer der Reste $R^{11}$ bis $R^{15}$ gemeinsam für eine verbrükkende Gruppe W stehen können, die diese Einheiten kovalent miteinander verbindet,

$R^{11}$ bis $R^{15}$
unabhängig voneinander für Wasserstoff, $C_{1-20}$-Alkyl, $C_{7-20}$-Aralkyl, $C_{7-20}$-Alkaryl, $C_{6-12}$-Aryl, wobei eines oder mehrere Kohlenstoffatome durch Heteroatome ersetzt sein können, $W'COO^-M^+$, $W'SO_3^-M^+$, $W'PO_3^{2-}M^+_2$, $W'NR'_3^+X^-$. W'OR', $W'NR'_2$, W'COOR', W'SR', $W'(CHR'CH_2O)_xR'$, $W'(CH_2NR')_xR'$, $W'(CH_2CH_2NR')_xR'$, $W'((CH_2)_4O)_xR'$, W'Halogen, $W'NO_2$, W'COR' oder W'CN stehen,
wobei in den Resten R und $R^{11}$ bis $R^{15}$ ein oder mehrere Wasserstoffatome durch Fluor ersetzt sein können,

W und W'
unabhängig voneinander für Einfachbindungen oder Brücken mit 1 bis 20 Kohlenstoffatomen stehen, die Bestandteil einer cyclischen oder aromatischen Gruppe sein können und durch Heteroatome unterbrochen sein können, wobei W auch für eine Polyoxyalkylen- oder Polyalkylenimin-Brücke mit mindestens 21 Kohlenstoffatomen stehen kann,

R' für Wasserstoff, $C_{1-20}$-Alkyl, Carbonylalkyl, Cycloalkyl oder Aryl steht,

M+ für ein Kationäquivalent steht,

$X^-$ für ein Anionäquivalent steht und,

x für eine ganze Zahl von 1 bis 240 steht,

wobei einer oder mehrere der Reste R und $R^{11}$ bis $R^{15}$ eine zusätzliche, zur Koordination befähigte dreibindige Phosphor- oder Stickstoffgruppe aufweisen können,
wobei in den Verbindungen der Formeln V auch einer der Reste R oder $R^{11}$ bis $R^{15}$ oder, falls vorhanden, eine Gruppe W auch für einen Polymerrest mit einem zahlenmittleren Molekulargewicht im Bereich von 500 bis 50000 stehen kann, aufgebaut aus Wiederholungseinheiten, die sich von Monomeren ableiten, die ausgewählt sind unter Mono- und Diolefinen, Vinylaromaten, Estern $\alpha,\beta$-ethylenisch ungesättigter Mono- und Dicarbonsäuren mit $C_{1-30}$-Alkanolen, N-Vinylamiden, N-Vinyllactamen, unter Ringöffnung polymerisierbaren heterocyclischen Verbindungen und Mischungen davon,
wobei man

$\alpha$) wenigstens ein Pyryliumsalz der allgemeinen Formel IX

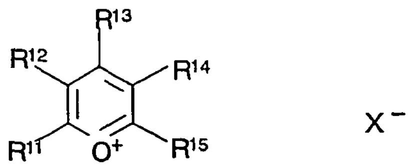

(IX)

worin $R^{11}$, $R^{12}$, $R^{13}$, $R^{14}$ und $R^{15}$ die zuvor angegebenen Bedeutungen besitzen und X- für ein Anionenäquivalent steht,
mit einer Verbindung der Formel $R\text{-}PH_2$ oder $H_2P\text{-}W\text{-}PH_2$ umsetzt, worin R und W die zuvor angegebenen Bedeutungen besitzen,

$\beta$) das/die in Schritt $\alpha$) erhaltene(n) Umsetzungsprodukt(e) mit Wasserstoff in Gegenwart eines Hydrierkatalysators hydriert, und

$\gamma$) das/die Hydrierungsprodukt(e) aus Schritt $\beta$) mit wenigstens einem Reduktionsmittel umsetzt,

wobei die Reihenfolge der Schritte $\beta$) und $\gamma$) beliebig ist.

Verfahren zur Herstellung von geeigneten Pyryliumsalzen sind beispielsweise in Houben-Weyl, Hetarene II, Teil 2, Herausgeber R. Kreher, Band E7b, S. 755ff, Thieme-Verlag, Stuttgart, beschrieben. Geeignete Pyryliumsalze und Verfahren zur ihrer Herstellung sind weiterhin in der DE-A-197 43 197 beschrieben, worauf hier in vollem Umfang Bezug genommen wird.
Geeignete Anionenäquivalente der Pyryliumsalze sind ausgewählt unter Ferraten, Zinkaten, Chloriden, Boraten, die gegebenenfalls mit einem $C_{1-6}$-Alkylrest substituiert sind, Triflaten, Trifluoracetaten, Tetrafluorboraten, Perchloraten, Hydrogensulfaten, Bromiden, Iodiden oder Gemischen davon. Vorzugsweise werden Tetrafluorborate eingesetzt. Die Reste $R^{11}$ bis $R^{15}$ der Pyryliumsalze entsprechen den zuvor angegebenen geeigneten und bevorzugten Resten $R^{11}$ bis $R^{15}$.

Bezüglich geeigneter und bevorzugter Reste R und W der einwertigen Phosphane $R-PH_2$ und zweiwertigen Phosphane $H_2P-W-PH_2$ wird auf die vorherigen Ausführungen Bezug genommen. Beim Einsatz von Verbindungen der Formel $H_2P-W-PH_2$ resultieren zweikernige, jeweils in 1-Position verbrückte Phosphacyclohexane.

Vorzugsweise steht der Rest R für $C_{1-20}$-Alkyl, wie beispielsweise Methyl, Ethyl, n-Propyl, n-Butyl, Pentyl, Hexyl, Heptyl, Octyl etc. oder $C_{5-8}$-Cycloalkyl, wie Cyclohexyl. Vorzugsweise steht R weiterhin für einen Polymerrest, wie zuvor beschrieben, besonders bevorzugt einen Polyalkenrest, wie einen Polyethylenrest oder Polyisobutenrest.

Die Reaktionstemperatur in Schritt $\alpha$) beträgt vorzugsweise etwa 50 bis 150°C, besonders bevorzugt 60 bis 140 °C.

Die Umsetzung kann in Gegenwart eines Lösungs- oder Verdünnungsmittels erfolgen. Geeignete Lösungs- oder Verdünnungsmittel sind beispielsweise $C_{1-6}$-Alkohole, wie Methanol, Ethanol, n-Propanol, Isopropanol, n-Butanol, Isobutanol, tert.-Butanol etc.

Schritt $\beta$)

Als Katalysatoren für die Hydrierung der in Schritt $\alpha$) erhaltenen Reaktionsprodukte eignen sich im Allgemeinen alle homogenen oder heterogenen Hydrierkatalysatoren, wie sie zuvor für die Hydrierung von Phosphabenzolen zu Phosphacyclohexenen und/oder Phosphacyclohexanen beschrieben wurden. Bevorzugt ist der Einsatz von Ruthenium-haltigen Katalysatoren, welche in Form von Salzen oder Komplexen eingesetzt werden können. Dazu zählt beispielsweise Ruthenium(III)acetylacetonat. Bevorzugt sind weiterhin heterogene Katalysatoren, wie an Aktivkohle gebundenes Pd.

Die Temperatur bei der Hydrierung liegt vorzugsweise in einem Bereich von 20 bis 250°C, besonders bevorzugt 50 bis 180 °C und insbesondere 60 bis 160°C. Der Reaktionsdruck liegt vorzugsweise zwischen Umgebungsdruck und 600 bar, besonders bevorzugt 5 bis 100 bar und insbesondere 10 bis 80 bar.

Die Hydrierung in Reaktionsschritt $\beta$) kann in Gegenwart von reinem Wasserstoff oder in Gegenwart von Wasserstoff-haltigen Gasgemischen, wie beispielsweise Synthesegas, erfolgen.

Schritt $\gamma$)

Geeignete Reduktionsmittel sind die üblichen, dem Fachmann zur Reduktion von Phosphinoxiden zu Phosphinen bekannten. Dazu zählen vorzugsweise Chlorsilane, wie Trichlorsilan und komplexe Hydride, wie $LiAlH_4$, $BH_3$/DMS (Dimethylsulfid), $LiAlH_4$/Cer(III).

Die Reihenfolge der Reaktionsschritte $\beta$) und $\gamma$), d. h. der Hydrierung und der Reduktion, ist beliebig.

Eine Isolierung des Liganden kann beispielsweise durch unmittelbare fraktionierte Destillation oder Kristallisation erfolgen.

Ein weiterer Gegenstand der Erfindung ist ein Katalysator, umfassend wenigstens einen Komplex eines Metalls der VIII. Nebengruppe mit wenigstens einem Phosphacyclohexan- Liganden, wie in Anspruch 13 definiert. Die Katalysatoren können zusätzlich wenigstens einen weiteren Liganden ausgewählt unter Halogeniden, Aminen, Carboxylaten, Acetylacetonat, Aryl- oder Alkylsulfonaten, Hydrid, CO, Olefinen, Dienen, Cycloolefinen, Nitrilen, N-haltigen Heterocyclen, Aromaten und Heteroaromaten, Ethern, $PF_3$, Phospholen, Phosphabenzolen sowie ein-, zwei- und mehrzähnigen Phosphin-, Phosphinit-, Phosphonit-, Phosphoramidit und Phosphitliganden aufweisen.

Vorzugsweise ist das Metall der VIII. Nebengruppe ausgewählt unter Ruthenium, Iridium, Rhodium, Nickel oder Palladium.

Die in der Hydroformylierung wirksamen Katalysatoren sind im Allgemeinen Übergangsmetallkomplexe der allgemeinen Formel $ML_n(CO)_m$, in denen M ein Element der VIII. Nebengruppe des Periodensystems darstellt, L mindestens ein zur Komplexbildung befähigter ein- oder mehrzähniger Phosphacyclohexanligand ist und n und m ganze Zahlen zwischen 1 und 3 darstellen. Im Übergangsmetallkomplex können noch weitere Reste wie Hydrido-, Alkyl- oder Acylreste als Liganden enthalten sein. Besonders bevorzugt ist die Verwendung von Rhodium als Übergangsmetall.

Der aktive Carbonylkomplex wird in der Regel in situ aus einem Übergangsmetallsalz, bevorzugt einem Rhodiumsalz, oder einer Übergangsmetallkomplexverbindung, bevorzugt einer Rhodiumkomplexverbindung, dem Liganden, Wasserstoff und Kohlenmonoxid gebildet; er kann aber auch getrennt hergestellt und eingesetzt werden.

Werden die Komplexkatalysatoren in situ erzeugt, setzt man besonders bevorzugt Precursorkomplexe wie Rhodiumdicarbonylacetonat, Rhodium(2-ethylhexanoat) oder Rhodiumacetat in Gegenwart der entsprechenden Phosphacyclohexanliganden ein. Alternativ können die Precursorkomplexe auch in Gegenwart geeigneter Phosphacyclohexanvorstufen eingesetzt werden, wobei unter den Reaktionsbedingungen auch der eigentliche Cokatalysator gebildet wird.

Die Zusammensetzung des im erfindungsmäßigen Hydroformylierungsverfahren eingesetzten Synthesegases $CO/H_2$ kann in weiten Bereichen variiert werden. Beispielsweise kann Synthesegas mit $CO/H_2$-Molverhältnissen von 5: 95 bis 90:10 erfolgreich eingesetzt werden, bevorzugt wird Synthesegas mit $CO/H_2$-Verhältnissen von 40:60 bis 70:30, besonders bevorzugt wird ein $CO/H_2$-Verhältnis von etwa 1:1 angewandt.

Die Hydroformylierung erfolgt in an sich bekannter Weise bei Temperaturen von 50 bis 180 °C und bei Drücken von 5 bis 100 bar. Die optimale Temperatur und der optimale Druck sind jedoch im Wesentlichen abhängig vom eingesetzten olefin.

Aufgrund ihrer höheren Reaktivität werden α-Olefine besonders bevorzugt bei Temperaturen von 80 bis 120 °C und Drücken von 10 bis 40 bar hydroformyliert. 1-Alkene werden vorzugsweise bei Temperaturen von 80 bis 120°C hydroformyliert. Der Druck liegt vorzugsweise in einem Bereich von 10 bis 40 bar. Olefine mit Vinyliden-Doppelbindung werden vorzugsweise bei 100 bis 150°C hydroformyliert. Auch hier beträgt der Druck bevorzugt 10 bis 40 bar. Eine Durchführung der Reaktion bei höheren Temperaturen und höheren Drücken als die zuvor angegebenen ist nicht ausgeschlossen.

Aufgrund ihrer geringeren Reaktivität werden interne und interne, an der Doppelbindung verzweigte Olefine besonders bevorzugt bei Temperaturen von 120 bis 180°C und Drücken von 40 bis 100 bar hydroformyliert.

Die Hydroformylierung erfolgt in der Regel in Gegenwart eines 1-bis 1000-fachen molaren Überschusses, vorzugsweise eines 2- bis 100-fachen Überschusses des Liganden, bezogen auf die eingesetzte Menge an Übergangsmetall.

Als Substrate für das erfindungsgemäße Hydroformylierungsverfahren kommen prinzipiell alle Verbindungen in betracht, welche eine oder mehrere ethylenisch ungesättigte Doppelbindungen enthalten. Dazu zählen Olefine, wie α-Olefine, interne geradkettige Olefine oder interne verzweigte olefine mit beliebiger Anzahl an C-Atomen, insbesondere jedoch solche mit 2 bis 14 C-Atomen und solche mit internen und internen verzweigten Doppelbindungen. Beispielhaft werden folgende Olefine genannt: Ethen, Propen, 1-Buten, 1-Hexen, 1-Octen, α-$C_{5-20}$-Olefine, 2-Buten, lineare interne $C_{5-20}$-Olefine und Isobuten.

Geeignete verzweigte, interne Olefine sind vorzugsweise $C_{4-20}$-Olefine, wie 2-Methyl-2-Buten, 2-Methyl-2-Penten, 3-Methyl-2-Penten, verzweigte, interne Hepten-Gemische, verzweigte, interne Octen-Gemische, verzweigte, interne Nonen-Gemische, verzweigte, interne Decen-Gemische, verzweigte, interne Undecen-Gemische, verzweigte, interne Dodecen-Gemische etc.

Geeignete zu hydroformylierende Olefine sind weiterhin $C_{5-8}$-Cycloalkene, wie Cyclopenten, Cyclohexen, Cyclohepten, Cycloocten und deren Derivate, wie z. B. deren $C_{1-20}$-Alkylderivate mit 1 bis 5 Alkylsubstituenten. Geeignete zu hydroformylierende Olefine sind weiterhin Vinylaromaten, wie Styrol, α-Methylstyrol, 4-Isobutylstyrol etc.

Geeignte zu hydroformylierende Olefine sind weiterhin ethylenisch ungesättigtes Polypropen und Polyisobuten.

Auch funktionelle Gruppen werden toleriert. Beispielhaft werden folgende Olefine genannt: 3-Pentennitril, 4-Pentennitril, 3-Pentensäureester, 4-Pentensäureester, Acrylsäureester, Vinylglycoldiacetat und Butendioldiacetat. Ebenfalls geeignete Substrate sind Di- und Polyene mit isolierten oder konjugierten Doppelbindungen. Beispielhaft werden folgende olefine genannt: 1,3-Butadien, 1,5-Hexadien, Vinylcyclohexen, Dicyclopentadien, 1,5,9-Cyclooctatrien, Butadienhomo- und copolymere.

Bevorzugt ist die zur Hydroformylierung eingesetzte ungesättigte Verbindung ausgewählt unter internen linearen Olefinen und Olefingemischen, die wenigstens ein internes lineares Olefin enthalten. Geeignete lineare (geradkettige) interne Olefine sind vorzugsweise $C_{4-20}$-Olefine, wie 2-Buten, 2-Penten, 2-Hexen, 3-Hexen, 2-Hepten, 3-Hepten, 2-Octen, 3-Octen, 4-Octen etc. und Mischungen davon.

Vorzugsweise wird in dem erfindungsgemäßen Hydroformylierungsverfahren ein großtechnisch zugängiges Olefingemisch eingesetzt, das insbesondere wenigstens ein internes lineares Olefin enthält. Dazu zählen z. B. die durch gezielte Ethen-Oligomerisierung in Gegenwart von Alkylaluminiumkatalysatoren erhaltenen ziegler-olefine. Dabei handelt es sich im Wesentlichen um unverzweigte Olefine mit endständiger Doppelbindung und gerader Kohlenstoffatomanzahl. Dazu zählen weiterhin die durch Ethen-oligomerisierung in Gegenwart verschiedener Katalysatorsysteme erhaltenen Olefine, z. B. die in Gegenwart von Alkylaluminiumchlorid/Titantetrachlorid-Katalysatoren erhaltenen, überwiegend linearen α-Olefine und die in Gegenwart von Nickel-Phosphinkomplex-Katalysatoren nach dem Shell Higher Olefin Process (SHOP) erhaltenen α-Olefine. Geeignete technisch zugängige Olefingemische werden weiterhin bei der Paraffin-Dehydrierung entsprechender Erdölfraktionen, z. B. der sog. Petroleum- oder Dieselölfraktionen, erhalten. Zur Überführung von Paraffinen, vorwiegend von n-Paraffinen in Olefine, werden im Wesentlichen drei Verfahren eingesetzt:

- thermisches Cracken (Steamcracken),
- katalytisches Dehydrieren und
- chemisches Dehydrieren durch Chlorieren und Dehydrochlorieren.

Dabei führt das thermische Cracken überwiegend zu α-Olefinen, während die anderen Varianten Olefingemische ergeben, die im Allgemeinen auch größere Anteile an Olefinen mit innenständiger Doppelbindung aufweisen.

Geeignete Olefingemische sind weiterhin die bei Metathese- bzw. Telomerisationsreaktionen erhaltenen olefine. Dazu zählen z. B. die Olefine aus dem Phillips-Triolefin-Prozess, einem modifizierten SHOP-Prozess aus Ethylen-Oligomerisierung, Doppelbindungs-Isomerisierung und anschließender Metathese (Ethenolyse).

Geeignete in dem erfindungsgemäßen Hydroformylierungsverfahren einsetzbare technische Olefingemische sind weiterhin ausgewählt unter Dibutenen, Tributenen, Tetrabutenen, Dipropenen, Tripropenen, Tetrapropenen, Mischungen von Butenisomeren, insbesondere Raffinat II, Dihexenen, Dimeren und Oligomeren aus dem Dimersol®-Prozess von IFP, Octolprozess® von Hüls, Polygasprozess etc.

Bevorzugt sind auch 1-Buten-haltige Kohlenwasserstoffgemische wie Raffinat II. Geeignete 1-Buten-haltige Kohlenwasserstoffgemische können einen Anteil an gesättigten Kohlenwasserstoffen aufweisen. Vorteilhaft sind Gemische mit einem geringen Anteil an hochsiedenden Komponenten.

Die Umsetzung kann in Gegenwart eines Lösungsmittels durchgeführt werden. Geeignet sind beispielsweise solche, welche aus der Gruppe von Ethern, überkritischem $CO_2$, Fluorkohlenwasserstoffen oder Alkylaromaten, wie Toluol und Xylol, ausgewählt sind. Das Lösungsmittel kann aber auch ein polares Lösungsmittel sein. Geeignet sind beispielsweise solche, welche aus der Gruppe von Alkoholen, Dimethylacetamid, Dimethylformamid oder N-Methylpyrrolidon ausgewählt sind. Ebenso ist es möglich, die Umsetzung in Gegenwart eines hochsiedenden Kondensationsprodukts, z. B. eines oligomeren Aldehyds, insbesondere in Gegenwart eines oligomeren des herzustellenden Aldehyds, durchzuführen, der hier auch als Lösungsmittel fungiert. Auch eine Durchführung der Reaktion in einer zweiphasigen Mischung ist möglich.

Da die erfindungsgemäß verwendeten Katalysatoren außer ihrer Hydroformylierungsaktivität häufig auch eine gewisse Aktivität in der Hydrierung von Aldehyden besitzen, können neben den Aldehyden auch die den Aldehyden entsprechenden Alkohole als Wertprodukte entstehen.

Der Austrag der Hydroformylierungsstufe wird vor seiner destillativen Aufarbeitung entspannt. Dabei wird nicht umgesetztes Synthesegas freigesetzt, das in der Hydroformylierung zurückgeführt werden kann. Entsprechendes gilt für das bei der Entspannung in die Gasphase übergegangene, nicht umgesetzte Olefin, das, gegebenenfalls nach destillativer Abtrennung darin enthaltener inerter Kohlenwasserstoffe, ebenfalls in die Hydroformylierung zurückgeführt werden kann. Die Destillation des entspannten Hydroformylierungsaustrages wird im Allgemeinen bei Drücken von 0,1 bis 1000 mbar absolut, vorzugsweise bei 1 bis 500 mbar und besonders bevorzugt von 10 bis 400 mbar durchgeführt.

Die Temperatur und der Druck, die in der Destillation eingestellt werden müssen, sind abhängig von der Art des Hydroformylierungsprodukts und der verwendeten Destillationsapparatur. Für das erfindungsgemäße Verfahren können im Allgemeinen beliebige Destillationsapparaturen verwendet werden. Bevorzugt werden jedoch Apparate eingesetzt, die niedrige Investitionskosten verursachen und, speziell für höhere Olefine, eine möglichst niedrige Destillationstemperatur erlauben, wie Dünnschichtverdampfer, wischblattverdampfer oder Fallfilmverdampfer, da mit höherer Temperatur die gebildeten Aldehyde im Reaktionsaustrag Folgereaktionen wie Aldolkondensationen eingehen können. Da diese Destillation im Wesentlichen der Abtrennung der Hydroformylierungsprodukte Aldehyd und Alkohol und gegebenenfalls noch vorhandener Leichtsieder, wie nicht umgesetztem Olefin und Inerten, von hochsiedenden Kondensationsprodukten der Aldehyde, sogenannten Hochsiedern, und dem Katalysator und überschüssigem Liganden dient, kann es zweckmäßig sein, die abgetrennten Hydroformylierungsprodukte und gegebenenfalls Olefine und Inerte, einer weiteren destillativen Reinigung, die auf herkömmliche Weise durchgeführt werden kann, zu unterziehen. Somit soll insbesondere eine Aufpegelung noch enthaltener Hochsieder vermieden werden.

Ein Vorteil des erfindungsgemäßen Verfahrens ist die Möglichkeit der Rückführung des Komplexkatalysators und des überschüssigen Liganden aus dem Destillationsrückstand des Reaktionsgemischs. Dabei kann entweder

a) der Destillationssumpf, der den Katalysator und überschüssigen Ligand enthält, insgesamt zurückgeführt werden, oder

b) der Katalysator und überschüssiger Ligand mit einem Lösungsmittel, in dem der Katalysator und der überschüssige Ligand unlöslich oder nahezu unlöslich sind, ausgefällt und nur das Fällungsprodukt zurückgeführt werden, oder

c) die im Destillationssumpf enthaltenen Hochsieder mittels Wasserdampfdestillation vom Katalysator und überschüssigem Liganden abgetrennt werden und nur der nach der Wasserdampfdestillation erhaltene Katalysator und überschüssige Liganden enthaltene Destillationssumpf in die Hydroformylierungsreaktion zurückgeführt werden, oder

d) der Katalysator und überschüssiger Ligand durch Ultrafiltration des Destillationssumpfs gewonnen und das Retentat zurückgeführt werden, oder

e) der Katalysator und überschüssiger Ligand durch Extraktion des Destillationssumpfs gewonnen werden,

wobei z. B. zur Vermeidung einer Anreicherung von Hochsiedern im Kreislauf eine Kombination wenigstens zweier der Methoden a) bis e) möglich ist.

Alternativ kann zwecks Vermeidung einer Aufpegelung von Hochsiedern im Reaktionsgemisch der Hydroformylierung auch ein Teil des Destillationssumpfes aus dem Verfahren von Zeit zu Zeit ausgeschleust werden und der weiteren Aufarbeitung zur Rückgewinnung des Übergangsmetalls der VIII. Nebengruppe des Periodensystems und gewünschtenfalls des verwendeten Liganden zurückgeführt werden. Bei einer solchen Vorgehensweise sollte eine der ausgeschleusten Menge an Übergangsmetall der VIII. Nebengruppe des Periodensystems und Ligand oder eine geeignete Ligandvorstufen, welche unter den Reaktionsbedingungen der Hydroformylierung den eigentlichen Liganden bildet, entsprechende Menge dieser Verbindung durch Zufuhr dieser Verbindungen in die Hydroformylierungsreaktion ergänzt werden.

Methode a)

Bevorzugt sind solche erfindungsgemäßen Liganden, die einen so hohen Siedepunkt haben, dass bei der Destillation des Reaktionsaustrages der gesamte Ligand-Übergangsmetall-Komplex und der gesamte oder zumindest der größte Teil nicht zur Komplexbildung benötigte Ligand im Sumpf der Destillation verbleiben und der Sumpf aus dieser Destillation zusammen mit frischem olefin in die Reaktion zurückgeführt werden kann.

Dieses Verfahren führt überraschenderweise zu ausgezeichneten Ergebnissen bei der Hydroformylierung mit destillativer Aufarbeitung des Reaktionsaustrages, da die zu verwendenden Liganden sehr gute Stabilisatoren für den thermolabilen Katalysator sind und selbst eine hohe thermische Stabilität aufweisen. Verluste des Liganden und der Übergangsmetallkomponente des Katalysators können weitgehend vermieden werden, selbst wenn eine sehr kostengünstige Destillation mit einer niedrigen Zahl von theoretischen Böden wie z. B. Dünnschichtverdampfer oder Fallfilmverdampfer eingesetzt wird.

Da bei dieser Variante des Verfahrens sämtliche hochsiedende Nebenprodukte wieder in die Reaktion gelangen, tritt eine gewisse Aufpegelung der Hochsieder ein und es kann erforderlich sein, kontinuierlich oder absatzweise eine Ausschleusung von Hochsiedern vorzunehmen. Dies kann z. B. dadurch geschehen, dass man nach den unten näher beschriebenen Varianten b) oder c) zumindest zeitweise eine Abtrennung des Katalysatorkomplexes und des überschüssigen Liganden aus dem Destillationssumpf vornimmt und den überwiegend Hochsieder enthaltenden Rest ausschleust.

Methode b)

Vorteilhaft für diese Methode der Fällung des Katalysatorkomplexes und des überschüssigen Liganden ist ein Lösungsmittel, das mit den organischen Bestandteilen des Destillationssumpfs des Reaktionsaustrags in einem weiten Bereich mischbar ist, in dem jedoch der Katalysatorkomplex und der Ligand unlöslich oder nahezu unlöslich sind, so dass es möglich wird, durch Wahl der Art und Menge des Lösungsmittels den Katalysatorkomplex und den Liganden auszufällen, die nach Abtrennung durch Dekantation oder Filtration in die Hydroformylierung zurückgeführt werden können.

Als Lösungsmittel kommt eine große Zahl von polaren Lösungsmittel, die vor allem Hydroxy-, Carbonyl-, Carbonsäureamid- oder Ether-Gruppen aufweisen, also Alkohole, Ketone, Amide oder Ether sowie Mischungen dieser Lösungsmittel oder Mischungen dieser Lösungsmittel mit Wasser in Betracht.

Die Art und Menge des anzuwendenden Lösungsmittels kann der Fachmann durch wenige Handversuche im Einzelnen ermitteln. Im Allgemeinen wird die Menge des Lösungsmittels möglichst niedrig gehalten, damit der Wiedergewinnungsaufwand möglichst gering ist. Demgemäß wird in der Regel, bezogen auf das Volumen des Destillationssumpfes, die 1- bis 50-fache, vorzugsweise die 3- bis 15-fache Menge benötigt.

Methode c)

Eine weitere Methode zur Ausschleusung von hochsiedenden Kondensationsprodukten der Aldehyde besteht darin, diese aus dem Sumpf der Destillation mittels wasserdampfdestillation abzutrennen. Die Wasserdampfdestillation des Destillationssumpfs kann diskontinuierlich, also absatzweise, oder kontinuierlich erfolgen, wobei die Wasserdampfdestillation in der Destillationsapparatur selbst oder einer separaten Vorrichtung zur Wasserdampfdestillation vorgenommen werden kann. Beispielsweise kann bei der diskontinuierlichen Ausgestaltung des Verfahrens der Destillationssumpf vor seiner Rückführung in die Hydroformylierung durch Durchleiten von wasserdampf ganz oder teilweise von hochsiedenden Kondensationsprodukten befreit werden, oder der Destillationssumpf kann je nach Hochsiederanfall von Zeit zu Zeit in einer separaten Apparatur einer wasserdampfdestillation unterzogen werden.

Die kontinuierliche Ausgestaltung der Methode kann z. B. so erfolgen, dass der Destillationssumpf oder ein Teil des Destillationssumpfs vor seiner Rückführung in die Hydroformylierung kontinuierlich einer wasserdampfdestillationsapparatur zugeführt und darin ganz oder teilweise von Hochsiedern befreit wird. Ebenso ist es möglich, die destillative Aufarbeitung des Hydroformylierungsaustrags von vornherein kontinuierlich in Gegenwart von Wasserdampf zu betreiben, um gleichzeitig mit dem Aldehyd und dem Alkohol auch die Hochsieder vom Katalysator und dem überschüssigen Liganden abzutrennen. Es versteht sich von selbst, dass bei einer solchen Vorgehensweise in einer nachfolgenden Fraktionier- und Destillationseinrichtung die Wertproduke von Hochsiedern und gegebenenfalls von Wasser geschieden werden müssen.

Die Wasserdampfdestillation erfolgt im Allgemeinen auf herkömmliche weise durch Einleiten von Wasserdampf in den Hochsieder enthaltenden Destillationssumpf und nachfolgende Kondensation des wasserdampfdestillats. Vorteilhaft wird dabei der Wasserdampf so durch den Destillationssumpf geleitet, dass er nicht im Destillationssumpf kondensiert. Dies kann durch Wahl der Druck- und/oder Temperaturbedingungen, unter denen die Wasserdampfdestillation durchgeführt wird, bewirkt werden. Dabei kann sowohl verminderter Druck angewendet werden oder bei Verwendung überhitzten Wasserdampfes auch erhöhter Druck. Im Allgemeinen wird die Wasserdampfdestillation bei einer Temperatur von 80 bis 200°C und bei einem Druck von 1 mbar bis 10 bar, vorzugsweise von 5 mbar bis 5 bar, vorgenommen. Dabei wird der Wasserdampf bezüglich der im Sumpf enthaltenen hochsiedenden Kondensationsprodukte der Aldehyde (Hochsieder) im Allgemeinen in einem Gewichtsverhältnis Wasserdampf:Hochsieder von 10:1 bis 1:10 durch den Destillationssumpf geleitet. Nach Beendigung der Wasserdampfdestillation kann der so ganz oder teilweise von Hochsiedern befreite Katalysator und überschüssigen Ligand enthaltende Destillationssumpf in die Hydroformylierung zurückgeführt werden.

Wie bereits vorstehend erwähnt, kann es zweckmäßig sein, die Methoden a) und b) bzw. a) und c) zu kombinieren.

Methode d)

Infolge des Unterschieds der Molekulargewichte der Katalysatorkomplexe und überschüssigen Liganden einerseits und der im Destillationssumpf verbleibenden Hochsieder andererseits ist es auch möglich, Katalysatorkomplexe und Liganden durch Ultrafiltration von den Hochsiedern abzutrennen. Dazu beträgt das mittlere Molekulargewicht des Liganden bevorzugt mehr als 500 Dalton, besonders bevorzugt mehr als 1000 Dalton.

Das Verfahren kann kontinuierlich oder diskontinuierlich betrieben werden. Ausgestaltungen derartiger Verfahren sind in der WO 99/36382 beschrieben, worauf hier Bezug genommen wird.

Nach einer geeigneten kontinuierlichen Arbeitsweise werden die Edukte in Anwesenheit vom Katalysator und Liganden in einem Reaktor umgesetzt. Der Reaktoraustrag wird in einer Destillationsapparatur in einen Destillatstrom, der die Oxoprodukte enthält, und in einen Rückstand getrennt. Der katalysatorhaltige Rückstand wird kontinuierlich einer Membranfiltration zugeleitet. Dabei wird der Rückstand, der Hochsieder (oder ein Gemisch Hochsieder, Edukte und Oxoprodukte), Katalysator und Liganden enthält, aufgearbeitet. Die Hochsieder (und gegebenenfalls Edukte und Oxoprodukte) permeieren durch die Membran. Der an Hochsieder (und gegebenenfalls an Edukten und Oxoprodukten) abgereicherte und an Katalysator und Liganden angereicherte Retentatstrom wird in die Hydroformylierung zurückgeführt.

Nach einer geeigneten diskontinuierlichen Fahrweise werden die Edukte in Anwesenheit vom Katalysator und Liganden in einem Reaktor umgesetzt. Am Ende der Reaktion wird der Reaktoraustrag in einer Destillationsapparatur in einen Destillatstrom, der die Oxoprodukte enthält, und in einen Rückstandstrom getrennt. Dieser katalysatorhaltige Rückstand aus der Destillation wird in einer Membranfiltration aufgearbeitet. Der an Hochsieder (und gegebenenfalls an Edukten und Oxoprodukten) abgereicherte und an Katalysator und Liganden angereicherte Destillationsrückstand wird am Ende der Ultrafiltration in den Reaktor für die nächste Charge der Hydroformylierung zurückgeführt.

Die Ultrafiltration kann einstufig oder mehrstufig (vorzugsweise zweistufig) betrieben werden. In jeder Stufe wird die Feedlösung z. B. mittels einer Druckpumpe auf Filtrationsdruck gebracht; die Überströmung, d. h. Benetzung, der Membran kann dann durch Rückführung eines Teils des Retentatstroms in einer zweiten Pumpe sichergestellt werden. Um einen nennenswerten Deckschichtaufbau aus dem Katalysator auf der Membranoberfläche zu vermeiden (Konzentrationspolarisation), der zu einer Abnahme des Permeatflusses führen kann, wird zwischen Membran und der katalysatorhaltigen Lösung vorzugsweise eine Relativgeschwindigkeit im Bereich von 0,1 bis 10 m/s eingehalten. Weitere geeignete Maßnahmen zur Vermeidung eines Deckschichtaufbaus sind z. B. mechanische Bewegung der Membran oder der Einsatz von Rühraggregaten zwischen den Membranen. In der mehrstufigen Variante wird der Permeatstrom einer Stufe der nachgeschalteten Stufe zugeleitet und der Retentatstrom dieser nachgeschalteten Stufe der vorherigen Stufe zugeleitet. Durch diese Aufarbeitung des Permeats ist eine bessere Rückhaltung des Katalysators und des Liganden erreichbar.

Im Fall einer mehrstufigen Ultrafiltration können die verschiedenen Stufen mit der gleichen oder mit unterschiedlichen Membranen ausgerüstet sein.

Die optimalen transmembranen Drücke zwischen Retentat und Permeat liegen im Wesentlichen abhängig von Durchmesser der Membranporen und der mechanischen Stabilität der Membran bei der Betriebstemperatur und liegen

je nach Membranart zwischen 0,5 bis 100 bar, vorzugsweise 10 bis 60 bar und bei einer Temperatur bis 200°C. Höhere transmembrane Drücke und höhere Temperaturen führen zu höheren Permeatflüssen. Die Überströmungsgeschwindigkeit in dem Modul beträgt in der Regel 1 bis 10, vorzugsweise 1 bis 4 m/s. Die Katalysatorkonzentration in der Feedlösung zur Membran beträgt vorzugsweise 5 bis 2000 ppm.

Für die Ultrafiltration kommen alle Membranen in Betracht, die im Reaktionssystem stabil sind. Die Trenngrenze der Membranen beträgt etwa 200 bis 20000 Dalton, besonders 500 bis 5000 Dalton. Die Trennschichten können aus organischen Polymeren, Keramik, Metall oder Kohlenstoff bestehen und müssen in dem Reaktionsmedium und bei der Prozesstemperatur stabil sein. Aus mechanischen Gründen sind die Trennschichten in der Regel auf einer ein- oder mehrschichtigen porösen Unterstruktur aus dem gleichen oder auch mehreren unterschiedlichen Materialien wie die Trennschicht aufgebracht. Beispiele sind:

| Trennschicht | Unterstruktur (gröber als Trennschicht |
|---|---|
| Metall | Metall |
| Keramik | Metall, Keramik oder Kohlenstoff |
| Polymer | Polymer, Metall, Keramik oder Keramik auf Metall |
| Kohlenstoff | Kohlenstoff, Metall oder Keramik |
| Keramik: z. B. $\alpha$-$Al_2O_3$, $\gamma$-$Al_2O_3$, $ZrO_2$, $TiO_2$, SiC, gemischte keramische werkstoffe<br>Polymer: z. B. PTFE, PVDF, Polysulfon, Polyethersulfon, Polyetheretherketon | |

Die Membranen können in Flach-, Rohr-, Multikanalelement-, Kapillar- oder Wickelgeometrie eingesetzt werden, für die entsprechende Druckgehäuse, die eine Trennung zwischen Retentat (katalysatorhaltig) und dem Permeat (katalysatorfreies Filtrat) erlauben, verfügbar sind.

In den folgenden Tabellen sind Beispiele von solchen Membranen zusammengestellt.

| Bezeichnung | Material | Trenngrenze | Hersteller |
|---|---|---|---|
| MPF-U20-S | Polysulfon | 20000 Dalton | Membran Products Kiryat Weizmar |
| K00X1040 | $TiO_2$ auf $Al_2O_3$-$TiO_2$ | 15000 Dalton | Tech-Sep |
| | $TiO_2$ | 500 bis 10000 Dalton | Inocermic Gesellschaft für Innovative Keramik mbH |
| | $TiO_2$ | 5000 Dalton | Société des Céramiques Techniques |
| | $TiO_2$ auf Edelstahl | | Graver Chemical Company |

| Hersteller | Membran | Trenngrenze (kD) Porendurchmesser (nm) |
|---|---|---|
| Atech innovations GmbH | UF/$TiO_2$ auf $\alpha$-$Al_2O_3$ | 20 kD |
| Rhodia/Orelis | NF/$ZrO_2$ auf Keramik | 1 kD |
| | UF/$ZrO_2$ oder $TiO_2$ auf Keramik | 15, 50, 150 kD |
| | UF/ZrO2 auf Kohlenstoff | 15, 50, 150 kD |
| USF Filtration & Separation | NF/$TiO_2$ auf Keramik | 1-5 kD |
| | UF/$ZrO_2$ auf Keramik | 20 nm |
| Graver Technologies | UF/Keramik auf Stahl | 20 kD |
| Inocermic GmbH | UF/$ZrO_2$ auf Keramik | 3 nm |
| | UF/$TiO_2$ auf Keramik | 5 nm |
| | NF/$TiO_2$ auf Keramik | 0,9 nm/0,5 kD |
| Osmonics/Desal | UF/PVDF | 10 kD |
| NADIR Filtrations GmbH | UF/Polyethersulfon | 5-150 kD |

Methode e)

Als weitere Variante kann auch zunächst eine destillative Abtrennung der Aldehyde/Alkohole durchgeführt werden, worauf der Katalysator-haltige Sumpf mit einem polaren Extraktionsmittel wie z. B. Wasser behandelt wird. Dabei tritt der Katalysator in die polare Phase über, während Hochsieder in der organischen Phase verbleiben. Vorzugsweise werden nach dieser Variante Katalysatoren mit wasserlöslichen (hydrophilen) Liganden oder mit Liganden, die sich in eine wasserlösliche Form überführen lassen, eingesetzt. Durch eine Reextraktion kann der Katalysator zurückgewonnen werden oder direkt als solches zurückgeführt werden. Alternativ kann der Katalysator durch ein unpolares Lösungsmittel extrahiert werden, worauf die Hochsieder abgetrennt werden.

Alternativ zu einem destillativen Aufarbeitungsverfahren kann auch die Aufarbeitung des Reaktionsaustrags durch Extraktion erfolgen. Dazu wird dem Reaktionsaustrag je nach Art des eingesetzten Katalysators ein polares oder unpolares Lösungsmittel zugesetzt, das mit dem Reaktionsaustrag oder mit wenigstens einem der gegebenenfalls im Reaktionsaustrag enthaltenen Lösungsmittel im Wesentlichen unmischbar ist. Gewünschtenfalsl kann dem Reaktionsaustrag zur Extraktion auch ein zweiphasiges Gemisch aus wenigstens einem polaren und wenigstens einem unpolaren Lösungsmittel zugesetzt werden. Im Falle einer zweiphasigen Reaktionsführung kann auf die Zugabe eines weiteren Lösungsmittels in der Regel verzichtet werden. Nach Phasentrennung in einem geeigneten Apparat erhält man eine Phase, welche die Hydroformylierungsprodukte und höhersiedende Kondensationsprodukte enthält, und eine Phase, welche den Katalysator enthält. Besonders geeignete polare Phasen sind Wasser und ionogene Flüssigkeiten, d. h. Salze, welche einen niedrigen Schmelzpunkt aufweisen. Bevorzugt werden bei einem derartigen Hydroformylierungsverfahren ionische oder polare Gruppen enthaltende Phosphacyclohexanliganden verwendet, so dass eine hohe Löslichkeit des Katalysators in der polaren Phase resultiert und ein "Leaching" des Katalysators in die organische Phase unterbunden oder zumindest weitgehend unterbunden wird. Geeignete Substituenten sind beispielsweise W'COO-$M^+$, W'$SO_3^-M^+$, W'$PO_3^{2-}M^{2+}$, W'NR'$_3^+X^-$, W'OR', WNR'$_2$, W'COOR', W'SR', W'(CHR'$CH_2O)_x$R', W'($CH_2$NR')$_x$R' und W'($CH_2CH_2$NR')$_x$R', wobei X-, $M^+$, R', W' und x die zuvor genannten Bedeutungen aufweisen.

Phosphacyclohexane mit unpolaren Resten können auch mit einem unpolaren Lösungsmittel durch Phasentrennung entfernt werden. Besonders geeignet sind Phosphacyclohexane mit lipophilen Resten. Auf diese Weise kann man ein "Leaching" des Katalysators zumindest weitestgehend unterbinden.

In einer bevorzugten Ausführungsform kann die Aufarbeitung des Reaktionsgemischs auch direkt über eine Ultrafiltration erfolgen. Dabei wird zur Abtrennung des Katalysators und Gewinnung eines katalysatorfreien Produkt- bzw. Hochsiederstroms der Syntheseaustrag, wie zuvor beschrieben, unter Druck mit einer Membran in Kontakt gebracht und Permeat (Filtrat) auf der Rückseite der Membran bei einem geringeren Druck als auf der Feedseite abgezogen. Man erhält ein Katalysatorkonzentrat (Retentat) und ein praktisch katalysatorfreies Permeat.

Wenn der Syntheseaustrag direkt dem Membranverfahren mit dem Synthesedruck zugeführt wird, kann der transmembrane Druck durch Anhebung des Permeatdrucks eingestellt werden.

Das erhaltene, im Wesentlichen katalysatorfreie Permeat kann durch übliche, dem Fachmann bekannte Verfahren, wie Destillation oder Kristallisation, weiter in Produkte und Hochsieder aufgetrennt werden.

Bei der Verwendung von $\alpha$-Olefinen als Feed können beim erfindungsgemäß verwendeten Hydroformylierungsverfahren im Vergleich zu einem entsprechenden Rhodium/Triphenylphosphan-katalysierten Hydroformylierungsverfahren höhere Isoaldehydanteile im Aldehydgemisch gebildet werden. Dies ist vorteilhaft für bestimmte Anwendungen, z. B. zur Herstellung von Neopentylglykol.

Die erfindungsgemäß verwendeten Phosphacyclohexanliganden besitzen eine hohe Stabilität unter Hydroformylierungsbedingungen. Der Katalysator kann über mehrere Wochen ohne Aktivitätsverlust und ohne Zufuhr (Nachpegelung) von Phosphacylcohexanligand oder einer entsprechenden Phosphacyclohexan-Vorstufe in einem kontinuierlichen Hydroformylierungsverfahren eingesetzt werden. Es wird kein Abbau des Liganden beobachtet.

Die erfindungsgemäß verwendeten Katalysatoren zeigen in der Hydroformylierung eine hohe Selektivität zu Aldehyden und Alkoholen. Die Paraffinbildung durch Hydrierung von Eduktalkenen ist im Vergleich zu einem Rhodium/Triphenylphosphan-katalysierten Hydroformylierungsverfahren deutlich geringer.

Im kontinuierlichen Hydroformylierungsverfahren mit Rhodium/Phosphacyclohexanen kann alternativ zur Ergänzung (Nachpegelung) des Phosphacyclohexanliganden auch das entsprechende Phosphabenzol oder eine andere Phosphacyclohexan-Vorstufe nachgepegelt werden, welche unter Hydroformylierungsbedingungen in Gegenwart von Olefin zum entsprechenden Phoshacyclohexan umgewandelt werden. Der Zusatz der entsprechenden Phosphabenzol-Vorstufe zu einer Rhodium/Phosphacyclohexan-katalysierten Hydroformylierung hat dabei nur einen untergeordneten Einfluss auf die Aktivität und die Selektivität der Katalyse.

In 1-Position unsubstituierte Phosphacyclohexane und -phosphacyclohexene sind in der Regel von geringerer katalytischer Aktivität. Durch Behandlung des Rhodium/Phosphacyclohexan-Rohkatalysatorgemisches mit einem Olefin oder Olefingemisch in Gegenwart von Synthesegas lassen sich derartige Verbindungen vernichten und ein noch aktiveres Katalysatorsystem erhalten. Dadurch resultieren aktivere Katalysatorgemische, wie ein Vergleich verschiedener Hydroformylierungsversuche zeigt.

Neben der Hydroformylierung ist der Katalysator auch in anderen geeigneten Umsetzungen einsetzbar. Beispiele sind die Hydroacylierung, Hydrocyanierung, Hydroamidierung, Hydroveresterung, Aminolyse, Alkoholyse, Hydrocarbonylierung, Hydroxycarbonylierung, Carbonylierung, Isomerisierung bzw. Transferhydrierung.

Die Erfindung wird nachstehend anhand von Beispielen näher erläutert:

Beispiele

Beispiel 1

Herstellung einer Rhodium/2,6-Bis(2,4-dimethylphenyl)-1-octyl-4-phenylphosphacyclohexan-Lösung

0.10 g (0.39 mmol) Rhodiumdicarbonylacetylacetonat, 1.0 g (2.6 mmol) 2,6-Bis(2,4-dimethylphenyl)-4-phenylphosphabenzol und 10 g (89 mmol) 1-Octen wurden unter Stickstoff-Inertgas in einem Schlenkrohr in 40 g Toluol gelöst. Die erhaltene Lösung wurde in einen mit Wasserstoff gespülten 100 ml Autoklaven überführt. Es wurden 5 bar Wasserstoff aufgepresst. Unter kräftigem Rühren mit einem Begasungsrührer wurde das Reaktionsgemisch auf 150 °c erhitzt. Der Wasserstoffdruck im Autoklaven wurde auf 80 bar erhöht. Das Reaktionsgemisch wurde dann 72 h bei 150 °c gerührt. Während der Reaktion wurde der Druck im Reaktor durch Nachpressen über einen Druckregler auf Druckniveau gehalten. Nach Abkühlen auf Raumtemperatur wurden zur Vervollständigung der Reaktion erneut 3.0 g (27 mmol) 1-Octen zugesetzt. Die Reaktion wurde für weitere 24 h bei 150 °C und 80 bar Wasserstoffdruck gemäß dem zuvor beschriebenen Verfahren betrieben. Nach Abkühlen auf Raumtemperatur resultierte eine gelb-braune, homogene Lösung. Die erhaltene Lösung wurde im Hochvakuum vollständig eingeengt und dann in 200 ml Toluol aufgenommen. Eine Analyse der Lösung lieferte einen Rhodium-Gehalt von 190 ppm und einen Phosphor-Gehalt von 400 ppm (P/Rh-Molverhältnis 7). Eine GC-Analyse (NP-Detektor, d. h. gegenüber Stickstoff und Phosphor sensitiver Detektor) zeigt einen Phosphabenzol-Umsatz von 97 % an. Die Ausbeute an 2,6-Bis(2,4-dimethylphenyl)-1-octyl-4-phenylphosphacyclohexan betrug 81 %, die Ausbeute an 2,6-Bis(2,4-dimethylphenyl)-1-octyl-4-phenylphosphacyclohexenen betrug 8 % und die Ausbeute an 2,6-Bis(2,4-dimethylphenyl)-4-phenylphosphacyclohexan betrug 0.2 %. Die Verbindungen konnten durch GC-MS-Analyse und $^{31}$P-NMR-Spektroskopie bestätigt werden.

Beispiel 2

Herstellung einer Rhodium/2,6-Bis(2,4-dimethylphenyl)-1-octyl-4-phenylphosphacyclohexan-Lösung

0.10 g (0.39 mmol) Rhodiumdicarbonylacetylacetonat, 1.0 g (2.6 mmol) 2,6-Bis(2,4-dimethylphenyl)-4-phenylphosphabenzol und 6.0 g (53 mmol) 1-Octen wurden unter Stickstoff-Inertgas in einem Schlenkrohr in 6.0 g Toluol gelöst. Die erhaltene Lösung wurde in einen mit Wasserstoff gespülten 100 ml Autoklaven überführt. Es wurden 5 bar Wasserstoff aufgepresst. Unter kräftigem Rühren mit einem Begasungsrührer wurde das Reaktionsgemisch auf 150 °c erhitzt. Der Wasserstoffdruck im Autoklaven wurde auf 80 bar erhöht. Das Reaktionsgemisch wurde dann 72 h bei 150 °C gerührt. Während der Reaktion wurde der Druck im Reaktor durch Nachpressen über einen Druckregler auf Druckniveau gehalten. Nach Abkühlen auf Raumtemperatur wurden zur Vervollständigung der Reaktion erneut 6.0 g (53 mmol) 1-Octen zugesetzt. Die Reaktion wurde für weitere 24 h bei 150 °c und 80 bar Wasserstoffdruck gemäß dem zuvor beschriebenen Verfahren betrieben. Nach Abkühlen auf Raumtemperatur resultierte eine gelb-braune, homogene Lösung. Der Reaktionsaustrag wurde dann im Hochvakuum vollständig eingeengt. Der verbleibende Rückstand wurde über Silica 60 mit einem Hexan/Ethylacetat-Gemisch (4:1) als Eluens chromatographiert. Die erhaltene Lösung wurde erneut im Hochvakuum vollständig eingeengt und anschließend über Silica 60 mit einem Hexan/Ethylacetat-Gemisch (9:1) als Eluens chromatographiert. Die erhaltene Lösung wurde im Hochvakuum vollständig eingeengt und dann in 50 ml Toluol aufgenommen. Eine Analyse der Lösung lieferte einen Rhodium-Gehalt von 340 ppm und einen Phosphor-Gehalt von 790 ppm (P/Rh-Molverhältnis 8). Eine GC-Analyse (NP-Detektor) zeigt folgende Zusammensetzung der Lösung: 2 % 2,6-Bis(2,4-dimethylphenyl)-4-phenylphosphabenzol, 86 % 2,6-Bis(2,4-dimethylphenyl)-1-octyl-4-phenylphosphacyclohexan, 10 % 2,6-Bis(2,4-dimethylphenyl)-1-octyl-4-phenylphosphacyclohexenen und 2 % 2,6-Bis(2,4-dimethylphenyl)-4-phenylphosphacyclohexan. Die Verbindungen konnten durch GC-MS-Analyse und $^{31}$P-NMR-Spektroskopie bestätigt werden.

Beispiel 3

Herstellung einer Rhodium/2,6-Bis(2,4-dimethylphenyl)-1-propyl-4-phenylphosphacyclohexan-Lösung

0.044 g (0.17 mmol) Rhodiumdicarbonylacetylacetonat und 1.5 g (3.9 mmol) 2,6-Bis(2,4-dimethylphenyl)-4-phenylphosphabenzol wurden unter Stickstoff-Inertgas in einem Schlenkrohr in 30 g Toluol gelöst. Die erhaltene Lösung

wurde in einen mit Wasserstoff gespülten 100 ml Autoklaven überführt. Über eine Schleuse wurden dann 6.0 g (143 mmol) Propen mit Wasserstoff-Überdruck in den Autoklaven gepresst. Darauf wurde mittels Wasserstoff ein Druck von 5 bar eingestellt. Unter kräftigem Rühren mit einem Begasungsrührer wurde das Reaktionsgemisch auf 150 °C erhitzt. Der Wasserstoffdruck im Autoklaven wurde auf 80 bar erhöht. Das Reaktionsgemisch wurde dann 72 h bei 150 °C gerührt. Während der Reaktion wurde der Druck im Reaktor durch Nachpressen über einen Druckregler auf Druckniveau gehalten. Nach Abkühlen auf Raumtemperatur wurden der Autoklav auf Normaldruck entspannt und es wurden der Reaktion erneut 8.0 g (190 mmol) Propen zugesetzt. Die Reaktion wurde für weitere 24 h bei 150 °C und 80 bar Wasserstoffdruck gemäß dem zuvor beschriebenen Verfahren betrieben. Nach Abkühlen auf Raumtemperatur wurde die erhaltene Lösung im Hochvakuum vollständig eingeengt und der verbleibende Rückstand wurde in 120 ml Toluol aufgenommen. Eine GC-Analyse (NP-Detektor) zeigt einen Phosphabenzol-Umsatz von 100 % an. Die Ausbeute an 2,6-Bis(2,4-dimethylphenyl)-1-propyl-4-phenylphosphacyclohexan betrug 61 % und die Ausbeute an 2,6-Bis(2,4-dimethylphenyl)-4-phenylphosphacyclohexan betrug 21 %. Die Verbindungen konnten durch GC-MS-Analyse und $^{31}$P-NMR-Spektroskopie bestätigt werden.

Beispiel 4

Herstellung einer Rhodium/2,6-Bis(2,4-dimethylphenyl)-1-isobutyl -4-phenylphosphacyclohexan-Lösung

0.044 g (0.17 mmol) Rhodiumdicarbonylacetylacetonat und 1.5 g (3.9 mmol) 2,6-Bis(2,4-dimethylphenyl)-4-phenylphosphabenzol wurden unter Stickstoff-Inertgas in einem Schlenkrohr in 30 g Toluol gelöst. Die erhaltene Lösung wurde in einen mit Wasserstoff gespülten 100 ml Autoklaven überführt. Über eine Schleuse wurden dann 6.0 g (107 mmol) Isobuten mit Wasserstoff-Überdruck in den Autoklaven gepresst. Darauf wurde mittels Wasserstoff ein Druck von 5 bar eingestellt. Unter kräftigem Rühren mit einem Begasungsrührer wurde das Reaktionsgemisch auf 150 °C erhitzt. Der Wasserstoffdruck im Autoklaven wurde auf 80 bar. Das Reaktionsgemisch wurde dann 72 h bei 150 °C gerührt. Während der Reaktion wurde der Druck im Reaktor durch Nachpressen über einen Druckregler auf Druckniveau gehalten. Nach Abkühlen auf Raumtemperatur wurden der Autoklav auf Normaldruck entspannt und es wurden der Reaktion erneut 6.0 g (107 mmol) Isobuten zugesetzt. Die Reaktion wurde für weitere 24 h bei 150 °C und 80 bar Wasserstoffdruck gemäß dem zuvor beschriebenen Verfahren betrieben. Nach Abkühlen auf Raumtemperatur resultierte eine gelb-braune, homogene Lösung. Eine GC-Analyse (NP-Detektor) zeigt einen Phosphabenzol-Umsatz von 47 % an. Die Ausbeute an 2,6-Bis(2,4-dimethylphenyl)-1-isobutyl-4-phenylphosphacyclohexan betrug 35 %, die Ausbeute an 2,6-Bis(2,4-dimethylphenyl)-1-isobutyl-4-phenylphospha-cyclohexenen betrug 14 % und die Ausbeute an 2,6-Bis(2,4-dimethylphenyl)-4-phenylphosphacyclohexan betrug 19 %. Die Verbindungen konnten durch GC-MS-Analyse und $^{31}$P-NMR-Spektroskopie bestätigt werden.

Beispiel 5

Herstellung einer Rhodium/1-Octyl-2,4,6-triphenylphosphacyclohexan-Lösung

0.10 g (0.39 mmol) Rhodiumdicarbonylacetylacetonat, 1.3 g (4.0 mmol) 2,4,6-Triphenylphosphabenzol und 10 g (89 mmol) 1-octen wurden unter Stickstoff-Inertgas in einem Schlenkrohr in 20 g Toluol gelöst. Die erhaltene Lösung wurde in einen mit Wasserstoff gespülten 100 ml Autoklaven überführt. Es wurden 5 bar Wasserstoff aufgepresst. Unter kräftigem Rühren mit einem Begasungsrührer wurde das Reaktionsgemisch auf 150 °C erhitzt. Der Wasserstoffdruck im Autoklaven wurde auf 80 bar erhöht. Das Reaktionsgemisch wurde 24 h bei 150 °C gerührt. Während der Reaktion wurde der Druck im Reaktor durch Nachpressen über einen Druckregler auf Druckniveau gehalten. Nach Abkühlen auf Raumtemperatur resultierte eine braune, homogene Lösung. Die erhaltene Lösung wurde im Hochvakuum vollständig eingeengt und dann in 200 ml Toluol aufgenommen. Eine Analyse der Lösung lieferte einen Rhodium-Gehalt von 200 ppm und einen Phosphor-Gehalt von 640 ppm (P/Rh-Molverhältnis 11). Eine GC-Analyse (NP-Detektor) zeigt einen Phosphabenzol-Umsatz von 98 % an. Die Ausbeute an 1-Octyl-2,4,6-triphenylphosphacyclohexanen betrug 76 % und die Ausbeute an 2,4,6-Triphenylphosphacyclohexan betrug 9 %. Die Verbindungen konnten durch GC-MS-Analyse und $^{31}$P-NMR-Spektroskopie bestätigt werden.

Beispiel 6

Herstellung einer Rhodium/2,6-Bis(2,4-dimethylphenyl)-1-octyl-4-phenylphosphacyclohexan-Lösung

0.40 g (1.55 mmol) Rhodiumdicarbonylacetylacetonat, 3.1 g (8.1 mmol) 2,6-Bis(2,4-dimethylphenyl)-4-phenylphosphabenzol und 10 g (89 mmol) 1-Octen wurden unter Stickstoff-Inertgas in einem Schlenkrohr in 10 g Toluol gelöst. Die erhaltene Lösung wurde in einen mit Wasserstoff gespülten 100 ml Autoklaven überführt. Es wurden 5 bar

Wasserstoff aufgepresst. Unter kräftigem Rühren mit einem Begasungsrührer wurde das Reaktionsgemisch auf 160 °C erhitzt. Der Wasserstoffdruck im Autoklaven wurde auf 60 bar erhöht. Das Reaktionsgemisch wurde dann 24 h bei 160 °C gerührt. Während der Reaktion wurde der Druck im Reaktor durch Nachpressen über einen Druckregler auf Druckniveau gehalten. Nach Abkühlen auf Raumtemperatur wurden zur Vervollständigung der Reaktion erneut 10 g (89 mmol) 1-Octen zugesetzt. Die Reaktion wurde für weitere 96 h bei 160 °C und 60 bar Wasserstoffdruck gemäß dem zuvor beschriebenen Verfahren betrieben. Nach der Reaktionszeit wurde der Autoklav abgekühlt, entspannt und entleert. Die erhaltene Lösung wurde im Hochvakuum vollständig eingeengt und der verbleibende Rückstand wurde in 200 g Toluol aufgenommen. Eine Analyse der Lösung lieferte einen Rhodium-Gehalt von 690 ppm und einen Phosphor-Gehalt von 1100 ppm (P/Rh-Molverhältnis 5). Eine GC-Analyse (NP-Detektor) zeigt einen Phosphabenzol-Umsatz von 95 % an. Die Ausbeute an 2,6-Bis(2,4-dimethylphenyl)-1-octyl-4-phenylphosphacyclohexan betrug 74 %, die Ausbeute an 2,6-Bis(2,4-dimethylphenyl)-1-octyl-4-phenylphosphacyclohexenen betrug 11 %, die Ausbeute an 2,6-Bis(2,4-dimethylphenyl)-4-phenylphosphacyclohexan betrug 0.7 %. Die Verbindungen konnten durch GC-MS-Analyse und $^{31}P$-NMR-Spektroskopie bestätigt werden.

Beispiel 7

Herstellung einer Rhodium/2,6-Bis(2,4-dimethylphenyl)-1-cyclohexyl-4-phenylphosphacyclohexan-Lösung

0.10 g (0.39 mmol) Rhodiumdicarbonylacetylacetonat, 1.5 g (3.9 mmol) 2,6-Bis(2,4-dimethylphenyl)-4-phenylphosphabenzol und 10 g (122 mmol) Cyclohexen wurden unter Stickstoff-Inertgas in einem Schlenkrohr in 20 g Toluol gelöst. Die erhaltene Lösung wurde in einen mit Wasserstoff gespülten 100 ml Autoklaven überführt. Darauf wurde mittels Wasserstoff ein Druck von 5 bar eingestellt. Unter kräftigem Rühren mit einem Begasungsrührer wurde das Reaktionsgemisch auf 160 °C erhitzt. Der Wasserstoffdruck im Autoklaven wurde auf 80 bar erhöht. Das Reaktionsgemisch wurde dann 24 h bei 160 °C gerührt. Während der Reaktion wurde der Druck im Reaktor durch Nachpressen über einen Druckregler auf Druckniveau gehalten. Nach Abkühlen auf Raumtemperatur wurden der Autoklav auf Normaldruck entspannt und es wurden der Reaktion erneut 10 g (122 mmol) Cyclohexen zugesetzt. Die Reaktion wurde für weitere 24 h bei 160 °C und 20 bar Wasserstoffdruck gemäß dem zuvor beschriebenen Verfahren betrieben. Nach Abkühlen auf Raumtemperatur wurden der Autoklav auf Normaldruck entspannt und es wurden der Reaktion erneut 10 g (122 mmol) Cyclohexen und 0.05 g (0.19 mmol) Rhodiumdicarbonylacetylacetonat zugesetzt. Die Reaktion wurde für weitere 24 h bei 160 °C und 20 bar Wasserstoffdruck gemäß dem zuvor beschriebenen Verfahren betrieben. Eine GC-Analyse (NP-Detektor) zeigt einen Phosphabenzol-Umsatz von 72 % an. Die Ausbeute an 2,6-Bis(2,4-dimethylphenyl)-1-cyclohexyl-4-phenylphosphacyclohexan betrug 29 %, die Ausbeute an 2,6-Bis(2,4-dimethylphenyl)-4-phenylphosphacyclohexan betrug 20 % und die Ausbeute an 2,6-Bis(2,4-dimethylphenyl)-4-phenylphosphacyclohexenen betrug 13 %. Die Verbindungen konnten durch GC-MS-Analyse und $^{31}P$-NMR-Spektroskopie bestätigt werden.

Beispiel 8

Herstellung einer Rhodium/2,6-Bis(2,4-dimethylphenyl)-1-octyl-4-phenylphosphacyclohexan-Lösung

0.15 g (0.58 mmol) Rhodiumdicarbonylacetylacetonat, 1.5 g (3.9 mmol) 2,6-Bis(2,4-dimethylphenyl)-4-phenylphosphabenzol und 10 g (89 mmol) 1-Octen wurden unter Stickstoff-Inertgas in einem Schlenkrohr in 20 g Toluol gelöst. Die erhaltene Lösung wurde in einen mit Wasserstoff gespülten 100 ml Autoklaven überführt. Es wurden 5 bar Wasserstoff aufgepresst. Unter kräftigem Rühren mit einem Begasungsrührer wurde das Reaktionsgemisch auf 150 °C erhitzt. Der Wasserstoffdruck im Autoklaven wurde auf 80 bar erhöht. Das Reaktionsgemisch wurde dann 72 h bei 150 °C gerührt. Während der Reaktion wurde der Druck im Reaktor durch Nachpressen über einen Druckregler auf Druckniveau gehalten. Nach Abkühlen auf Raumtemperatur wurden der Autoklav auf Normaldruck entspannt. Eine GC-Analyse (NP-Detektor) der Reaktionslösung zeigt einen Phosphabenzol-Umsatz von 97 % an. Die Ausbeute an 2,6-Bis(2,4-dimethylphenyl)-1-octyl-4-phenylphosphacyclohexan betrug 53 %, die Ausbeute an 2,6-Bis(2,4-dimethylphenyl)-1-octyl-4-phenylphosphacyclohexenen betrug 40 % und die Ausbeute an 2,6-Bis(2,4-dimethylphenyl)-4-phenylphosphacyclohexan betrug 1 %. Die Lösung wurde erneut mit 10 g (89 mmol) 1-Octen versetzt. Es wurden bei Raumtemperatur 5 bar $CO/H_2$ (1:1) aufgepresst. Unter kräftigem Rühren mit einem Begasungsrührer wurde das Reaktionsgemisch auf 180 °C erhitzt. Der $CO/H_2$(1:1)-Druck im Autoklaven wurde auf 80 bar erhöht. Das Reaktionsgemisch wurde dann 24 h bei 180 °C gerührt. Während der Reaktion wurde der Druck im Reaktor durch Nachpressen über einen Druckregler auf Druckniveau gehalten. Nach der Reaktionszeit wurde der Autoklav abgekühlt, entspannt und entleert. Die erhaltene Lösung wurde im Hochvakuum vollständig eingeengt und der verbleibende Rückstand wurde in 100 g Toluol aufgenommen. Eine Analyse der Lösung lieferte einen Rhodium-Gehalt von 510 ppm und einen Phosphor-Gehalt von 1200 ppm (P/Rh-Molverhältnis 8). Eine GC-Analyse (NP-Detektor) zeigt einen Phosphabenzol-Umsatz von 99 % an. Die Ausbeute an 2,6-Bis(2,4-dimethylphenyl)-1-octyl-4-phenylphosphacyclohexan betrug 51 % und die Ausbeute an 2,6-Bis(2,4-dimethylphenyl)-1-octyl-4-phenylphosphacyclohexenen betrug 42 %. Die Verbindungen konnten durch GC-MS-Analyse und $^{31}P$-NMR-Spektroskopie bestätigt werden. 2,6-Bis(2,4-dimethylphenyl)-4-phenylphosphacyclohexan war nicht nachweisbar.

Beispiel 9

Herstellung einer Rhodium/1-Octyl-2,4,6-triphenylphosphacyclohexan-Lösung

0.15 g (0.58 mmol) Rhodiumdicarbonylacetylacetonat, 1.5 g (4.6 mmol) 2,4,6-Triphenylphosphabenzol und 10 g (89 mmol) 1-Octen wurden unter Stickstoff-Inertgas in einem Schlenkrohr in 20 g Toluol gelöst. Die erhaltene Lösung wurde in einen mit Wasserstoff gespülten 100 ml Autoklaven überführt. Es wurden 5 bar Wasserstoff aufgepresst. Unter kräftigem Rühren mit einem Begasungsrührer wurde das Reaktionsgemisch auf 150 °C erhitzt. Der Wasserstoffdruck im Autoklaven wurde auf 80 bar erhöht. Das Reaktionsgemisch wurde dann 72 h bei 150 °C gerührt. Während der Reaktion wurde der Druck im Reaktor durch Nachpressen über einen Druckregler auf Druckniveau gehalten. Nach Abkühlen auf Raumtemperatur wurden der Autoklav auf Normaldruck entspannt. Eine GC-Analyse (NP-Detektor) der Reaktionslösung zeigt einen Phosphabenzol-Umsatz von 98 % an. Die Ausbeute an 1-Octyl-2,4,6-triphenylphosphacyclohexan betrug 93 %, die Ausbeute an 2,4,6-Triphenylphosphacyclohexenen betrug 0.2 % und die Ausbeute an 2,4,6-Triphenylphosphacyclohexan betrug 0.1 %. Die Lösung wurde erneut mit 5 g (45 mmol) 1-Octen versetzt. Es wurden bei Raumtemperatur 5 bar $CO/H_2$ (1:1) aufgepresst. Unter kräftigem Rühren mit einem Begasungsrührer wurde das Reaktionsgemisch auf 100 °C erhitzt. Der $CO/H_2$(1:1)-Druck im Autoklaven wurde auf 20 bar erhöht. Das Reaktionsgemisch wurde dann 12 h bei 100 °C gerührt. Während der Reaktion wurde der Druck im Reaktor durch Nachpressen über einen Druckregler auf Druckniveau gehalten. Nach der Reaktionszeit wurde der Autoklav abgekühlt, entspannt und entleert. Die erhaltene Lösung wurde im Hochvakuum vollständig eingeengt und der verbleibende Rückstand wurde in 100 g Toluol aufgenommen. Eine Analyse der Lösung lieferte einen Rhodium-Gehalt von 720 ppm und einen Phosphor-Gehalt von 1500 ppm (P/Rh-Molverhältnis 7). Eine GC-Analyse (NP-Detektor) zeigt einen Phosphabenzol-Umsatz von 98 % an. Die Ausbeute an 1-Octyl-2,4,6-triphenylphosphacyclohexan betrug 95 %. Die Verbindungen konnten durch GC-MS-Analyse und $^{31}P$-NMR-Spektroskopie bestätigt werden. 2,4,6-Triphenylphosphacyclohexan und 2,4,6-Triphenylphosphacyclohexene waren nicht nachweisbar.

Beispiel 10

Herstellung einer Rhodium/3-Benzyl-1-octyl-2,4,6-triphenylphosphacyclohexan-Lösung

0.27 g (1.05 mmol) Rhodiumdicarbonylacetylacetonat, 3.0 g (7.2 mmol) 3-Benzyl-2,4,6-triphenylphosphabenzol und 10 g (89 mmol) 1-Octen wurden unter Stickstoff-Inertgas in einem Schlenkrohr in 10 g Toluol gelöst. Die erhaltene Lösung wurde in einen mit Wasserstoff gespülten 100 ml Autoklaven überführt. Es wurden 5 bar Wasserstoff aufgepresst. Unter kräftigem Rühren mit einem Begasungsrührer wurde das Reaktionsgemisch auf 160 °C erhitzt. Der Wasserstoffdruck im Autoklaven wurde auf 80 bar erhöht. Das Reaktionsgemisch wurde dann 72 h bei 160 °C gerührt. Während der Reaktion wurde der Druck im Reaktor durch Nachpressen über einen Druckregler auf Druckniveau gehalten. Nach Abkühlen auf Raumtemperatur wurden der Autoklav auf Normaldruck entspannt. Eine GC-Analyse (NP-Detektor) der Reaktionslösung zeigt einen Phosphabenzol-Umsatz von 96 % an. Die Ausbeute an 3-Benzyl-1-octyl-2,4,6-triphenylphosphacyclohexan betrug 86 % und die Ausbeute an 3-Benzyl-1-octyl-2,4,6-triphenylphosphacyclohexenen betrug 8 %. Die Lösung wurde erneut mit 5 g (45 mmol) 1-Octen versetzt. Es wurden bei Raumtemperatur 5 bar $CO/H_2$ (1:1) aufgepresst. Unter kräftigem Rühren mit einem Begasungsrührer wurde das Reaktionsgemisch auf 100 °C erhitzt. Der $CO/H_2$(1:1)-Druck im Autoklaven wurde auf 20 bar erhöht. Das Reaktionsgemisch wurde dann 24 h bei 100 °C gerührt. Während der Reaktion wurde der Druck im Reaktor durch Nachpressen über einen Druckregler auf Druckniveau gehalten. Nach der Reaktionszeit wurde der Autoklav abgekühlt, entspannt und entleert. Die erhaltene Lösung wurde im Hochvakuum vollständig eingeengt und der verbleibende Rückstand wurde in 50 g Toluol aufgenommen. Eine Analyse der Lösung lieferte einen Rhodium-Gehalt von 1400 ppm und einen Phosphor-Gehalt von 3000 ppm (P/Rh-Molverhältnis 7). Eine GC-Analyse (NP-Detektor) zeigt einen Phosphabenzol-Umsatz von 96 % an. Die Ausbeute an 3-Benzyl-1-octyl-2,4,6-triphenylphosphacyclohexan betrug 80 % und die Ausbeute an 3-Benzyl-1-octyl-2,4,6-triphenylphosphacyclohexenen betrug 10 %. Die Verbindungen konnten durch GC-MS-Analyse und $^{31}P$-NMR-Spektroskopie bestätigt werden. 3-Benzyl-2,4,6-triphenylphosphacyclohexan und 3-Benzyl-2,4,6-triphenylphosphacyclohexene waren nicht nachweisbar.

Beispiel 11

Herstellung einer Rhodium/1-Octyl-2,3,5,6-tetraphenylphosphacyclohexan-Lösung

0.27 g (1.05 mmol) Rhodiumdicarbonylacetylacetonat, 3.0 g (7.5 mmol) 2,3,5,6-Tetraphenylphosphabenzol und 10 g (89 mmol) 1-Octen wurden unter Stickstoff-Inertgas in einem Schlenkrohr in 10 g Toluol gelöst. Die erhaltene Lösung wurde in einen mit Wasserstoff gespülten 100 ml Autoklaven überführt. Es wurden 5 bar Wasserstoff aufgepresst. Unter kräftigem Rühren mit einem Begasungsrührer wurde das Reaktionsgemisch auf 160 °C erhitzt. Der Wasserstoffdruck im Autoklaven wurde auf 80 bar erhöht. Das Reaktionsgemisch wurde dann 72 h bei 160 °C gerührt. Während der Reaktion wurde der Druck im Reaktor durch Nachpressen über einen Druckregler auf Druckniveau gehalten. Nach der Reaktionszeit wurde der Autoklav abgekühlt, entspannt und entleert. Die erhaltene Lösung wurde im Hochvakuum vollständig eingeengt und der verbleibende Rückstand wurde in 50 g Toluol aufgenommen. Eine Analyse der Lösung lieferte einen Rhodium-Gehalt von 285 ppm und einen Phosphor-Gehalt von 590 ppm (P/Rh-Molverhältnis 7). Eine GC-Analyse (NP-Detektor) der Reaktionslösung zeigt einen Phosphabenzol-Umsatz von 100 % an. Die Ausbeute an 1-Octyl-2,3,5,6-tetraphenylphosphacyclohexan betrug 92 % und die Ausbeute an 1-Octyl-2,3,5,6-tetraphenylphosphacyclohexenen betrug 3 %. Die Verbindungen konnten durch GC-MS-Analyse und $^{31}P$-NMR-Spektroskopie bestätigt werden. 2,3,5,6-Tetraphenylphosphacyclohexan und 2,3,5,6-Tetraphenylphosphacyclohexene waren nicht nachweisbar.

Allgemeine Versuchsbeschreibung zur Durchführung diskontinuierlicher Hydroformylierungsversuche

Variante A:

Katalysator oder Katalysatorlösung und Lösungsmittel wurden unter Stickstoff-Inertgas in einem Schlenkrohr gemischt. Die erhaltene Lösung wurde in einen mit $CO/H_2$ (1:1) gespülten 70 ml oder 100 ml fassenden Autoklaven überführt. Es wurden bei Raumtemperatur 2-5 bar $CO/H_2$ (1:1) aufgepresst. Unter kräftigem Rühren mit einem Begasungsrührer wurde das Reaktionsgemisch innerhalb von 30 min auf die gewünschte Temperatur erhitzt. Über eine Schleuse wurde dann das eingesetzte Olefin mit $CO/H_2$-Überdruck in den Autoklaven gepresst. Darauf wurde sofort mittels $CO/H_2$ (1:1) der gewünschte Reaktionsdruck eingestellt. Während der Reaktion wurde der Druck im Reaktor durch Nachpressen über einen Druckregler auf Druckniveau gehalten. Nach der Reaktionszeit wurde der Autoklav abgekühlt, entspannt und entleert. Eine Analyse des Reaktionsgemisches wurde mittels GC unter Verwendung von Korrekturfaktoren durchgeführt.

Variante B:

Katalysator oder Katalysatorlösung und Lösungsmittel wurden unter Stickstoff-Inertgas in einem Schlenkrohr gemischt. Die erhaltene Lösung wurde in einen mit $CO/H_2$ (1:1) gespülten 70 ml oder 100 ml fassenden Autoklaven überführt. Es wurden 3-5 bar $CO/H_2$ (1:1) kalt aufgepresst. Unter kräftigen Rühren mit einem Begasungsrührer wurde das Reaktionsgemisch innerhalb von 30 min auf Reaktionstemperatur erhitzt. Nach der Katalysatorpräformierung wurde der Autoklav abgekühlt und entspannt. Über eine Schleuse wurde dann das eingesetzte Olefin mit $CO/H_2$-Überdruck in den Autoklaven gepresst. Das Reaktionsgemisch wurde innerhalb von 30 min erneut auf die gewünschte Temperatur erhitzt. Darauf wurde sofort mittels $CO/H_2$ (1:1) der gewünschte Reaktionsdruck eingestellt. Während der Reaktion wurde der Druck im Reaktor durch Nachpressen über einen Druckregler auf Druckniveau gehalten. Nach der Reaktionszeit wurde der Autoklav abgekühlt, entspannt und entleert. Eine Analyse des Reaktionsgemisches wurde mittels GC unter Verwendung von Korrekturfaktoren durchgeführt.

Beispiel 12

Niederdruck-Hydroformylierung von 1-Octen

Ausgehend von 15.8 g Katalysatorlösung aus Beispiel 1, 25.4 g (226 mmol) 1-Octen (Reinheit: 95 %, Restmenge: n-Octene mit interner Doppelbindung) und 9.2 g Toluol erhielt man bei 90 °C, 10 bar $CO/H_2$ und 4 h gemäß der allgemeinen Versuchsdurchführung (Variante A) einen Umsatz von 1-Octen von 100 %. Die Ausbeute an Nonanalen betrug 97 %, die Selektivität zu n-Nonanal (n-Anteil) betrug 61 % und die Selektivität zu n-Nonanal und 2-Methyloctanal ($\alpha$-Anteil) betrug 96 %.

Beispiel 13

Niederdruck-Hydroformylierung von 1-Octen

Ausgehend von 8.8 g Katalysatorlösung aus Beispiel 2, 25.4 g (226 mmol) 1-Octen (Reinheit: 95 %, Restmenge: n-Octene mit interner Doppelbindung) und 16.2 g Toluol erhielt man bei 160 °C, 10 bar $CO/H_2$ und 4 h gemäß der allgemeinen Versuchsdurchführung (Variante A) einen Umsatz von 1-Octen von 98 %. Die Ausbeute an Nonanalen betrug 30 %, die Selektivität zu n-Nonanal (n-Anteil) betrug 48 % und die Selektivität zu n-Nonanal und 2-Methyloctanal ($\alpha$-Anteil) betrug 83 %. Die Ausbeute an Nonanolen betrug 1 %.

Beispiel 14

Niederdruck-Hydroformylierung von 1-Octen

Ausgehend von 8.8 g Katalysatorlösung aus Beispiel 2, 25.9 g (231 mmol) 1-Octen (Reinheit: 95 %, Restmenge: n-Octene mit interner Doppelbindung) und 16.2 g Toluol erhielt man bei 90 °C, 10 bar $CO/H_2$ und 4 h gemäß der allgemeinen Versuchsdurchführung (Variante A) einen Umsatz von 1-Octen von 100 %. Die Ausbeute an Nonanalen betrug 92 %, die Selektivität zu n-Nonanal (n-Anteil) betrug 65 % und die Selektivität zu n-Nonanal und 2-Methyloctanal ($\alpha$-Anteil) betrug 99 %.

Beispiel 15

Niederdruck-Hydroformylierung von 1-Octen (Rückführung)

Hydroformylierungsprodukte und Toluol im Reaktionsgemisch von Beispiel 14 wurden bei 100 °C unter Vakuum abdestilliert. Der verbleibende Rückstand wurde unter Luftausschluss in 25.0 g Toluol gelöst. Die erhaltene Lösung wurde in einen mit $CO/H_2$ (1:1) gespülten 100 ml Autoklaven überführt. Es wurden 5 bar $CO/H_2$ (1:1) kalt aufgespresst. Unter kräftigem Rühren mit einem Begasungsrührer wurde das Reaktionsgemisch innerhalb von 30 min auf 90 °C erhitzt. Über eine Schleuse wurden dann 25.3 g (225 mmol) 1-Octen mit $CO/H_2$-Überdruck in den Autoklaven gepresst Daraufhin wurde sofort mittels $CO/H_2$ (1:1) ein Reaktionsdruck von 10 bar eingestellt. Während der Reaktion wurde der Druck im Reaktor durch Nachpressen über einen Druckregler auf Druckniveau gehalten. Nach der Reaktionszeit von 4 h wurde der Autoklav abgekühlt, entspannt und entleert. Eine Analyse wurde mittels GC unter Verwendung von Korrekturfaktoren durchgeführt. Der Umsatz von 1-Octen betrug 98 %, die Ausbeute an Nonanalen betrug 93 %, die Selektivität zu n-Nonanal (n-Anteil) betrug 65 % und die Selektivität zu n-Nonanal und 2-Methyloctanal ($\alpha$-Anteil) betrug 99 %.

Beispiel 16

Niederdruck-Hydroformylierung von 2-Octen

Ausgehend von 9.2 g Katalysatorlösung aus Beispiel 1, 15.2 g (135 mmol) 2-Octen (Verhältnis cis:trans = 80:20) und 5.4 g Toluol erhielt man bei 160 °C, 10 bar $CO/H_2$ und 4 h gemäß der allgemeinen Versuchsdurchführung (Variante A) einen Umsatz von 2-Octen von 93 %. Die Ausbeute an Nonanalen betrug 64 %, die Selektivität zu n-Nonanal (n-Anteil) betrug 35 % und die Selektivität zu n-Nonanal und 2-Methyloctanal ($\alpha$-Anteil) betrug 72 %. Die Ausbeute an Nonanolen betrug 14 %, die Selektivität zu n-Nonanol (n-Anteil) betrug 39 % und die Selektivität zu n-Nonanal und 2-Methyloctanal ($\alpha$-Anteil) betrug 72 %.

Beispiel 17

Mitteldruck-Hydroformylierung von 2-Octen

Ausgehend von 15.8 g Katalysatorlösung aus Beispiel 1, 25.8 g (230 mmol) 2-Octen (Verhältnis cis:trans = 80:20) und 9.2 g Toluol erhielt man bei 160 °C, 60 bar $CO/H_2$ und 30 min gemäß der allgemeinen Versuchsdurchführung (Variante A) einen Umsatz von 2-Octen von 100 %. Die Ausbeute an Nonanalen betrug 95 %, die Selektivität zu n-Nonanal (n-Anteil) betrug 24 % und die Selektivität zu n-Nonanal und 2-Methyloctanal ($\alpha$-Anteil) betrug 63 %. Die Ausbeute an Nonanolen betrug 4 %, die Selektivität zu n-Nonanol (n-Anteil) betrug 25 % und die Selektivität zu n-Nonanal und 2-Methyloctanal ($\alpha$-Anteil) betrug 71 %.

Vergleichsbeispiel 1

Mitteldruck-Hydroformylierung von 2-Octen

Ausgehend von 7.6 mg (0.029 mmol) Rhodiumdicarbonylacetylacetonat, 0.155 g (0.59 mmol) Triphenylphosphan, 25.2 g (225 mmol) 2-Octen (Verhältnis cis:trans = 75:24) und 25.0 g Toluol erhielt man bei 160 °C, 60 bar $CO/H_2$ und 30 min gemäß der allgemeinen Versuchsdurchführung (Variante A) einen Umsatz von 2-Octen von 53 %. Die Ausbeute an Nonanalen betrug 37 %, die Selektivität zu n-Nonanal (n-Anteil) betrug 18 % und die Selektivität zu n-Nonanal und 2-Methyloctanal ($\alpha$-Anteil) betrug 79 %. Die Ausbeute an Nonanolen betrug 0 %.

Beispiel 18

Mitteldruck-Hydroformylierung von Isobuten

4.0 g Katalysatorlösung aus Beispiel 1 wurden im Ölpumpenvakuum bei ca. 80 °C bis zur Trockene eingedampft. Ausgehend vom verbleibenden Katalysatorrückstand, 5.7 g (102 mmol) Isobuten und 6.0 g Toluol erhielt man bei 160 °C, 40 bar Gesamtdruck (Eigendruck des Olefins und $CO/H_2$) und 4 h gemäß der allgemeinen Versuchsdurchführung (Variante B) einen Umsatz von Isobuten von 84 %. Die Ausbeute an 3-Methylbutanal betrug 60 % und die Ausbeute an 3-Methylbutanol betrug 21 %.

Vergleichsbeispiel 2

Mitteldruck-Hydroformylierung von Isobuten

Ausgehend von 2.1 mg (0.008 mmol) Rhodiumdicarbonylacetylacetonat, 37.7 mg (0.144 mmol) Triphenylphosphan, 5.0 g (89 mmol) Isobuten und 6.0 g Toluol erhielt man bei 160 °C, 40 bar Gesamtdruck (Eigendruck des Olefins und $CO/H_2$) und 4 h gemäß der allgemeinen Versuchsdurchführung (Variante B) einen Umsatz von Isobuten von 12 %. Die Ausbeute an 3-Methylbutanal betrug 11 % und die Ausbeute an 3-Methylbutanol betrug 0 %.

Beispiel 19

Mitteldruck-Hydroformylierung von Dimerbuten

15.8 g Katalysatorlösung aus Beispiel 1 wurden im Ölpumpenvakuum bei ca. 80 °C bis zur Trockene eingedampft. Ausgehend vom verbleibenden Katalysatorrückstand, 25.8 g (230 mmol) Dimerbuten (erhalten durch Nickel-katalysierte Dimerisierung von n-Butenen, Verzweigungsgrad 1.06) und 25.0 g Texanol® erhielt man bei 160 °C und 60 bar $CO/H_2$ gemäß der allgemeinen Versuchsdurchführung (Variante A) die in Tabelle 1 aufgeführten Ergebnisse.

Beispiel 20

Mitteldruck-Hydroformylierung von Dimerbuten

16.0 g Katalysatorlösung aus Beispiel 1 wurden im Ölpumpenvakuum bei ca. 80 °C bis zur Trockene eingedampft. Ausgehend vom verbleibenden Katalysatorrückstand, 24.7 g (220 mmol) Dimerbuten (erhalten durch Nickel-katalysierte Dimerisierung von n-Butenen, Verzweigungsgrad 1.06) und 24.7 g Texanol® erhielt man bei 140 °C und 60 bar $CO/H_2$ gemäß der allgemeinen Versuchsdurchführung (Variante A) die in Tabelle 1 aufgeführten Ergebnisse.

Beispiel 21

Mitteldruck-Hydroformylierung von Dimerbuten

16.0 g Katalysatorlösung aus Beispiel 1 wurden im Ölpumpenvakuum bei ca. 80 °C bis zur Trockene eingedampft. Ausgehend vom verbleibenden Katalysatorrückstand, 21.6 g (192 mmol) Dimerbuten (erhalten durch Nickel-katalysierte Dimerisierung von n-Butenen, Verzweigungsgrad 1.06) und 24.8 g Texanol® erhielt man bei 120 °C und 60 bar $CO/H_2$ gemäß der allgemeinen Versuchsdurchführung (Variante A) die in Tabelle 1 aufgeführten Ergebnisse.

Beispiel 22

Niederdruck-Hydroformylierung von Dimerbuten

16.0 g Katalysatorlösung aus Beispiel 1 wurden im Ölpumpenvakuum bei ca. 80 °C bis zur Trockene eingedampft. Ausgehend vom verbleibenden Katalysatorrückstand, 24.4 g (217 mmol) Dimerbuten (erhalten durch Nickel-katalysierte Dimerisierung von n-Butenen, Verzweigungsgrad 1.06) und 24.8 g Texanol® erhielt man bei 160 °C und 20 bar $CO/H_2$ gemäß der allgemeinen Versuchsdurchführung (Variante A) die in Tabelle 1 aufgeführten Ergebnisse.

Die folgenden Vergleichsbeispiele mit nicht erfindungsgemäßen Triphenylphosphan und nicht erfindungsgemäßen sterisch anspruchsvollen Trialkylphosphanen als Cokatalysatoren zeigen eine deutlich niedrigere Aktivität, insbesondere in der Hydroformylierung von internen verzweigten Olefinen, als die erfindungsgemäß verwendeten Phosphacyclohexancokatalysatoren:

Vergleichsbeispiel 3

Mitteldruck-Hydroformylierung von Dimerbuten

Ausgehend von 7.5 mg (0.029 mmol) Rhodiumdicarbonylacetylacetonat, 0.157 g (0.60 mmol) Triphenylphosphan, 25.1 g (224 mmol) Dimerbuten (erhalten durch Nickel-katalysierte Dimerisierung von n-Butenen, Verzweigungsgrad 1.06) und 25.0 g Texanol® erhielt man bei 160 °C, 60 bar $CO/H_2$ gemäß der allgemeinen Versuchsdurchführung (Variante A) die in Tabelle 2 aufgeführten Ergebnisse.

Vergleichsbeispiel 4

Mitteldruck-Hydroformylierung von Dimerbuten

Ausgehend von 7.8 mg Rhodiumdicarbonylacetylacetonat (0.030 mmol), 83.0 mg (0.296 mmol) Tricyclohexylphosphan, 25.0 g (223 mmol) Dimerbuten (erhalten durch Nickel-katalysierte Dimerisierung von n-Butenen, Verzweigungsgrad 1.06) und 25.0 g Texanol® erhielt man bei 160 °C und 60 bar $CO/H_2$ gemäß der allgemeinen Versuchsdurchführung (Variante A) die in Tabelle 2 aufgeführten Ergebnisse.

Vergleichsbeispiel 5

Mitteldruck-Hydroformylierung von Dimerbuten

Ausgehend von 8.1 mg Rhodiumdicarbonylacetylacetonat (0.031 mmol), 42.9 mg (0.246 mmol) n-Butyldi-n-propylphosphan, 24.6 g (219 mmol) Dimerbuten (erhalten durch Nickel-katalysierte Dimerisierung von n-Butenen, Verzweigungsgrad 1.06) und 24.7 g Texanol® erhielt man bei 160 °C und 60 bar $CO/H_2$ gemäß der allgemeinen Versuchsdurchführung (Variante A) die in Tabelle 2 aufgeführten Ergebnisse.

Vergleichsbeispiel 6

Mitteldruck-Hydroformylierung von Dimerbuten

Ausgehend von 8.2 mg Rhodiumdicarbonylacetylacetonat (0.032 mmol), 46.0 mg (0.227 mmol) Tri-tert-butylphosphan, 24.6 g (219 mmol) Dimerbuten (erhalten durch Nickel-katalysierte Dimerisierung von n-Butenen, Verzweigungsgrad 1.06) und 24.7 g Texanol® erhielt man bei 160 °C und 60 bar $CO/H_2$ gemäß der allgemeinen Versuchsdurchführung (Variante A) die in Tabelle 2 aufgeführten Ergebnisse.

Vergleichsbeispiel 7

Mitteldruck-Hydroformylierung von Dimerbuten

Ausgehend von 7.8 mg Rhodiumdicarbonylacetylacetonat (0.030 mmol), 69.2 mg (0.223 mmol) 9-Dodecyl-9-phosphabicyclo[3.3.1]nonan, 26.8 g (239 mmol) Dimerbuten (erhalten durch Nickel-katalysierte Dimerisierung von n-Butenen, Verzweigungsgrad 1.06) und 25.0 g Texanol® erhielt man bei 160 °C und 60 bar $CO/H_2$ gemäß der allgemeinen Versuchsdurchführung (Variante A) die in Tabelle 2 aufgeführten Ergebnisse.

Beispiel 23

Mitteldruck-Hydroformylierung von 2-Octen

Ausgehend von 4.3 g Katalysatorlösung aus Beispiel 6, 25.4 g (226 mmol) 2-Octen (Verhältnis cis:trans = 80: 20) und 20.7 g Toluol erhielt man bei 160 °C, 60 bar $CO/H_2$ und 4 h gemäß der allgemeinen Versuchsdurchführung (Variante A) einen Umsatz von 2-Octen von 100 %. Die Ausbeute an Nonanalen betrug 98 %, die Selektivität zu n-Nonanal (n-Anteil) betrug 4 %. Die Selektivität zu n-Nonanal und 2-Methyloctanal ($\alpha$-Anteil) betrug 57 %.

Beispiel 24

Mitteldruck-Hydroformylierung von Dimerbuten

8.8 g Katalysatorlösung aus Beispiel 2 wurden im Ölpumpenvakuum bei ca. 80 °C bis zur Trockene eingedampft. Ausgehend vom verbleibenden Katalysatorrückstand, 25.6 g (228 mmol) Dimerbuten (erhalten durch Nickel-katalysierte Dimerisierung von n-Butenen, Verzweigungsgrad 1.06) und 25.0 g Texanol® erhielt man bei 160 °C und 60 bar $CO/H_2$ gemäß der allgemeinen Versuchsdurchführung (Variante A) einen Umsatz von Dimerbuten von 71 % nach 1 h und 94 % nach 4 h. Die Ausbeute an Nonanalen betrug 65 % nach 1 h und 75 % nach 4 h. Die Ausbeute an Nonanolen betrug 3 % nach 1 h und 15 % nach 4 h.

Beispiel 25

Mitteldruck-Hydroformylierung von 1-Octen

Ausgehend von 5.9 g Katalysatorlösung aus Beispiel 8, 25.1 g (224 mmol) 1-Octen (Reinheit: 95 %, Restmenge: n-Octene mit interner Doppelbindung) und 19.1 g Toluol erhielt man bei 100 °C, 60 bar $CO/H_2$ und 2 h gemäß der allgemeinen Versuchsdurchführung (Variante A) einen Umsatz von 1-Octen von 100 %. Die Ausbeute an Nonanalen betrug 99 %, die Selektivität zu n-Nonanal (n-Anteil) betrug 62 % und die Selektivität zu n-Nonanal und 2-Methyloctanal ($\alpha$-Anteil) betrug 96 %.

Beispiel 26

Mitteldruck-Hydroformylierung von Dimerbuten

6.0 g Katalysatorlösung aus Beispiel 8 wurden im Ölpumpenvakuum bei ca. 100 °C bis zur Trockene eingedampft. Ausgehend vom verbleibenden Katalysatorrückstand, 24.0 g (214 mmol) Dimerbuten (erhalten durch Nickel-katalysierte Dimerisierung von n-Butenen, Verzweigungsgrad 1.06) und 25.0 g Texanol® erhielt man bei 160 °C und 60 bar $CO/H_2$ gemäß der allgemeinen Versuchsdurchführung (Variante A) einen Umsatz von Dimerbuten von 89 % nach 1 h und 97 % nach 4 h. Die Ausbeute an Nonanalen betrug 78 % nach 1 h und 61 % nach 4 h. Die Ausbeute an Nonanolen betrug 8 % nach 1 h und 28 % nach 4 h.

Beispiel 27

Mitteldruck-Hydroformylierung von Dimerbuten

6.0 g Katalysatorlösung aus Beispiel 8 wurden im Ölpumpenvakuum bei ca. 100 °C bis zur Trockene eingedampft. Ausgehend vom verbleibenden Katalysatorrückstand, 24.0 g (214 mmol) Dimerbuten (erhalten durch Nickel-katalysierte Dimerisierung von n-Butenen, Verzweigungsgrad 1.06) und 23.8 g Texanol® erhielt man bei 160 °C, 60 bar $CO/H_2$ und 24 h gemäß der allgemeinen Versuchsdurchführung (Variante A) einen Umsatz von Dimerbuten von 97 %. Die Ausbeute an Nonanalen betrug 30 % und die Ausbeute an Nonanolen betrug 45 %.

Beispiel 28

Mitteldruck-Hydroformylierung von Dimerbuten (Rückführung) Hydroformylierungsprodukte im Reaktionsgemisch von Beispiel 27 wurden bei 100 °C unter Vakuum abdestilliert. Zurück blieb eine klare, dunkelgelbe Lösung, bestehend aus Aktivkatalysator, Texanol® und reaktionseigenen Hochsiedern. Hierzu wurden unter Luftausschluss 25 g Texanol® gegeben. Die erhaltene Lösung wurde in einen mit $CO/H_2$ (1:1) gespülten 100 ml Autoklaven überführt. Es wurden 5 bar $CO/H_2$ (1:1) kalt aufgespresst. Unter kräftigem Rühren mit einem Begasungsrührer wurde das Reaktionsgemisch innerhalb von 30 min auf 160 °C erhitzt. Über eine Schleuse wurden dann 24.0 g (214 mmol) Dimerbuten (erhalten durch Nickel-katalysierte Dimerisierung von n-Butenen, Verzweigungsgrad 1.06) mit $CO/H_2$-Überdruck in den Autoklaven gepresst Daraufhin wurde sofort mittels $CO/H_2$ (1:1) ein Reaktionsdruck von 60 bar eingestellt. Während der Reaktion wurde der Druck im Reaktor durch Nachpressen über einen Druckregler auf Druckniveau gehalten. Nach der Reaktionszeit von 4 h wurde der Autoklav abgekühlt, entspannt und entleert. Eine Analyse wurde mittels GC unter Verwendung von Korrekturfaktoren durchgeführt. Der Umsatz von Dimerbuten betrug 77 % nach 1 h und 96 % nach 4 h. Die Ausbeute an Nonanalen betrug 60 % nach 1 h und 60 % nach 4 h. Die Ausbeute an Nonanolen betrug 19 % nach 1 h und 32 % nach 4 h.

Beispiel 29

Mitteldruck-Hydroformylierung von Dimerbuten

15.0 g Katalysatorlösung aus Beispiel 5 wurden im Ölpumpenvakuum bei ca. 80 °C bis zur Trockene eingedampft. Ausgehend vom verbleibenden Katalysatorrückstand, 25.4 g (226 mmol) Dimerbuten (erhalten durch Nickel-katalysierte Dimerisierung von n-Butenen, Verzweigungsgrad 1.06) und 25.0 g Texanol® erhielt man bei 160 °C und 60 bar $CO/H_2$ gemäß der allgemeinen Versuchsdurchführung (Variante A) einen Umsatz von Dimerbuten von 52 % nach 1 h und 82 % nach 4 h. Die Ausbeute an Nonanalen betrug 48 % nach 1 h und 78 % nach 4 h. Die Ausbeute an Nonanolen betrug 3 % nach 1 h und 5 % nach 4 h.

Beispiel 30

Mitteldruck-Hydroformylierung von Dimerbuten

2.8 g Katalysatorlösung aus Beispiel 5 wurden im Ölpumpenvakuum bei ca. 80 °C bis zur Trockene eingedampft. Ausgehend vom verbleibenden Katalysatorrückstand, 7.4 mg (0.029 mmol) Rhodiumdicarbonylacetylacetonat, 25.2 g (225 mmol) Dimerbuten (erhalten durch Nickel-katalysierte Dimerisierung von n-Butenen, Verzweigungsgrad 1.06) und 25.0 g Texanol® erhielt man bei 160 °C und 60 bar $CO/H_2$ gemäß der allgemeinen Versuchsdurchführung (Variante A) einen Umsatz von Dimerbuten von 77 % nach 1 h und 87 % nach 4 h. Die Ausbeute an Nonanalen betrug 58 % nach 1 h und 44 % nach 4 h. Die Ausbeute an Nonanolen betrug 15 % nach 1 h und 29 % nach 4 h.

Beispiel 31

Mitteldruck-Hydroformylierung von Dimerbuten

4.2 g Katalysatorlösung aus Beispiel 9 wurden im Ölpumpenvakuum bei ca. 80 °C bis zur Trockene eingedampft. Ausgehend vom verbleibenden Katalysatorrückstand, 23.7 g (225 mmol) Dimerbuten (erhalten durch Nickel-katalysierte Dimerisierung von n-Butenen, Verzweigungsgrad 1.06) und 25.0 g Texanol® erhielt man bei 160 °C und 60 bar $CO/H_2$ gemäß der allgemeinen Versuchsdurchführung (Variante A) einen Umsatz von Dimerbuten von 68 % nach 1 h und 92 % nach 4 h. Die Ausbeute an Nonanalen betrug 65 % nach 1 h und 78 % nach 4 h. Die Ausbeute an Nonanolen betrug 2 % nach 1 h und 13 % nach 4 h.

Beispiel 32

Niederdruck-Hydroformylierung von 1-Octen

Ausgehend von 4.2 g Katalysatorlösung aus Beispiel 9, 25.6 g (226 mmol) 1-Octen (Reinheit: 97 %, Restmenge: n-Octene mit interner Doppelbindung) und 25.0 g Toluol erhielt man bei 90 °C, 10 bar $CO/H_2$ und 3 h gemäß der allgemeinen Versuchsdurchführung (Variante A) einen Umsatz von 1-Octen von 100 %. Die Ausbeute an Nonanalen betrug 96 %, die Selektivität zu n-Nonanal (n-Anteil) betrug 62 % und die Selektivität zu n-Nonanal und 2-Methyloctanal ($\alpha$-Anteil) betrug 98 %.

Beispiel 33

Mitteldruck-Hydroformylierung von Dimerbuten

4.2 g Katalysatorlösung aus Beispiel 11 wurden im Ölpumpenvakuum bei ca. 80 °C bis zur Trockene eingedampft. Ausgehend vom verbleibenden Katalysatorrückstand, 23.8 g (212 mmol) Dimerbuten (erhalten durch Nickel-katalysierte Dimerisierung von n-Butenen, Verzweigungsgrad 1.06) und 25.0 g Texanol® erhielt man bei 160 °C und 60 bar $CO/H_2$ gemäß der allgemeinen Versuchsdurchführung (Variante A) einen Umsatz von Dimerbuten von 54 % nach 1 h und 83 % nach 4 h. Die Ausbeute an Nonanalen betrug 53 % nach 1 h und 75 % nach 4 h. Die Ausbeute an Nonanolen betrug 1 % nach 1 h und 7 % nach 4 h.

Beispiel 34

Niederdruck-Hydroformylierung von 1-Octen

Ausgehend von 7.8 mg (0.030 mmol) Rhodiumdicarbonylacetylacetonat, 0.145 g (0.57 mmol) 1-Cyclohexyl-2,2,6,6-tetramethylphosphacyclohexan-4-on, 24.8 g (221 mmol) 1-Octen (Reinheit: 95 %, Restmenge: n-Octene mit interner Doppelbindung) und 24.8 g Toluol erhielt man bei 90 °C, 10 bar $CO/H_2$ und 4 h gemäß der allgemeinen Versuchsdurchführung (Variante A) einen Umsatz von 1-Octen von 91 %. Die Ausbeute an Nonanalen betrug 84 %, die Selektivität zu n-Nonanal (n-Anteil) betrug 55 % und die Selektivität zu n-Nonanal und 2-Methyloctanal ($\alpha$-Anteil) betrug 100 %.

Beispiel 35

Mitteldruck-Hydroformylierung von Dimerbuten

Ausgehend von 7.4 mg (0.029 mmol) Rhodiumdicarbonylacetylacetonat, 0.080 g (0.31 mmol) 1-Cyclohexyl-2,2,6,6-tetramethylphosphacyclohexan-4-on, 20.7 g (184 mmol) Dimerbuten (erhalten durch Nickel-katalysierte Dimerisierung von n-Butenen, Verzweigungsgrad 1.06) und 24.7 g Texanol® erhielt man bei 160 °C, 60 bar $CO/H_2$ und 4 h gemäß der allgemeinen Versuchsdurchführung (Variante A) einen Dimerbuten-Umsatz von 72 %. Die Ausbeute an Nonanalen betrug 69 % und die Ausbeute an Nonanolen betrug 3 %.

Beispiel 36

Niederdruck-Hydroformylierung von 1-Octen

Ausgehend von 7.4 mg (0.029 mmol) Rhodiumdicarbonylacetylacetonat, 0.127 g (0.33 mmol) 1-Octyl-2,6-diphenylphosphacyclohexan-4-on, 25.0 g (223 mmol) 1-Octen (Reinheit: 95 %, Restmenge: n-Octene mit interner Doppelbindung) und 24.8 g Toluol erhielt man bei 90 °C, 10 bar $CO/H_2$ und 4 h gemäß der allgemeinen Versuchsdurchführung (Variante A, jedoch 180 min Katalysatorpräformierung) einen Umsatz von 1-Octen von 75 %. Die Ausbeute an Nonanalen betrug 71 %, die Selektivität zu n-Nonanal (n-Anteil) betrug 66 % und die Selektivität zu n-Nonanal und 2-Methyloctanal ($\alpha$-Anteil) betrug 100 %.

Beispiel 37

Mitteldruck-Hydroformylierung von Dimerbuten

Ausgehend von 7.7 mg (0.030 mmol) Rhodiumdicarbonylacetylacetonat, 0.112 g (0.29 mmol) 1-Octyl-2,6-diphenylphosphacyclohexan-4-on, 20.7 g (184 mmol) Dimerbuten (erhalten durch Nickel-katalysierte Dimerisierung von n-Butenen, Verzweigungsgrad 1.06) und 24.7 g Texanol® erhielt man bei 160 °C, 60 bar $CO/H_2$ und 4 h gemäß der allgemeinen Versuchsdurchführung (Variante A) einen Dimerbuten-Umsatz von 79 %. Die Ausbeute an Nonanalen betrug 73 % und die Ausbeute an Nonanolen betrug 4 %.

Beispiel 38

Niederdruck-Hydroformylierung von 1-Octen

Ausgehend von 9,8 mg (0,038 mmol) Rhodiumdicarbonylacetylacetonat, 0,162 g (0,65 mmol) 1-Phenyl-2,2,6,6-tetramethylphosphacyclohexan-4-on, 25,7 g (229 mmol) 1-Octen (Reinheit: 95 %, Restmenge: n-Octene mit interner Doppelbindung) und 25 g Toluol erhielt man bei 90°C, 10 bar $CO/H_2$ und 4 h gemäß der allgemeinen Versuchsdurchführung (Variante A) einen Umsatz von 1-Octen von 100 %. Die Ausbeute an Nonanalen betrug 97 %, die Selektivität zu n-Nonanal (n-Anteil) betrug 58 % und die Selektivität zu n-Nonanal und 2-Methyloctanal ($\alpha$-Anteil) betrug 95 %.

Beispiel 39

Kontinuierliche Niederdruck-Hydroformylierung von Propen

Zur kontinuierlichen Niederdruck-Hydroformylierung wurde eine Miniplant-Anlage gemäß Figur 1 eingesetzt.
In einen Druckreaktor werden Synthesegas und ein Olefinzulauf geführt. Der Austrag des Druckreaktors wird zunächst in einen Druckabscheider, nachfolgend in einen Flashbehälter geführt. Der flüssige Austrag des Flashbehälters wird dann in einen Wischblattverdampfer geführt. Im Wischblattverdampfer fällt das Produkt als Destillat an. Des Weiteren wird dem Wischblattverdampfer ein flüssiger Katalysator-haltiger Austrag entnommen und in den Reaktor zurückgeführt. Am Druckabscheider und Flashbehälter wird über Kopf ein Abgasstrom (Sprudelgas) entnommen und abgeleitet.
In der Miniplant-Anlage wurde Propen mit dem Katalysatorsystem Rhodium/2,6-Bis(2,4-dimethylphenyl)-4-phenyl-1-propylphospacyclohexan kontinuierlich hydroformyliert. Der Cokatalysator wurde in situ aus 2,6-Bis(2,4-dimethylphenyl)-4-phenylphosphabenzol durch kontinuierliches Betreiben der Rhodium-katalysierten Hydroformylierung wie nachstehend beschrieben bei einer Reaktortemperaturen von 84-89 °C und einem Reaktordruck von 20 bar hergestellt. In einem Reaktor wurden stündlich 328 g Propen in Gegenwart von 80-120 ppm Rhodium und in situ erzeugtem 2,6-Bis(2,4-dimethylphenyl)-4-phenyl-1-propylphospacyclohexan als Cokatalysator [P/Rh-Verhältnis = 11 (mol:mol)] - die Mengenangaben sind auf die Menge des Reaktionsgemisches bezogen - und 1 kg Texanol® als Lösungsmittel bei einer Reaktorinnentemperatur von 82-100 °C und einem Gesamtdruck von 20 bar hydroformyliert. Dem Reaktor wurde ein $CO/H_2$(1:1)-Gasgemisch in solcher Menge zugeführt, dass eine konstante Abgasmenge über einen Druckabscheider von ca. 120 Nl/h resultierte. Der flüssige Reaktoraustrag wurde in einem Flashbehälter entspannt und über einen Wischblattverdampfer in Butyraldehyd-Destillat und das katalysatorhaltige Sumpfprodukt aufgetrennt. Das katalysatorhaltige Produkt wurde kontinuierlich in den Reaktor zurückgeführt. Die erzielten Umsätze, Selektivitäten und Raum-Zeit-Ausbeuten sind in Tabelle 3 und in Figur 2 dokumentiert. Darin bedeuten A die Aldehydausbeute, B die Bilanztage, RZ die Raum-Zeit-Ausbeute. Die Kästchen beziehen sich auf die Aldehydausbeute, die Rauten auf die Raum-Zeit-Ausbeute.

Tabelle 3: Umsätze, Selektivitäten und Raum-Zeit-Ausbeuten bei der kontinuierlichen Hydroformylierung von Propen (20 bar Gesamtdruck, L/Rh = 11, ca. 100 ppm Rh) ohne Berücksichtigung der Hochsiederbildung

| Temperatur | [°C] | 90 | 100 |
|---|---|---|---|
| Propen (Umsatz) | [%] | 75.4 | 92.4 |
| Butyraldehyd (Selektivität) | [%] | 99.8 | 99.8 |
| n-Anteil (Selektivität) | [%] | 58.7 | 58.2 |
| Propan (Selektivität) | [%] | 0.1 | 0.1 |
| Butanole (Selektivität) | [%] | 0.1 | 0.1 |
| Raum-Zeit-Ausbeute | [g/l·h] | 247 | 304 |

Vergleichsbeispiel 8

Kontinuierliche Niederdruck-Hydroformylierung von Propen

In der Miniplant-Anlage gemäß Beispiel 39 wurde Propen mit dem Katalysatorsystem Rhodium/Triphenylphosphan hydroformyliert. In einem Reaktor wurden stündlich 328 g Propen in Gegenwart von 100-120 ppm Rhodium und 22 g Triphenylphosphan als Cokatalysator [P/Rh-Verhältnis = 20 (mol:mol)] - die Mengenangaben sind auf die Menge des Reaktionsgemisches bezogen - und 1 kg Texanol® als Lösungsmittel bei einer Reaktorinnentemperatur von 100 °C und einem Gesamtdruck von 20 bar hydroformyliert. Dem Reaktor wurde ein $CO/H_2$(1:1)-Gasgemisch in einer Menge zugeführt, so dass eine konstante Abgasmenge über einen Druckabscheider von ca. 120 Nl/h resultiert. Der flüssige Reaktoraustrag wurde in einem Flashbehälter entspannt und über einen Wischblattverdampfer in Butyraldehyd-Destillat und das katalysatorhaltige Sumpfprodukt aufgetrennt. Das katalysatorhaltige Produkt wurde kontinuierlich in den Reaktor zurückgeführt. Die erzielten Umsätze, Selektivitäten und Raum-Zeit-Ausbeuten sind in Tabelle 4 dokumentiert.

Tabelle 4: Umsätze, Selektivitäten und Raum-Zeit-Ausbeuten bei der kontinuierlichen Hydroformylierung von Propen (20 bar Gesamtdruck, L/Rh = 20, ca. 100 ppm Rh) ohne Berücksichtigung der Hochsiederbildung

| Temperatur | [°C] | 100 |
|---|---|---|
| Propen (Umsatz) | [%] | 71.4 |

(fortgesetzt)

| Temperatur | [°C] | 100 |
|---|---|---|
| Butyraldehyd (Selektivität) | [%] | 97.9 |
| n-Anteil (Selektivität) | [%] | 68.8 |
| Propan (Selektivität) | [%] | 0.8 |
| Butanole (Selektivität) | [%] | 1.3 |
| Raum-Zeit-Ausbeute | [g/l·h] | 231 |

Tabelle 1: Hydroformylierung von Dimerbuten (erhalten durch Nickel-katalysierte Dimerisierung von n-Butenen, Verzweigungsgrad 1.06) mit Phosphacyclohexanen als Cokatalysatoren

| Beispiel | T [°C] | p [bar] | Reaktionszeit [h] | Dimerbuten (Umsatz) [%] | Nonanale (Ausbeute) [%] | Nonanole (Ausbeute) [%] [1/min] | TOF* (Olefin) [1/h] |
|---|---|---|---|---|---|---|---|
| 19 | 160 | 60 | 1 | 89 | 78 | 8 | 6350 |
| | | | 4 | 97 | 61 | 28 | |
| 20 | 140 | 60 | 1 | 66 | 65 | 1 | 4920 |
| | | | 4 | 91 | 86 | 5 | |
| 21 | 120 | 60 | 1 | 47 | 47 | 0 | 3720 |
| | | | 4 | 68 | 67 | 1 | |
| 22 | 160 | 20 | 1 | 60 | 54 | 6 | 4440 |
| | | | 4 | 82 | 59 | 21 | |
| 31 | 160 | 60 | 1 | 68 | 65 | 2 | 4860 |
| | | | 4 | 92 | 78 | 13 | |

*

TOF (turnover frequency) = Produkt [mol] / (Katalysator [mol] × Zeit [h])

Tabelle 2: Hydroformylierung von Dimerbuten (erhalten durch Nickel-katalysierte Dimerisierung von n-Butenen, Verzweigungsgrad 1.06) mit Triphenylphosphan und Trialkylphosphanen als Cokatalysatoren

| Vergleichsbeispiel | T [°C] | p [bar] | Reaktionszeit [h] | Dimerbuten (Umsatz) [%] | Nonanale (Ausbeute) [%] | Nonanole (Ausbeute) [%] | TOF* (Olefin) [1/h] |
|---|---|---|---|---|---|---|---|
| 3 | 160 | 60 | 1 | 4 | 3 | 0 | 292 |
| | | | 4 | 9 | 4 | 0 | |
| 4 | 160 | 60 | 1 | 51 | 50 | 1 | 3780 |
| | | | 4 | 70 | 66 | 5 | |
| 5 | 160 | 60 | 1 | 58 | 57 | 1 | 4020 |
| | | | 4 | 79 | 73 | 6 | |
| 6 | 160 | 60 | 1 | 63 | 57 | 7 | 4320 |
| | | | 4 | 91 | 58 | 33 | |
| 7 | 160 | 60 | 1 | 52 | 47 | 0 | 4080 |
| | | | 4 | 71 | 68 | 3 | |

* TOF (turnover frequency) = Produkt [mol] / (Katalysator [mol] × Zeit [h])

Beispiel 40

Herstellung einer Rhodium/2,4,6-Tris(4-methylphenyl)-1-octyl-phosphacyclohexan-Lösung

0.05 g (0.19 mmol) Rhodiumdicarbonylacetylacetonat, 0.5 g (1.4 mmol) 2,4,6-Tris(4-methylphenyl)phosphabenzol und 15 g (134 mmol) 1-Octen wurden unter Stickstoff-Inertgas in einem Schlenkrohr in 10 g Toluol gelöst. Die erhaltene Lösung wurde in einen mit Wasserstoff gespülten 100 ml Autoklaven überführt. Es wurden 5 bar Wasserstoff aufgepresst. Unter kräftigem Rühren mit einem Begasungsrührer wurde das Reaktionsgemisch auf 160 °C erhitzt. Der Wasserstoffdruck im Autoklaven wurde auf 80 bar erhöht. Das Reaktionsgemisch wurde dann 72 h bei 160 °C gerührt. Während der Reaktion wurde der Druck im Reaktor durch Nachpressen über einen Druckregler auf Druckniveau gehalten. Die Reaktionslösung wurde auf Raumtemperatur abgekühlt und als solche weiter verwendet. Eine Analyse der Lösung lieferte einen Rhodium-Gehalt von 680 ppm und einen Phosphor-Gehalt von 1500 ppm (P/Rh-Molverhältnis 7). Eine GC-Analyse (NP-Detektor) zeigt einen Phosphabenzol-Umsatz von 97 % an. Die Ausbeute an 2,4,6-Tris(4-methylphenyl)-1-octylphosphacyclohexan betrug 97 %. Das Produkt konnte zusätzlich durch $^{31}P$-NMR-Spektroskopie bestätigt werden.

Beispiel 41

Herstellung einer Rhodium/2,6-Bis(4-tert-butylphenyl)-1-octyl-4-phenylphosphacyclohexan-Lösung

0.17 g (0.66 mmol) Rhodiumdicarbonylacetylacetonat, 2.0 g (4.6 mmol) 2,6-Bis(4-tert-butylphenyl)-4-phenylphosphabenzol und 20 g (178 mmol) 1-Octen wurden unter Stickstoff-Inertgas in einem Schlenkrohr in 10 g Toluol gelöst. Die erhaltene Lösung wurde in einen mit Wasserstoff gespülten 100 ml Autoklaven überführt. Es wurden 5 bar Wasserstoff aufgepresst. Unter kräftigem Rühren mit einem Begasungsrührer wurde das Reaktionsgemisch auf 160 °C erhitzt. Der Wasserstoffdruck im Autoklaven wurde auf 80 bar erhöht. Das Reaktionsgemisch wurde dann 72 h bei 160 °C gerührt. Während der Reaktion wurde der Druck im Reaktor durch Nachpressen über einen Druckregler auf Druckniveau gehalten. Nach Abkühlen auf Raumtemperatur wurden zur Vervollständigung der Reaktion erneut 20 ml 1-Octen zugesetzt. Die Reaktion wurde für weitere 24 h bei 160 °C und 80 bar Wasserstoffdruck gemäß dem zuvor beschriebenen Verfahren betrieben. Die Reaktionslösung wurde auf Raumtemperatur abgekühlt, im Hochvakuum auf ca. ein Viertel des ursprünglichen Volumens eingeengt und anschließend mit 20 ml Toluol verdünnt. Eine Analyse der Lösung lieferte einen Rhodium-Gehalt von 2100 ppm und einen Phosphor-Gehalt von 4300 ppm (P/Rh-Molverhältnis 7). Eine GC-Analyse (NP-Detektor) zeigt einen Phosphabenzol-Umsatz von 95 % an. Die Ausbeute an 2,6-Bis(4-tert-butylphenyl)-1-octyl-4-phenylphosphacyclohexan betrug 95 %. Das Produkt konnte zusätzlich durch $^{31}P$-NMR-Spektroskopie bestätigt werden.

Beispiel 42

Herstellung einer Rhodium/2,6-Bis(2,4-dimethylphenyl)-1-octyl-4-phenylphosphacyclohexan-Lösung

0.88 g (3.4 mmol) Rhodiumdicarbonylacetylacetonat, 13 g (34 mmol) 2,6-Bis(2,4-dimethylphenyl)-4-phenylphosphabenzol und 85.8 g (765 mmol) 1-Octen wurden unter Stickstoff-Inertgas in einem Schlenkrohr in 50 ml Xylol gelöst. Die erhaltene Lösung wurde in einen mit Wasserstoff gespülten 300 ml Autoklaven überführt. Es wurden 10 bar Wasserstoff aufgepresst. Unter kräftigem Rühren mit einem Begasungsrührer wurde das Reaktionsgemisch auf 160 °C erhitzt. Der Wasserstoffdruck im Autoklaven wurde auf 80 bar erhöht. Das Reaktionsgemisch wurde dann 72 h bei 160 °C gerührt. Während der Reaktion wurde der Druck im Reaktor durch Nachpressen über einen Druckregler auf Druckniveau gehalten. Die Reaktionslösung wurde auf Raumtemperatur abgekühlt und als solche weiter verwendet. Eine Analyse der Lösung lieferte einen Rhodium-Gehalt von 2400 ppm und einen Phosphor-Gehalt von 7200 ppm (P/Rh-Molverhältnis 10). Eine GC-Analyse (NP-Detektor) zeigt einen Phosphabenzol-Umsatz von 97 % an. Die Ausbeute an 2,6-Bis(2,4-dimethylphenyl)-1-octyl-4-phenylphosphacyclohexanen und -hexenen betrug 95 % und die Ausbeute an 2,6-Bis(2,4-dimethylphenyl)-4-phenylphosphacyclohexan betrug 0.3 %. Die Verbindungen konnten durch GC-MS-Analyse und $^{31}P$-NMR-Spektroskopie bestätigt werden.

Beispiel 43

Herstellung einer Rhodium/2,6-Bis(2,4-dimethylphenyl)-1-octyl-4-phenylphosphacyclohexan-Lösung

0.90 g (3.5 mmol) Rhodiumdicarbonylacetylacetonat, 10 g (26 mmol) 2,6-Bis(2,4-dimethylphenyl)-4-phenylphosphabenzol und 85.8 g (765 mmol) 1-Octen wurden unter Stickstoff-Inertgas in einem Schlenkrohr in 50 ml Toluol gelöst. Die erhaltene Lösung wurde in einen mit Wasserstoff gespülten 300 ml Autoklaven überführt. Es wurden 10 bar

Wasserstoff aufgepresst. Unter kräftigem Rühren mit einem Begasungsrührer wurde das Reaktionsgemisch auf 160 °C erhitzt. Der Wasserstoffdruck im Autoklaven wurde auf 80 bar erhöht. Das Reaktionsgemisch wurde dann 72 h bei 160 °C gerührt.

Während der Reaktion wurde der Druck im Reaktor durch Nachpressen über einen Druckregler auf Druckniveau gehalten. Nach Abkühlen auf Raumtemperatur wurde die erhaltene Lösung im Hochvakuum vollständig eingeengt und dann in 70 ml Toluol aufgenommen. Eine Analyse der Lösung lieferte einen Rhodium-Gehalt von 3600 ppm und einen Phosphor-Gehalt von 8400 ppm (P/Rh-Molverhältnis 8). Eine GC-Analyse (NP-Detektor) zeigt einen Phosphabenzol-Umsatz von 96 % an. Die Ausbeute an 2,6-Bis(2,4-dimethylphenyl)-1-octyl-4-phenylphosphacyclohexan betrug 88 %, die Ausbeute an 2,6-Bis(2,4-dimethylphenyl)-1-octyl-4-phenylphosphacyclohexenen betrug 6 % und die Ausbeute an 2,6-Bis(2,4-dimethylphenyl)-4-phenylphosphacyclohexan betrug 0.2 %. Die Verbindungen konnten durch GC-MS-Analyse und $^{31}$P-NMR-Spektroskopie bestätigt werden.

Beispiel 44

Herstellung einer Rhodium/4-Phenyl-2,6-bis(2-naphthyl)-1-octyl-phosphacyclohexan-Lösung

0.26 g (1.0 mmol) Rhodiumdicarbonylacetylacetonat, 3.0 g (7.1 mmol) 4-Phenyl-2,6-bis(2-naphthyl)phosphabenzol und 10 g (89 mmol) 1-Octen wurden unter Stickstoff-Inertgas in einem Schlenkrohr in 10 g Toluol gelöst. Die erhaltene Lösung wurde in einen mit Wasserstoff gespülten 100 ml Autoklaven überführt. Es wurden 5 bar Wasserstoff aufgepresst. Unter kräftigem Rühren mit einem Begasungsrührer wurde das Reaktionsgemisch auf 160 °C erhitzt. Der Wasserstoffdruck im Autoklaven wurde auf 80 bar erhöht. Das Reaktionsgemisch wurde dann 96 h bei 160 °C gerührt. Während der Reaktion wurde der Druck im Reaktor durch Nachpressen über einen Druckregler auf Druckniveau gehalten. Die Reaktionslösung wurde auf Raumtemperatur abgekühlt und als solche weiter verwendet. Eine Analyse der Lösung lieferte einen Rhodium-Gehalt von 2100 ppm und einen Phosphor-Gehalt von 4300 ppm (P/Rh-Molverhältnis 7). Eine GC-Analyse (NP-Detektor) zeigt einen Phosphabenzol-Umsatz von 90 % an. Die Ausbeute an 4-Phenyl-2,6-bis(2-naphthyl)-1-octylphosphacyclohexan betrug 89 %. Das Produkt konnte zusätzlich durch $^{31}$P-NMR-Spektroskopie bestätigt werden.

Beispiel 45

Herstellung einer Rhodium/2,6-Bis(2-methylphenyl)-1-octyl-4-phenylphosphacyclohexan-Lösung

0.14 g (0.54 mmol) Rhodiumdicarbonylacetylacetonat, 1.5 g (4.3 mmol) 2,6-Bis(2-methylphenyl)-4-phenylphosphabenzol und 10 g (89 mmol) 1-Octen wurden unter Stickstoff-Inertgas in einem Schlenkrohr in 10 g Toluol gelöst. Die erhaltene Lösung wurde in einen mit Wasserstoff gespülten 100 ml Autoklaven überführt. Es wurden 5 bar Wasserstoff aufgepresst. Unter kräftigem Rühren mit einem Begasungsrührer wurde das Reaktionsgemisch auf 160 °C erhitzt. Der Wasserstoffdruck im Autoklaven wurde auf 80 bar erhöht. Das Reaktionsgemisch wurde dann 72 h bei 160 °C gerührt. Während der Reaktion wurde der Druck im Reaktor durch Nachpressen über einen Druckregler auf Druckniveau gehalten. Nach Abkühlen auf Raumtemperatur wurden zur Vervollständigung der Reaktion erneut 10 g (89 mmol) 1-Octen zugesetzt. Die Reaktion wurde für weitere 48 h bei 160 °C und 80 bar Wasserstoffdruck gemäß dem zuvor beschriebenen Verfahren betrieben. Nach Abkühlen auf Raumtemperatur wurde der Autoklav auf Normaldruck entspannt. Eine GC-Analyse (NP-Detektor) der Reaktionslösung zeigt einen Phosphabenzol-Umsatz von 99 % an. Die Ausbeute an 2,6-Bis (2-methylphenyl)-1-octyl-4-phenylphosphacyclohexan betrug 98 %. Die Lösung wurde erneut mit 5 g (45 mmol) 1-Octen versetzt. Es wurden bei Raumtemperatur 5 bar $CO/H_2$ (1:1) aufgepresst. Unter kräftigem Rühren mit einem Begasungsrührer wurde das Reaktionsgemisch auf 100 °C erhitzt. Der $CO/H_2$(1:1)-Druck im Autoklaven wurde auf 20 bar erhöht. Das Reaktionsgemisch wurde dann 12 h bei 100 °C gerührt. Während der Reaktion wurde der Druck im Reaktor durch Nachpressen über einen Druckregler auf Druckniveau gehalten. Nach der Reaktionszeit wurde der Autoklav abgekühlt, entspannt und entleert. Die erhaltene Lösung wurde im Hochvakuum vollständig eingeengt und der verbleibende Rückstand wurde in 30 ml Toluol aufgenommen. Eine Analyse der Lösung lieferte einen Rhodium-Gehalt von 710 ppm und einen Phosphor-Gehalt von 1600 ppm (P/Rh-Molverhältnis 7). Eine GC-Analyse (NP-Detektor) zeigt einen Phosphabenzol-Umsatz von 99 % an. Die Ausbeute an 2,6-Bis(2-methylphenyl)-1-octyl-4-phenylphosphacyclohexan betrug 96 %. Das Produkt konnte zusätzlich durch $^{31}$P-NMR-Spektroskopie bestätigt werden.

Beispiel 46

Herstellung einer Rhodium/Phosphacyclohexan-Lösung eines Phosphacyclohexans der Formel A

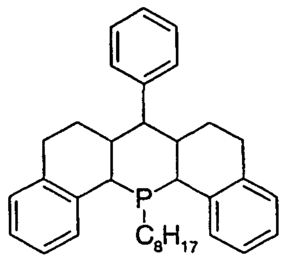

A

0.02 g (0.08 mmol) Rhodiumdicarbonylacetylacetonat, 0.20 g (0.53 mmol) 10-Phenyl-1,2,7,8-dibenzo-3,4,5,6-tetrahydro-9-phosphaanthracen und 10 g (89 mmol) 1-Octen wurden unter Stickstoff-Inertgas in einem Schlenkrohr in 10 g Toluol gelöst. Die erhaltene Lösung wurde in einen mit Wasserstoff gespülten 300 ml Autoklaven überführt. Es wurden 5 bar Wasserstoff aufgepresst. Unter kräftigem Rühren mit einem Begasungsrührer wurde das Reaktionsgemisch auf 160 °C erhitzt. Der Wasserstoffdruck im Autoklaven wurde auf 80 bar erhöht. Das Reaktionsgemisch wurde dann 72 h bei 160 °C gerührt. Während der Reaktion wurde der Druck im Reaktor durch Nachpressen über einen Druckregler auf Druckniveau gehalten. Nach der Reaktionszeit wurde der Autoklav abgekühlt, entspannt und entleert. Die erhaltene Lösung wurde im Hochvakuum vollständig eingeengt und der verbleibende Rückstand wurde in 10 ml Toluol aufgenommen. Eine Analyse der Lösung lieferte einen Rhodium-Gehalt von 165 ppm und einen Phosphor-Gehalt von 920 ppm (P/Rh-Molverhältnis 19). Eine GC-Analyse (NP-Detektor) zeigt einen Phosphabenzol-Umsatz von 99 % an. Die Ausbeute an dem Phosphacyclohexan der Formel A betrug 94 %. Die Verbindung konnten durch GC-MS-Analyse und $^{31}P$-NMR-Spektroskopie bestätigt werden.

Beispiel 47

Herstellung von 2,6-Bis(2,4-dimethylphenyl)-1-octyl-4-phenylphosphacyclohexan

0.30 g (1.2 mmol) Rhodiumdicarbonylacetylacetonat, 3.0 g (7.9 mmol) 2,6-Bis(2,4-dimethylphenyl)-4-phenylphosphabenzol und 10 g (89 mmol) 1-Octen wurden unter Stickstoff-Inertgas in einem Schlenkrohr in 10 g Toluol gelöst. Die erhaltene Lösung wurde in einen mit Wasserstoff gespülten 100 ml Autoklaven überführt. Es wurden 5 bar Wasserstoff aufgepresst. Unter kräftigem Rühren mit einem Begasungsrührer wurde das Reaktionsgemisch auf 160 °C erhitzt. Der Wasserstoffdruck im Autoklaven wurde auf 80 bar erhöht. Das Reaktionsgemisch wurde dann 96 h bei 160 °C gerührt. Während der Reaktion wurde der Druck im Reaktor durch Nachpressen über einen Druckregler auf Druckniveau gehalten. Nach Abkühlen auf Raumtemperatur wurden zur Vervollständigung der Reaktion erneut 10 g (89 mmol) 1-Octen zugesetzt. Die Reaktion wurde für weitere 24 h bei 160 °C und 80 bar Wasserstoffdruck gemäß dem zuvor beschriebenen Verfahren betrieben. Nach Abkühlen auf Raumtemperatur wurde der Autoklav auf Normaldruck entspannt. Die Lösung wurde erneut mit 5 g (45 mmol) 1-Octen versetzt. Es wurden bei Raumtemperatur 5 bar $CO/H_2$ (1:1) aufgepresst. Unter kräftigem Rühren mit einem Begasungsrührer wurde das Reaktionsgemisch auf 100 °C erhitzt. Der $CO/H_2$(1:1)-Druck im Autoklaven wurde auf 20 bar erhöht. Das Reaktionsgemisch wurde dann 12 h bei 100 °C gerührt. Während der Reaktion wurde der Druck im Reaktor durch Nachpressen über einen Druckregler auf Druckniveau gehalten. Nach der Reaktionszeit wurde der Autoklav abgekühlt, entspannt und entleert.

20 g der zuvor erhaltenen Katalysatorlösung wurden im Ölpumpenvakuum bei ca. 80 °C bis zur Trockene eingedampft. Der verbleibenden Rückstand wurde einer Kugelrohrdestillation unterworfen. Bei 270-280 °C/0.07 mbar erhielt man 1.42 g des Produktes in Form eines gelben Öles. Die Konstitution des Produktes konnte durch GC-Analyse (NP-Detektor), $^{31}P$-, $^{1}H$- und $^{13}C$-NMR-Spektroskopie bestätigt werden. Das Destillat wurde in 7 g Toluol aufgenommen. Eine Analyse der Lösung lieferte einen Phosphor-Gehalt von 9400 ppm.

Beispiel 48

Herstellung von 1-Octyl-2,4,6-triphenylphosphacyclohexan

0.09 g (0.35 mmol) Rhodiumdicarbonylacetylacetonat, 0.95 g (2.9 mmol) 2,4,6-Triphenylphosphabenzol und 10 g (89 mmol) 1-Octen wurden unter Stickstoff-Inertgas in einem Schlenkrohr in 10 g Toluol gelöst. Die erhaltene Lösung wurde in einen mit Wasserstoff gespülten 100 ml Autoklaven überführt. Es wurden 5 bar Wasserstoff aufgepresst. Unter kräftigem Rühren mit einem Begasungsrührer wurde das Reaktionsgemisch auf 160 °C erhitzt. Der Wasserstoffdruck im Autoklaven wurde auf 80 bar erhöht. Das Reaktionsgemisch wurde dann 96 h bei 160 °C gerührt. Während

der Reaktion wurde der Druck im Reaktor durch Nachpressen über einen Druckregler auf Druckniveau gehalten.

20 g der zuvor erhaltenen Katalysatorlösung wurden im Ölpumpenvakuum bei ca. 80 °C bis zur Trockene eingedampft. Der verbleibenden Rückstand wurde einer Kugelrohrdestillation unterworfen. Bei 270-280 °C/0.07 mbar erhielt man 0.45 g des Produktes in Form eines hellgelben Öles, welches bei Raumtemperatur langsam zu einem Feststoff erstarrte. Die Konstitution des Produktes konnte durch GC-Analyse (NP-Detektor), $^{31}P$-, $^{1}H$- und $^{13}C$-NMR-Spektroskopie bestätigt werden. Das Destillat wurde in 7.25 g Toluol aufgenommen. Eine Analyse der Lösung lieferte einen Phosphor-Gehalt von 4100 ppm.

Beispiel 49

Herstellung einer Rhodium/2,6-Bis(2,5-dimethylphenyl)-1-octyl-4-phenylphosphacyclohexan-Lösung

0.045 g (0.17 mmol) Rhodiumdicarbonylacetylacetonat, 0.57 g (1.5 mmol) 2,6-Bis(2,5-dimethylphenyl)-4-phenylphosphabenzol und 120 g (766 mmol) 1-Octen wurden unter Stickstoff-Inertgas in einem Schlenkrohr in 50 ml Xylol gelöst. Die erhaltene Lösung wurde in einen mit Wasserstoff gespülten 300 ml Autoklaven überführt. Es wurden 10 bar Wasserstoff aufgepresst. Unter kräftigem Rühren mit einem Begasungsrührer wurde das Reaktionsgemisch auf 160 °C erhitzt. Der Wasserstoffdruck im Autoklaven wurde auf 80 bar erhöht. Das Reaktionsgemisch wurde dann 72 h bei 160 °C gerührt. Während der Reaktion wurde der Druck im Reaktor durch Nachpressen über einen Druckregler auf Druckniveau gehalten. Die Reaktionslösung wurde auf Raumtemperatur abgekühlt und im Ölpumpenvakuum auf ca. 50 ml eingeengt. Eine Analyse der Lösung lieferte einen Rhodium-Gehalt von 480 ppm und einen Phosphor-Gehalt von 1200 ppm (P/Rh-Molverhältnis 8). Eine GC-Analyse (NP-Detektor) zeigt einen Phosphabenzol-Umsatz von 100 % an. Die Ausbeute an 2,6-Bis(2,5-dimethylphenyl)-1-octyl-4-phenylphosphacyclohexan betrug 95 %. Das Produkt konnte zusätzlich durch $^{31}P$-NMR-Spektroskopie bestätigt werden.

Beispiel 50

Herstellung einer Rhodium/2,6-Bis(2,4-dimethylphenyl)-1-dodecyl-4-phenylphosphacyclohexan-Lösung

0.88 g (3.4 mmol) Rhodiumdicarbonylacetylacetonat, 13 g (34 mmol) 2,6-Bis(2,4-dimethylphenyl)-4-phenylphosphabenzol und 91 g (541 mmol) 1-Dodecen wurden unter Stickstoff-Inertgas in einem Schlenkrohr in 50 ml Xylol gelöst. Die erhaltene Lösung wurde in einen mit Wasserstoff gespülten 300 ml Autoklaven überführt. Es wurden 10 bar Wasserstoff aufgepresst. Unter kräftigem Rühren mit einem Begasungsrührer wurde das Reaktionsgemisch auf 160 °C erhitzt. Der Wasserstoffdruck im Autoklaven wurde auf 80 bar erhöht. Das Reaktionsgemisch wurde dann 72 h bei 160 °C gerührt. Während der Reaktion wurde der Druck im Reaktor durch Nachpressen über einen Druckregler auf Druckniveau gehalten. Nach Abkühlen auf Raumtemperatur wurde der Autoklav auf Normaldruck entspannt, und anschließend im Ölpumpenvakuum unter leichtem Erwärmen auf ca. 40 ml eingeengt. Zur Vervollständigung der Reaktion wurde die Reaktionslösung erneut mit 91 g (541 mmol) 1-Dodecen versetzt. Die Reaktion wurde für weitere 24 h bei 160 °C und 80 bar Wasserstoffdruck gemäß dem zuvor beschriebenen Verfahren betrieben. Die Reaktionslösung wurde auf Raumtemperatur abgekühlt und als solche weiter verwendet. Eine Analyse der Lösung lieferte einen Rhodium-Gehalt von 2500 ppm und einen Phosphor-Gehalt von 7400 ppm (P/Rh-Molverhältnis 8). Eine GC-Analyse (NP-Detektor) zeigt einen Phosphabenzol-Umsatz von 89 % an. Die Ausbeute an 2,6-Bis(2,4-dimethylphenyl)-1-dodecyl-4-phenylphosphacyclohexan betrug 85 %. Die Verbindung konnte durch GC-MS-Analyse und $^{31}P$-NMR-Spektroskopie bestätigt werden.

Beispiel 51

Mitteldruck-Hydroformylierung von Dimerbuten

4.4 g Katalysatorlösung aus Beispiel 40 wurden im Ölpumpenvakuum bei ca. 80 °C bis zur Trockene eingedampft. Ausgehend vom verbleibenden Katalysatorrückstand, 24.8 g (221 mmol) Dimerbuten (erhalten durch Nickelkatalysierte Dimerisierung von n-Butenen, Verzweigungsgrad 1.06) und 25.0 g Texanol® erhielt man bei 140 °C und 80 bar $CO/H_2$ gemäß der allgemeinen Versuchsdurchführung (Variante A) einen Umsatz von Dimerbuten von 55 % nach 1 h und 84 % nach 4 h. Die Ausbeute an Nonanalen betrug 55 % nach 1 h und 82 % nach 4 h. Die Ausbeute an Nonanolen betrug 0 % nach 1 h und 2 % nach 4 h.

Beispiel 52

Mitteldruck-Hydroformylierung von Dimerbuten

4.4 g Katalysatorlösung aus Beispiel 40 wurden im Ölpumpenvakuum bei ca. 80 °C bis zur Trockene eingedampft. Ausgehend vom verbleibenden Katalysatorrückstand, 24.8 g (221 mmol) Dimerbuten (erhalten durch Nickelkatalysierte Dimerisierung von n-Butenen, Verzweigungsgrad 1.06) und 25.0 g Texanol® erhielt man bei 160 °C und 60 bar $CO/H_2$ gemäß der allgemeinen Versuchsdurchführung (Variante A) einen Umsatz von Dimerbuten von 82 % nach 1 h und 96 % nach 4 h. Die Ausbeute an Nonanalen betrug 76 % nach 1 h und 69 % nach 4 h. Die Ausbeute an Nonanolen betrug 5 % nach 1 h und 22 % nach 4 h.

Beispiel 53

Mitteldruck-Hydroformylierung von Dimerbuten

1.4 g Katalysatorlösung aus Beispiel 41 wurden im Ölpumpenvakuum bei ca. 80 °C bis zur Trockene eingedampft. Ausgehend vom verbleibenden Katalysatorrückstand, 24.8 g (221 mmol) Dimerbuten (erhalten durch Nickelkatalysierte Dimerisierung von n-Butenen, Verzweigungsgrad 1.06) und 25.0 g Texanol® erhielt man bei 140 °C und 80 bar $CO/H_2$ gemäß der allgemeinen Versuchsdurchführung (Variante A) einen Umsatz von Dimerbuten von 45 % nach 1 h und 70 % nach 4 h. Die Ausbeute an Nonanalen betrug 45 % nach 1 h und 70 % nach 4 h.

Beispiel 54

Niederdruck-Hydroformylierung von Dimerbuten

0.88 g Katalysatorlösung aus Beispiel 42 wurden im Ölpumpenvakuum bei ca. 80 °C bis zur Trockene eingedampft. Ausgehend vom verbleibenden Katalysatorrückstand, 1.3 mg Rhodiumdicarbonylacetylacetonat, 21.5 g (192 mmol) Dimerbuten (erhalten durch Nickel-katalysierte Dimerisierung von n-Butenen, Verzweigungsgrad 1.06) und 23.2 g Texanol® erhielt man bei 110 °C und 20 bar $CO/H_2$ gemäß der allgemeinen Versuchsdurchführung (Variante A) einen Umsatz von Dimerbuten von 29 % nach 1 h und 46 % nach 4 h. Die Ausbeute an Nonanalen betrug 29 % nach 1 h und 46 % nach 4 h.

Beispiel 55

Niederdruck-Hydroformylierung von Dimerbuten

0.89 g Katalysatorlösung aus Beispiel 42 wurden im Ölpumpenvakuum bei ca. 80 °C bis zur Trockene eingedampft. Ausgehend vom verbleibenden Katalysatorrückstand, 1.7 mg Rhodiumdicarbonylacetylacetonat, 24.7 g (220 mmol) Dimerbuten (erhalten durch Nickel-katalysierte Dimerisierung von n-Butenen, Verzweigungsgrad 1.06) und 24.7 g Texanol® erhielt man bei 140 °C und 20 bar $CO/H_2$ gemäß der allgemeinen Versuchsdurchführung (Variante A) einen Umsatz von Dimerbuten von 44 % nach 1 h und 70 % nach 4 h. Die Ausbeute an Nonanalen betrug 43 % nach 1 h und 66 % nach 4 h. Die Ausbeute an Nonanolen betrug 1 % nach 1 h und 4 % nach 4 h.

Beispiel 56

Mitteldruck-Hydroformylierung von Dimerbuten

0.32 g Katalysatorlösung aus Beispiel 43 wurden im Ölpumpenvakuum bei ca. 80 °C bis zur Trockene eingedampft. Ausgehend vom verbleibenden Katalysatorrückstand, 4.6 mg Rhodiumdicarbonylacetylacetonat, 26.0 g (232 mmol) Dimerbuten (erhalten durch Nickel-katalysierte Dimerisierung von n-Butenen, Verzweigungsgrad 1.06) und 25.0 g Texanol® erhielt man bei 140 °C und 80 bar $CO/H_2$ gemäß der allgemeinen Versuchsdurchführung (Variante A) einen Umsatz von Dimerbuten von 85 % nach 1 h und 93 % nach 4 h. Die Ausbeute an Nonanalen betrug 82 % nach 1 h und 85 % nach 4 h. Die Ausbeute an Nonanolen betrug 3 % nach 1 h und 12 % nach 4 h.

Beispiel 57

Mitteldruck-Hydroformylierung von Dimerbuten

2.2 g Katalysatorlösung aus Beispiel 10 wurden im Ölpumpenvakuum bei ca. 80 °C bis zur Trockene eingedampft. Ausgehend vom verbleibenden Katalysatorrückstand, 24.8 g (221 mmol) Dimerbuten (erhalten durch Nickel-katalysierte Dimerisierung von n-Butenen, Verzweigungsgrad 1.06) und 25.0 g Texanol® erhielt man bei 160 °C und 60 bar $CO/H_2$ gemäß der allgemeinen Versuchsdurchführung (Variante A) einen Umsatz von Dimerbuten von 75 % nach 1 h und 95 % nach 4 h. Die Ausbeute an Nonanalen betrug 70 % nach 1 h und 69 % nach 4 h. Die Ausbeute an Nonanolen betrug 4 % nach 1 h und 21 % nach 4 h.

Beispiel 58

Mitteldruck-Hydroformylierung von Dimerbuten

1.4 g Katalysatorlösung aus Beispiel 44 wurden im Ölpumpenvakuum bei ca. 80 °C bis zur Trockene eingedampft. Ausgehend vom verbleibenden Katalysatorrückstand, 25.1 g (224 mmol) Dimerbuten (erhalten durch Nickel-katalysierte Dimerisierung von n-Butenen, Verzweigungsgrad 1.06) und 25.0 g Texanol® erhielt man bei 160 °C und 60 bar $CO/H_2$ gemäß der allgemeinen Versuchsdurchführung (Variante A) einen Umsatz von Dimerbuten von 52 % nach 1 h und 88 % nach 4 h. Die Ausbeute an Nonanalen betrug 51 % nach 1 h und 77 % nach 4 h. Die Ausbeute an Nonanolen betrug 1 % nach 1 h und 9 % nach 4 h.

Beispiel 59

Mitteldruck-Hydroformylierung von Dimerbuten

4.3 g Katalysatorlösung aus Beispiel 45 wurden im Ölpumpenvakuum bei ca. 80 °C bis zur Trockene eingedampft. Ausgehend vom verbleibenden Katalysatorrückstand, 25.4 g (226 mmol) Dimerbuten (erhalten durch Nickel-katalysierte Dimerisierung von n-Butenen, Verzweigungsgrad 1.06) und 25.0 g Texanol® erhielt man bei 160 °C und 60 bar $CO/H_2$ gemäß der allgemeinen Versuchsdurchführung (Variante A) einen Umsatz von Dimerbuten von 31 % nach 1 h und 53 % nach 4 h. Die Ausbeute an Nonanalen betrug 31 % nach 1 h und 50 % nach 4 h. Die Ausbeute an Nonanolen betrug 0 % nach 1 h und 1 % nach 4 h.

Beispiel 60

Mitteldruck-Hydroformylierung von Dimerbuten

6.0 g Katalysatorlösung aus Beispiel 46 wurden im Ölpumpenvakuum bei ca. 80 °C bis zur Trockene eingedampft. Ausgehend vom verbleibenden Katalysatorrückstand, 8.8 g (78 mmol) Dimerbuten (erhalten durch Nickel-katalysierte Dimerisierung von n-Butenen, Verzweigungsgrad 1.06) und 8.3 g Texanol® erhielt man bei 160 °C und 60 bar $CO/H_2$ gemäß der allgemeinen Versuchsdurchführung (Variante A) einen Umsatz von Dimerbuten von 50 % nach 1 h und 97 % nach 24 h. Die Ausbeute an Nonanalen betrug 43 % nach 1 h und 44 % nach 24 h. Die Ausbeute an Nonanolen betrug 1 % nach 1 h und 41 % nach 24 h.

Beispiel 61

Mitteldruck-Hydroformylierung von Dimerbuten

0.68 g Ligandlösung aus Beispiel 47 wurden im Ölpumpenvakuum bis zur Trockene eingedampft. Ausgehend vom verbleibenden Liganden, 7.5 mg (0.029 mmol) Rhodiumdicarbonylacetylacetonat, 26.0 (232 mmol) Dimerbuten (erhalten durch Nickel-katalysierte Dimerisierung von n-Butenen, Verzweigungsgrad 1.06) und 25.0 g Texanol® erhielt man bei 160 °C und 60 bar $CO/H_2$ gemäß der allgemeinen Versuchsdurchführung (Variante A) einen Umsatz von Dimerbuten von 80 % nach 1 h und 96 % nach 4 h. Die Ausbeute an Nonanalen betrug 75 % nach 1 h und 75 % nach 4 h. Die Ausbeute an Nonanolen betrug 4 % nach 1 h und 17% nach 4 h.

Beispiel 62

Mitteldruck-Hydroformylierung von Dimerbuten

0.68 g Ligandlösung aus Beispiel 47 wurden im Ölpumpenvakuum bis zur Trockene eingedampft. Ausgehend vom verbleibenden Liganden, 7.4 mg (0.029 mmol) Rhodiumdicarbonylacetylacetonat, 25.3 (225 mmol) Dimerbuten (erhalten durch Nickel-katalysierte Dimerisierung von n-Butenen, Verzweigungsgrad 1.06) und 25.0 g Texanol® erhielt man bei 140 °C und 80 bar $CO/H_2$ gemäß der allgemeinen Versuchsdurchführung (Variante A) einen Umsatz von Dimerbuten von 75 % nach 1 h und 95 % nach 4 h. Die Ausbeute an Nonanalen betrug 73 % nach 1 h und 88 % nach 4 h. Die Ausbeute an Nonanolen betrug 1 % nach 1 h und 5% nach 4 h.

Beispiel 63

Mitteldruck-Hydroformylierung von Dimerbuten

1.54 g Ligandlösung aus Beispiel 48 wurden im Ölpumpenvakuum bis zur Trockene eingedampft. Ausgehend vom verbleibenden Liganden, 7.4 mg (0.029 mmol) Rhodiumdicarbonylacetylacetonat, 25.6 (228 mmol) Dimerbuten (erhalten durch Nickel-katalysierte Dimerisierung von n-Butenen, Verzweigungsgrad 1.06) und 25.0 g Texanol® erhielt man bei 160 °C und 60 bar $CO/H_2$ gemäß der allgemeinen Versuchsdurchführung (Variante A) einen Umsatz von Dimerbuten von 75 % nach 1 h und 95 % nach 4 h. Die Ausbeute an Nonanalen betrug 70 % nach 1 h und 75 % nach 4 h. Die Ausbeute an Nonanolen betrug 4 % nach 1 h und 16 % nach 4 h.

Beispiel 64

Mitteldruck-Hydroformylierung von Dimerbuten

1.55 g Ligandlösung aus Beispiel 48 wurden im Ölpumpenvakuum bis zur Trockene eingedampft. Ausgehend vom verbleibenden Liganden, 7.5 mg (0.029 mmol) Rhodiumdicarbonylacetylacetonat, 25.5 (227 mmol) Dimerbuten (erhalten durch Nickel-katalysierte Dimerisierung von n-Butenen, Verzweigungsgrad 1.06) und 25.0 g Texanol® erhielt man bei 140 °C und 80 bar $CO/H_2$ gemäß der allgemeinen Versuchsdurchführung (Variante A) einen Umsatz von Dimerbuten von 60 % nach 1 h und 87 % nach 4 h. Die Ausbeute an Nonanalen betrug 59 % nach 1 h und 81 % nach 4 h. Die Ausbeute an Nonanolen betrug 1 % nach 1 h und 3 % nach 4 h.

Beispiel 65

Mitteldruck-Hydroformylierung von Dimerbuten

6.3 g Katalysatorlösung aus Beispiel 49 wurden im Ölpumpenvakuum bei ca. 80 °C bis zur Trockene eingedampft. Ausgehend vom verbleibenden Katalysatorrückstand, 25.7 g (229 mmol) Dimerbuten (erhalten durch Nickel-katalysierte Dimerisierung von n-Butenen, Verzweigungsgrad 1.06) und 25.1 g Texanol® erhielt man bei 140 °C und 80 bar $CO/H_2$ gemäß der allgemeinen Versuchsdurchführung (Variante A) einen Umsatz von Dimerbuten von 52 % nach 1 h und 83 % nach 4 h. Die Ausbeute an Nonanalen betrug 51 % nach 1 h und 76 % nach 4 h. Die Ausbeute an Nonanolen betrug 0 % % nach 1 h und 3 % nach 4 h.

Beispiel 66

Mitteldruck-Hydroformylierung von Dimerbuten

0.32 g Katalysatorlösung aus Beispiel 43 wurden im Ölpumpenvakuum bei ca. 80 °C bis zur Trockene eingedampft. Ausgehend vom verbleibenden Katalysatorrückstand, 4.6 mg (0.018 mmol) Rhodiumdicarbonylacetylacetonat, 26.0 g (232 mmol) Dimerbuten (erhalten durch Nickel-katalysierte Dimerisierung von n-Butenen, Verzweigungsgrad 1.06) und 25.0 g Texanol® erhielt man bei 140 °C und 80 bar $CO/H_2$ gemäß der allgemeinen Versuchsdurchführung (Variante A) einen Umsatz von Dimerbuten von 85 % nach 1 h und 97 % nach 4 h. Die Ausbeute an Nonanalen betrug 82 % nach 1 h und 85 % nach 4 h. Die Ausbeute an Nonanolen betrug 3 % nach 1 h und 12 % nach 4 h.

Beispiel 67

Mitteldruck-Hydroformylierung von Dimerbuten

0.32 g Katalysatorlösung aus Beispiel 43 wurden im Ölpumpenvakuum bei ca. 80 °C bis zur Trockene eingedampft. Ausgehend vom verbleibenden Katalysatorrückstand, 4.6 mg (0.018 mmol) Rhodiumdicarbonylacetylacetonat, 26.0 g (232 mmol) Dimerbuten (erhalten durch Nickel-katalysierte Dimerisierung von n-Butenen, Verzweigungsgrad 1.06) und 25.0 g Texanol® erhielt man bei 140 °C und 80 bar $CO/H_2$ gemäß der allgemeinen Versuchsdurchführung (Variante A) einen Umsatz von Dimerbuten von 85 % nach 1 h und 97 % nach 4 h. Die Ausbeute an Nonanalen betrug 82 % nach 1 h und 85 % nach 4 h. Die Ausbeute an Nonanolen betrug 3 % nach 1 h und 12 % nach 4 h.

Beispiel 68

Mitteldruck-Hydroformylierung einer $C_{12}/C_{14}$-Alphaolefinen

0.33 g Katalysatorlösung aus Beispiel 43 wurden im Ölpumpenvakuum bei ca. 80 °C bis zur Trockene eingedampft. Ausgehend vom verbleibenden Katalysatorrückstand, 4.7 mg (0.018 mmol) Rhodiumdicarbonylacetylacetonat, 25.4 g $C_{12}/C_{14}$-Alphaolefine (Molverhältnis $C_{12}$-Olefine:$C_{14}$-Olefine = 70:30; Zusammensetzung: 90 % 1-Alkene, 2 % interne Olefine und 8 % Vinylidene) und 25.0 g Toluol erhielt man bei 100 °C und 80 bar $CO/H_2$ gemäß der allgemeinen Versuchsdurchführung (Variante A) einen Umsatz von $C_{12}/C_{14}$-Alphaolefinen von 93 % nach 1 h und 97 % nach 4 h. Die Ausbeute an Aldehyden betrug 93 % nach 1 h und 97 % nach 4 h.

Beispiel 69

Mitteldruck-Hydroformylierung von $C_{12}/C_{14}$-Alphaolefinen

0.83 g Katalysatorlösung aus Beispiel 43 wurden im Ölpumpenvakuum bei ca. 80 °C bis zur Trockene eingedampft. Ausgehend vom verbleibenden Katalysatorrückstand, 25.2 g $C_{12}/C_{14}$-Alphaolefine (Molverhältnis $C_{12}$-Olefine: $C_{14}$-Olefine = 70:30; Zusammensetzung: 90 % 1-Alkene, 2 % interne Olefine und 8 % Vinylidene) und 25.0 g Toluol erhielt man bei 120 °C und 80 bar $CO/H_2$ gemäß der allgemeinen Versuchsdurchführung (Variante A) einen Umsatz von $C_{12}/C_{14}$-Alphaolefinen von 98 % nach 1 h und 99 % nach 4 h. Die Ausbeute an Aldehyden betrug 97 % nach 1 h und 98 % nach 4 h. Die Ausbeute an Alkoholen betrug 0 % nach 1 h und 1 % nach 4 h.

Beispiel 70

Mitteldruck-Hydroformylierung von $C_{12}/C_{14}$-Alphaolefinen

0.84 g Katalysatorlösung aus Beispiel 43 wurden im Ölpumpenvakuum bei ca. 80 °C bis zur Trockene eingedampft. Ausgehend vom verbleibenden Katalysatorrückstand, 25.5 g $C_{12}/C_{14}$-Alphaolefine (Molverhältnis $C_{12}$-Olefine: $C_{14}$-Olefine = 70:30; Zusammensetzung: 90 % 1-Alkene, 2 % interne Olefine und 8 % Vinylidene) und 25.0 g Toluol erhielt man bei 140 °C und 80 bar $CO/H_2$ gemäß der allgemeinen Versuchsdurchführung (Variante A) einen Umsatz von $C_{12}/C_{14}$-Alphaolefinen von 98 % nach 1 h. Die Ausbeute an Aldehyden betrug 97 % nach 1 h. Die Ausbeute an Alkoholen betrug 1 % nach 1 h.

Beispiel 71

Niederdruck-Hydroformylierung von $C_{12}/C_{14}$-Alphaolefinen

0.84 g Katalysatorlösung aus Beispiel 43 wurden im Ölpumpenvakuum bei ca. 80 °C bis zur Trockene eingedampft. Ausgehend vom verbleibenden Katalysatorrückstand, 24.9 g $C_{12}/C_{14}$-Alphaolefine (Molverhältnis $C_{12}$-Olefine: $C_{14}$-Olefine = 70:30; Zusammensetzung: 90 % 1-Alkene, 2 % interne Olefine und 8 % Vinylidene) und 25.1 g Toluol erhielt man bei 110 °C und 20 bar $CO/H_2$ gemäß der allgemeinen Versuchsdurchführung (Variante A) einen Umsatz von $C_{12}/C_{14}$-Alphaolefinen von 93 % nach 1 h und 99 % nach 4 h. Die Ausbeute an Aldehyden betrug 93 % nach 1 h und 98 % nach 4 h. Die Ausbeute an Alkoholen betrug 0 % nach 1 h und 1 % nach 4 h.

Beispiel 72

Niederdruck-Hydroformylierung von 2-Octen

0.83 g Katalysatorlösung aus Beispiel 43 wurden im Ölpumpenvakuum bei ca. 80 °C bis zur Trockene eingedampft. Ausgehend vom verbleibenden Katalysatorrückstand, 25.5 g (227 mmol) 2-Octen (Verhältnis cis:trans = 80:20) und 25.1 g Toluol erhielt man bei 110 °C, 20 bar $CO/H_2$ und 3 h gemäß der allgemeinen Versuchsdurchführung (Variante A) einen Umsatz von 2-Octen von 99 %. Die Ausbeute an Nonanalen betrug 97 %, die Selektivität zu n-Nonanal (n-Anteil) betrug 5 % und die Selektivität zu n-Nonanal und 2-Methyloctanal ($\alpha$-Anteil) betrug 58 %.

Beispiel 73

Niederdruck-Hydroformylierung von $C_{12}/C_{14}$-Alphaolefinen

0.83 g Katalysatorlösung aus Beispiel 43 wurden im Ölpumpenvakuum bei ca. 80 °C bis zur Trockene eingedampft. Ausgehend vom verbleibenden Katalysatorrückstand, 25.1 g $C_{12}/C_{14}$-Alphaolefine (Molverhältnis $C_{12}$-Olefine: $C_{14}$-Olefine = 70:30; Zusammensetzung: 90 % 1-Alkene, 2 % interne Olefine und 8 % Vinylidene) und 25.0 g Toluol erhielt man bei 140 °C und 20 bar $CO/H_2$ gemäß der allgemeinen Versuchsdurchführung (Variante A) einen Umsatz von $C_{12}/C_{14}$-Alphaolefinen von 95 % nach 1 h und 98 % nach 4 h. Die Ausbeute an Aldehyden betrug 93 % nach 1 h und 87 % nach 4 h. Die Ausbeute an Alkoholen betrug 2 % nach 1 h und 10 % nach 4 h.

Beispiel 74

Niederdruck-Hydroformylierung von $C_{12}/C_{14}$-Alphaolefinen

0.83 g Katalysatorlösung aus Beispiel 43 wurden im Ölpumpenvakuum bei ca. 80 °C bis zur Trockene eingedampft. Ausgehend vom verbleibenden Katalysatorrückstand, 26.0 g $C_{12}/C_{14}$-Alphaolefine (Molverhältnis $C_{12}$-Olefine: $C_{14}$-Olefine = 68:32; Zusammensetzung: 84 % 1-Alkene, 4 % interne Olefine und 12 % Vinylidene) und 25.1 g Toluol erhielt man bei 80 °C und 20 bar $CO/H_2$ gemäß der allgemeinen Versuchsdurchführung (Variante A) einen Umsatz von $C_{12}/C_{14}$-Alphaolefinen von 61 % nach 1 h und 87 % nach 4 h. Die Ausbeute an Aldehyden betrug 61 % nach 1 h und 87 % nach 4 h.

Beispiel 75

Niederdruck-Hydroformylierung von $C_{12}/C_{14}$-Alphaolefinen

0.84 g Katalysatorlösung aus Beispiel 43 wurden im Ölpumpenvakuum bei ca. 80 °C bis zur Trockene eingedampft. Ausgehend vom verbleibenden Katalysatorrückstand, 24.9 g $C_{12}/C_{14}$-Alphaolefine (Molverhältnis $C_{12}$-Olefine: $C_{14}$-Olefine = 68:32; Zusammensetzung: 84 % 1-Alkene, 4 % interne Olefine und 12 % Vinylidene) und 25.0 g Toluol erhielt man bei 110 °C und 20 bar $CO/H_2$ gemäß der allgemeinen Versuchsdurchführung (Variante A) einen Umsatz von $C_{12}/C_{14}$-Alphaolefinen von 83 % nach 0.5 h und 95 % nach 4 h. Die Ausbeute an Aldehyden betrug 83 % nach 0.5 h und 95 % nach 4 h. Die Ausbeute an Alkoholen betrug 0 % nach 0.5 h und 1 % nach 4 h.

Beispiel 76

Niederdruck-Hydroformylierung von $C_{12}/C_{14}$-Alphaolefinen

0.83 g Katalysatorlösung aus Beispiel 43 wurden im Ölpumpenvakuum bei ca. 80 °C bis zur Trockene eingedampft. Ausgehend vom verbleibenden Katalysatorrückstand, 25.0 g $C_{12}/C_{14}$-Alphaolefine (Molverhältnis $C_{12}$-Olefine: $C_{14}$-Olefine = 68:32; Zusammensetzung: 84 % 1-Alkene, 4 % interne Olefine und 12 % Vinylidene) und 25.1 g Toluol erhielt man bei 140 °C und 20 bar $CO/H_2$ gemäß der allgemeinen Versuchsdurchführung (Variante A) einen Umsatz von $C_{12}/C_{14}$-Alphaolefinen von 89 % nach 1 h und 94 % nach 4 h. Die Ausbeute an Aldehyden betrug 87 % nach 1 h und 86 % nach 4 h. Die Ausbeute an Alkoholen betrug 2 % nach 1 h und 8 % nach 4 h.

Beispiel 77

Niederdruck-Hydroformylierung von Diisobuten

0.83 g Katalysatorlösung aus Beispiel 43 wurden im Ölpumpenvakuum bei ca. 80 °C bis zur Trockene eingedampft. Ausgehend vom verbleibenden Katalysatorrückstand, 25.5 g (227 mmol) Diisobuten (Zusammensetzung: 73 % 2,4,4-Trimethyl-1-penten, 18 % 2,4,4-Trimethyl-2-penten, 9 % interne, verzweigte Octene) und 25.1 g Toluol erhielt man bei 110 °C und 20 bar $CO/H_2$ gemäß der allgemeinen Versuchsdurchführung (Variante A) einen Umsatz von Diisobuten von 27 % nach 4 h und 69 % nach 24 h. Die Ausbeute an Nonanalen betrug 24 % nach 4 h und 64 % nach 24 h. Die Ausbeute an Alkoholen betrug 1 % nach 4 h und 4 % nach 24 h.

Beispiel 78

Niederdruck-Hydroformylierung von $C_{12}/C_{14}$-Alphaolefinen

1.22 g Katalysatorlösung aus Beispiel 50 wurden im Ölpumpenvakuum bei ca. 80 °C bis zur Trockene eingedampft. Ausgehend vom verbleibenden Katalysatorrückstand, 25.1 g $C_{12}/C_{14}$-Alphaolefine (Molverhältnis $C_{12}$-Olefine: $C_{14}$-Olefine = 68:32; Zusammensetzung: 84 % 1-Alkene, 4 % interne Olefine und 12 % Vinylidene) und 25.0 g Toluol erhielt man bei 110 °C und 20 bar $CO/H_2$ gemäß der allgemeinen Versuchsdurchführung (Variante A) einen Umsatz von $C_{12}/C_{14}$-Alphaolefinen von 88 % nach 1 h und 95 % nach 4 h. Die Ausbeute an Aldehyden betrug 88 % nach 1 h und 94 % nach 4 h. Die Ausbeute an Alkoholen betrug 0 % nach 1 h und 1 % nach 4 h.

Beispiel 79

Mitteldruck-Hydroformylierung von Dimerbuten

0.37 g Katalysatorlösung aus Beispiel 50 wurden im Ölpumpenvakuum bei ca. 80 °C bis zur Trockene eingedampft. Ausgehend vom verbleibenden Katalysatorrückstand, 5.6 mg (0.022 mmol) Rhodiumdicarbonylacetylacetonat, 25.6 g (228 mmol) Dimerbuten (erhalten durch Nickel-katalysierte Dimerisierung von n-Butenen, Verzweigungsgrad 1.06) und 25.0 g Texanol® erhielt man bei 140 °C und 80 bar $CO/H_2$ gemäß der allgemeinen Versuchsdurchführung (Variante A) einen Umsatz von Dimerbuten von 87 % nach 1 h und 96 % nach 4 h. Die Ausbeute an Nonanalen betrug 83 % nach 1 h und 79 % nach 4 h. Die Ausbeute an Nonanolen betrug 3 % nach 1 h und 16 % nach 4 h.

Beispiel 80

Mitteldruck-Hydroformylierung von Diisobuten

0.33 g Katalysatorlösung aus Beispiel 43 wurden im Ölpumpenvakuum bei ca. 80 °C bis zur Trockene eingedampft. Ausgehend vom verbleibenden Katalysatorrückstand, 4.8 mg (0.019 mmol) Rhodiumdicarbonylacetylacetonat, 25.4 g (226 mmol) Diisobuten (Zusammensetzung: 73 % 2,4,4-Trimethyl-1-penten, 18 % 2,4,4-Trimethyl-2-penten, 9 % interne, verzweigte Octene) und 24.9 g Toluol erhielt man bei 140 °C und 80 bar $CO/H_2$ gemäß der allgemeinen Versuchsdurchführung (Variante A) einen Umsatz von Diisobuten von 76 % nach 1 h und 99 % nach 4 h. Die Ausbeute an Nonanalen betrug 70 % nach 1 h und 88 % nach 4 h. Die Ausbeute an Alkoholen betrug 4 % nach 1 h und 9 % nach 4 h.

Beispiel 81

Niederdruck-Hydroformylierung von Diisobuten

0.84 g Katalysatorlösung aus Beispiel 43 wurden im Ölpumpenvakuum bei ca. 80 °C bis zur Trockene eingedampft. Ausgehend vom verbleibenden Katalysatorrückstand, 25.6 g (228 mmol) Diisobuten (Zusammensetzung: 73 % 2,4,4-Trimethyl-1-penten, 18 % 2,4,4-Trimethyl-2-penten, 9 % interne, verzweigte Octene) und 25.0 g Toluol erhielt man bei 140 °C und 20 bar $CO/H_2$ gemäß der allgemeinen Versuchsdurchführung (Variante A) einen Umsatz von Diisobuten von 72 % nach 4 h und 97 % nach 24 h. Die Ausbeute an Nonanalen betrug 62 % nach 4 h und 53 % nach 24 h. Die Ausbeute an Alkoholen betrug 7 % nach 4 h und 31 % nach 24 h.

Beispiel 82

Niederdruck-Hydroformylierung von 2-Ethyl-1-hexen

0.83 g Katalysatorlösung aus Beispiel 43 wurden im Ölpumpenvakuum bei ca. 80 °C bis zur Trockene eingedampft. Ausgehend vom verbleibenden Katalysatorrückstand, 25.2 g (225 mmol) 2-Ethyl-1-hexen und 25.0 g Toluol erhielt man bei 80 °C und 20 bar $CO/H_2$ gemäß der allgemeinen Versuchsdurchführung (Variante A) einen Umsatz von 2-Ethyl-1-hexen von 87 % nach 4 h und 98 % nach 24 h. Die Ausbeute an Nonanalen betrug 86 % nach 4 h und 93 % nach 24 h. Die Ausbeute an Alkoholen betrug 1 % nach 4 h und 5 % nach 24 h.

Beispiel 83

Niederdruck-Hydroformylierung von 2-Ethyl-1-hexen

1.02 g Katalysatorlösung aus Beispiel 42 wurden im Ölpumpenvakuum bei ca. 80 °C bis zur Trockene eingedampft. Ausgehend vom verbleibenden Katalysatorrückstand, 20.4 g (182 mmol) 2-Ethyl-1-hexen und 25.0 g Toluol erhielt man bei 140 °C und 20 bar $CO/H_2$ gemäß der allgemeinen Versuchsdurchführung (Variante A) einen Umsatz von 2-Ethyl-1-hexen von 56 % nach 4 h und 88 % nach 24 h. Die Ausbeute an Nonanalen betrug 52 % nach 4 h und 57 % nach 24 h. Die Ausbeute an Alkoholen betrug 2 % nach 4 h und 21 % nach 24 h.

Beispiel 84

Niederdruck-Hydroformylierung von 1-Octen

1.25 g Katalysatorlösung aus Beispiel 42 wurden im Ölpumpenvakuum bei ca. 80 °C bis zur Trockene eingedampft. Ausgehend vom verbleibenden Katalysatorrückstand, 25.0 g (223 mmol) 1-Octen und 25.0 g Toluol erhielt man bei 110 °C, 20 bar $CO/H_2$ und 3 h gemäß der allgemeinen Versuchsdurchführung (Variante A) einen Umsatz von 1-Octen von 99 %. Die Ausbeute an Nonanalen betrug 98 %, die Selektivität zu n-Nonanal (n-Anteil) betrug 59 % und die Selektivität zu n-Nonanal und 2-Methyloctanal ($\alpha$-Anteil) betrug 95 %.

Beispiel 85

Kontinuierliche Mitteldruck-Hydroformylierung von Dimerbuten

In einer Miniplant-Anlage gemäß Figur 1, jedoch unter Verwendung einer Kaskade bestehend aus zwei Reaktoren, wurde Dimerbuten (erhalten durch Nickel-katalysierte Dimerisierung von n-Butenen, Verzweigungsgrad 1.06) mit dem Katalysatorsystem Rhodium/2,6-Bis(2,4-dimethylphenyl)-1-octyl-4-phenylphosphacyclohexan kontinuierlich hydroformyliert.

Der Katalysator Rhodium/2,6-Bis(2,4-dimethylphenyl)-1-octyl-4-phenylphosphacyclohexan wurde durch Rhodium-katalysierte Hydrierung von 2,6-Bis(2,4-dimethylphenyl)-4-phenylphosphabenzol in Gegenwart von Dimerbuten hergestellt. Die Anlage wurde kontinuierlich, entsprechend wie nachstehend beschrieben für die Hydroformylierung, betrieben, jedoch mit dem Unterschied, dass die Temperatur in beiden Reaktoren 150 °C betrug und ein Wasserstoffdruck von 80 bar eingestellt wurde.

Zur kontinuierlichen Hydroformylierung wurden stündlich 300 g Dimerbuten in Gegenwart von 15-60 ppm Rhodium und dem zuvor erzeugtem 2,6-Bis(2,4-dimethylphenyl)-1-octyl-4-phenylphosphacyclohexan als Cokatalysator [P/Rh-Verhältnis = 3-4 (mol:mol)] - die Mengenangaben sind auf die Menge des Reaktionsgemisches bezogen - und 1 kg Texanol® als Lösungsmittel bei einer Reaktorinnentemperatur von 140 °C und einem $CO/H_2$-Druck von 80 bar in beiden Reaktoren hydroformyliert. Den Reaktoren wurde ein $CO/H_2$(1:1)-Gasgemisch in einer Menge zugeführt, so dass eine konstante Abgasmenge über einen Druckabscheider von ca. 75 Nl/h resultiert. Der flüssige Reaktoraustrag wurde in einem Flashbehälter entspannt und über einen Wischblattverdampfer in ein Destillat, hauptsächlich bestehend aus Nonanalen, Nonanolen und $C_8$-Kohlenwasserstoffen und einen katalysatorhaltigen Sumpf aufgetrennt. 200 g des katalysatorhaltigen Sumpfes wurden kontinuierlich in den ersten Reaktor der zweistufigen Reaktionskaskade zurückgeführt. Die Anlage konnte über 24 Bilanztage stabil und ohne Aktivitätsverlust betrieben werden. Ein Abbau des Liganden konnte nicht beobachtet werden. Am 14. Bilanztag wurden 500 ml des katalysatorhaltigen Sumpfes ausgeschleust, und am 21. Bilanztag wurden 760 ml des katalysatorhaltigen Sumpfes ausgeschleust. Die erzielten Umsätze, Selektivitäten und Raum-Zeit-Ausbeuten sind in Tabelle 5 und Figur 3 dokumentiert.

Tabelle 5: Umsätze, Selektivitäten und Raum-Zeit-Ausbeuten bei der kontinuierlichen Hydroformylierung von Dimerbuten (80 bar $CO/H_2$-Gesamtdruck, L/Rh = 3-4, ca. 15-60 ppm Rh) ohne Berücksichtigung der Hochsiederbildung

| | | |
|---|---|---|
| Dimerbuten (Umsatz) | [%] | 81.3 |
| Nonanale/-ole (Ausbeute) | [%] | 79.8 |
| Nonanale (Ausbeute) | [%] | 76.8 |
| Nonanole (Ausbeute) | [%] | 2.9 |
| Raum-Zeit-Ausbeute | [g/l·h] | 89.1 |

Die folgenden Versuche 86 bis 95 wurden unter Stickstoff oder Argon als Schutzgas durchgeführt.

Beispiel 86

Herstellung von 2,6-Bis(2,4-dimethylphenyl)-4-phenylphosphacyclohexan aus 2,6-Bis(2,4-dimethylphenyl)-4-phenylphosphabenzol

Eine Lösung von 10.0 g (26.3 mmol) 2,6-Bis(2,4-dimethylphenyl)-4-phenylphosphabenzol, 1.0 g (2.5 mmol) Ruthenium(III)acetylacetonat und 5 ml Toluol in 100 ml Cyclohexan wurde in einen mit Wasserstoff gespülten Autoklaven überführt. Es wurden bei Raumtemperatur 10 bar Wasserstoff aufgepresst. Unter Rühren mit einem Begasungsrührer wurde das Reaktionsgemisch auf 160 °C erhitzt. Darauf wurde mittels Wasserstoff ein Reaktionsdruck von 80 bar eingestellt. Während der Reaktion wurde der Druck im Autoklaven durch Nachpressen über einen Druckregler auf Druckniveau gehalten. Nach 4 d Reaktionszeit wurde der Autoklav abgekühlt, entspannt und entleert. Der weiße Feststoff im Reaktionsgemisch wurde durch Filtration isoliert. Die erhaltene Reaktionslösung wurde dann im Hochvakuum vollständig eingeengt. Der Rückstand wurde in wenig Cyclohexan gerührt. Der dabei abgeschiedene weiße Feststoff wurde abfiltriert und mehrmals mit wenig Cyclohexan gewaschen. Insgesamt konnten 4.0 g (39 % d. Th.) des Produkts in Form eines weißen Feststoffs isoliert werden.

Beispiel 87

Herstellung von 2,6-Bis(2,4-dimethylphenyl)-1-octyl-4-phenylphosphacyclohexan aus 2,6-Bis(2,4-dimethylphenyl)-4-phenylphosphacyclohexan

Eine Suspension von 0.5 g (1.3 mmol) 2,6-Bis(2,4-dimethylphenyl)-4-phenylphosphacyclohexan in 30 g (267 mmol) 1-Octen wird unter 3 bar Stickstoffdruck 6 h bei 120 °C temperiert. Die GC-Analyse des Reaktionsgemischs zeigt die Bildung von 2,6-Bis(2,4-dimethylphenyl)-1-octyl-4-phenylphosphacyclohexan in einer Ausbeute von 33 % an.

Beispiel 88

Herstellung von 2,6-Bis(2,4-dimethylphenyl)-1-octyl-4-phenylphosphacyclohexan aus 2,6-Bis(2,4-dimethylphenyl)-4-phenylphosphacyclohexan

Zu einem Gemisch aus 0.5 g (1.3 mmol) 2,6-Bis(2,4-dimethylphenyl)-4-phenylphosphacyclohexan und 0.30 g (2.7 mmol) 1-Octen in 0.8 ml Toluol tropft man bei einer Temperatur von 82 °C über einen Zeitraum von 4 h hinweg eine Lösung von 4 mg (0.024 mmol) 2,2'-Azoisobutyronitril (Vazo 64) in 0.8 ml Toluol. Es resultiert eine klare, schwach gelbe Lösung. Die GC-Analyse des Reaktionsgemischs zeigt die Bildung von 2,6-Bis(2,4-dimethylphenyl)-1-octyl-4-phenylphosphacyclohexan in einer Ausbeute von 80 % an.

Beispiel 89

Herstellung von 2,6-Bis(2,4-dimethylphenyl)-1-[2-(2-Methoxyethoxy)ethyl]-4-phenylphosphacyclohexan aus 2,6-Bis (2,4-dimethylphenyl)-4-phenylphosphacyclohexan

Zu einem Gemisch aus 0.30 g (0.77 mmol) 2,6-Bis(2,4-dimethylphenyl)-4-phenylphosphacyclohexan und 0.096 g (0.94 mmol) Ethylenglycolmethylvinylether in 0.8 ml Toluol tropft man bei einer Temperatur von 82 °C über einen Zeitraum von 4 h hinweg eine Lösung von 4 mg (0.024 mmol) 2,2'-Azoisobutyronitril (Vazo 64) in 0.8 ml Toluol. Es resultiert eine klare, schwach rote Lösung. Die GC-Analyse des Reaktionsgemischs zeigt die Bildung von 2,6-Bis(2,4-dimethylphenyl)-1-[2-(2-Methoxyethoxy)ethyl]-4-phenylphosphacyclohexan in einer Ausbeute von 61 % an.

Beispiel 90

Herstellung von 1,3-Di[2,6-bis(2,4-dimethylphenyl)-4-phenylphosphacyclohexan-1-yl]-propan aus 2,6-Bis(2,4-dimethylphenyl)-4-phenylphosphacyclohexan

Eine Suspension von 3.1 g (7.8 mmol) 2,6-Bis(2,4-dimethylphenyl)-4-phenylphosphacyclohexan (Beispiel 86) und 3.7 ml einer 2.5-molaren Butyllithium-Lösung in Hexan in einer Lösung aus 15 ml Hexan und 11 ml Tetrahydrofuran wurde 24 h bei ca. 30 °C gerührt. Das Reaktionsgemisch wurde anschließend mit 0.43 g (3.8 mmol) 1,3-Dichlorpropan versetzt und nachfolgend weitere 4 h bei Raumtemperatur gerührt. Nach Filtration des Reaktionsaustrags wurde die erhaltene Lösung mehrfach mit jeweils 50 ml Wasser gewaschen. Die organische Phase wurde im Hochvakuum unter gelindem Erwärmen zur Trockene eingeengt und der Rückstand anschließend über neutralem Aluminiumoxid mit Toluol als Fließmittel gesäult. Nach Einengen der toluolischen Lösung im Hochvakuum und mehrfachem Waschen des Rückstands mit Pentan erhielt man 2.2 g (35 % d. Th.) des Produkts in Form eines weißen, feinkristallinen Pulvers.

Beispiel 91

Herstellung von 1-Butyl-2,6-bis(2,4-dimethylphenyl)-4-phenylphosphacyclohexan aus 2,6-Bis(2,4-dimethylphenyl)-4-phenyl-phosphabenzol

Zu einer auf ca. 10 °C gekühlten Lösung von 3.1 g (8.2 mmol) 2,6-Bis(2,4-dimethylphenyl)-4-phenylphosphabenzol in 100 ml Toluol tropft man langsam unter Rühren 3.9 g einer 15%igen Lösung von n-Butyllithium in n-Hexan. Die Lösung wird dann weitere 20 h bei Raumtemperatur gerührt. Zur Vervollständigung der Reaktion wurden nochmals 1.5 g einer 15%igen Lösung von n-Butyllithium in n-Hexan zugegeben und die Lösung erneut 20 h bei Raumtemperatur gerührt. Die erhaltene Lösung wurde dann unter kräftigem Rühren mit 100 ml Wasser versetzt. Die organische Phase wurde abgetrennt und analysiert. GC-Analyse (NP-Detektor) und $^{31}$P-NMR-Spektroskopie ($\delta$ = -55 und -40 ppm) zeigen die selektive Bildung isomerer 1-Butyl-2,6-bis(2,4-dimethylphenyl)-4-phenylphosphacyclohexadiene an. 40 g der zuvor erhaltenen toluolischen Lösung wurden Zusammen mit 0.10 g (0.25 mmol) Ruthenium(III)acetylacetonat in einem mit Wasserstoff gespülten 100 ml Autoklaven überführt und bei Raumtemperatur 5 bar Wasserstoff aufgepresst. Unter kräftigem Rühren mit einem Begasungsrührer wurde das Reaktionsgemisch innerhalb von 30 min auf 160 °C erhitzt. Darauf wurde mittels Wasserstoff ein Reaktionsdruck von 80 bar eingestellt. Während der Reaktion wurde der Druck im Autoklaven durch Nachpressen über einen Druckregler auf Druckniveau gehalten. Nach 24 h Reaktionszeit wurde der Autoklav abgekühlt, entspannt und entleert. GC-Analyse (NP-Detektor) und $^{31}$P-NMR-Spektroskopie ($\delta$ = -29) der Reaktionslösung zeigen die selektive Bildung von 1-Butyl-2,6-bis(2,4-dimethylphenyl)-4-phenylphosphacyclohexan an. Nach Entfernen der flüchtigen Bestandteile im Ölpumpenvakuum und Kugelrohrdestillation des erhaltenen Rückstands bei 300 °C und 2 mbar erhält man 0.4 g des Produkts.

Beispiel 92

Herstellung von 1-(2-Methylpropyl-2,6-bis(2,4-dimethylphenyl)-4-phenylphosphacyclohexan aus 2,6-Bis(2,4-dimethylphenyl)-4-phenylphosphabenzol

Zu einer Lösung von 0.74 g (2.0 mmol) 2,6-Bis(2,4-dimethylphenyl)-4-phenylphosphabenzol in 50 ml Toluol tropft man bei Raumtemperatur langsam unter Rühren 3.0 ml einer 1.3 M-Lösung von sek.-Butyllithium in Cyclohexan. Die Lösung wird dann für weitere 3 h bei Raumtemperatur gerührt. Zur Vervollständigung der Reaktion wurden nochmals 3.0 ml einer 1.3 M-Lösung von sek.-Butyllithium in Cyclohexan zugegeben und die Lösung weitere 17 h bei Raumtemperatur gerührt. Die erhaltene Lösung wurde dann unter kräftigem Rühren mit 70 ml Wasser versetzt. Die organische Phase wurde abgetrennt und analysiert. Eine GC-Analyse (NP-Detektor) zeigt die selektive Bildung isomerer 1-(2-Methylpropyl)-2,6-bis(2,4-dimethylphenyl)-4-phenylphosphacyclohexadiene an.

40 g der zuvor erhaltenen toluolischen Lösung wurden zusammen mit 0.10 g (0.25 mmol) Ruthenium(III) acetylacetonat in einem mit Wasserstoff gespülten 100 ml Autoklaven überführt und bei Raumtemperatur 5 bar Wasserstoff aufgepresst. Unter kräftigem Rühren mit einem Begasungsrührer wurde das Reaktionsgemisch innerhalb von 30 min auf 160 °C erhitzt. Darauf wurde mittels Wasserstoff ein Reaktionsdruck von 70 bar eingestellt. Während der Reaktion wurde der Druck im Autoklaven durch Nachpressen über einen Druckregler auf Druckniveau gehalten. Nach 24 h Reaktionszeit wurde der Autoklav abgekühlt, entspannt und entleert. GC-Analyse (NP-Detektor) und $^{31}$P-NMR-Spektroskopie ($\delta$ = -18 und -14) der Reaktionslösung zeigen die selektive Bildung isomerer 1-(2-Methylpropyl)-2,6-bis (2,4-dimethylphenyl)-4-phenylphosphacyclohexane an. Nach Entfernen der flüchtigen Bestandteile im Ölpumpenvakuum und Kugelrohrdestillation des erhaltenen Rückstands bei 300 °C und 2 mbar erhält man 0.2 g des Produkts.

Beispiel 93

Herstellung von 1-Butyl-2,6-bis(2,4-dimethylphenyl)-4-phenylphosphacyclohexan aus 2,6-Bis(2,4-dimethylphenyl)-4-phenyl-phosphabenzol

Zu 3.1 g (128 mmol) Magnesiumspänen in 20 ml THF (Tetrahydrofuran) tropft man langsam eine Lösung von 5.91 g (64 mmol) Butylchlorid in 40 ml THF. Das Reaktionsgemisch wird anschließend 2 h unter Rückfluss gekocht. 7.0 g der erhaltenen Reaktionslösung werden dann bei Raumtemperatur zu einer Lösung von 1.65 g (4.3 mmol) 2,6-Bis (2,4-dimethylphenyl)-4-phenylphosphabenzol in 20 ml THF getropft. Die Lösung wird dann für weitere 16 h bei Raumtemperatur gerührt. Zur Vervollständigung der Reaktion wurden nochmals 7.1 g der Grignard-Lösung zugegeben und das Reaktionsgemisch weitere 4 h bei Raumtemperatur gerührt. Die erhaltene Lösung wird dann unter kräftigem Rühren mit 30 ml Wasser versetzt. GC-Analyse (NP-Detektor) und $^{31}$P-NMR-Spektroskopie (δ = -55 und -40 ppm) zeigen die selektive Bildung isomerer 1-Butyl-2,6-bis(2,4-dimethylphenyl)-4-phenylphosphacyclohexadiene an.

Der erhaltene Reaktionsaustrag wurde zusammen mit 0.17 g (0.43 mmol) Ruthenium(III)acetylacetonat in einem mit Wasserstoff gespülten 100 ml Autoklaven überführt und bei Raumtemperatur 5 bar Wasserstoff aufgepresst. Unter kräftigem Rühren mit einem Begasungsrührer wurde das Reaktionsgemisch innerhalb von 30 min auf 120 °C erhitzt. Darauf wurde mittels Wasserstoff ein Reaktionsdruck von 60 bar eingestellt. Während der Reaktion wurde der Druck im Autoklaven durch Nachpressen über einen Druckregler auf Druckniveau gehalten. Nach 24 h Reaktionszeit wurde der Autoklav abgekühlt, entspannt und entleert. Der Reaktionsaustrag wurde mit 50 ml Toluol versetzt. Die organische Phase wurde abgetrennt und die wässrige Phase zweimal mit Toluol nachgewaschen. Nach Entfernen der flüchtigen Bestandteile der vereinigten organischen Phasen im Ölpumpenvakuum und Kugelrohrdestillation des erhaltenen Rückstands bei 250 °C und 0.07 mbar erhält man 0.62 g des Produkts. GC-Analyse (NP-Detektor) und $^{31}$P-NMR-Spektroskopie (δ = -31) bestätigen die Bildung von 1-Butyl-2,6-bis(2,4-dimethylphenyl)-4-phenylphosphacylcohexan.

Beispiel 94

Herstellung von 1-Polyethylen-2,6-bis(2,4-dimethylphenyl)-4-phenylphosphacyclohexadien aus 2,6-Bis(2,4-dimethylphenyl)-4-phenylphosphabenzol

Eine Lösung von 1,38 g (5.0 mmol) einer 2.5 M-Lösung von n-Butyllithium in Hexan und 0.62 g (5.3 mmol) N,N,N',N'-Tetramethylethylendiamin in 30 ml Hexan wurden in einen mit Inertgas gespülten Autoklaven gefüllt. Unter kräftigem Rühren der Reaktionslösung mit einem Begasungsrührer wurden bei Raumtemperatur 2 bar Ethen aufgepresst. Während der Reaktion wurde der Druck im Autoklaven durch Nachpressen über einen Druckregler auf Druckniveau gehalten. Nach 6 h Reaktionszeit wurde der Autoklav entspannt und mit Inertgas gespült. Über eine Schleuse wurde dann eine Lösung von 1.52 g (4.0 mmol) 2,6-Bis(2,4-dimethylphenyl)-4-phenylphosphabenzol in 10 ml Toluol zugegeben. Das Reaktionsgemisch wurde 12 h bei 80 °C gerührt, anschließend mit 50 ml Wasser versetzt und zusätzlich mit 20 ml Toluol verdünnt. Danach wurden alle flüchtigen Bestandteile im Ölpumpenvakuum entfernt. $^{31}$P-NMR-Spektroskopie (δ = -50, -40) des erhaltenen Rückstands zeigt die selektive Bildung isomerer 1-Polyethylen-2,6-bis(2,4-dimethylphenyl)-4-phenylphosphacyclohexadiene an.

Beispiel 95

Umsetzung von 2,6-Bis(2,4-dimethylphenyl)-4-phenylpyryliumtetrafluoroborat mit Octylphosphan, Hydrierung und Reduktion zum 1-Octyl-2,6-bis(2,4-dimethylphenyl)-4-phenylphosphacyclohexan

In einen mit Argon-Schutzgas gespülten Autoklaven wurde 20 g (44 mmol) 2,6-Bis(2,4-dimethylphenyl)-4-phenylpyryliumtetrafluoroborat in 120 ml Butanol gelöst, mit 10 g (68 mmol) Octylphosphan versetzt und anschließend 6 h bei 120 °C gerührt. Es wurden weitere 5 g (34 mmol) Octylphosphan zugegeben und die Reaktionslösung wurde für weitere 6 h bei 120 °C gerührt. Nach Abkühlen auf Raumtemperatur wurde der unlösliche Feststoff, bei dem es sich um unumgesetztes Pyryliumsalz handelte, durch Filtration abgetrennt. Die erhaltene Lösung wurde dann im Vakuum unter Erwärmen bis zur Trockene eingeengt. Der Rückstand wurde in einem Gemisch aus Toluol und Dichlormethan gelöst und die Lösung wurde solange mit Wasser gewaschen, bis die wässrige Phase pH-neutral war. Die erhaltene Lösung wurde nach Filtration über Kieselgel im Vakuum unter leichtem Erwärmen bis zur Trockene eingeengt. Der erhaltene Feststoff wurde mehrmals mit wenig Pentan gewaschen und anschließend im Vakuum getrocknet. Man erhielt 22 g eines grünen Feststoffs.

6.33 g des zuvor erhaltenen Feststoffs wurden in 150 ml Toluol gelöst, mit 1.0 g (2.5 mmol) Ruthenium(III)acetylacetonat versetzt und in einen mit Wasserstoff gespülten 100 ml Autoklaven überführt. Es wurden bei Raumtemperatur 10 bar Wasserstoff aufgepresst. Unter Rühren mit einem Begasungsrührer wurde das Reaktionsgemisch innerhalb von 30 min auf 140 °C erhitzt. Darauf wurde mit Wasserstoff ein Reaktionsdruck von 80 bar eingestellt. Während der Reaktion wurde der Druck im Autoklaven durch Nachpressen über einen Druckregler auf Druckniveau gehalten. Nach 72 h wurde der Autoklav abgekühlt, entspannt und entleert.

Unter Argon-Schutzgas wurden 30 g der zuvor erhaltenen Lösung in 200 g Toluol auf ca. -5 °C abgekühlt. Hierzu gibt man langsam 2.0 g (20 mmol) Triethylamin und anschließend 1.3 g (9.8 mmol) Trichlorsilan. Das Reaktionsgemisch wurde anschließend 24 h bei Raumtemperatur gerührt. Es wurde weitere 1.5 g (11.1 mmol) Trichlorsilan zugegeben und die Reaktionslösung 24 h bei Raumtemperatur gerührt. Das Reaktionsgemisch wurde dann über Kieselgur filtriert. Die erhaltene Lösung wurde im Vakuum auf ein Viertel des ursprünglichen Volumens eingeengt und dann mit 100 ml wässriger Natronlauge versetzt. Die organische Phase wurde abgetrennt und über ein Aluminiumoxidbett filtriert. Nach Entfernen der flüchtigen Bestandteile im Ölpumpenvakuum und Kugelrohrdestillation des erhaltenen Rückstands bei 300 °C und 0.03 mbar erhält man 0.12 g des Produkts. GC-Analyse (NP-Detektor) und $^{31}P$-NMR-Spektroskopie bestätigen die Bildung von 1-Octyl-2,6-bis(2,4-dimethylphenyl)-4-phenylphosphacyclohexan.

Beispiel 96

Herstellung von 1-Dodecyl-2,6-bis(2,4-dimethylphenyl)-4-phenylphosphacyclohexan aus 2,6-Bis(2,4-dimethylphenyl)-4-phenylphosphacyclohexan

Zu einem Gemisch aus 2.02 g (5.2 mmol) 2,6-Bis(2,4-dimethylphenyl)-4-phenylphosphacyclohexan und 1.20 g (7.1 mmol) 1-Dodecen in 1.5 g Toluol tropft man bei einer Temperatur von 92 °C über einen Zeitraum von 10 h hinweg 13.2 g einer Lösung von 0.039 g (0.24 mmol) 2,2'-Azoisobutyronitril (Vazo 64) in 19.3 g Toluol. Anschließend rührt man weitere 10 h bei 92 °C nach. Es resultiert eine klare, graue Lösung. Die erhaltene Reaktionslösung wurde im Hochvakuum bis zur Trockene eingeengt. Der verbleibende Feststoff wurde in 140 ml Ethanol suspendiert und dann mit 60 ml Dichlormethan versetzt, wobei eine klare Lösung resultiert. Die Lösung wurde im Hochvakuum solange eingeengt, bis sich das Produkt als weißer Feststoff abschied. Der abgeschiedene Feststoff wurde abfiltriert und im Hochvakuum getrocknet. Es konnten 1.9 g (65.5 d. Th.) des Produkts in Form eines weißen Feststoffs isoliert werden.

Beispiel 97

Herstellung von 2,6-Bis(2,4-dimethylphenyl)-4-phenyl-1-[methylpoly(ethylenglycol)-500]phosphacyclohexan aus 2,6-Bis(2,4-dimethylphenyl)-4-phenylphosphacyclohexan

Zu einem Gemisch aus 0.15 g (0.39 mmol) 2,6-Bis(2,4-dimethylphenyl)-4-phenylphosphacyclohexan und 0.85 g (1.45 mmol) Poly(ethylenglycol)-500-methyl-vinylether in 1 ml Toluol tropft man bei einer Temperatur von 82 °C über einen Zeitraum von 18 h hinweg 9 ml einer Lösung von 0.035 g (0.21 mmol) 2,2'-Azoisobutyronitril (Vazo 64) in 16 ml Toluol. Es resultiert eine klare, gelbe Lösung. $^{31}P$-NMR-Spektroskopie zeigt die selektive Bildung isomerer 2,6-Bis(2,4-dimethylphenyl)-4-phenyl-1-[methylpoly(ethylenglycol)]phosphacyclohexane in einer Ausbeute von ca. 90 % an.

Beispiel 98

Herstellung von 2,6-Bis(2,4-dimethylphenyl)-4-phenyl-1-[methylpoly(ethylenglycol)-500]phosphacyclohexan aus 2,6-Bis(2,4-dimethylphenyl)-4-phenylphosphacyclohexan)

Zu einem Gemisch aus 0.15 g (0.39 mmol) 2,6-Bis(2,4-dimethylphenyl)-4-phenylphosphacyclohexan und 0.32 g (0.54 mmol) Poly(ethylenglycol)-500-methyl-vinylether in 1 ml Toluol tropft man bei einer Temperatur von 93 °C über einen Zeitraum von 18 h hinweg 11.8 ml einer Lösung von 0.096 g (0.58 mmol) 2,2'-Azoisobutyronitril (Vazo 64) in 38 ml Toluol. Es resultiert eine klare, gelbe Lösung. $^{31}P$-NMR-Spektroskopie zeigt die selektive Bildung isomerer 2,6-Bis (2,4-dimethylphenyl)-4-phenyl-1-[methylpoly(ethylenglycol)]phosphacyclohexane in einer Ausbeute von ca. 66 % an. Der erhaltene Austrag wurde mit 5 ml Wasser und bei Raumtemperatur für 1 h kräftig gerührt. Nach Phasentrennung ergab sich für die organische Phase ein Phosphor-Gehalt von 1000 ppm und für die wässrige Phase ein Phosphor-Gehalt von 80 ppm.

Beispiel 99

Herstellung von 1-Dodecyl-2,6-bis(2,4-dimethylphenyl)-4-phenylphosphacyclohexan aus 2,6-Bis(2,4-dimethylphenyl)-4-phenylphosphabenzol

Zu 9.4 g (387 mmol) Magnesiumspänen in 100 ml THF tropft man langsam unter Rühren 50.4 g (202 mmol) 1-Dodecylbromid. Nach Beendigung des Zutropfens wurde das Reaktionsgemisch mit weiteren 100 ml THF verdünnt und dann 2 h unter Rückfluss gekocht. Die erhaltene graue Lösung wurde zu einer Lösung von 53.2 g 2,6-Bis(2,4-dimethylphenyl)-4-phenylphosphabenzol in 100 ml THF getropft, wobei eine tiefrote Reaktionslösung resultiert, die 16 h bei Raumtemperatur nachgerührt und anschließend unter kräftigem Rühren mit 350 ml Wasser gequenscht wurde. Zur besseren Phasentrennung wurde die Emulsion mit 200 ml Toluol und 1500 ml Ethylacetat versetzt und anschließend viermal mit je 500 ml einer wässrigen, gesättigten Ammoniumchlorid-Lösung gewaschen. Die erhaltene organische Phase wurde dann im Ölpumpenvakuum bis zur Trockene eingeengt. Der erhaltene Rückstand wurde in 150 ml Toluol aufgenommen. $^{31}$P-NMR-Spektroskopie zeigt die selektive Bildung isomerer 1-Dodecyl-2,6-bis(2,4-dimethylphenyl)-4-phenylphosphacyclohexadiene an.

125 g der zuvor erhaltenen toluolischen Lösung wurden zusammen mit 0.52 g (1.3 mmol) Ruthenium(III) acetylacetonat und 50 ml 1-Octen in einem mit Wasserstoff gespülten 300 ml Autoklaven überführt. Es wurden bei Raumtemperatur 10 bar Wasserstoff aufgepresst. Unter kräftigem Rühren mit einem Begasungsrührer wurde das Reaktionsgemisch innerhalb von 30 min auf 110 °C erhitzt. Darauf wurde mittels Wasserstoff ein Reaktionsdruck von 80 bar eingestellt. Während der Reaktion wurde der Druck im Autoklaven durch Nachpressen über einen Druckregler auf Druckniveau gehalten. Nach 72 h Reaktionszeit wurde der Autoklav abgekühlt, entspannt und entleert. Die erhaltene Lösung wurde über Aluoxid filtriert, anschließend im Ölpumpenvakuum eingeengt und in Toluol aufgenommen. $^{31}$P-NMR-Spektroskopie ($\delta$= -30) der Lösung zeigt die selektive Bildung isomerer 1-Dodecyl-2,6-bis(2,4-dimethylphenyl)-4-phenylphosphacyclohexane an. Eine Analyse der Lösung lieferte einen Ruthenium-Gehalt von 190 ppm und einen Phosphor-Gehalt von 11000 ppm (P/Ru-Molverhältnis 192).

Beispiel 100

Herstellung von 1-Polyethylen-2,6-bis(2,4-dimethylphenyl)-4-phenylphosphacyclohexadien aus 2,6-Bis(2,4-dimethylphenyl)-4-phenylphosphabenzol

Eine Lösung von 2.68 g (9.65 mmol) einer 2.5 M-Lösung von n-Butyllithium in Hexan und 1.22 g (10.5 mmol) N,N,N',N'-Tetramethylethylendiamin in 60 ml Cyclohexan, welches zuvor über Natrium getrocknet wurde, wurden in einen mit Inertgas gespülten Glasautoklaven überführt. Unter kräftigem Rühren der Reaktionslösung mit einem Begasungsrührer wurden bei Raumtemperatur 2 bar Ethen aufgepresst. Während der Reaktion wurde der Druck im Autoklaven durch Nachpressen über einen Druckregler auf Druckniveau gehalten. Nach 12 h Reaktionszeit wurde der Autoklav entspannt und mit Inertgas gespült. Über eine Schleuse wurde dann eine Lösung von 2.08 g (5.47 mmol) 2,6-Bis(2,4-dimethylphenyl)-4-phenylphosphabenzol in 100 ml Cyclohexan zugegeben. Das Reaktionsgemisch wurde 20 h bei 80 °C gerührt, anschließend mit 50 ml Methanol versetzt und zusätzlich mit 120 ml Toluol verdünnt. Die Reaktionslösung wurde dann dreimal mit je 50 ml Wasser gewaschen. Danach wurden alle flüchtigen Bestandteile im Ölpumpenvakuum entfernt. Der verbleibende Rückstand wurde in 100 ml Methanol und 60 ml Dichlormethan gelöst. Die Lösung wurde im Hochvakuum solange eingeengt, bis sich ein weißer Feststoff abschied. Der abgeschiedene Feststoff wurde abfiltriert, mit wenig Methanol nachgewaschen und im Hochvakuum getrocknet. Zur Abtrennung von nicht-umgesetztem Polyethylen wurde der Feststoff erneut in 100 ml Toluol und 60 ml Dichlormethan aufgerührt und dann über Kieselgur abfiltriert. Die erhaltene orangefarbene Lösung wurde im Hochvakuum bis zur Trokkene eingeengt, und anschließend in 80 ml Methanol und 80 ml Dichlormethan gelöst. Die Lösung wurde im Hochvakuum solange eingeengt, bis sich ein weißer Feststoff abschied. Der abgeschiedene Feststoff wurde abfiltriert, mit wenig Methanol nachgewaschen und im Hochvakuum getrocknet. Es konnten 1.42 g des Produktes in Form eines blassgelben Feststoffes isoliert werden. $^{31}$P-NMR-Spektroskopie ($\delta$= -40, -50) zeigt die selektive Bildung isomerer 1-Polyethylen-2,6-bis(2,4-dimethylphenyl)-4-phenylphosphacyclohexadiene an. Eine GPC-Analyse (Eichung mit Polybutadien-Standard der Fa. Polymer Laboratories) lieferte ein zahlengemitteltes Molekulargewicht $M_n$ von ca. 830.

Beispiel 101

Herstellung einer Rhodium/1-Polybutylen-2,6-bis(2,4-dimethylphenyl)-4-phenylphosphacyclohexan-Lösung aus 2,6-Bis (2,4-dimethylphenyl)-4-phenylphosphabenzol

In einem 10 1-Kessel wurden 2400 g Cyclohexan, 12 ml THF und 180 g (3.33 mol) Butadien vorgelegt und

anschließend mit 11.4 ml (16 mmol) einer 1.4 M-Lösung von sek.-Buthyllithium in Cyclohexan versetzt. Die Lösung wurde 4 h bei ca. 40 °C gerührt und anschließend mit 4.0 g (10.5 mmol) 2,6-Bis(2,4-dimethylphenyl)-4-phenylphosphabenzol versetzt. Man rührt 24 h bei 60 °C und versetzt dann das Reaktionsgemisch mit einem Ethanol/Wasser-Gemisch. $^{31}$P-NMR-Spektroskopie des erhaltenen Reaktionsgemisches zeigt die selektive Bildung isomerer 1-Polybutadien-2,6-bis(2,4-dimethylphenyl)-4-phenylphosphacyclohexadiene in einer Ausbeute von ca. 94 % an. Eine Analyse der Lösung lieferte einen Phosphor-Gehalt von 135 ppm. Eine GPC-Analyse (Eichung mit einem Polystyrol-Eichkit der Fa. Polymer Laboratories und Umrechnung auf Polybutadien mit Mark-Houwink-Konstanten) lieferte ein zahlengemitteltes Molekulargewicht $M_n$ von ca. 12000.

299 g der zuvor erhaltenen Lösung wurde im Ölpumpenvakuum auf ca. 50 ml eingeengt. Die verbleibende Lösung wurde zusammen mit 34 mg (130 mmol) Rhodiumdicarbonylacetylacetonat, 35 ml 1-Octen und 75 ml Toluol in einen mit Wasserstoff gespülten 300 ml Autoklaven überführt. Es wurden bei Raumtemperatur 10 bar Wasserstoff aufgepresst. Unter kräftigem Rühren mit einem Begasungsrührer wurde das Reaktionsgemisch innerhalb von 30 min auf 80 °C erhitzt. Darauf wurde mittels Wasserstoff ein Reaktionsdruck von 80 bar eingestellt. Während der Reaktion wurde der Druck im Autoklaven durch Nachpressen über einen Druckregler auf Druckniveau gehalten. Nach 24 h Reaktionszeit wurde die Reaktionstemperatur unter Beibehalten des Reaktionsdruckes auf 160 °C erhöht. Nach einer weiteren Reaktionszeit von 24 h wurde der Autoklav abgekühlt, entspannt und entleert. Die erhaltene Lösung wurde mit 65 g Oxoöl 9N versetzt, und dann im Ölpumpenvakuum bei ca. 150 °C von Leichtsiedern befreit. $^{31}$P-NMR-Spektroskopie ($\delta$= -30) der Lösung zeigt die selektive Bildung isomerer 1-Polybutylen-2,6-bis(2,4-dimethylphenyl)-4-phenylphosphacyclohexane an. Eine Analyse der Lösung lieferte einen Rhodium-Gehalt von 155 ppm und einen Phosphor-Gehalt von 380 ppm (P/Rh-Molverhältnis 8).

**Patentansprüche**

1. Verfahren zur Hydroformylierung von Olefinen durch Umsetzung von Olefinen mit CO und $H_2$ in Gegenwart eines Rhodiumkomplexes als Katalysator bei Temperaturen von 50 bis 180°C und Drücken von 5 bis 100 bar, wobei der Katalysator wenigstens einen Liganden mit wenigstens einem unverbrückten Phosphacyclohexan-Strukturelement umfasst, ausgewählt unter
Phosphacyclohexanen der allgemeinen Formeln I und II

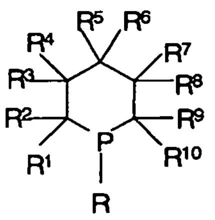

(I)

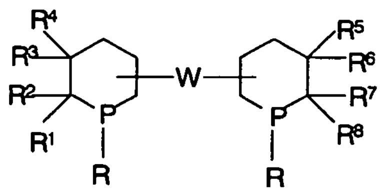

(II)

mit der Bedeutung

R
Wasserstoff, $C_{1-100}$-Alkyl, Cycloalkyl, Heterocycloalkyl, $C_{7-20}$-Aralkyl, $C_{7-20}$-Alkaryl, $C_{6-12}$-Aryl, Hetaryl, $W'COO^-M^+$, $W'SO_3^-M^+$, $W'PO_3^{2-}M^+_2$, $W'NR'_3{}^+X^-$, W'OR', $W'NR'_2$, W'COOR', W'SR', $W'(CHR'CH_2O)$,R', W'$(CH_2NR')_XR'$, $W'(CH_2CH_2NR')_xR'$, $W'((CH_2)_4O)_XR'$ oder W'COR',
wobei in der Formel II die Reste R auch anstelle von oder zusätzlich zu der Gruppe W zusammen eine Brücke mit 1 bis 20 Kohlenstoffatomen bilden können, die Bestandteil einer cyclischen oder aromatischen Gruppe sein kann und durch Heteroatome unterbrochen sein kann,
wobei in der Formel II die Reste R auch anstelle von oder zusätzlich zu der Gruppe W zusammen für eine Polyoxyalkylen- oder Polyalkylenimin-Brücke mit mindestens 21 Kohlenstoffatomen stehen können,
**R**$^1$ bis $R^{10}$
unabhängig voneinander Wasserstoff, $C_{1-20}$-Alkyl, $C_{7-20}$-Aralkyl, $C_{7-20}$-Alkaryl, $C_{6-12}$-Aryl, wobei eines oder mehrere Kohlenstoffatome durch Heteroatome ersetzt sein können, $W'COO^-M^+$, $W'SO_3^-M^+$, $W'PO_3^{2-}M^+_2$, $W'NR'_3{}^+X^-$, W'OR', $W'NR'_2$, W'COOR', W'SR', $W'(CHR'CH_2O)_xR'$, $W'(CH_2NR')_xR'$, $W'(CH_2CH_2NR')_xR'$, W'($(CH_2)_4O)_xR'$, W'Halogen, $W'NO_2$, W'COR' oder W'CN, wobei mindestens zwei der Reste $R^1$ bis $R^{10}$ von Was-

serstoff verschieden sind,
wobei in den Resten R und $R^1$ bis $R^{10}$ ein oder mehrere Wasserstoffatome durch Fluor ersetzt sein können,
W und W' unabhängig voneinander Einfachbindungen oder Brücken mit 1 bis 20 Kohlenstoffatomen, die Bestandteil einer cyclischen oder aromatischen Gruppe sein können und durch Heteroatome unterbrochen sein können,
wobei W auch für eine Polyoxyalkylen- oder Polyalkylenimin-Brücke mit mindestens 21 Kohlenstoffatomen stehen kann,

R' Wasserstoff, $C_{1-20}$-Alkyl, Carbonylalkyl, Cycloalkyl oder Aryl,
M+ Kationäquivalent,
$X^-$ Anionäquivalent,
x Zahl von 1 bis 240,

wobei zwei geminale Reste $R^1$ bis $R^{10}$ eine Oxo-Gruppe bilden können und einer oder mehrere der Reste R und $R^1$ bis $R^{10}$ eine zusätzliche, zur Koordination befähigte dreibindige Phosphor- oder Stickstoffgruppierung aufweisen können,
wobei jeweils zwei vicinale Reste zu anellierten aliphatischen Ringen verbunden sein können,
wobei in den Verbindungen der allgemeinen Formel II auch zwei oder mehr Brücken W vorliegen können und wobei die nicht an die Brücke(n) W gebundenen Atome der Phosphacyclohexanringe auch wie für $R^1$ bis $R^{10}$ definiert substituiert sein können,
wobei in den Verbindungen der Formel I auch einer der Reste R oder $R^1$ bis $R^{10}$ und in den Verbindungen der Formel II auch einer der Reste R oder $R^1$ bis $R^8$ oder die beiden Reste R gemeinsam oder eine Gruppe W auch für einen Polymerrest mit einem zahlenmittleren Molekulargewicht im Bereich von 500 bis 50000 stehen kann, aufgebaut aus Wiederholungseinheiten, die sich von Monomeren ableiten, die ausgewählt sind unter Mono- und Diolefinen, Vinylaromaten, Estern $\alpha,\beta$-ethylenisch ungesättigter Mono- und Dicarbonsäuren mit $C_1$-$C_{30}$-Alkanolen, N-Vinylamiden, N-Vinyllactamen, unter Ringöffnung polymerisierbaren heterocyclischen Verbindungen und Mischungen davon.

**2.** Verfahren nach Anspruch 1, **dadurch gekennzeichnet, dass** die Verbindung der allgemeinen Formel I ausgewählt ist aus Verbindungen der allgemeinen Formel III

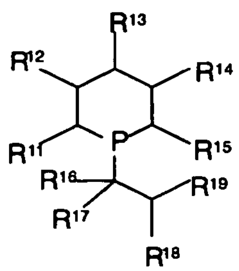

(III)

mit der Bedeutung:

$R^{11}$ bis $R^{19}$
unabhängig voneinander Wasserstoff, $C_{1-20}$-Alkyl, $C_{7-20}$-Aralkyl, $C_{7-20}$-Alkaryl, $C_{6-12}$-Aryl, wobei eines oder mehrere Kohlenstoffatome durch Heteroatome ersetzt sein können, $W'COO^-M^+$, $W'SO_3^-M^+$, $W'PO_3^{2-}M^+_2$, $W'NR'_3{}^+X^-$, W'OR', $W'NR'_2$, W'COOR', W'SR', $W'(CHR'CH_2O)_xR'$, $W'(CH_2NR')_xR'$, $W'(CH_2CH_2NR')_xR'$,

wobei jeweils zwei vicinale Reste $R^{11}$ bis $R^{15}$ und/oder $R^{17}$ und $R^{18}$ und/oder $R^{16}$ und $R^{17}$ und/oder $R^{16}$ und $R^{19}$ und/oder $R^{18}$ und $R^{19}$ zu Ringen verbunden sein können,

W' Einfachbindung oder Brücke mit 1 bis 20 Kohlenstoffatomen, die Bestandteil einer cyclischen oder aromatischen Gruppe sein kann,
R' Wasserstoff oder $C_{1-6}$-Alkyl,
M+ Kation,

$X^-$ Anion,
x Zahl von 1 bis 240,

wobei einer oder mehrere der Reste $R^{11}$ bis $R^{19}$ eine zusätzliche, zur Koordination befähigte dreibindige Phosphor- oder Stickstoffgruppierung aufweisen können,
wobei $R^{18}$ auch $-W'-CR^{20}=CR^{21}R^{22}$, wobei $R^{20}$, $R^{21}$, $R^{22}$ wie vorstehend für $R^{11}$ bis $R^{19}$ definiert sind, bedeuten kann.

**3.** Verfahren nach Anspruch 1, wobei die Verbindungen der Formeln I und II ausgewählt sind unter Verbindungen der allgemeinen Formeln

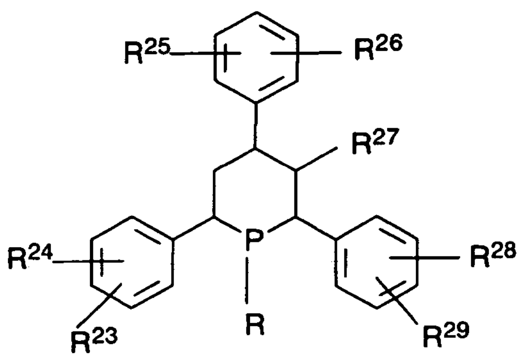

(I.a)

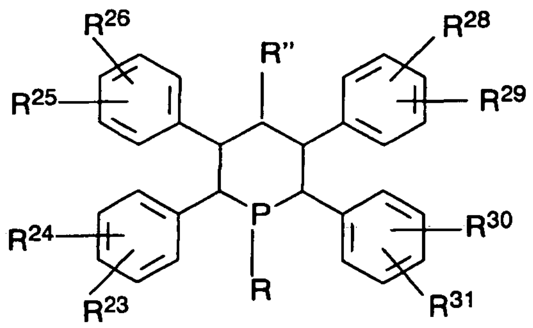

(I.b)

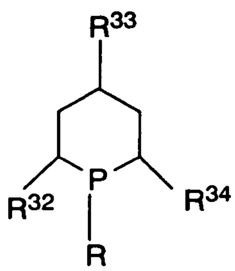

(I.c)

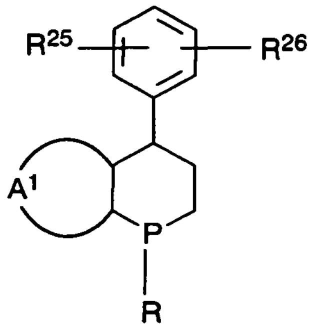

(I.d)

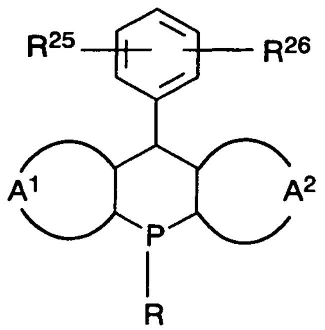

(I.e)

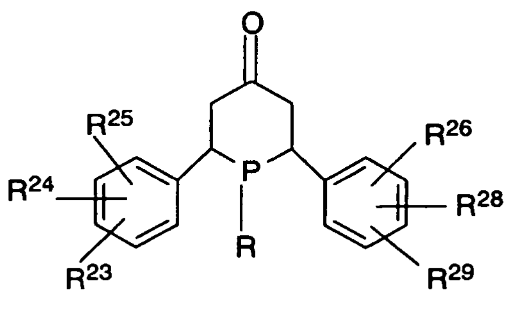

(I.f)

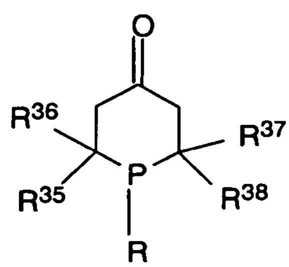

(I.g)

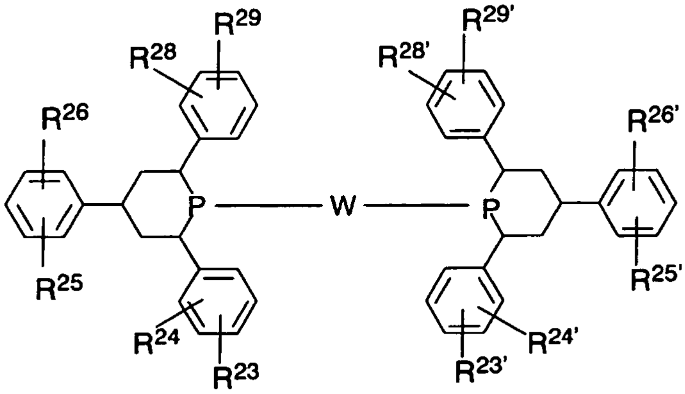

(II.a)

worin

R für $C_{1-20}$-Alkyl, Cycloalkyl, $C_{6-12}$-Aryl, $W'(CHR'CH_2O)_xR'$, $W'((CH_2)_4O)_xR'$ oder einen Polymerrest mit einem zahlenmittleren Molekulargewicht im Bereich von 500 bis 50000, der aufgebaut ist aus Ethylen und/oder Butadien, steht, wobei

W' für eine Einfachbindung oder $C_{1-4}$-Alkylen steht, und
R' für Wasserstoff oder $C_{1-20}$-Alkyl steht,
x für eine ganze Zahl von 1 bis 240 steht,

$R^{23}$, $R^{23'}$, $R^{24}$, $R^{24'}$, $R^{25}$, $R^{25'}$, $R^{26}$, $R^{26'}$, $R^{28}$, $R^{28'}$, $R^{29}$, $R^{29'}$, $R^{30}$ und $R^{31}$ unabhängig voneinander für Wasserstoff, Alkyl, Alkoxy, Carboxyl, Carboxylat, Trifluormethyl, $-SO_3H$, Sulfonat, $NE^1E^2$ oder Alkylen-$NE^1E^2$ stehen, wobei $E^1$ und $E^2$ unabhängig voneinander für Wasserstoff, Alkyl oder Cycloalkyl stehen,
$R^{27}$ für Wasserstoff, Alkyl, Cycloalkyl, Aryl oder Aralkyl steht,
$R^{32}$, $R^{33}$, $R^{34}$, $R^{35}$, $R^{36}$, $R^{37}$ und $R^{38}$ unabhängig voneinander für Alkyl oder Cycloalkyl stehen,
R" für Wasserstoff oder Phenyl steht,
$A^1$ und $A^2$ zusammen mit den benachbarten Kohlenstoffatomen des Phosphacyclohexans, an die sie gebunden wird, für ein anelliertes Ringsystem mit je 1 oder 2 weiteren Ringen stehen,
W für eine Brücke mit 1 bis 20 Kohlenstoffatomen steht, die durch Heteroatome unterbrochen sein kann.

4. Verfahren nach einem der vorhergehenden Ansprüche, wobei der Rest R für einen Polyoxyalkylen- oder Polyalkyleniminrest steht.

5. Verfahren nach einem der Ansprüche 1 bis 3, wobei in den Verbindungen der Formel I einer der Reste R oder $R^1$ bis $R^{10}$ und in den Verbindungen der Formel II einer der Reste R oder $R^1$ bis $R^8$ oder die beiden Reste R gemeinsam oder die Gruppe W für einen Polymerrest mit einem zahlenmittleren Molekulargewicht im Bereich von 500 bis 50000 steht, wobei die Wiederholungseinheiten dieser Polymerreste sich von Monomeren ableiten, die ausgewählt sind unter Mono- und Diolefinen, Vinylaromaten, Estern $\alpha,\beta$-ethylenisch ungesättigter Mono- und Dicarbonsäuren mit $C_1$-$C_{30}$-Alkanolen, N-Vinylamiden, N-Vinyllactamen, unter Ringöffnung polymerisierbaren heterocyclischen Verbindungen und Mischungen davon.

6. Verfahren nach einem der Ansprüche 1 bis 3, wobei in den Verbindungen der Formel I einer der Reste R oder $R^1$ bis $R^{10}$ und in den Verbindungen der Formel II einer der Reste R oder $R^1$ bis $R^8$ oder die beiden Reste R gemeinsam oder eine Gruppe W für einen Polyolefinrest steht.

7. Verfahren nach einem der Ansprüche 1 bis 6, wobei der Komplexkatalysator und überschüssige Liganden aus dem Destillationssumpf des Reaktionsgemischs rückgeführt werden, wobei der Katalysator und überschüssiger Ligand durch Ultrafiltration des Destillationssumpfs gewonnen und das Retentat zurückgeführt wird.

8. Verfahren nach einem der Ansprüche 1 bis 6, wobei die Aufarbeitung des Reaktionsgemischs direkt über eine Ultrafiltration erfolgt.

9. Phosphacyclohexane der allgemeinen Formel I, II oder III, wie sie in einem der Ansprüche 1 bis 3 definiert sind, wobei mindestens drei der Reste $R^1$ bis $R^{10}$ von Wasserstoff verschieden sind und wobei mindestens zwei der Reste R und $R^1$ bis $R^{10}$ cyclische Strukturen, die aliphatisch, aromatisch oder heterocyclisch sein können, enthalten, ausgenommen:

   - Verbindungen der Formel I, worin $R^5$ und $R^6$ gemeinsam für eine Oxo-Gruppe stehen oder einer der Reste $R^5$ oder $R^6$ für Hydroxyl und der andere für Wasserstoff steht.

10. Phosphacyclohexane, wie in einem der Ansprüche 4 bis 6 definiert.

11. Komplexe von Übergangsmetallen der VIII. Nebengruppe des Periodensystems der Elemente mit Phosphacyclohexanliganden, wie sie in einem der Ansprüche 1 bis 6 definiert sind, wobei mindestens drei der Reste $R^1$ bis $R^{10}$ von Wasserstoff verschieden sind und wobei mindestens zwei der Reste R und $R^1$ bis $R^{10}$ cyclische Strukturen, die aliphatisch, aromatisch oder heterocyclisch sein können, enthalten.

12. Komplexe der allgemeinen Formel X

$$ML_n(CO)_m \qquad X$$

mit der Bedeutung

M Übergangsmetall der VIII. Nebengruppe des Periodensystems der Elemente, vorzugsweise Rhodium,
L Phosphacyclohexanligand, wie er in einem der Ansprüche 1 bis 6 definiert ist, wobei mindestens drei der Reste $R^1$ bis $R^{10}$ von Wasserstoff verschieden sind und wobei mindestens zwei der Reste R und $R^1$ bis $R^{10}$ cyclische Strukturen, die aliphatisch, aromatisch oder heterocyclisch sein können, enthalten,
n,m unabhängig ganze Zahlen von 1 bis 3.

13. Katalysator, umfassend wenigstens einen Komplex eines Metalls der VIII. Nebengruppe mit wenigstens einem Liganden wie in einem der Ansprüche 1 bis 6 definiert, wobei mindestens drei der Reste $R^1$ bis $R^{10}$ von Wasserstoff verschieden sind und wobei mindestens zwei der Reste R und $R^1$ bis $R^{10}$ cyclische Strukturen, die aliphatisch, aromatisch oder heterocyclisch sein können, enthalten.

14. Katalysator nach Anspruch 13, der zusätzlich wenigstens einen weiteren Liganden, ausgewählt unter Halogeniden, Aminen, Carboxylaten, Acetylacetonat, Aryl- oder Alkylsulfonaten, Hydrid, CO, Olefinen, Dienen, Cycloolefinen, Nitrilen, N-haltigen Heterocyclen, Aromaten und Heteroaromaten, Ethern, $PF_3$, Phospholen, Phosphabenzolen sowie ein-, zwei- und mehrzähnigen Phosphin-, Phosphinit-, Phosphonit-, Phosphoramidit und Phosphitliganden aufweist.

15. Katalysator nach einem der Ansprüche 13 oder 14, **dadurch gekennzeichnet, dass** das Metall der VIII. Nebengruppe ausgewählt ist unter Ruthenium, Iridium, Rhodium, Nickel oder Palladium.

16. Verfahren zur Herstellung von Komplexen gemäß Anspruch 12 durch Umsetzung eines Komplexvorläufers des Metalls M mit den Liganden L in Gegenwart eines CO-haltigen Gases.

17. Verwendung von Komplexen gemäß Anspruch 11 oder 12 zur Hydroacylierung, Hydrocyanierung, Hydroamidierung, Hydroveresterung, Aminolyse, Alkoholyse, Hydrocarbonylierung, Hydroxycarbonylierung, Carbonylierung, Isomerisierung oder Transferhydrierung von Olefinen.

## Claims

1. A process for the hydroformylation of olefins by reacting olefins with CO and $H_2$ in the presence of a rhodium complex as catalyst at from 50 to 180°C and pressures of from 5 to 100 bar, where the catalyst comprises at least one ligand having at least one unbridged phosphacyclohexane structural element selected from among phosphacyclohexanes of the formulae I and II

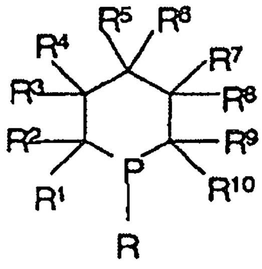

(I)

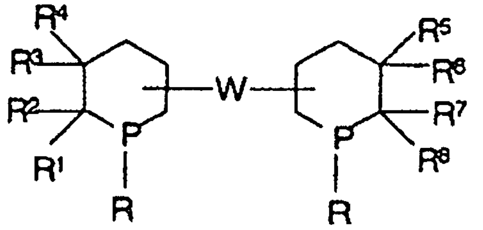

(II)

where

R

is hydrogen, $C_{1-100}$-alkyl, cycloalkyl, heterocycloalkyl, $C_{7-20}$-aralkyl, $C_{7-20}$-alkaryl, $C_{6-12}$-aryl, hetaryl, $W'COO^-M^+$, $W'SO_3^-M^+$, $W'PO_3^{2-}M^+_2$, $W'NR'_3{}^+X^-$, W'OR', $W'NR'_2$, W'COOR', W'SR', $W'(CHR'CH_2O)_xR'$, $W'(CH_2NR')_xR'$ $W'(CH_2CH_2NR')_xR'$, $W'((CH_2)_4O)_xR'$ or W'COR',

where the radicals R in the formula II may also, in place of or in addition to the group W, together form a bridge having from 1 to 20 carbon atoms which may be part of a cyclic or aromatic group and may be interrupted by heteroatoms,

where the radicals R in the formula II may also, in place of or in addition to the group W, together be a polyoxyalkylene or polyalkylenimine bridge having at least 21 carbon atoms,

$R^1$ to $R^{10}$

are each, independently of one another, hydrogen, $C_{1-20}$-alkyl, $C_{7-20}$-aralkyl, $C_{7-20}$-alkaryl, $C_{6-12}$-aryl, where one or more carbon atoms may be replaced by heteroatoms, $W'COO^-M^+$, $W'SO_3^-M^+$, $W'PO_3^{2-}M^+_2$, $W'NR'_3{}^+X^-$, W'OR', $W'NR'_2$, W'COOR', W'SR', W' $(CHR'CH_2O)_xR'$, $W'(CH_2NR')_xR'$, $W'(CH_2CH_2NR')_xR'$, $W'((CH_2)_4O)_xR'$, W'halogen, $W'NO_2$, W'COR' or W'CN, where at least two of the radicals $R^1$ to $R^{10}$ are different from hydrogen,

where one or more hydrogen atoms in the radicals R and $R^1$ to $R^{10}$ may be replaced by fluorine,

W and W' are, independently of one another, single bonds or bridges having from 1 to 20 carbon atoms which may be part of a cyclic or aromatic group and may be interrupted by heteroatoms,

where W may also be a polyoxyalkylene or polyalkylenimine bridge having at least 21 carbon atoms,

R' is hydrogen, $C_{1-20}$-alkyl, carbonylalkyl, cycloalkyl or aryl,

M+ is a cation equivalent,

$X^-$ is an anion equivalent,

x is from 1 to 240,

where two geminal radicals $R^1$ to $R^{10}$ may form an oxo group and one or more of the radicals R and $R^1$ to $R^{10}$ may bear an additional trivalent phosphorus or nitrogen group capable of coordination,

where in each case two vicinal radicals may be joined to form fused aliphatic rings,

where two or more bridges W may also be present in the compounds of the formula II and the atoms of the phosphacyclohexane rings which are not bound to the bridge(s) W may also bear substituents defined as for $R^1$ to $R^{10}$'

where one of the radicals R or $R^1$ to $R^{10}$ in the compounds of the formula I and one of the radicals R or $R^1$ to $R^8$ or both radicals R together or a group W in the compounds of the formula II may also be a polymer radical having a number average molecular weight in the range from 500 to 50 000 and made up of repeating units derived from monomers selected from among monoolefins and diolefins, vinylaromatics, esters of $\alpha,\beta$-ethylenically unsaturated monocarboxylic and dicarboxylic acids with $C_1$-$C_{30}$-alkanols, N-vinyl amides, N-vinyllactams, heterocyclic compounds which can be polymerized with ring opening and mixtures thereof.

**2.** The process according to claim 1, wherein the compound of the formula I is selected from among compounds of the formula III

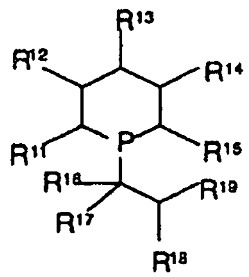

(III)

where:

$R^{11}$ to $R^{19}$
are each, independently of one another, hydrogen, $C_{1-20}$-alkyl, $C_{7-20}$-aralkyl, $C_{7-20}$-alkaryl, $C_{6-12}$-aryl,

where one or more carbon atoms may be replaced by heteroatoms, $W'COO^{-}M^{+}$, $W'SO_3^{-}M^{+}$, $W'PO_3^{2-}M^{+}_2$, $W'NR'_3{}^{+}X^{-}$, W'OR', $W'NR'_2$, W'COOR', W'SR', $W'(CHR'CH_2O)_x R'$, $W'(CH_2NR')_xR'$, $W'(CH_2CH_2NR')_xR'$,
where in each case two vicinal radicals $R^{11}$ to $R^{15}$ and/or $R^{17}$ and $R^{18}$ and/or $R^{16}$ and $R^{17}$ and/or $R^{16}$ and $R^{19}$ and/or $R^{18}$ and $R^{19}$ may be joined to form rings,

W' is a single bond or a bridge having from 1 to 20 carbon atoms which may be part of a cyclic or aromatic group,
R' is hydrogen or $C_{1-6}$-alkyl,
M+ is a cation,
$X^{-}$ is an anion,
x is from 1 to 240,

where one or more of the radicals $R^{11}$ to $R^{19}$ may bear an additional trivalent phosphorus or nitrogen group capable of coordination, and $R^{18}$ may also be $-W'-CR^{20}=CR^{21}R^{22}$, where $R^{20}$, $R^{21}$, $R^{22}$ are as defined above for $R^{11}$ to $R^{19}$.

**3.** The process according to claim 1, wherein the compounds of the formulae I and II are selected from among compounds of the formulae

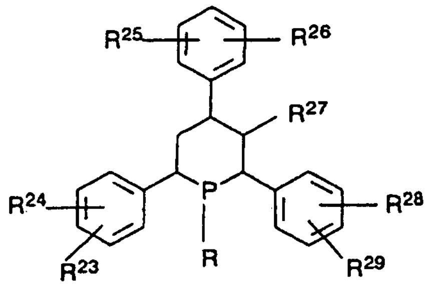

(I.a)

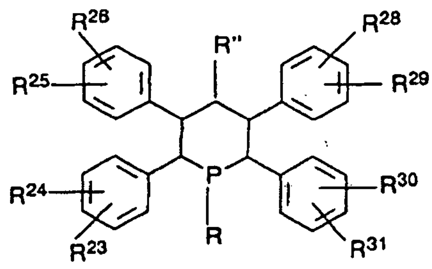

(I.b)

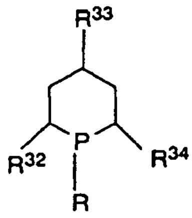

(I.c)

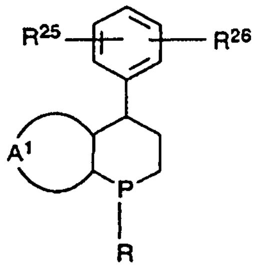

(I.d)

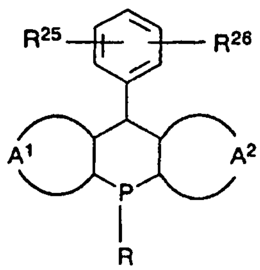

(I.e)

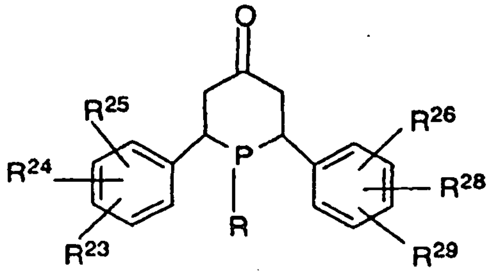

(I.f)

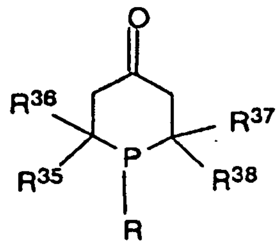

(I.g)

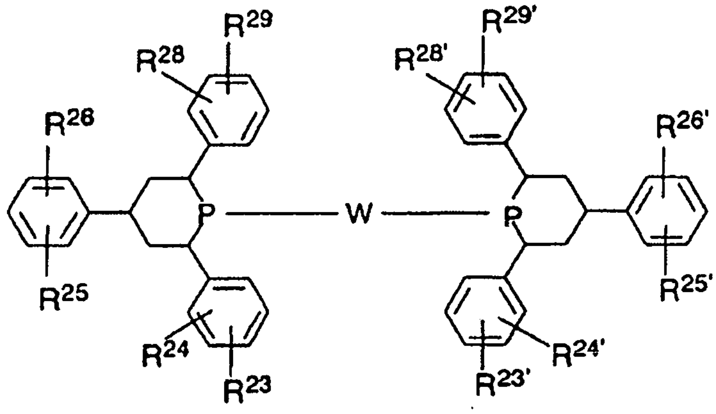

(II.a)

where

R is $C_{1-20}$-alkyl, cycloalkyl, $C_{6-12}$-aryl, $W'(CHR'CH_2O)_xR'$, $W'((CH2_2)_4O)_xR'$ or a polymer radical having a number average molecular weight in the range from 500 to 50 000 and made up of ethylene and/or butadiene, where

W' is a single bond or $C_{1-4}$-alkylene, and
R' is hydrogen or $C_{1-20}$-alkyl,
x is an integer from 1 to 240,

$R^{23}$, $R^{23'}$,$R^{24}$, $R^{24'}$, $R^{25}$, $R^{25'}$, $R^{26}$, $R^{26'}$, $R^{28}$, $R^{28'}$, $R^{29}$, $R^{29'}$, $R^{30}$ and $R^{31}$ are each, independently of one another, hydrogen, alkyl, alkoxy, carboxyl, carboxylate, trifluoromethyl, $-SO_3H$, sulfonate, $NE^1E^2$ or alkylene-$NE^1E^2$, where $E^1$ and $E^2$ are each, independently of one another, hydrogen, alkyl or cycloalkyl,
$R^{27}$ is hydrogen, alkyl, cycloalkyl, aryl or aralkyl,
$R^{32}$, $R^{33}$, $R^{34}$, $R^{35}$, $R^{36}$, $R^{37}$ and $R^{38}$ are each, independently of one another, alkyl or cycloalkyl,

R" is hydrogen or phenyl,
$A^1$ and $A^2$ together with the adjacent carbon atoms of the phosphacyclohexane to which they are bound form a fused-on ring system having in each case 1 or 2 further rings,
W is a bridge having from 1 to 20 carbon atoms which may be interrupted by heteroatoms.

**4.** The process according to any of the preceding claims, wherein the radical R is a polyoxyalkylene or polyalkylenimine radical.

**5.** The process according to any of claims 1 to 3, wherein one of the radicals R or $R^1$ to $R^{10}$ in the compounds of the formula I and one of the radicals R or $R^1$ to $R^8$ or the two radicals R together or the group W in the compounds of the formula II is a polymer radical having a number average molecular weight in the range from 500 to 50 000, where the repeating units of these polymer radicals are derived from monomers selected from among monoolefins and diolefins, vinylaromatics, esters of $\alpha,\beta$-ethylenically unsaturated monocarboxylic and dicarboxylic acids with $C_1$-$C_{30}$-alkanols, N-vinyl amides, N-vinyllactams, heterocyclic compounds which can be polymerized with ring opening and mixtures thereof.

**6.** The process according to any of claims 1 to 3, wherein one of the radicals R or $R^1$ to $R^{10}$ in the compounds of the formula I and one of the radicals R or $R^1$ to $R^8$ or the two radicals R together or a group W in the compounds of the formula II is a polyolefin radical.

**7.** The process according to any of claims 1 to 6, wherein the catalyst complex and excess ligands are recirculated from the distillation bottoms of the reaction mixture, with the catalyst and excess ligand being recovered from the distillation bottoms by ultrafiltration and the retentate being recirculated.

**8.** The process according to any of claims 1 to 6, wherein the work-up of the reaction mixture is carried out directly via an ultrafiltration.

**9.** A phosphacyclohexane of the formula I, II or III as defined in any of claims 1 to 3 in which at least three of the radicals $R^1$ to $R^{10}$ are different from hydrogen and at least two of the radicals R and $R^1$ to $R^{10}$ comprise cyclic structures which may be aliphatic, aromatic or heterocyclic, with the exception of:

- compounds of the formula I in which $R^5$ and $R^6$ together form an oxo group or one of the radicals $R^5$ or $R^6$ is hydroxyl and the other is hydrogen.

**10.** A phosphacyclohexane as defined in any of claims 4 to 6.

**11.** A complex of a transition metal of transition group VIII of the Periodic Table of the Elements with phosphacyclohexane ligands as defined in any of claims 1 to 6 in which at least three of the radicals $R^1$ to $R^{10}$ are different from hydrogen and at least two of the radicals R and $R^1$ to $R^{10}$ comprise cyclic structures which may be aliphatic, aromatic or heterocyclic.

**12.** A complex of the formula X

$$ML_n(CO)_m \qquad X$$

where

M is a transition metal of transition group VIII of the Periodic Table of the Elements, preferably rhodium,
L is a phosphacyclohexane ligand as defined in any of claims 1 to 6 in which at least three of the radicals $R^1$ to $R^{10}$ are different from hydrogen and at least two of the radicals R and $R^1$ to $R^{10}$ comprise cyclic structures which may be aliphatic, aromatic or heterocyclic,
n, m are, independently of one another, integers from 1 to 3.

**13.** A catalyst comprising at least one complex of a metal of transition group VIII with at least one ligand as defined in any of claims 1 to 6 in which at least three of the radicals $R^1$ to $R^{10}$ are different from hydrogen and at least two of the radicals R and $R^1$ to $R^{10}$ comprise cyclic structures which may be aliphatic, aromatic or heterocyclic.

**14.** The catalyst according to claim 13 which further comprises at least one additional ligand selected from among

halides, amines, carboxylates, acetylacetonate, arylsulfonates and alkylsulfonates, hydride, CO, olefins, dienes, cycloolefins, nitriles, N-containing heterocycles, aromatics and heteroaromatics, ethers, $PF_3$, phospholes, phosphabenzenes and monodentate, bidentate and polydentate phosphine, phosphinite, phosphonite, phosphoramidite and phosphite ligands.

**15.** The catalyst according to claim 13 or 14, wherein the metal of transition group VIII is selected from among ruthenium, iridium, rhodium, nickel and palladium.

**16.** A process for preparing complexes according to claim 12 by reacting a precursor complex of the metal M with the ligands L in the presence of a CO-containing gas.

**17.** The use of complexes according to claim 11 or 12 for the hydroacylation, hydrocyanation, hydroamidation, hydroesterification, aminolysis, alcoholysis, hydrocarbonylation, hydroxycarbonylation, carbonylation, isomerization or transfer hydrogenation of olefins.

**Revendications**

**1.** Procédé d'hydroformylation d'oléfines consistant à faire réagir les oléfines avec du CO et du $H_2$ en présence d'un complexe de rhodium comme catalyseur, à des températures de 50 à 180 °C et à des pressions de 5 à 100 bars, dans lequel le catalyseur comprend au moins un ligand ayant au moins un élément structurel de phosphacyclohexane non ponté, choisi parmi les phosphacyclohexanes de formules générales I et II suivantes :

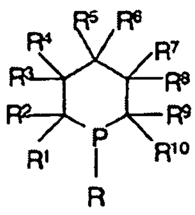

(I)

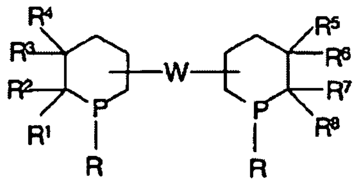

(II)

dans lesquelles :

R représente :

l'hydrogène, un groupement alkyle en $C_{1-100}$, cycloalkyle, hétérocycloalkyle, aralkyle en $C_{7-20}$, alkaryle en $C_{7-20}$, aryle en $C_{6-12}$, hétaryle, $W'COO^-M^+$, $W'SO_3^-M^+$, $W'PO_3^{2-}M^+_2$, $W'NR'_3^-X^+$, W'OR', $W'NR'_2$, W'COOR', W'SR', $W'(CHR'CH_2O)_xR'$, $W'(CH_2NR')_XR'$, $W'(CH_2CH_2NR')_xR'$, $W'((CH_2)_4O)_xR'$ ou W'COR',
les radicaux R dans la formule II pouvant également former, ensemble à la place du groupement W ou en plus de celui-ci, un pont ayant 1 à 20 atomes de carbone, qui peut faire partie d'un groupement cyclique ou aromatique et qui peut être interrompu par des hétéroatomes,
les radicaux R dans la formule II pouvant également représenter, ensemble à la place du groupement W ou en plus de celui-ci, un pont de type polyoxyalkylène ou polyalkylène-imine ayant au moins 21 atomes de carbone,

$R^1$ à $R^{10}$ représentent :

indépendamment l'un de l'autre, l'hydrogène, un groupement alkyle en $C_{1-20}$, aralkyle en $C_{7-20}$, alkaryle en $C_{7-20}$, aryle en $C_{6-12}$, dans lequel un ou plusieurs atomes de carbone peuvent être substitués par des hétéroatomes, un groupement $W'COO^-M^+$, $W'SO_3^-M^+$, $W'PO_3^{2-}M^+_2$, $W'NR'_3^+X^-$, W'OR', $W'NR'_2$, W'COOR', W'SR', $W'(CHR'CH_2O)_xR'$, $W'(CH_2NR')_xR'$, $W'(CH_2CH_2NR')_xR'$, $W'((CH_2)_4O)_xR'$, W'halogène, $W'NO_2$, W'COR' ou W'CN, au moins deux des radicaux $R^1$ à $R^{10}$ étant différents de l'hydrogène,
un ou plusieurs atomes d'hydrogène dans les radicaux R et $R^1$ à $R^{10}$ pouvant être substitués par du fluor,
W et W' peuvent représenter, indépendamment l'un de l'autre, des liaisons simples ou des ponts ayant 1 à 20 atomes de carbone, qui peuvent faire partie d'un groupement cyclique ou aromatique et qui peuvent

être interrompus par des hétéroatomes,
W pouvant également représenter un pont polyoxyalkylène ou polyalkylène-imine ayant au moins 21 atomes de carbone,

R' représente l'hydrogène, un groupement alkyle en $C_{1-20}$, carbonylalkyle, cycloalkyle ou aryle,
$M^+$ représente un équivalent de cation,
$X^-$ représente un équivalent d'anion,
x représente un nombre entier de 1 à 240,

deux radicaux géminaux $R^1$ à $R^{10}$ pouvant former un groupement oxo, et un ou plusieurs des radicaux R et $R^1$ à $R^{10}$ pouvant présenter un groupement phosphore ou azote trivalent supplémentaire capable d'établir des liaisons de coordination,
deux radicaux vicinaux pouvant, respectivement, être reliés sous la forme de cycles aliphatiques condensés,
deux ponts W ou plus pouvant être présents dans les composés de formule générale II, et les atomes des cycles de phosphacyclohexane non liés au(x) pont(s) W pouvant également être substitués de la manière définie pour les radicaux $R^1$ à $R^{10}$,
l'un des radicaux R ou $R^1$ à $R^{10}$ dans les composés de formule I, l'un des radicaux R ou $R^1$ à $R^8$ ou, conjointement, les deux radicaux R, ou un groupement W dans les composés de formule II, pouvant également représenter un radical polymère ayant un poids moléculaire moyen en nombre dans la plage de 500 à 50 000, constitué d'unités répétées qui dérivent de monomères qui sont choisis parmi des monooléfines et des dioléfines, des substances vinylaromatiques, des esters d'acides monocarboxyliques et dicarboxyliques à insaturation $\alpha,\beta$-éthylénique avec des alcanols en $C_1$-$C_{30}$, des N-vinylamides, des N-vinyllactames, des composés hétérocycliques que l'on peut polymériser par ouverture de cycle et leurs mélanges.

2. Procédé selon la revendication 1, **caractérisé en ce que** le composé de formule générale I est choisi parmi les composés de formule générale III :

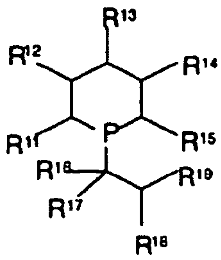

(III)

dans laquelle :

$R^{11}$ à $R^{19}$ représentent :

indépendamment l'un de l'autre, l'hydrogène, un groupement alkyle en $C_{1-20}$, aralkyle en $C_{7-20}$, alkaryle en $C_{7-20}$, aryle en $C_{6-12}$, dans lequel un ou plusieurs atomes de carbone peuvent être substitués par des hétéroatomes, un groupement $W'COO^-M^+$, $W'SO_3^-M^+$, $W'PO_3^{2-}M^+_2$, $W'NR'_3{}^+X^-$, W'OR', $W'NR'_2$, W'COOR', W'SR', $W'(CHR'CH_2O)_xR'$, $W'(CH_2NR')_xR'$, $W'(CH_2CH_2NR')_xR'$,
deux radicaux vicinaux $R^{11}$ à $R^{15}$ et/ou $R^{17}$ et $R^{18}$ et/ou $R^{16}$ et $R^{17}$ et/ou $R^{16}$ et $R^{19}$ et/ou $R^{18}$ et $R^{19}$ pouvant, respectivement, être reliés sous la forme de cycles,

W' représente une simple liaison ou un pont ayant 1 à 20 atomes de carbone, qui peut faire partie d'un groupement cyclique ou aromatique,
R' représente l'hydrogène ou un alkyle en $C_{1-6}$,
$M^+$ représente un cation,
$X^-$ représente un anion,
x représente un nombre de 1 à 240,

un ou plusieurs des radicaux $R^{11}$ à $R^{19}$ pouvant présenter un groupement phosphore ou azote trivalent

supplémentaire capable d'établir des liaisons de coordination, $R^{18}$ pouvant également représenter un groupement -W'-$CR^{20}$=$CR^{21}R^{22}$, dans lequel les radicaux $R^{20}$, $R^{21}$, $R^{22}$ sont tels que définis précédemment pour les radicaux $R^{11}$ à $R^{19}$.

**3.** Procédé selon la revendication 1, dans lequel les composés des formules I et II sont choisis parmi les composés de formules générales suivantes :

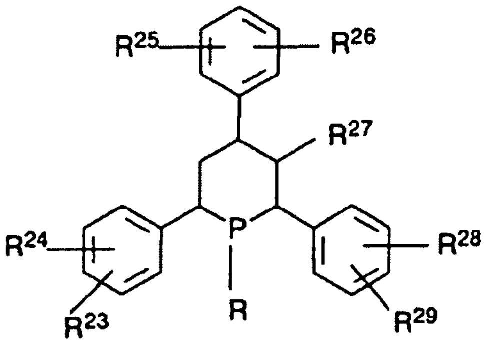

(I.a)

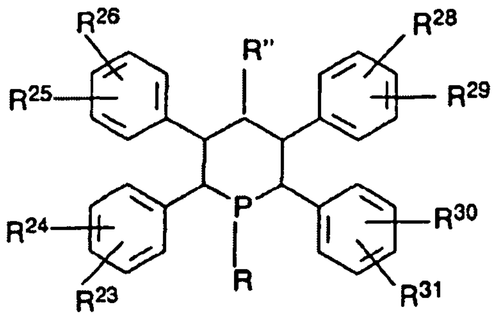

(I.b)

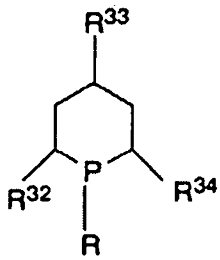

(I.c)

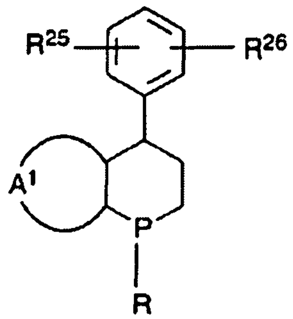

(I.d)

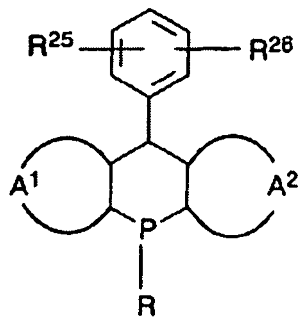

(I.e)

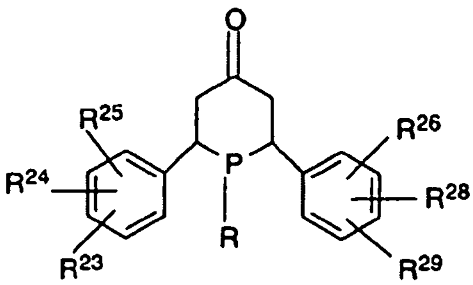

(I.f)

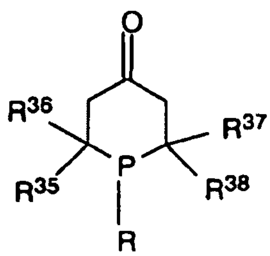

(I.g)

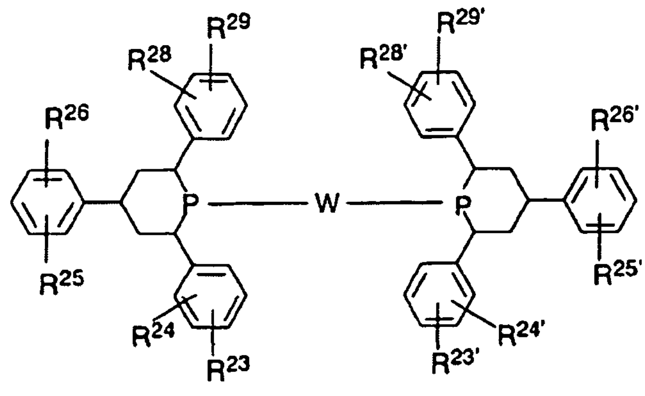

(II.a)

dans lesquelles :

R représente un groupement alkyle en $C_{1-20}$, cycloalkyle, aryle en $C_{6-12}$, W' $(CHR'CH_2O)_xR'$, $W'((CH_2)_4O)_xR'$ ou un radical polymère ayant un poids moléculaire moyen en nombre dans la plage de 500 à 50 000, qui est constitué d'éthylène et/ou de butadiène, dans lequel
W' représente une simple liaison ou un groupement alkylène en $C_{1-4}$, et
R' représente l'hydrogène ou un alkyle en $C_{1-20}$,
x représente un nombre entier de 1 à 240,
$R^{23}$, $R^{23'}$, $R^{24}$, $R^{24'}$, $R^{25}$, $R^{25'}$, $R^{26}$, $R^{26'}$, $R^{28}$, $R^{28'}$, $R^{29}$, $R^{29'}$, $R^{30}$ et $R^{31}$ représentent, indépendamment l'un de l'autre, l'hydrogène, un groupement alkyle, alcoxy, carboxyle, carboxylate, trifluorométhyle, $-SO_3H$, sulfonate, $NE^1E^2$ ou alkylène-$NE^1E^2$, $E^1$ et $E^2$ représentant, indépendamment l'un de l'autre, l'hydrogène, un groupement alkyle ou cycloalkyle,
$R^{27}$ représente l'hydrogène, un groupement alkyle, cycloalkyle, aryle ou aralkyle,
$R^{32}$, $R^{33}$, $R^{34}$, $R^{35}$, $R^{36}$, $R^{37}$ et $R^{38}$ représentent, indépendamment l'un de l'autre, un groupement alkyle ou cycloalkyle,
R" représente l'hydrogène ou un phényle,
$A^1$ et $A^2$ représentent, conjointement avec les atomes de carbone voisins du groupement phosphacyclohexane auxquels ils sont liés, un système de cycles condensés avec, respectivement, 1 ou 2 autres cycles,
W représente un pont ayant 1 à 20 atomes de carbone, qui peut être interrompu par des hétéroatomes.

**4.** Procédé selon l'une quelconque des revendications précédentes, dans lequel le radical R représente un radical de polyoxyalkylène ou de polyalkylène-imine.

**5.** Procédé selon l'une quelconque des revendications 1 à 3, dans lequel l'un des radicaux R ou $R^1$ à $R^{10}$ dans les composés de formule I, et l'un des radicaux R ou $R^1$ à $R^8$ ou, conjointement, les deux radicaux R, ou le groupement W dans les composés de formule II, représentent un radical polymère ayant un poids moléculaire moyen en nombre dans la plage de 500 à 50 000, dans lequel les unités répétées de ces radicaux polymères dérivent de monomères qui sont choisis parmi des monooléfines et des dioléfines, des composés vinylaromatiques, des esters d'acides monocarboxyliques et dicarboxyliques à insaturation $\alpha,\beta$-éthylénique avec des alcanols en $C_{1-30}$, des N-vinylamides, des N-vinyllactames, des composés hétérocycliques pouvant être polymérisés par ouverture de cycle et leurs mélanges.

**6.** Procédé selon l'une quelconque des revendications 1 à 3, dans lequel l'un des radicaux R ou $R^1$ à $R^{10}$ dans les composés de formule I, et l'un des radicaux R ou $R^1$ à $R^8$ ou, conjointement, les deux radicaux R ou un groupement W dans les composés de formule II, représentent un radical de polyoléfine.

**7.** Procédé selon l'une quelconque des revendications 1 à 6, dans lequel le catalyseur complexe et les ligands en excès provenant du bas de la colonne de distillation du mélange réactionnel sont recyclés, le catalyseur et le ligand en excès étant récupérés par le biais d'une ultrafiltration du bas de colonne de distillation et le rétentat étant réinjecté.

**8.** Procédé selon l'une quelconque des revendications 1 à 6, dans lequel le traitement du mélange réactionnel se fait directement par ultrafiltration.

**9.** Phosphacyclohexanes de formules générales I, II ou III, tels qu'ils sont définis selon l'une quelconque des revendications 1 à 3, dans lesquels au moins trois des radicaux $R^1$ à $R^{10}$ sont différents de l'hydrogène et dans lesquels au moins deux des radicaux R et $R^1$ à $R^{10}$ contiennent des structures cycliques qui peuvent être aliphatiques, aromatiques ou hétérocycliques, exception faite :

- des composés de formule I, dans lesquels $R^5$ et $R^6$ représentent conjointement un groupement oxo ou dans lesquels l'un des radicaux $R^5$ ou $R^6$ représente un hydroxyle et l'autre l'hydrogène.

**10.** Phosphacyclohexanes, tels qu'ils sont définis selon l'une quelconque des revendications 4 à 6.

**11.** Complexes de métaux de transition du sous-groupe VIII du système périodique des éléments avec des ligands de phosphacyclohexane, tels qu'ils sont définis selon l'une quelconque des revendications 1 à 6, dans lesquels au moins trois des radicaux $R^1$ à $R^{10}$ sont différents de l'hydrogène et dans lesquels au moins deux des radicaux R et $R^1$ à $R^{10}$ contiennent des structures cycliques qui peuvent être aliphatiques, aromatiques ou hétérocycliques.

**12.** Complexes de formule générale X :

$$ML_n(CO)_m \qquad X$$

dans laquelle :

M représente un métal de transition du sous-groupe VIII du système périodique des éléments, de préférence, le rhodium,
L représente un ligand de phosphacyclohexane, tel que défini selon l'une des revendications 1 à 6, dans lequel au moins trois des radicaux $R^1$ à $R^{10}$ sont différents de l'hydrogène et dans lequel au moins deux des radicaux R et $R^1$ à $R^{10}$ contiennent des structures cycliques qui peuvent être aliphatiques, aromatiques ou hétérocycliques,
n, m représentent, indépendamment l'un de l'autre, des nombres entiers de 1 à 3.

**13.** Catalyseur, comprenant au moins un complexe d'un métal du sous-groupe VIII avec au moins un ligand, tel que défini selon l'une des revendications 1 à 6, dans lequel au moins trois des radicaux $R^1$ à $R^{10}$ sont différents de l'hydrogène et dans lequel au moins deux des radicaux R et $R^1$ à $R^{10}$ contiennent des structures cycliques, qui peuvent être aliphatiques, aromatiques ou hétérocycliques.

**14.** Catalyseur selon la revendication 13, qui présente en outre au moins un autre ligand, choisi parmi des halogénures, des amines, des carboxylates, l'acétylacétonate, des arylsulfonates ou des alkylsulfonates, des hydrures, le CO, des oléfines, des diènes, des cyclooléfines, des nitriles, des hétérocycles contenant de l'azote, des composés aromatiques et hétéroaromatiques, des éthers, le $PF_3$, des phosphols, des phosphabenzènes, ainsi que des ligands de phosphine, de phosphinite, de phosphonite, de phosphoramidite et de phosphite du type monodentate, bidentate ou multidentate.

**15.** Catalyseur selon l'une quelconque des revendications 13 ou 14, **caractérisé en ce que** le métal du sous-groupe VIII est choisi parmi le ruthénium, l'iridium, le rhodium, le nickel ou le palladium.

**16.** Procédé de fabrication de complexes selon la revendication 12, consistant à faire réagir un précurseur complexe du métal M avec les ligands L en présence d'un gaz contenant du CO.

**17.** Utilisation de complexes selon la revendication 11 ou 12, pour effectuer une réaction d'hydroacylation, d'hydrocyanation, d'hydroamidation, d'hydroestérification, d'aminolyse, d'alcoolyse, d'hydrocarbonylation, d'hydroxycarbonylation, de carbonylation, d'isomérisation ou d'hydrogénation par transfert, d'oléfines.

Fig. 1

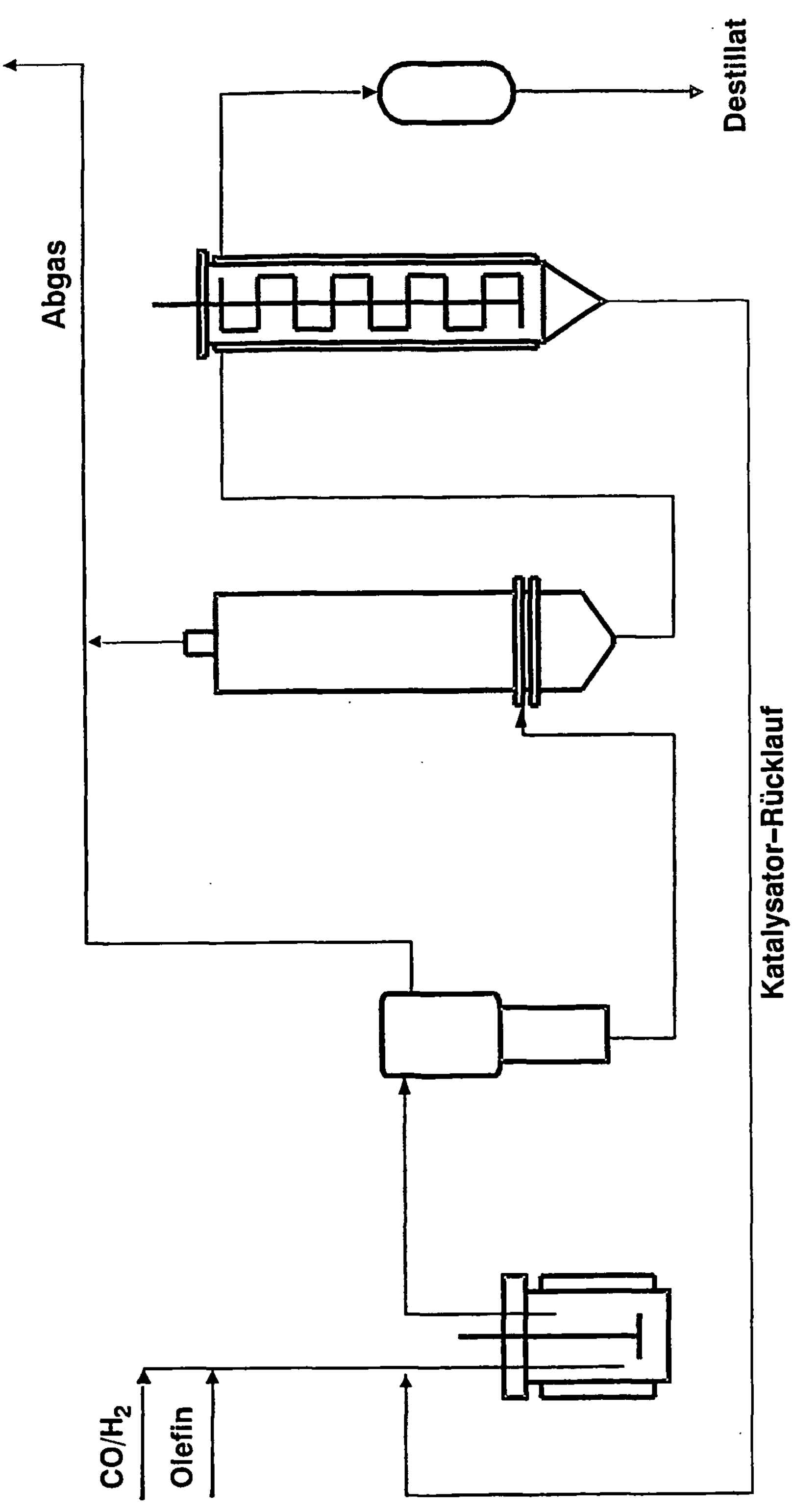

Fig. 2

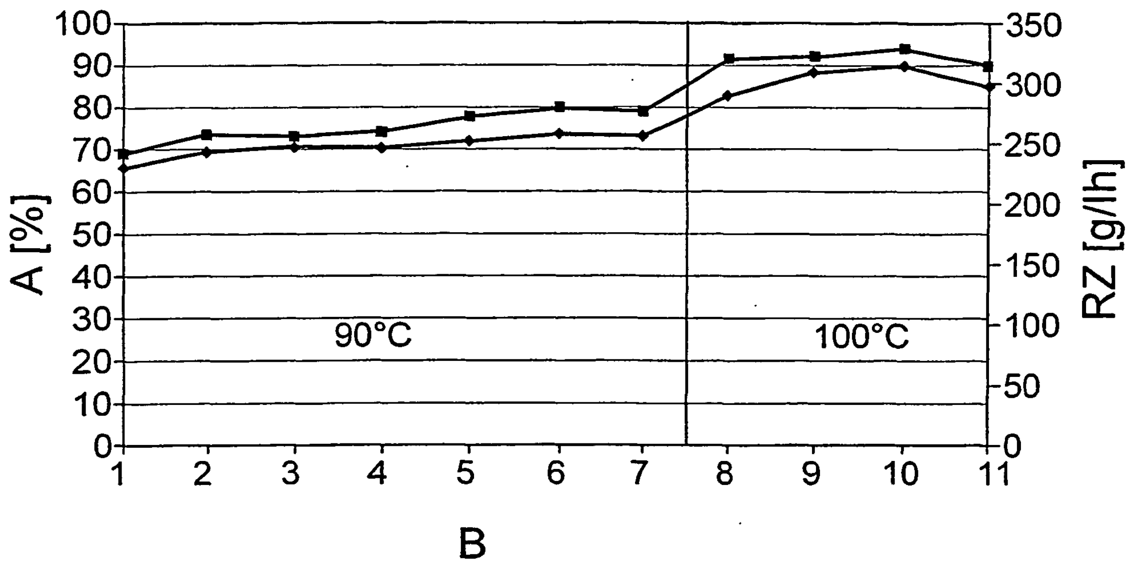
100
90
80
70
60
50
40
30
20
10
0
A [%]
350
300
250
200
150
100
50
0
RZ [g/lh]
90°C
100°C
1
2
3
4
5
6
7
8
9
10
11
B

Fig. 3

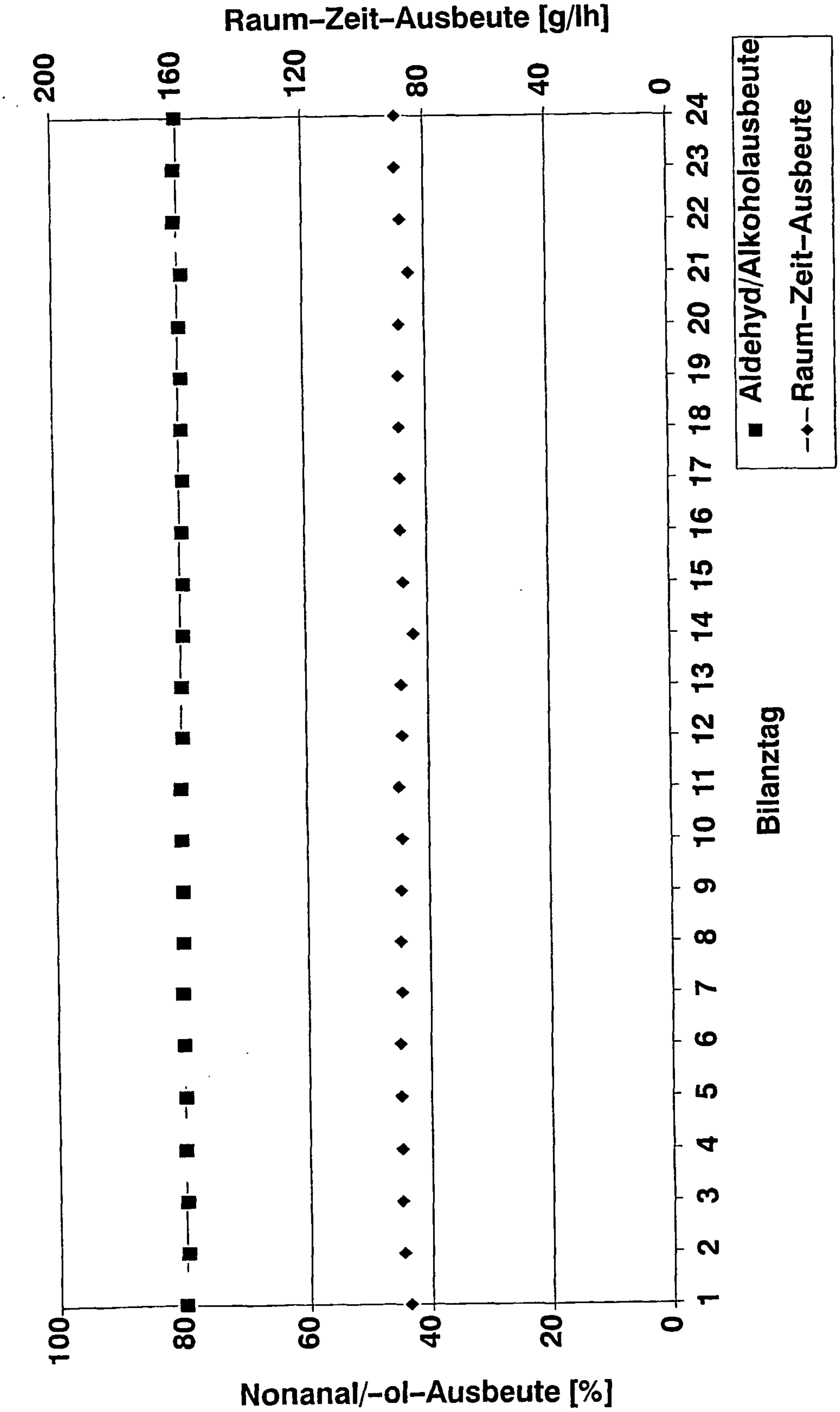
Raum-Zeit-Ausbeute [g/lh]
200
160
120
80
40
0
Nonanal/-ol-Ausbeute [%]
100
80
60
40
20
0
Bilanztag
1
2
3
4
5
6
7
8
9
10
11
12
13
14
15
16
17
18
19
20
21
22
23
24
Aldehyd/Alkoholausbeute
Raum-Zeit-Ausbeute

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9746507 A
- DE 19743197 A
- US 3105096 A
- WO 0053305 A
- WO 0052017 A
- US 3496204 A
- EP 0646588 A
- EP 0982314 A
- WO 0026220 A
- US 5177 A
- US 044 A
- JP 61291532 A
- DE 19621967 A
- WO 9936382 A


### In der Beschreibung aufgeführte Nicht-Patentliteratur


- **J. FALBE.** New Syntheses With Carbon Monoxide. Springer, 1980, 55
- **J. FALBE.** New Syntheses With Carbon Monoxide. Springer, 1980, 95
- **M. BELLER ; B. CORNILS ; C. D. FROHNING ; C. W. KOHLPAINTNER.** *J. Mol. Catal.,* 1995, vol. 104, 17
- **J. A. MOULIJN ; P. W. N. M. LEEUWEN ; R. A. VAN SANTEN.** Catalysis, An integrated Approach to Homogeneous, Heterogenous and Industrial Catalysis. Elsevier, 1993
- **K. ISSLEIB ; SIEFRIED HÄUSLER.** *Chem. Ber.,* 1961, vol. 94, 113-117
- **R. P. WELCHER ; N. E. DAY.** *J. Org. Chem.,* 1962, vol. 27, 1824-1827
- **G. MÄRKL et al.** *Tetrahedron Letters,* vol. 9, 645-648
- **K. SOMMER.** *Z. anorg. allg. Chem.,* 1970, vol. 376 (37), 56-62
- **S. D. PASTOR et al.** *J. Org. Chem.,* 1984, vol. 49, 2906-2909
- **M. M. RAUHUT et al.** *J. Org. Chem.,* 1961, vol. 26, 5138
- **G. MÄRKL ; A. MERZ.** *Tetrahedron Letters,* 1971, (17), 1215-1218
- **G. MÄRKL et al.** *Angew. Chem.,* 1967, vol. 79, 59
- **A. J. ASHE ; T. W. SMITH.** *Tetrahedron Letters,* 1977, (5), 407-410
- **D. E. BERGBREITER et al.** J. Polym. Sci. *Polym. Chem.,* 1989, vol. 27, 4205-4226
- **C. C. PRICE et al.** *J. Am. Chem. Soc.,* 1966, vol. 88, 1034-1037
- *Chem. Ber.,* 1961, vol. 94, 113-117
- *J. Org. Chem.,* 1962, vol. 27, 1824-1827
- *Tetrahedron Letters,* 1970, 645-648
- **G. MÄRKL.** Multiple Bonds and Low Coordination in Phosphorus Chemistry. Thieme, 1990, 220
- **H. L. HSIEH ; R. P. QUIRK.** Anionic Polyerisation, Principles and Practical Applications. Marcel Dekker-Verlag, 1996, vol. 5, 93-116
- ANIONIC POLYERISATION, PRINCIPLES AND PRACTICAL APPLICATIONS. 131-258
- ANIONIC POLYERISATION, PRINCIPLES AND PRACTICAL APPLICATIONS. 261-368
- ANIONIC POLYERISATION, PRINCIPLES AND PRACTICAL APPLICATIONS. vol. 22, 621-638
- ANIONIC POLYERISATION, PRINCIPLES AND PRACTICAL APPLICATIONS. vol. 23, 641-684
- ANIONIC POLYERISATION, PRINCIPLES AND PRACTICAL APPLICATIONS. vol. 24, 685-710